# EUROPEAN PATENT APPLICATION

(11) **EP 2 407 488 A2**
(43) Date of publication of application: **18.01.2012**
(21) Application number: 10751048.9
(22) Date of filing: 12.03.2010
(51) Int. Cl.: C07K 19/00, C07K 14/435, C12N 15/62, C12N 15/63, C12N 5/074, C12N 5/10

(54) **ESTABLISHMENT OF INDUCED PLURIPOTENT STEM CELL USING CELL-PERMEABLE REPROGRAMMING TRANSCRIPTION FACTOR FOR CUSTOMIZED STEM CELL THERAPY**

(30) Priority: 12.03.2009 US 159461 P
(71) Applicant: Procell Therapeutics Inc., Seoul 152-779 (KR); Jo, Daewoong, Seoul 135-857 (KR)
(72) Inventor: JO, Daewoong, Brentwood, TN 37027 (US); LIM, Jung-Hee, Seoul 153-832 (KR); KIM, Jungeun, Seoul 152-809 (KR); JEONG, Sooyoung, Goyang-si Gyeonggi-do 412-230 (KR); JUNG, Inhee, Hanam-si Gyeonggi-do 465-709 (KR)
(74) Representative: Schwabe - Sandmair - Marx
(86) International application number: PCT/KR2010/001559
(87) International publication number: WO 2010/104357

(57) **Abstract**

The present invention relates to a reprogramming transcription factor recombinant protein in which a macromolecule transduction domain (MTD) is fused to a reprogramming transcription factor to obtain cell permeability. The present invention also relates to a polynucleotide for coding said reprogramming transcription factor recombinant protein and to an expression vector of said cell-permeable reprogramming transcription factor recombinant protein. Treating a somatic cell with the cell-permeable reprogramming transcription factor recombinant protein induces the reprogramming of the stem cell-specific gene of the somatic cell, and thus can be effectively used in the establishment of an induced pluripotent stem cell (iPS cell) having characteristics similar to those of an embryonic stem cell in terms of morphology and genetics.

Sequence Listing

<110>	Procell Therapeutics Inc.
	JO, Daewoong

<120>	Establishment of Induced Pluripotent Stem (iPS) Cell Line With
	Reprogramming CP-TFs for Patient-Specific Stem Cell Therapy

<130>	FC103121

<150>	US 61/159,461
<151>	2009-03-12

<160>	252

<170>	KopatentIn 1.71

<210>	1
<211>	918
<212>	DNA
<213>	Human

<400>	1
atgagtgtgg atccagcttg tccccaaagc ttgccttgct ttgaagcatc cgactgtaaa	60

gaatcttcac ctatgcctgt gatttgtggg cctgaagaaa actatccatc cttgcaaatg	120

tcttctgctg agatgcctca cacggagact gtctctcctc ttccttcctc catggatctg	180

cttattcagg acagccctga ttcttccacc agtcccaaag gcaaacaacc cacttctgca	240

gagaagagtg tcgcaaaaaa ggaagacaag gtcccggtca agaaacagaa gaccagaact	300

gtgttctctt ccacccagct gtgtgtactc aatgatagat ttcagagaca gaaatacctc	360

agcctccagc agatgcaaga actctccaac atcctgaacc tcagctacaa acaggtgaag	420

acctggttcc agaaccagag aatgaaatct aagaggtggc agaaaaacaa ctggccgaag	480

aatagcaatg gtgtgacgca gaaggcctca gcacctacct accccagcct ttactcttcc	540

taccaccagg gatgcctggt gaacccgact gggaaccttc caatgtggag caaccagacc	600

tggaacaatt caacctggag caaccagacc cagaacatcc agtcctggag caaccactcc 660

tggaacactc agacctggtg cacccaatcc tggaacaatc aggcctggaa cagtcccttc 720

tataactgtg gagaggaatc tctgcagtcc tgcatgcagt tccagccaaa ttctcctgcc 780

agtgacttgg aggctgcctt ggaagctgct ggggaaggcc ttaatgtaat acagcagacc 840

actaggtatt ttagtactcc acaaaccatg gatttattcc taaactactc catgaacatg 900

caacctgaag acgtgtga 918

<210> 2
<211> 305
<212> PRT
<213> Human

<400> 2
Met Ser Val Asp Pro Ala Cys Pro Gln Ser Leu Pro Cys Phe Glu Ala
1               5                   10                  15

Ser Asp Cys Lys Glu Ser Ser Pro Met Pro Val Ile Cys Gly Pro Glu
            20                  25                  30

Glu Asn Tyr Pro Ser Leu Gln Met Ser Ser Ala Glu Met Pro His Thr
        35                  40                  45

Glu Thr Val Ser Pro Leu Pro Ser Ser Met Asp Leu Leu Ile Gln Asp
    50                  55                  60

Ser Pro Asp Ser Ser Thr Ser Pro Lys Gly Lys Gln Pro Thr Ser Ala
65                  70                  75                  80

Glu Asn Ser Val Ala Lys Lys Glu Asp Lys Val Pro Val Lys Lys Gln
                85                  90                  95

Lys Thr Arg Thr Val Phe Ser Ser Thr Gln Leu Cys Val Leu Asn Asp
            100                 105                 110

Arg Phe Gln Arg Gln Lys Tyr Leu Ser Leu Gln Gln Met Gln Glu Leu
        115                 120                 125

Ser Asn Ile Leu Asn Leu Ser Tyr Lys Gln Val Lys Thr Trp Phe Gln
    130                 135                 140

Asn Gln Arg Met Lys Ser Lys Arg Trp Gln Lys Asn Asn Trp Pro Lys
145                 150                 155                 160

Asn Ser Asn Gly Val Thr Gln Lys Ala Ser Ala Pro Thr Tyr Pro Ser
                165                 170                 175

Leu Tyr Ser Ser Tyr His Gln Gly Cys Leu Val Asn Pro Thr Gly Asn
            180                 185                 190

Leu Pro Met Trp Ser Asn Gln Thr Trp Asn Asn Ser Thr Trp Ser Asn
        195                 200                 205

Gln Thr Gln Asn Ile Gln Ser Trp Ser Asn His Ser Trp Asn Thr Gln
    210                 215                 220

Thr Trp Cys Thr Gln Ser Trp Asn Asn Gln Ala Trp Asn Ser Pro Phe
225                 230                 235                 240

Tyr Asn Cys Gly Glu Glu Ser Leu Gln Ser Cys Met Gln Phe Gln Pro
                245                 250                 255

Asn Ser Pro Ala Ser Asp Leu Glu Ala Ala Leu Glu Ala Ala Gly Glu
            260                 265                 270

Gly Leu Asn Val Ile Gln Gln Thr Thr Arg Tyr Phe Ser Thr Pro Gln
        275                 280                 285

Thr Met Asp Leu Phe Leu Asn Tyr Ser Met Asn Met Gln Pro Glu Asp
    290                 295                 300

Val
305

<210> 3
<211> 1083
<212> DNA
<213> Human

<400> 3
atggcgggac acctggcttc ggatttcgcc ttctcgcccc ctccaggtgg tggaggtgat 60

gggccagggg ggccggagcc gggctgggtt gatcctcgga cctggctaag cttccaaggc 120

cctcctggag ggccaggaat cgggccgggg gttgggccag gctctgaggt gtgggggatt 180

cccccatgcc ccccgccgta tgagttctgt ggggggatgg cgtactgtgg gccccaggtt 240

ggagtggggc tagtgcccca aggcggcttg gagacctctc agcctgaggg cgaagcagga 300

gtcggggtgg agagcaactc cgatggggcc tccccggagc cctgcaccgt cacccctggt 360

gccgtgaagc tggagaagga gaagctggag caaaacccgg aggagtccca ggacatcaaa 420

gctctgcaga aagaactcga gcaatttgcc aagctcctga agcagaagag gatcaccctg 480

ggatatacac aggccgatgt ggggctcacc ctgggggttc tatttgggaa ggtattcagc 540

caaacgacca tctgccgctt tgaggctctg cagcttagct tcaagaacat gtgtaagctg 600

cggcccttgc tgcagaagtg ggtggaggaa gctgacaaca atgaaaatct tcaggagata 660

tgcaaagcag aaaccctcgt gcaggcccga aagagaaagc gaaccagtat cgagaaccga 720

gtgagaggca acctggagaa tttgttcctg cagtgcccga aacccacact gcagcagatc 780

agccacatcg cccagcagct tgggctcgag aaggatgtgg tccgagtgtg gttctgtaac 840

cggcgccaga agggcaagcg atcaagcagc gactatgcac aacgagagga ttttgaggct 900

gctgggtctc ctttctcagg gggaccagtg tcctttcctc tggccccagg gccccatttt 960

ggtaccccag gctatgggag ccctcacttc actgcactgt actcctcggt ccctttccct 1020

gagggggaag cctttccccc tgtctccgtc accactctgg gctctcccat gcattcaaac 1080

tga 1083

<210> 4
<211> 360
<212> PRT
<213> Human

<400> 4
Met Ala Gly His Leu Ala Ser Asp Phe Ala Phe Ser Pro Pro Pro Gly
1               5                   10                  15

Gly Gly Gly Asp Gly Pro Gly Gly Pro Glu Pro Gly Trp Val Asp Pro
            20                  25                  30

Arg Thr Trp Leu Ser Phe Gln Gly Pro Pro Gly Gly Pro Gly Ile Gly
        35                  40                  45

Pro Gly Val Gly Pro Gly Ser Glu Val Trp Gly Ile Pro Pro Cys Pro
    50                  55                  60

Pro Pro Tyr Glu Phe Cys Gly Gly Met Ala Tyr Cys Gly Pro Gln Val
65                  70                  75                  80

Gly Val Gly Leu Val Pro Gln Gly Gly Leu Glu Thr Ser Gln Pro Glu
                85                  90                  95

Gly Glu Ala Gly Val Gly Val Glu Ser Asn Ser Asp Gly Ala Ser Pro
            100                 105                 110

Glu Pro Cys Thr Val Thr Pro Gly Ala Val Lys Leu Glu Lys Glu Lys
        115                 120                 125

Leu Glu Gln Asn Pro Glu Glu Ser Gln Asp Ile Lys Ala Leu Gln Lys
    130                 135                 140

Glu Leu Glu Gln Phe Ala Lys Leu Leu Lys Gln Lys Arg Ile Thr Leu

145                 150                 155                 160

Gly Tyr Thr Gln Ala Asp Val Gly Leu Thr Leu Gly Val Leu Phe Gly
                165                 170                 175

Lys Val Phe Ser Gln Thr Thr Ile Cys Arg Phe Glu Ala Leu Gln Leu
            180                 185                 190

Ser Phe Lys Asn Met Cys Lys Leu Arg Pro Leu Leu Gln Lys Trp Val
        195                 200                 205

Glu Glu Ala Asp Asn Asn Glu Asn Leu Gln Glu Ile Cys Lys Ala Glu
    210                 215                 220

Thr Leu Val Gln Ala Arg Lys Arg Lys Arg Thr Ser Ile Glu Asn Arg
225                 230                 235                 240

Val Arg Gly Asn Leu Glu Asn Leu Phe Leu Gln Cys Pro Lys Pro Thr
                245                 250                 255

Leu Gln Gln Ile Ser His Ile Ala Gln Gln Leu Gly Leu Glu Lys Asp
            260                 265                 270

Val Val Arg Val Trp Phe Cys Asn Arg Arg Gln Lys Gly Lys Arg Ser
        275                 280                 285

Ser Ser Asp Tyr Ala Gln Arg Glu Asp Phe Glu Ala Ala Gly Ser Pro
    290                 295                 300

Phe Ser Gly Gly Pro Val Ser Phe Pro Leu Ala Pro Gly Pro His Phe
305                 310                 315                 320

Gly Thr Pro Gly Tyr Gly Ser Pro His Phe Thr Ala Leu Tyr Ser Ser
                325                 330                 335

Val Pro Phe Pro Glu Gly Glu Ala Phe Pro Pro Val Ser Val Thr Thr
            340                 345                 350

Leu Gly Ser Pro Met His Ser Asn
        355                 360

<210> 5
<211> 954
<212> DNA
<213> Human

<400> 5
atgtacaaca tgatggagac ggagctgaag ccgccgggcc cgcagcaaac ttcggggggc 60

ggcggcggca actccaccgc ggcggcggcc ggcggcaacc agaaaaacag cccggaccgc 120

gtcaagcggc ccatgaatgc cttcatggtg tggtcccgcg ggcagcggcg caagatggcc 180

caggagaacc ccaagatgca caactcggag atcagcaagc gcctgggcgc cgagtggaaa 240

cttttgtcgg agacggagaa gcggccgttc atcgacgagg ctaagcggct gcgagcgctg 300

cacatgaagg agcacccgga ttataaatac cggccccggc ggaaaaccaa gacgctcatg 360

aagaaggata agtacacgct gcccggcggg ctgctggccc ccggcggcaa tagcatggcg 420

agcggggtcg gggtgggcgc cggcctgggc gcgggcgtga accagcgcat ggacagttac 480

gcgcacatga acggctggag caacggcagc tacagcatga tgcaggacca gctgggctac 540

ccgcagcacc cgggcctcaa tgcgcacggc gcagcgcaga tgcagcccat gcaccgctac 600

gacgtgagcg ccctgcagta caactccatg accagctcgc agacctacat gaacggctcg 660

cccacctaca gcatgtccta ctcgcagcag ggcacccctg gcatggctct tggctccatg 720

ggttcggtgg tcaagtccga ggccagctcc agcccccctg tggttacctc ttcctcccac 780

tccagggcgc cctgccaggc cggggacctc cgggacatga tcagcatgta tctccccggc 840

gccgaggtgc cggaacccgc cgcccccagc agacttcaca tgtcccagca ctaccagagc 900

ggcccggtgc ccggcacggc cattaacggc acactgcccc tctcacacat gtga 954

<210>    6

<211>    317

<212>    PRT

<213>    Human

<400>    6

<210> 7
<211> 1413
<212> DNA
<213> Human

<400> 7
atggctgtca gcgacgcgct gctcccatct ttctccacgt tcgcgtctgg cccggcggga 60

agggagaaga cactgcgtca agcaggtgcc ccgaataacc gctggcggga ggagctctcc 120

cacatgaagc gacttccccc agtgcttccc ggccgcccct atgacctggc ggcggcgacc 180

gtggccacag acctggagag cggcggagcc ggtgcggctt gcggcggtag caacctggcg 240

cccctacctc ggagagagac cgaggagttc aacgatctcc tggacctgga ctttattctc 300

tccaattcgc tgacccatcc tccggagtca gtggccgcca ccgtgtcctc gtcagcgtca 360

gcctcctctt cgtcgtcgcc gtcgagcagc ggccctgcca gcgcgccctc cacctgcagc 420

ttcacctatc cgatccgggc cgggaacgac ccgggcgtgg cgccgggcgg cacgggcgga 480

ggcctcctct atggcaggga gtccgctccc cctccgacgg ctcccttcaa cctggcggac 540

atcaacgacg tgagcccctc gggcggcttc gtggccgagc tcctgcggcc agaattggac 600

ccggtgtaca ttccgccgca gcagccgcag ccgccaggtg gcgggctgat gggcaagttc 660

gtgctgaagg cgtcgctgag cgcccctggc agcgagtacg gcagcccgtc ggtcatcagc 720

gtcagcaaag gcagccctga cggcagccac ccggtggtgg tggcgcccta caacggcggg 780

ccgccgcgca cgtgccccaa gatcaagcag gaggcggtct cttcgtgcac ccacttgggc 840

gctggacccc ctctcagcaa tggccaccgg ccggctgcac acgacttccc cctggggcgg 900

cagctcccca gcaggactac cccgaccctg ggtcttgagg aagtgctgag cagcagggac 960

tgtcaccctg ccctgccgct tcctcccggc ttccatcccc acccggggcc caattaccca 1020

tccttcctgc ccgatcagat gcagccgcaa gtcccgccgc tccattacca agagctcatg 1080

ccacccggtt cctgcatgcc agaggagccc aagccaaaga ggggaagacg atcgtggccc 1140

cggaaaagga ccgccaccca cacttgtgat tacgcgggct gcggcaaaac ctacacaaag 1200

agttcccatc tcaaggcaca cctgcgaacc cacacaggtg agaaacctta ccactgtgac 1260

tgggacggct gtggatggaa attcgcccgc tcagatgaac tgaccaggca ctaccgtaaa 1320

cacacggggc accgcccgtt ccagtgccaa aaatgcgacc gagcattttc caggtcggac 1380

cacctcgcct tacacatgaa gaggcatttt taa                                    1413

<210>    8
<211>    470
<212>    PRT
<213>    Human

<400>    8
Met Ala Val Ser Asp Ala Leu Leu Pro Ser Phe Ser Thr Phe Ala Ser
1               5                   10                  15

Gly Pro Ala Gly Arg Glu Lys Thr Leu Arg Gln Ala Gly Ala Pro Asn
            20                  25                  30

Asn Arg Trp Arg Glu Glu Leu Ser His Met Lys Arg Leu Pro Pro Val
        35                  40                  45

Leu Pro Gly Arg Pro Tyr Asp Leu Ala Ala Ala Thr Val Ala Thr Asp
    50                  55                  60

Leu Glu Ser Gly Gly Ala Gly Ala Ala Cys Gly Gly Ser Asn Leu Ala
65                  70                  75                  80

Pro Leu Pro Arg Arg Glu Thr Glu Glu Phe Asn Asp Leu Leu Asp Leu
                85                  90                  95

Asp Phe Ile Leu Ser Asn Ser Leu Thr His Pro Pro Glu Ser Val Ala
            100                 105                 110

Ala Thr Val Ser Ser Ser Ala Ser Ala Ser Ser Ser Ser Ser Pro Ser
        115                 120                 125

Ser Ser Gly Pro Ala Ser Ala Pro Ser Thr Cys Ser Phe Thr Tyr Pro
    130                 135                 140

Ile Arg Ala Gly Asn Asp Pro Gly Val Ala Pro Gly Gly Thr Gly Gly
145                 150                 155                 160

Gly Leu Leu Tyr Gly Arg Glu Ser Ala Pro Pro Pro Thr Ala Pro Phe

                165                 170                 175

Asn Leu Ala Asp Ile Asn Asp Val Ser Pro Ser Gly Gly Phe Val Ala
            180                 185                 190

Glu Leu Leu Arg Pro Glu Leu Asp Pro Val Tyr Ile Pro Pro Gln Gln
        195                 200                 205

Pro Gln Pro Pro Gly Gly Gly Leu Met Gly Lys Phe Val Leu Lys Ala
    210                 215                 220

Ser Leu Ser Ala Pro Gly Ser Glu Tyr Gly Ser Pro Ser Val Ile Ser
225                 230                 235                 240

Val Ser Lys Gly Ser Pro Asp Gly Ser His Pro Val Val Val Ala Pro
                245                 250                 255

Tyr Asn Gly Gly Pro Pro Arg Thr Cys Pro Lys Ile Lys Gln Glu Ala
            260                 265                 270

Val Ser Ser Cys Thr His Leu Gly Ala Gly Pro Pro Leu Ser Asn Gly
        275                 280                 285

His Arg Pro Ala Ala His Asp Phe Pro Leu Gly Arg Gln Leu Pro Ser
    290                 295                 300

Arg Thr Thr Pro Thr Leu Gly Leu Glu Glu Val Leu Ser Ser Arg Asp
305                 310                 315                 320

Cys His Pro Ala Leu Pro Leu Pro Pro Gly Phe His Pro His Pro Gly
                325                 330                 335

Pro Asn Tyr Pro Ser Phe Leu Pro Asp Gln Met Gln Pro Gln Val Pro
            340                 345                 350

Pro Leu His Tyr Gln Glu Leu Met Pro Pro Gly Ser Cys Met Pro Glu
        355                 360                 365

Glu Pro Lys Pro Lys Arg Gly Arg Arg Ser Trp Pro Arg Lys Arg Thr
    370                 375                 380

Ala Thr His Thr Cys Asp Tyr Ala Gly Cys Gly Lys Thr Tyr Thr Lys
385                 390                 395                 400

Ser Ser His Leu Lys Ala His Leu Arg Thr His Thr Gly Glu Lys Pro
                405                 410                 415

Tyr His Cys Asp Trp Asp Gly Cys Gly Trp Lys Phe Ala Arg Ser Asp
            420                 425                 430

Glu Leu Thr Arg His Tyr Arg Lys His Thr Gly His Arg Pro Phe Gln
        435                 440                 445

Cys Gln Lys Cys Asp Arg Ala Phe Ser Arg Ser Asp His Leu Ala Leu
    450                 455                 460

His Met Lys Arg His Phe
465                 470

<210>    9
<211>    1368
<212>    DNA
<213>    Human

<400>    9
atgctggatt tttttcgggt agtggaaaac cagcagcctc ccgcgacgat gcccctcaac        60

gttagcttca ccaacaggaa ctatgacctc gactacgact cggtgcagcc gtatttctac       120

tgcgacgagg aggagaactt ctaccagcag cagcagcaga gcgagctgca gcccccggcg       180

cccagcgagg atatctggaa gaaattcgag ctgctgccca ccccgcccct gtcccctagc       240

cgccgctccg ggctctgctc gccctcctac gttgcggtca cacccttctc ccttcgggga       300

gacaacgacg gcggtggcgg gagcttctcc acggccgacc agctggagat ggtgaccgag       360

ctgctgggag gagacatggt gaaccagagt ttcatctgcg acccggacga cgagaccttc       420

atcaaaaaca tcatcatcca ggactgtatg tggagcggct tctcggccgc cgccaagctc 480

gtctcagaga agctggcctc ctaccaggct gcgcgcaaag acagcggcag cccgaacccc 540

gcccgcggcc acagcgtctg ctccacctcc agcttgtacc tgcaggatct gagcgccgcc 600

gcctcagagt gcatcgaccc ctcggtggtc ttcccctacc ctctcaacga cagcagctcg 660

cccaagtcct gcgcctcgca agactccagc gccttctctc cgtcctcgga ttctctgctc 720

tcctcgacgg agtcctcccc gcagggcagc cccgagcccc tggtgctcca tgaggagaca 780

ccgcccacca ccagcagcga ctctgaggag gaacaagaag atgaggaaga aatcgatgtt 840

gtttctgtgg aaaagaggca ggctcctggc aaaaggtcag agtctggatc accttctgct 900

ggaggccaca gcaaacctcc tcacagccca ctggtcctca agaggtgcca cgtctccaca 960

catcagcaca actacgcagc gcctccctcc actcggaagg actatcctgc tgccaagagg 1020

gtcaagttgg acagtgtcag agtcctgaga cagatcagca acaaccgaaa atgcaccagc 1080

cccaggtcct cggacaccga ggagaatgtc aagaggcgaa cacacaacgt cttggagcgc 1140

cagaggagga acgagctaaa acggagcttt tttgccctgc gtgaccagat cccggagttg 1200

gaaaacaatg aaaaggcccc caaggtagtt atccttaaaa aagccacagc atacatcctg 1260

tccgtccaag cagaggagca aaagctcatt tctgaagagg acttgttgcg gaaacgacga 1320

gaacagttga aacacaaact tgaacagcta cggaactctt gtgcgtaa 1368

<210> 10
<211> 455
<212> PRT
<213> Human

<400> 10
Met Leu Asp Phe Phe Arg Val Val Glu Asn Gln Gln Pro Pro Ala Thr

1               5                   10                  15

Met Pro Leu Asn Val Ser Phe Thr Asn Arg Asn Tyr Asp Leu Asp Tyr
            20                  25                  30

Asp Ser Val Gln Pro Tyr Phe Tyr Cys Asp Glu Glu Glu Asn Phe Tyr
        35                  40                  45

Gln Gln Gln Gln Gln Ser Glu Leu Gln Pro Pro Ala Pro Ser Glu Asp
    50                  55                  60

Ile Trp Lys Lys Phe Glu Leu Leu Pro Thr Pro Pro Leu Ser Pro Ser
65                  70                  75                  80

Arg Arg Ser Gly Leu Cys Ser Pro Ser Tyr Val Ala Val Thr Pro Phe
                85                  90                  95

Ser Leu Arg Gly Asp Asn Asp Gly Gly Gly Gly Ser Phe Ser Thr Ala
            100                 105                 110

Asp Gln Leu Glu Met Val Thr Glu Leu Leu Gly Gly Asp Met Val Asn
        115                 120                 125

Gln Ser Phe Ile Cys Asp Pro Asp Asp Glu Thr Phe Ile Lys Asn Ile
    130                 135                 140

Ile Ile Gln Asp Cys Met Trp Ser Gly Phe Ser Ala Ala Ala Lys Leu
145                 150                 155                 160

Val Ser Glu Lys Leu Ala Ser Tyr Gln Ala Ala Arg Lys Asp Ser Gly
                165                 170                 175

Ser Pro Asn Pro Ala Arg Gly His Ser Val Cys Ser Thr Ser Ser Leu
            180                 185                 190

Tyr Leu Gln Asp Leu Ser Ala Ala Ala Ser Glu Cys Ile Asp Pro Ser
        195                 200                 205

Val Val Phe Pro Tyr Pro Leu Asn Asp Ser Ser Ser Pro Lys Ser Cys
    210                 215                 220

Ala Ser Gln Asp Ser Ser Ala Phe Ser Pro Ser Ser Asp Ser Leu Leu
225                 230                 235                 240

Ser Ser Thr Glu Ser Ser Pro Gln Gly Ser Pro Glu Pro Leu Val Leu
                245                 250                 255

His Glu Glu Thr Pro Pro Thr Thr Ser Ser Asp Ser Glu Glu Glu Gln
            260                 265                 270

Glu Asp Glu Glu Glu Ile Asp Val Val Ser Val Glu Lys Arg Gln Ala
        275                 280                 285

Pro Gly Lys Arg Ser Glu Ser Gly Ser Pro Ser Ala Gly Gly His Ser
    290                 295                 300

Lys Pro Pro His Ser Pro Leu Val Leu Lys Arg Cys His Val Ser Thr
305                 310                 315                 320

His Gln His Asn Tyr Ala Ala Pro Pro Ser Thr Arg Lys Asp Tyr Pro
                325                 330                 335

Ala Ala Lys Arg Val Lys Leu Asp Ser Val Arg Val Leu Arg Gln Ile
            340                 345                 350

Ser Asn Asn Arg Lys Cys Thr Ser Pro Arg Ser Ser Asp Thr Glu Glu
        355                 360                 365

Asn Val Lys Arg Arg Thr His Asn Val Leu Glu Arg Gln Arg Arg Asn
    370                 375                 380

Glu Leu Lys Arg Ser Phe Phe Ala Leu Arg Asp Gln Ile Pro Glu Leu
385                 390                 395                 400

Glu Asn Asn Glu Lys Ala Pro Lys Val Val Ile Leu Lys Lys Ala Thr
                405                 410                 415

Ala Tyr Ile Leu Ser Val Gln Ala Glu Glu Gln Lys Leu Ile Ser Glu
            420                 425                 430

Glu Asp Leu Leu Arg Lys Arg Arg Glu Gln Leu Lys His Lys Leu Glu
        435                 440                 445

Gln Leu Arg Asn Ser Cys Ala
    450                 455

<210>   11
<211>   630
<212>   DNA
<213>   Human

<400>   11
atgggctccg tgtccaacca gcagtttgca ggtggctgcg ccaaggcggc agaagaggcg        60

cccgaggagg cgccggagga cgcggcccgg gcggcggacg agcctcagct gctgcacggt       120

gcgggcatct gtaagtggtt caacgtgcgc atggggttcg gcttcctgtc catgaccgcc       180

cgcgccgggg tcgcgctcga ccccccagtg gatgtctttg tgcaccagag taagctgcac       240

atggaagggt tccggagctt gaaggagggt gaggcagtgg agttcacctt taagaagtca       300

gccaagggtc tggaatccat ccgtgtcacc ggacctggtg gagtattctg tattgggagt       360

gagaggcggc caaaaggaaa gagcatgcag aagcgcagat caaaaggaga caggtgctac       420

aactgtggag gtctagatca tcatgccaag gaatgcaagc tgccacccca gcccaagaag       480

tgccacttct gccagagcat cagccatatg gtagcctcat gtccgctgaa ggcccagcag       540

ggccctagtg cacagggaaa gccaacctac tttcgagagg aagaagaaga aatccacagc       600

cctaccctgc tcccggaggc acagaattga                                        630

<210>   12
<211>   209
<212>   PRT
<213>   Human

<400>    12

Met Gly Ser Val Ser Asn Gln Gln Phe Ala Gly Gly Cys Ala Lys Ala
1               5                   10                  15

Ala Glu Glu Ala Pro Glu Glu Ala Pro Glu Asp Ala Ala Arg Ala Ala
            20                  25                  30

Asp Glu Pro Gln Leu Leu His Gly Ala Gly Ile Cys Lys Trp Phe Asn
        35                  40                  45

Val Arg Met Gly Phe Gly Phe Leu Ser Met Thr Ala Arg Ala Gly Val
    50                  55                  60

Ala Leu Asp Pro Pro Val Asp Val Phe Val His Gln Ser Lys Leu His
65                  70                  75                  80

Met Glu Gly Phe Arg Ser Leu Lys Glu Gly Glu Ala Val Glu Phe Thr
                85                  90                  95

Phe Lys Lys Ser Ala Lys Gly Leu Glu Ser Ile Arg Val Thr Gly Pro
            100                 105                 110

Gly Gly Val Phe Cys Ile Gly Ser Glu Arg Arg Pro Lys Gly Lys Ser
        115                 120                 125

Met Gln Lys Arg Arg Ser Lys Gly Asp Arg Cys Tyr Asn Cys Gly Gly
    130                 135                 140

Leu Asp His His Ala Lys Glu Cys Lys Leu Pro Pro Gln Pro Lys Lys
145                 150                 155                 160

Cys His Phe Cys Gln Ser Ile Ser His Met Val Ala Ser Cys Pro Leu
                165                 170                 175

Lys Ala Gln Gln Gly Pro Ser Ala Gln Gly Lys Pro Thr Tyr Phe Arg
            180                 185                 190

Glu Glu Glu Glu Glu Ile His Ser Pro Thr Leu Leu Pro Glu Ala Gln
        195                 200                 205

Asn

<210> 13
<211> 15
<212> DNA
<213> Artificial Sequence

<220>
<223> SV40 large T antigen-derived NLS cDNA Sequence

<400> 13
aagaagaaga ggaag 15

<210> 14
<211> 5
<212> PRT
<213> Artificial Sequence

<220>
<223> SV40 large T antigen-derived NLS Amino Acid Sequence

<400> 14
Lys Lys Lys Arg Lys
1               5

<210> 15
<211> 27
<212> DNA
<213> Artificial Sequence

<220>
<223> JO-84 MTD cDNA Sequence

<400>    15
ctggtggcgg cgctgctggc ggtgctg                                          27

<210>    16
<211>    9
<212>    PRT
<213>    Artificial Sequence

<220>
<223>    JO-84 MTD Amino Acid Sequence

<400>    16
Leu Val Ala Ala Leu Leu Ala Val Leu
1               5

<210>    17
<211>    24
<212>    DNA
<213>    Artificial Sequence

<220>
<223>    JO-86 MTD cDNA Sequence

<400>    17
ctggcggtgc tggcggcggc gccg                                             24

<210>    18
<211>    8
<212>    PRT
<213>    Artificial Sequence

<220>
<223>    JO-86 MTD Amino Acid Sequence

<400> 18
Leu Ala Val Leu Ala Ala Ala Pro
1               5

<210> 19
<211> 933
<212> DNA
<213> Artificial Sequence

<220>
<223> NLS-Nanog cDNA Sequence

<400> 19
atgaagaaga agaggaagag tgtggatcca gcttgtcccc aaagcttgcc ttgctttgaa 60

gcatccgact gtaaagaatc ttcacctatg cctgtgattt gtgggcctga agaaaactat 120

ccatccttgc aaatgtcttc tgctgagatg cctcacacgg agactgtctc tcctcttccc 180

tcctccatgg atctgcttat tcaggacagc cctgattctt ccaccagtcc caaaggcaaa 240

caacccactt ctgcagagaa tagtgtcgca aaaaaggaag acaaggtccc agtcaagaaa 300

cagaagacca gaactgtgtt ctcttccacc cagctgtgtg tactcaatga tagatttcag 360

agacagaaat acctcagcct ccagcagatg caagaactct ccaacatcct gaacctcagc 420

tacaaacagg tgaagacctg gttccagaac cagagaatga aatctaagag gtggcagaaa 480

aacaactggc cgaagaatag caatggtgtg acgcagaagg cctcagcacc tacctacccc 540

agcctctact cttcctacca ccagggatgc ctggtgaacc cgactgggaa ccttccaatg 600

tggagcaacc agacctggaa caattcaacc tggagcaacc agacccagaa catccagtcc 660

tggagcaacc actcctggaa cactcagacc tggtgcaccc aatcctggaa caatcaggcc 720

tggaacagtc ccttctataa ctgtggagag gaatctctgc agtcctgcat gcagttccag 780

ccaaattctc ctgccagtga cttggaggct gctttggaag ctgctgggga aggccttaat 840

gtaatacagc agaccactag gtattttagt actccacaaa ccatggattt attcctaaac 900

tactccatga acatgcaacc tgaagacgtg tga 933

<210> 20
<211> 310
<212> PRT
<213> Artificial Sequence

<220>
<223> NLS-Nanog Amino Acid Sequence

<400> 20
Met Lys Lys Lys Arg Lys Ser Val Asp Pro Ala Cys Pro Gln Ser Leu
1               5                   10                  15

Pro Cys Phe Glu Ala Ser Asp Cys Lys Glu Ser Ser Pro Met Pro Val
            20                  25                  30

Ile Cys Gly Pro Glu Glu Asn Tyr Pro Ser Leu Gln Met Ser Ser Ala
        35                  40                  45

Glu Met Pro His Thr Glu Thr Val Ser Pro Leu Pro Ser Ser Met Asp
    50                  55                  60

Leu Leu Ile Gln Asp Ser Pro Asp Ser Ser Thr Ser Pro Lys Gly Lys
65                  70                  75                  80

Gln Pro Thr Ser Ala Glu Asn Ser Val Ala Lys Lys Glu Asp Lys Val
                85                  90                  95

Pro Val Lys Lys Gln Lys Thr Arg Thr Val Phe Ser Ser Thr Gln Leu
            100                 105                 110

Cys Val Leu Asn Asp Arg Phe Gln Arg Gln Lys Tyr Leu Ser Leu Gln
        115                 120                 125

Gln Met Gln Glu Leu Ser Asn Ile Leu Asn Leu Ser Tyr Lys Gln Val
    130                 135                 140

Lys Thr Trp Phe Gln Asn Gln Arg Met Lys Ser Lys Arg Trp Gln Lys
145                 150                 155                 160

Asn Asn Trp Pro Lys Asn Ser Asn Gly Val Thr Gln Lys Ala Ser Ala
                165                 170                 175

Pro Thr Tyr Pro Ser Leu Tyr Ser Ser Tyr His Gln Gly Cys Leu Val
            180                 185                 190

Asn Pro Thr Gly Asn Leu Pro Met Trp Ser Asn Gln Thr Trp Asn Asn
        195                 200                 205

Ser Thr Trp Ser Asn Gln Thr Gln Asn Ile Gln Ser Trp Ser Asn His
    210                 215                 220

Ser Trp Asn Thr Gln Thr Trp Cys Thr Gln Ser Trp Asn Asn Gln Ala
225                 230                 235                 240

Trp Asn Ser Pro Phe Tyr Asn Cys Gly Glu Glu Ser Leu Gln Ser Cys
                245                 250                 255

Met Gln Phe Gln Pro Asn Ser Pro Ala Ser Asp Leu Glu Ala Ala Leu
            260                 265                 270

Glu Ala Ala Gly Glu Gly Leu Asn Val Ile Gln Gln Thr Thr Arg Tyr
        275                 280                 285

Phe Ser Thr Pro Gln Thr Met Asp Leu Phe Leu Asn Tyr Ser Met Asn
    290                 295                 300

Met Gln Pro Glu Asp Val
305                 310

<210>    21
<211>    960
<212>    DNA
<213>    Artificial Sequence

<220>
<223>    NLS-JO-84 MTD-Nanog cDNA Sequence

<400>    21
atgaagaaga agaggaagct ggtggcggcg ctgctggcgg tgctgagtgt ggatccagct        60

tgtccccaaa gcttgccttg ctttgaagca tccgactgta aagaatcttc acctatgcct       120

gtgatttgtg ggcctgaaga aaactatcca tccttgcaaa tgtcttctgc tgagatgcct       180

cacacggaga ctgtctctcc tcttccctcc tccatggatc tgcttattca ggacagccct       240

gattcttcca ccagtcccaa aggcaaacaa cccacttctg cagagaatag tgtcgcaaaa       300

aaggaagaca aggtcccagt caagaaacag aagaccagaa ctgtgttctc ttccacccag       360

ctgtgtgtac tcaatgatag atttcagaga cagaaatacc tcagcctcca gcagatgcaa       420

gaactctcca acatcctgaa cctcagctac aaacaggtga agacctggtt ccagaaccag       480

agaatgaaat ctaagaggtg gcagaaaaac aactggccga agaatagcaa tggtgtgacg       540

cagaaggcct cagcacctac ctaccccagc ctctactctt cctaccacca gggatgcctg       600

gtgaacccga ctgggaacct tccaatgtgg agcaaccaga cctggaacaa ttcaacctgg       660

agcaaccaga cccagaacat ccagtcctgg agcaaccact cctggaacac tcagacctgg       720

tgcacccaat cctggaacaa tcaggcctgg aacagtccct tctataactg tggagaggaa       780

tctctgcagt cctgcatgca gttccagcca aattctcctg ccagtgactt ggaggctgct       840

ttggaagctg ctggggaagg ccttaatgta atacagcaga ccactaggta ttttagtact       900

ccacaaacca tggatttatt cctaaactac tccatgaaca tgcaacctga agacgtgtga 960

960

<210> 22
<211> 319
<212> PRT
<213> Artificial Sequence

<220>
<223> NLS-JO-84 MTD-Nanog Amino Acid Sequence

<400> 22
Met Lys Lys Lys Arg Lys Leu Val Ala Ala Leu Leu Ala Val Leu Ser
1               5                   10                  15

Val Asp Pro Ala Cys Pro Gln Ser Leu Pro Cys Phe Glu Ala Ser Asp
            20                  25                  30

Cys Lys Glu Ser Ser Pro Met Pro Val Ile Cys Gly Pro Glu Glu Asn
        35                  40                  45

Tyr Pro Ser Leu Gln Met Ser Ser Ala Glu Met Pro His Thr Glu Thr
    50                  55                  60

Val Ser Pro Leu Pro Ser Ser Met Asp Leu Leu Ile Gln Asp Ser Pro
65                  70                  75                  80

Asp Ser Ser Thr Ser Pro Lys Gly Lys Gln Pro Thr Ser Ala Glu Asn
                85                  90                  95

Ser Val Ala Lys Lys Glu Asp Lys Val Pro Val Lys Lys Gln Lys Thr
            100                 105                 110

Arg Thr Val Phe Ser Ser Thr Gln Leu Cys Val Leu Asn Asp Arg Phe
        115                 120                 125

Gln Arg Gln Lys Tyr Leu Ser Leu Gln Gln Met Gln Glu Leu Ser Asn

    130                 135                 140

Ile Leu Asn Leu Ser Tyr Lys Gln Val Lys Thr Trp Phe Gln Asn Gln
145                 150                 155                 160

Arg Met Lys Ser Lys Arg Trp Gln Lys Asn Asn Trp Pro Lys Asn Ser
                165                 170                 175

Asn Gly Val Thr Gln Lys Ala Ser Ala Pro Thr Tyr Pro Ser Leu Tyr
            180                 185                 190

Ser Ser Tyr His Gln Gly Cys Leu Val Asn Pro Thr Gly Asn Leu Pro
        195                 200                 205

Met Trp Ser Asn Gln Thr Trp Asn Asn Ser Thr Trp Ser Asn Gln Thr
    210                 215                 220

Gln Asn Ile Gln Ser Trp Ser Asn His Ser Trp Asn Thr Gln Thr Trp
225                 230                 235                 240

Cys Thr Gln Ser Trp Asn Asn Gln Ala Trp Asn Ser Pro Phe Tyr Asn
                245                 250                 255

Cys Gly Glu Glu Ser Leu Gln Ser Cys Met Gln Phe Gln Pro Asn Ser
            260                 265                 270

Pro Ala Ser Asp Leu Glu Ala Ala Leu Glu Ala Ala Gly Glu Gly Leu
        275                 280                 285

Asn Val Ile Gln Gln Thr Thr Arg Tyr Phe Ser Thr Pro Gln Thr Met
    290                 295                 300

Asp Leu Phe Leu Asn Tyr Ser Met Asn Met Gln Pro Glu Asp Val
305                 310                 315

<210> 23
<211> 960
<212> DNA
<213> Artificial Sequence

<220>
<223>    NLS-Nanog-JO-84 MTD cDNA Sequence

<400>    23
atgaagaaga agaggaagag tgtggatcca gcttgtcccc aaagcttgcc ttgctttgaa      60

gcatccgact gtaaagaatc ttcacctatg cctgtgattt gtgggcctga agaaaactat     120

ccatccttgc aaatgtcttc tgctgagatg cctcacacgg agactgtctc tcctcttccc     180

tcctccatgg atctgcttat tcaggacagc cctgattctt ccaccagtcc caaaggcaaa     240

caacccactt ctgcagagaa tagtgtcgca aaaaaggaag acaaggtccc agtcaagaaa     300

cagaagacca gaactgtgtt ctcttccacc cagctgtgtg tactcaatga tagatttcag     360

agacagaaat acctcagcct ccagcagatg caagaactct ccaacatcct gaacctcagc     420

tacaaacagg tgaagacctg gttccagaac cagagaatga aatctaagag gtggcagaaa     480

aacaactggc cgaagaatag caatggtgtg acgcagaagg cctcagcacc tacctacccc     540

agcctctact cttcctacca ccagggatgc ctggtgaacc cgactgggaa ccttccaatg     600

tggagcaacc agacctggaa caattcaacc tggagcaacc agacccagaa catccagtcc     660

tggagcaacc actcctggaa cactcagacc tggtgcaccc aatcctggaa caatcaggcc     720

tggaacagtc ccttctataa ctgtggagag gaatctctgc agtcctgcat gcagttccag     780

ccaaattctc ctgccagtga cttggaggct gctttggaag ctgctgggga aggccttaat     840

gtaatacagc agaccactag gtattttagt actccacaaa ccatggattt attcctaaac     900

tactccatga acatgcaacc tgaagacgtg ctggtggcgg cgctgctggc ggtgctgtga     960

                                                                      960

<210>    24
<211>    319
<212>    PRT
<213>    Artificial Sequence

<220>
<223>    NLS-Nanog-JO-84 MTD Amino Acid Sequence

<400>    24
Met Lys Lys Lys Arg Lys Ser Val Asp Pro Ala Cys Pro Gln Ser Leu
1               5                   10                  15

Pro Cys Phe Glu Ala Ser Asp Cys Lys Glu Ser Ser Pro Met Pro Val
            20                  25                  30

Ile Cys Gly Pro Glu Glu Asn Tyr Pro Ser Leu Gln Met Ser Ser Ala
        35                  40                  45

Glu Met Pro His Thr Glu Thr Val Ser Pro Leu Pro Ser Ser Met Asp
    50                  55                  60

Leu Leu Ile Gln Asp Ser Pro Asp Ser Ser Thr Ser Pro Lys Gly Lys
65                  70                  75                  80

Gln Pro Thr Ser Ala Glu Asn Ser Val Ala Lys Lys Glu Asp Lys Val
                85                  90                  95

Pro Val Lys Lys Gln Lys Thr Arg Thr Val Phe Ser Ser Thr Gln Leu
            100                 105                 110

Cys Val Leu Asn Asp Arg Phe Gln Arg Gln Lys Tyr Leu Ser Leu Gln
        115                 120                 125

Gln Met Gln Glu Leu Ser Asn Ile Leu Asn Leu Ser Tyr Lys Gln Val
    130                 135                 140

Lys Thr Trp Phe Gln Asn Gln Arg Met Lys Ser Lys Arg Trp Gln Lys
145                 150                 155                 160

Asn Asn Trp Pro Lys Asn Ser Asn Gly Val Thr Gln Lys Ala Ser Ala
                165                 170                 175

Pro Thr Tyr Pro Ser Leu Tyr Ser Ser Tyr His Gln Gly Cys Leu Val
            180                 185                 190

Asn Pro Thr Gly Asn Leu Pro Met Trp Ser Asn Gln Thr Trp Asn Asn
        195                 200                 205

Ser Thr Trp Ser Asn Gln Thr Gln Asn Ile Gln Ser Trp Ser Asn His
    210                 215                 220

Ser Trp Asn Thr Gln Thr Trp Cys Thr Gln Ser Trp Asn Asn Gln Ala
225                 230                 235                 240

Trp Asn Ser Pro Phe Tyr Asn Cys Gly Glu Glu Ser Leu Gln Ser Cys
                245                 250                 255

Met Gln Phe Gln Pro Asn Ser Pro Ala Ser Asp Leu Glu Ala Ala Leu
            260                 265                 270

Glu Ala Ala Gly Glu Gly Leu Asn Val Ile Gln Gln Thr Thr Arg Tyr
        275                 280                 285

Phe Ser Thr Pro Gln Thr Met Asp Leu Phe Leu Asn Tyr Ser Met Asn
    290                 295                 300

Met Gln Pro Glu Asp Val Leu Val Ala Ala Leu Leu Ala Val Leu
305                 310                 315

<210> 25
<211> 987
<212> DNA
<213> Artificial Sequence

<220>
<223> NLS-JO-84 MTD-Nanog-JO-84 MTD cDNA Sequence

<400>    25
atgaagaaga agaggaagct ggtggcggcg ctgctggcgg tgctgagtgt ggatccagct        60

tgtccccaaa gcttgccttg ctttgaagca tccgactgta aagaatcttc acctatgcct       120

gtgatttgtg ggcctgaaga aaactatcca tccttgcaaa tgtcttctgc tgagatgcct       180

cacacggaga ctgtctctcc tcttccctcc tccatggatc tgcttattca ggacagccct       240

gattcttcca ccagtcccaa aggcaaacaa cccacttctg cagagaatag tgtcgcaaaa       300

aaggaagaca aggtcccagt caagaaacag aagaccagaa ctgtgttctc ttccacccag       360

ctgtgtgtac tcaatgatag atttcagaga cagaaatacc tcagcctcca gcagatgcaa       420

gaactctcca acatcctgaa cctcagctac aaacaggtga agacctggtt ccagaaccag       480

agaatgaaat ctaagaggtg gcagaaaaac aactggccga agaatagcaa tggtgtgacg       540

cagaaggcct cagcacctac ctaccccagc ctctactctt cctaccacca gggatgcctg       600

gtgaacccga ctgggaacct tccaatgtgg agcaaccaga cctggaacaa ttcaacctgg       660

agcaaccaga cccagaacat ccagtcctgg agcaaccact cctggaacac tcagacctgg       720

tgcacccaat cctggaacaa tcaggcctgg aacagtccct tctataactg tggagaggaa       780

tctctgcagt cctgcatgca gttccagcca aattctcctg ccagtgactt ggaggctgct       840

ttggaagctg ctggggaagg ccttaatgta atacagcaga ccactaggta ttttagtact       900

ccacaaacca tggatttatt cctaaactac tccatgaaca tgcaacctga agacgtgctg       960

gtggcggcgc tgctggcggt gctgtga                                           987

<210>    26
<211>    328
<212>    PRT

<213>    Artificial Sequence

<220>
<223>    NLS-JO-84 MTD-Nanog-JO-84 MTD Amino Acid Sequence

<400>    26
Met Lys Lys Lys Arg Lys Leu Val Ala Ala Leu Leu Ala Val Leu Ser
1               5                   10                  15

Val Asp Pro Ala Cys Pro Gln Ser Leu Pro Cys Phe Glu Ala Ser Asp
            20                  25                  30

Cys Lys Glu Ser Ser Pro Met Pro Val Ile Cys Gly Pro Glu Glu Asn
        35                  40                  45

Tyr Pro Ser Leu Gln Met Ser Ser Ala Glu Met Pro His Thr Glu Thr
    50                  55                  60

Val Ser Pro Leu Pro Ser Ser Met Asp Leu Leu Ile Gln Asp Ser Pro
65                  70                  75                  80

Asp Ser Ser Thr Ser Pro Lys Gly Lys Gln Pro Thr Ser Ala Glu Asn
                85                  90                  95

Ser Val Ala Lys Lys Glu Asp Lys Val Pro Val Lys Lys Gln Lys Thr
            100                 105                 110

Arg Thr Val Phe Ser Ser Thr Gln Leu Cys Val Leu Asn Asp Arg Phe
        115                 120                 125

Gln Arg Gln Lys Tyr Leu Ser Leu Gln Gln Met Gln Glu Leu Ser Asn
    130                 135                 140

Ile Leu Asn Leu Ser Tyr Lys Gln Val Lys Thr Trp Phe Gln Asn Gln
145                 150                 155                 160

Arg Met Lys Ser Lys Arg Trp Gln Lys Asn Asn Trp Pro Lys Asn Ser
                165                 170                 175

Asn Gly Val Thr Gln Lys Ala Ser Ala Pro Thr Tyr Pro Ser Leu Tyr
            180                 185                 190

Ser Ser Tyr His Gln Gly Cys Leu Val Asn Pro Thr Gly Asn Leu Pro
        195                 200                 205

Met Trp Ser Asn Gln Thr Trp Asn Asn Ser Thr Trp Ser Asn Gln Thr
    210                 215                 220

Gln Asn Ile Gln Ser Trp Ser Asn His Ser Trp Asn Thr Gln Thr Trp
225                 230                 235                 240

Cys Thr Gln Ser Trp Asn Asn Gln Ala Trp Asn Ser Pro Phe Tyr Asn
                245                 250                 255

Cys Gly Glu Glu Ser Leu Gln Ser Cys Met Gln Phe Gln Pro Asn Ser
            260                 265                 270

Pro Ala Ser Asp Leu Glu Ala Ala Leu Glu Ala Ala Gly Glu Gly Leu
        275                 280                 285

Asn Val Ile Gln Gln Thr Thr Arg Tyr Phe Ser Thr Pro Gln Thr Met
    290                 295                 300

Asp Leu Phe Leu Asn Tyr Ser Met Asn Met Gln Pro Glu Asp Val Leu
305                 310                 315                 320

Val Ala Ala Leu Leu Ala Val Leu
                325

<210> 27
<211> 957
<212> DNA
<213> Artificial Sequence

<220>
<223> NLS-JO-86 MTD-Nanog cDNA Sequence

<400> 27
atgaagaaga agaggaagct ggcggtgctg gcggcggcgc cgagtgtgga tccagcttgt 60

ccccaaagct tgccttgctt tgaagcatcc gactgtaaag aatcttcacc tatgcctgtg 120

atttgtgggc ctgaagaaaa ctatccatcc ttgcaaatgt cttctgctga gatgcctcac 180

acggagactg tctctcctct tccctcctcc atggatctgc ttattcagga cagccctgat 240

tcttccacca gtcccaaagg caaacaaccc acttctgcag agaatagtgt cgcaaaaaag 300

gaagacaagg tcccagtcaa gaaacagaag accagaactg tgttctcttc cacccagctg 360

tgtgtactca atgatagatt tcagagacag aaatacctca gcctccagca gatgcaagaa 420

ctctccaaca tcctgaacct cagctacaaa caggtgaaga cctggttcca gaaccagaga 480

atgaaatcta agaggtggca gaaaaacaac tggccgaaga atagcaatgg tgtgacgcag 540

aaggcctcag cacctaccta ccccagcctc tactcttcct accaccaggg atgcctggtg 600

aacccgactg ggaaccttcc aatgtggagc aaccagacct ggaacaattc aacctggagc 660

aaccagaccc agaacatcca gtcctggagc aaccactcct ggaacactca gacctggtgc 720

acccaatcct ggaacaatca ggcctggaac agtcccttct ataactgtgg agaggaatct 780

ctgcagtcct gcatgcagtt ccagccaaat tctcctgcca gtgacttgga ggctgctttg 840

gaagctgctg gggaaggcct taatgtaata cagcagacca ctaggtattt tagtactcca 900

caaaccatgg atttattcct aaactactcc atgaacatgc aacctgaaga cgtgtga 957

<210> 28
<211> 318
<212> PRT
<213> Artificial Sequence

<220>

<223>    NLS-JO-86 MTD-Nanog Amino Acid Sequence

<400>    28

Met Lys Lys Lys Arg Lys Leu Ala Val Leu Ala Ala Ala Pro Ser Val
1               5                   10                  15

Asp Pro Ala Cys Pro Gln Ser Leu Pro Cys Phe Glu Ala Ser Asp Cys
            20                  25                  30

Lys Glu Ser Ser Pro Met Pro Val Ile Cys Gly Pro Glu Glu Asn Tyr
        35                  40                  45

Pro Ser Leu Gln Met Ser Ser Ala Glu Met Pro His Thr Glu Thr Val
    50                  55                  60

Ser Pro Leu Pro Ser Ser Met Asp Leu Leu Ile Gln Asp Ser Pro Asp
65                  70                  75                  80

Ser Ser Thr Ser Pro Lys Gly Lys Gln Pro Thr Ser Ala Glu Asn Ser
                85                  90                  95

Val Ala Lys Lys Glu Asp Lys Val Pro Val Lys Lys Gln Lys Thr Arg
            100                 105                 110

Thr Val Phe Ser Ser Thr Gln Leu Cys Val Leu Asn Asp Arg Phe Gln
        115                 120                 125

Arg Gln Lys Tyr Leu Ser Leu Gln Gln Met Gln Glu Leu Ser Asn Ile
    130                 135                 140

Leu Asn Leu Ser Tyr Lys Gln Val Lys Thr Trp Phe Gln Asn Gln Arg
145                 150                 155                 160

Met Lys Ser Lys Arg Trp Gln Lys Asn Asn Trp Pro Lys Asn Ser Asn
                165                 170                 175

Gly Val Thr Gln Lys Ala Ser Ala Pro Thr Tyr Pro Ser Leu Tyr Ser
            180                 185                 190

Ser Tyr His Gln Gly Cys Leu Val Asn Pro Thr Gly Asn Leu Pro Met
        195                 200                 205

Trp Ser Asn Gln Thr Trp Asn Asn Ser Thr Trp Ser Asn Gln Thr Gln
    210                 215                 220

Asn Ile Gln Ser Trp Ser Asn His Ser Trp Asn Thr Gln Thr Trp Cys
225                 230                 235                 240

Thr Gln Ser Trp Asn Asn Gln Ala Trp Asn Ser Pro Phe Tyr Asn Cys
                245                 250                 255

Gly Glu Glu Ser Leu Gln Ser Cys Met Gln Phe Gln Pro Asn Ser Pro
            260                 265                 270

Ala Ser Asp Leu Glu Ala Ala Leu Glu Ala Ala Gly Glu Gly Leu Asn
        275                 280                 285

Val Ile Gln Gln Thr Thr Arg Tyr Phe Ser Thr Pro Gln Thr Met Asp
    290                 295                 300

Leu Phe Leu Asn Tyr Ser Met Asn Met Gln Pro Glu Asp Val
305                 310                 315

<210>    29
<211>    957
<212>    DNA
<213>    Artificial Sequence

<220>
<223>    NLS-Nanog-JO-86 MTD cDNA Sequence

<400>    29
atgaagaaga agaggaagag tgtggatcca gcttgtcccc aaagcttgcc ttgctttgaa        60

gcatccgact gtaaagaatc ttcacctatg cctgtgattt gtgggcctga agaaaactat       120

ccatccttgc aaatgtcttc tgctgagatg cctcacacgg agactgtctc tcctcttccc       180

tcctccatgg atctgcttat tcaggacagc cctgattctt ccaccagtcc caaaggcaaa 240

caacccactt ctgcagagaa tagtgtcgca aaaaaggaag acaaggtccc agtcaagaaa 300

cagaagacca gaactgtgtt ctcttccacc cagctgtgtg tactcaatga tagatttcag 360

agacagaaat acctcagcct ccagcagatg caagaactct ccaacatcct gaacctcagc 420

tacaaacagg tgaagacctg gttccagaac cagagaatga aatctaagag gtggcagaaa 480

aacaactggc cgaagaatag caatggtgtg acgcagaagg cctcagcacc tacctacccc 540

agcctctact cttcctacca ccagggatgc ctggtgaacc cgactgggaa ccttccaatg 600

tggagcaacc agacctggaa caattcaacc tggagcaacc agacccagaa catccagtcc 660

tggagcaacc actcctggaa cactcagacc tggtgcaccc aatcctggaa caatcaggcc 720

tggaacagtc ccttctataa ctgtggagag gaatctctgc agtcctgcat gcagttccag 780

ccaaattctc ctgccagtga cttggaggct gctttggaag ctgctgggga aggccttaat 840

gtaatacagc agaccactag gtattttagt actccacaaa ccatggattt attcctaaac 900

tactccatga acatgcaacc tgaagacgtg ctggcggtgc tggcggcggc gccgtga 957

<210> 30
<211> 318
<212> PRT
<213> Artificial Sequence

<220>
<223> NLS-Nanog-JO-86 MTD Amino Acid Sequence

<400> 30
Met Lys Lys Lys Arg Lys Ser Val Asp Pro Ala Cys Pro Gln Ser Leu
1               5                   10                  15

Pro Cys Phe Glu Ala Ser Asp Cys Lys Glu Ser Ser Pro Met Pro Val
            20                  25                  30

Ile Cys Gly Pro Glu Glu Asn Tyr Pro Ser Leu Gln Met Ser Ser Ala
        35                  40                  45

Glu Met Pro His Thr Glu Thr Val Ser Pro Leu Pro Ser Ser Met Asp
    50                  55                  60

Leu Leu Ile Gln Asp Ser Pro Asp Ser Ser Thr Ser Pro Lys Gly Lys
65                  70                  75                  80

Gln Pro Thr Ser Ala Glu Asn Ser Val Ala Lys Lys Glu Asp Lys Val
                85                  90                  95

Pro Val Lys Lys Gln Lys Thr Arg Thr Val Phe Ser Ser Thr Gln Leu
            100                 105                 110

Cys Val Leu Asn Asp Arg Phe Gln Arg Gln Lys Tyr Leu Ser Leu Gln
        115                 120                 125

Gln Met Gln Glu Leu Ser Asn Ile Leu Asn Leu Ser Tyr Lys Gln Val
    130                 135                 140

Lys Thr Trp Phe Gln Asn Gln Arg Met Lys Ser Lys Arg Trp Gln Lys
145                 150                 155                 160

Asn Asn Trp Pro Lys Asn Ser Asn Gly Val Thr Gln Lys Ala Ser Ala
                165                 170                 175

Pro Thr Tyr Pro Ser Leu Tyr Ser Ser Tyr His Gln Gly Cys Leu Val
            180                 185                 190

Asn Pro Thr Gly Asn Leu Pro Met Trp Ser Asn Gln Thr Trp Asn Asn
        195                 200                 205

Ser Thr Trp Ser Asn Gln Thr Gln Asn Ile Gln Ser Trp Ser Asn His
    210                 215                 220

Ser Trp Asn Thr Gln Thr Trp Cys Thr Gln Ser Trp Asn Asn Gln Ala
225                 230                 235                 240

Trp Asn Ser Pro Phe Tyr Asn Cys Gly Glu Glu Ser Leu Gln Ser Cys
                245                 250                 255

Met Gln Phe Gln Pro Asn Ser Pro Ala Ser Asp Leu Glu Ala Ala Leu
            260                 265                 270

Glu Ala Ala Gly Glu Gly Leu Asn Val Ile Gln Gln Thr Thr Arg Tyr
        275                 280                 285

Phe Ser Thr Pro Gln Thr Met Asp Leu Phe Leu Asn Tyr Ser Met Asn
    290                 295                 300

Met Gln Pro Glu Asp Val Leu Ala Val Leu Ala Ala Ala Pro
305                 310                 315

<210>   31
<211>   981
<212>   DNA
<213>   Artificial Sequence

<220>
<223>   NLS-JO-86 MTD-Nanog-JO-86 MTD cDNA Sequence

<400>   31
atgaagaaga agaggaagct ggcggtgctg gcggcggcgc cgagtgtgga tccagcttgt        60

ccccaaagct tgccttgctt tgaagcatcc gactgtaaag aatcttcacc tatgcctgtg       120

atttgtgggc ctgaagaaaa ctatccatcc ttgcaaatgt cttctgctga gatgcctcac       180

acggagactg tctctcctct tccctcctcc atggatctgc ttattcagga cagccctgat       240

tcttccacca gtcccaaagg caaacaaccc acttctgcag agaatagtgt cgcaaaaaag       300

gaagacaagg tcccagtcaa gaaacagaag accagaactg tgttctcttc cacccagctg       360

tgtgtactca atgatagatt tcagagacag aaatacctca gcctccagca gatgcaagaa 420

ctctccaaca tcctgaacct cagctacaaa caggtgaaga cctggttcca gaaccagaga 480

atgaaatcta agaggtggca gaaaaacaac tggccgaaga atagcaatgg tgtgacgcag 540

aaggcctcag cacctaccta ccccagcctc tactcttcct accaccaggg atgcctggtg 600

aacccgactg ggaaccttcc aatgtggagc aaccagacct ggaacaattc aacctggagc 660

aaccagaccc agaacatcca gtcctggagc aaccactcct ggaacactca gacctggtgc 720

acccaatcct ggaacaatca ggcctggaac agtcccttct ataactgtgg agaggaatct 780

ctgcagtcct gcatgcagtt ccagccaaat tctcctgcca gtgacttgga ggctgctttg 840

gaagctgctg gggaaggcct taatgtaata cagcagacca ctaggtattt tagtactcca 900

caaaccatgg atttattcct aaactactcc atgaacatgc aacctgaaga cgtgctggcg 960

gtgctggcgg cggcgccgtg a 981

<210> 32
<211> 326
<212> PRT
<213> Artificial Sequence

<220>
<223> NLS-JO-86 MTD-Nanog-JO-86 MTD Amino Acid Sequence

<400> 32
Met Lys Lys Lys Arg Lys Leu Ala Val Leu Ala Ala Ala Pro Ser Val
1               5                   10                  15

Asp Pro Ala Cys Pro Gln Ser Leu Pro Cys Phe Glu Ala Ser Asp Cys
            20                  25                  30

Lys Glu Ser Ser Pro Met Pro Val Ile Cys Gly Pro Glu Glu Asn Tyr
        35                  40                  45

Pro Ser Leu Gln Met Ser Ser Ala Glu Met Pro His Thr Glu Thr Val
    50                  55                  60

Ser Pro Leu Pro Ser Ser Met Asp Leu Leu Ile Gln Asp Ser Pro Asp
65                  70                  75                  80

Ser Ser Thr Ser Pro Lys Gly Lys Gln Pro Thr Ser Ala Glu Asn Ser
                85                  90                  95

Val Ala Lys Lys Glu Asp Lys Val Pro Val Lys Lys Gln Lys Thr Arg
            100                 105                 110

Thr Val Phe Ser Ser Thr Gln Leu Cys Val Leu Asn Asp Arg Phe Gln
        115                 120                 125

Arg Gln Lys Tyr Leu Ser Leu Gln Gln Met Gln Glu Leu Ser Asn Ile
    130                 135                 140

Leu Asn Leu Ser Tyr Lys Gln Val Lys Thr Trp Phe Gln Asn Gln Arg
145                 150                 155                 160

Met Lys Ser Lys Arg Trp Gln Lys Asn Asn Trp Pro Lys Asn Ser Asn
                165                 170                 175

Gly Val Thr Gln Lys Ala Ser Ala Pro Thr Tyr Pro Ser Leu Tyr Ser
            180                 185                 190

Ser Tyr His Gln Gly Cys Leu Val Asn Pro Thr Gly Asn Leu Pro Met
        195                 200                 205

Trp Ser Asn Gln Thr Trp Asn Asn Ser Thr Trp Ser Asn Gln Thr Gln
    210                 215                 220

Asn Ile Gln Ser Trp Ser Asn His Ser Trp Asn Thr Gln Thr Trp Cys
225                 230                 235                 240

Thr Gln Ser Trp Asn Asn Gln Ala Trp Asn Ser Pro Phe Tyr Asn Cys

245                 250                 255

Gly Glu Glu Ser Leu Gln Ser Cys Met Gln Phe Gln Pro Asn Ser Pro
            260                 265                 270

Ala Ser Asp Leu Glu Ala Ala Leu Glu Ala Ala Gly Glu Gly Leu Asn
        275                 280                 285

Val Ile Gln Gln Thr Thr Arg Tyr Phe Ser Thr Pro Gln Thr Met Asp
    290                 295                 300

Leu Phe Leu Asn Tyr Ser Met Asn Met Gln Pro Glu Asp Val Leu Ala
305                 310                 315                 320

Val Leu Ala Ala Ala Pro
                325

<210>   33
<211>   993
<212>   DNA
<213>   Artificial Sequence

<220>
<223>   His-NLS-Nanog cDNA Sequence

<400>   33
atgggcagca gccatcatca tcatcatcac agcagcggcc tggtgccgcg cggcagccat      60

atgaagaaga agaggaagag tgtggatcca gcttgtcccc aaagcttgcc ttgctttgaa     120

gcatccgact gtaaagaatc ttcacctatg cctgtgattt gtgggcctga agaaaactat     180

ccatccttgc aaatgtcttc tgctgagatg cctcacacgg agactgtctc tcctcttccc     240

tcctccatgg atctgcttat tcaggacagc cctgattctt ccaccagtcc caaaggcaaa     300

caacccactt ctgcagagaa tagtgtcgca aaaaaggaag acaaggtccc agtcaagaaa     360

cagaagacca gaactgtgtt ctcttccacc cagctgtgtg tactcaatga tagatttcag 420

agacagaaat acctcagcct ccagcagatg caagaactct ccaacatcct gaacctcagc 480

tacaaacagg tgaagacctg gttccagaac cagagaatga aatctaagag gtggcagaaa 540

aacaactggc cgaagaatag caatggtgtg acgcagaagg cctcagcacc tacctacccc 600

agcctctact cttcctacca ccagggatgc ctggtgaacc cgactgggaa ccttccaatg 660

tggagcaacc agacctggaa caattcaacc tggagcaacc agacccagaa catccagtcc 720

tggagcaacc actcctggaa cactcagacc tggtgcaccc aatcctggaa caatcaggcc 780

tggaacagtc ccttctataa ctgtggagag gaatctctgc agtcctgcat gcagttccag 840

ccaaattctc ctgccagtga cttggaggct gctttggaag ctgctgggga aggccttaat 900

gtaatacagc agaccactag gtattttagt actccacaaa ccatggattt attcctaaac 960

tactccatga acatgcaacc tgaagacgtg tga 993

<210> 34
<211> 330
<212> PRT
<213> Artificial Sequence

<220>
<223> His-NLS-Nanog Amino Acid Sequence

<400> 34
Met Gly Ser Ser His His His His His His Ser Ser Gly Leu Val Pro
1               5                   10                  15

Arg Gly Ser His Met Lys Lys Lys Arg Lys Ser Val Asp Pro Ala Cys
            20                  25                  30

Pro Gln Ser Leu Pro Cys Phe Glu Ala Ser Asp Cys Lys Glu Ser Ser

35                  40                  45

Pro Met Pro Val Ile Cys Gly Pro Glu Glu Asn Tyr Pro Ser Leu Gln
    50                  55                  60

Met Ser Ser Ala Glu Met Pro His Thr Glu Thr Val Ser Pro Leu Pro
65                  70                  75                  80

Ser Ser Met Asp Leu Leu Ile Gln Asp Ser Pro Asp Ser Ser Thr Ser
                85                  90                  95

Pro Lys Gly Lys Gln Pro Thr Ser Ala Glu Asn Ser Val Ala Lys Lys
            100                 105                 110

Glu Asp Lys Val Pro Val Lys Lys Gln Lys Thr Arg Thr Val Phe Ser
        115                 120                 125

Ser Thr Gln Leu Cys Val Leu Asn Asp Arg Phe Gln Arg Gln Lys Tyr
    130                 135                 140

Leu Ser Leu Gln Gln Met Gln Glu Leu Ser Asn Ile Leu Asn Leu Ser
145                 150                 155                 160

Tyr Lys Gln Val Lys Thr Trp Phe Gln Asn Gln Arg Met Lys Ser Lys
                165                 170                 175

Arg Trp Gln Lys Asn Asn Trp Pro Lys Asn Ser Asn Gly Val Thr Gln
            180                 185                 190

Lys Ala Ser Ala Pro Thr Tyr Pro Ser Leu Tyr Ser Ser Tyr His Gln
        195                 200                 205

Gly Cys Leu Val Asn Pro Thr Gly Asn Leu Pro Met Trp Ser Asn Gln
    210                 215                 220

Thr Trp Asn Asn Ser Thr Trp Ser Asn Gln Thr Gln Asn Ile Gln Ser
225                 230                 235                 240

Trp Ser Asn His Ser Trp Asn Thr Gln Thr Trp Cys Thr Gln Ser Trp
                245                 250                 255

Asn Asn Gln Ala Trp Asn Ser Pro Phe Tyr Asn Cys Gly Glu Glu Ser
            260                 265                 270

Leu Gln Ser Cys Met Gln Phe Gln Pro Asn Ser Pro Ala Ser Asp Leu
        275                 280                 285

Glu Ala Ala Leu Glu Ala Ala Gly Glu Gly Leu Asn Val Ile Gln Gln
    290                 295                 300

Thr Thr Arg Tyr Phe Ser Thr Pro Gln Thr Met Asp Leu Phe Leu Asn
305                 310                 315                 320

Tyr Ser Met Asn Met Gln Pro Glu Asp Val
                325                 330

<210> 35
<211> 1020
<212> DNA
<213> Artificial Sequence

<220>
<223> His-NLS-JO-84 MTD-Nanog cDNA Sequence

<400> 35
atgggcagca gccatcatca tcatcatcac agcagcggcc tggtgccgcg cggcagccat 60

atgaagaaga agaggaagct ggtggcggcg ctgctggcgg tgctgagtgt ggatccagct 120

tgtccccaaa gcttgccttg ctttgaagca tccgactgta aagaatcttc acctatgcct 180

gtgatttgtg ggcctgaaga aaactatcca tccttgcaaa tgtcttctgc tgagatgcct 240

cacacggaga ctgtctctcc tcttccctcc tccatggatc tgcttattca ggacagccct 300

gattcttcca ccagtcccaa aggcaaacaa cccacttctg cagagaatag tgtcgcaaaa 360

aaggaagaca aggtcccagt caagaaacag aagaccagaa ctgtgttctc ttccacccag 420

ctgtgtgtac tcaatgatag atttcagaga cagaaatacc tcagcctcca gcagatgcaa 480

gaactctcca acatcctgaa cctcagctac aaacaggtga agacctggtt ccagaaccag 540

agaatgaaat ctaagaggtg gcagaaaaac aactggccga agaatagcaa tggtgtgacg 600

cagaaggcct cagcacctac ctaccccagc ctctactctt cctaccacca gggatgcctg 660

gtgaacccga ctgggaacct tccaatgtgg agcaaccaga cctggaacaa ttcaacctgg 720

agcaaccaga cccagaacat ccagtcctgg agcaaccact cctggaacac tcagacctgg 780

tgcacccaat cctggaacaa tcaggcctgg aacagtccct tctataactg tggagaggaa 840

tctctgcagt cctgcatgca gttccagcca aattctcctg ccagtgactt ggaggctgct 900

ttggaagctg ctggggaagg ccttaatgta atacagcaga ccactaggta ttttagtact 960

ccacaaacca tggatttatt cctaaactac tccatgaaca tgcaacctga agacgtgtga 1020

1020

<210> 36
<211> 339
<212> PRT
<213> Artificial Sequence

<220>
<223> His-NLS-JO-84 MTD-Nanog Amino Acid Sequence

<400> 36
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Ser Val Asp Pro Ala Cys Pro Gln Ser Leu Pro Cys Phe
        35                  40                  45

Glu Ala Ser Asp Cys Lys Glu Ser Ser Pro Met Pro Val Ile Cys Gly
    50                  55                  60

Pro Glu Glu Asn Tyr Pro Ser Leu Gln Met Ser Ser Ala Glu Met Pro
65                  70                  75                  80

His Thr Glu Thr Val Ser Pro Leu Pro Ser Ser Met Asp Leu Leu Ile
                85                  90                  95

Gln Asp Ser Pro Asp Ser Ser Thr Ser Pro Lys Gly Lys Gln Pro Thr
            100                 105                 110

Ser Ala Glu Asn Ser Val Ala Lys Lys Glu Asp Lys Val Pro Val Lys
        115                 120                 125

Lys Gln Lys Thr Arg Thr Val Phe Ser Ser Thr Gln Leu Cys Val Leu
    130                 135                 140

Asn Asp Arg Phe Gln Arg Gln Lys Tyr Leu Ser Leu Gln Gln Met Gln
145                 150                 155                 160

Glu Leu Ser Asn Ile Leu Asn Leu Ser Tyr Lys Gln Val Lys Thr Trp
                165                 170                 175

Phe Gln Asn Gln Arg Met Lys Ser Lys Arg Trp Gln Lys Asn Asn Trp
            180                 185                 190

Pro Lys Asn Ser Asn Gly Val Thr Gln Lys Ala Ser Ala Pro Thr Tyr
        195                 200                 205

Pro Ser Leu Tyr Ser Ser Tyr His Gln Gly Cys Leu Val Asn Pro Thr
    210                 215                 220

Gly Asn Leu Pro Met Trp Ser Asn Gln Thr Trp Asn Asn Ser Thr Trp
225                 230                 235                 240

Ser Asn Gln Thr Gln Asn Ile Gln Ser Trp Ser Asn His Ser Trp Asn

                245                 250                 255

Thr Gln Thr Trp Cys Thr Gln Ser Trp Asn Asn Gln Ala Trp Asn Ser
            260                 265                 270

Pro Phe Tyr Asn Cys Gly Glu Glu Ser Leu Gln Ser Cys Met Gln Phe
        275                 280                 285

Gln Pro Asn Ser Pro Ala Ser Asp Leu Glu Ala Ala Leu Glu Ala Ala
    290                 295                 300

Gly Glu Gly Leu Asn Val Ile Gln Gln Thr Thr Arg Tyr Phe Ser Thr
305                 310                 315                 320

Pro Gln Thr Met Asp Leu Phe Leu Asn Tyr Ser Met Asn Met Gln Pro
                325                 330                 335

Glu Asp Val

<210> 37
<211> 1020
<212> DNA
<213> Artificial Sequence

<220>
<223> His-NLS-Nanog-JO-84 MTD cDNA Sequence

<400> 37
atgggcagca gccatcatca tcatcatcac agcagcggcc tggtgccgcg cggcagccat 60

atgaagaaga agaggaagag tgtggatcca gcttgtcccc aaagcttgcc ttgctttgaa 120

gcatccgact gtaaagaatc ttcacctatg cctgtgattt gtgggcctga agaaaactat 180

ccatccttgc aaatgtcttc tgctgagatg cctcacacgg agactgtctc tcctcttccc 240

tcctccatgg atctgcttat tcaggacagc cctgattctt ccaccagtcc caaaggcaaa 300

caacccactt ctgcagagaa tagtgtcgca aaaaaggaag acaaggtccc agtcaagaaa 360

cagaagacca gaactgtgtt ctcttccacc cagctgtgtg tactcaatga tagatttcag 420

agacagaaat acctcagcct ccagcagatg caagaactct ccaacatcct gaacctcagc 480

tacaaacagg tgaagacctg gttccagaac cagagaatga aatctaagag gtggcagaaa 540

aacaactggc cgaagaatag caatggtgtg acgcagaagg cctcagcacc tacctacccc 600

agcctctact cttcctacca ccagggatgc ctggtgaacc cgactgggaa ccttccaatg 660

tggagcaacc agacctggaa caattcaacc tggagcaacc agacccagaa catccagtcc 720

tggagcaacc actcctggaa cactcagacc tggtgcaccc aatcctggaa caatcaggcc 780

tggaacagtc ccttctataa ctgtggagag gaatctctgc agtcctgcat gcagttccag 840

ccaaattctc ctgccagtga cttggaggct gctttggaag ctgctgggga aggccttaat 900

gtaatacagc agaccactag gtattttagt actccacaaa ccatggattt attcctaaac 960

tactccatga acatgcaacc tgaagacgtg ctggtggcgg cgctgctggc ggtgctgtga 1020

1020

<210> 38
<211> 339
<212> PRT
<213> Artificial Sequence

<220>
<223> His-NLS-Nanog-JO-84 MTD Amino Acid Sequence

<400> 38
Met Gly Ser Ser His His His His His His Ser Ser Gly Leu Val Pro
1               5                   10                  15

Arg Gly Ser His Met Lys Lys Lys Arg Lys Ser Val Asp Pro Ala Cys
            20                  25                  30

Pro Gln Ser Leu Pro Cys Phe Glu Ala Ser Asp Cys Lys Glu Ser Ser
        35                  40                  45

Pro Met Pro Val Ile Cys Gly Pro Glu Glu Asn Tyr Pro Ser Leu Gln
    50                  55                  60

Met Ser Ser Ala Glu Met Pro His Thr Glu Thr Val Ser Pro Leu Pro
65                  70                  75                  80

Ser Ser Met Asp Leu Leu Ile Gln Asp Ser Pro Asp Ser Ser Thr Ser
                85                  90                  95

Pro Lys Gly Lys Gln Pro Thr Ser Ala Glu Asn Ser Val Ala Lys Lys
            100                 105                 110

Glu Asp Lys Val Pro Val Lys Lys Gln Lys Thr Arg Thr Val Phe Ser
        115                 120                 125

Ser Thr Gln Leu Cys Val Leu Asn Asp Arg Phe Gln Arg Gln Lys Tyr
    130                 135                 140

Leu Ser Leu Gln Gln Met Gln Glu Leu Ser Asn Ile Leu Asn Leu Ser
145                 150                 155                 160

Tyr Lys Gln Val Lys Thr Trp Phe Gln Asn Gln Arg Met Lys Ser Lys
                165                 170                 175

Arg Trp Gln Lys Asn Asn Trp Pro Lys Asn Ser Asn Gly Val Thr Gln
            180                 185                 190

Lys Ala Ser Ala Pro Thr Tyr Pro Ser Leu Tyr Ser Ser Tyr His Gln
        195                 200                 205

Gly Cys Leu Val Asn Pro Thr Gly Asn Leu Pro Met Trp Ser Asn Gln
    210                 215                 220

Thr Trp Asn Asn Ser Thr Trp Ser Asn Gln Thr Gln Asn Ile Gln Ser
225                 230                 235                 240

Trp Ser Asn His Ser Trp Asn Thr Gln Thr Trp Cys Thr Gln Ser Trp
                245                 250                 255

Asn Asn Gln Ala Trp Asn Ser Pro Phe Tyr Asn Cys Gly Glu Glu Ser
            260                 265                 270

Leu Gln Ser Cys Met Gln Phe Gln Pro Asn Ser Pro Ala Ser Asp Leu
        275                 280                 285

Glu Ala Ala Leu Glu Ala Ala Gly Glu Gly Leu Asn Val Ile Gln Gln
    290                 295                 300

Thr Thr Arg Tyr Phe Ser Thr Pro Gln Thr Met Asp Leu Phe Leu Asn
305                 310                 315                 320

Tyr Ser Met Asn Met Gln Pro Glu Asp Val Leu Val Ala Ala Leu Leu
                325                 330                 335

Ala Val Leu

<210>    39
<211>    1047
<212>    DNA
<213>    Artificial Sequence

<220>
<223>    His-NLS-JO-84 MTD-Nanog-JO-84 MTD cDNA Sequence

<400>    39
atgggcagca gccatcatca tcatcatcac agcagcggcc tggtgccgcg cggcagccat        60

atgaagaaga agaggaagct ggtggcggcg ctgctggcgg tgctgagtgt ggatccagct       120

tgtccccaaa gcttgccttg ctttgaagca tccgactgta aagaatcttc acctatgcct       180

gtgatttgtg ggcctgaaga aaactatcca tccttgcaaa tgtcttctgc tgagatgcct 240

cacacggaga ctgtctctcc tcttccctcc tccatggatc tgcttattca ggacagccct 300

gattcttcca ccagtcccaa aggcaaacaa cccacttctg cagagaatag tgtcgcaaaa 360

aaggaagaca aggtcccagt caagaaacag aagaccagaa ctgtgttctc ttccacccag 420

ctgtgtgtac tcaatgatag atttcagaga cagaaatacc tcagcctcca gcagatgcaa 480

gaactctcca acatcctgaa cctcagctac aaacaggtga agacctggtt ccagaaccag 540

agaatgaaat ctaagaggtg gcagaaaaac aactggccga agaatagcaa tggtgtgacg 600

cagaaggcct cagcacctac ctaccccagc ctctactctt cctaccacca gggatgcctg 660

gtgaacccga ctgggaacct tccaatgtgg agcaaccaga cctggaacaa ttcaacctgg 720

agcaaccaga cccagaacat ccagtcctgg agcaaccact cctggaacac tcagacctgg 780

tgcacccaat cctggaacaa tcaggcctgg aacagtccct tctataactg tggagaggaa 840

tctctgcagt cctgcatgca gttccagcca aattctcctg ccagtgactt ggaggctgct 900

ttggaagctg ctggggaagg ccttaatgta atacagcaga ccactaggta ttttagtact 960

ccacaaacca tggatttatt cctaaactac tccatgaaca tgcaacctga agacgtgctg 1020

gtggcggcgc tgctggcggt gctgtga 1047

<210> 40
<211> 348
<212> PRT
<213> Artificial Sequence

<220>
<223> His-NLS-JO-84 MTD-Nanog-JO-84 MTD Amino Acid Sequence

<400>    40
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Ser Val Asp Pro Ala Cys Pro Gln Ser Leu Pro Cys Phe
        35                  40                  45

Glu Ala Ser Asp Cys Lys Glu Ser Ser Pro Met Pro Val Ile Cys Gly
    50                  55                  60

Pro Glu Glu Asn Tyr Pro Ser Leu Gln Met Ser Ser Ala Glu Met Pro
65                  70                  75                  80

His Thr Glu Thr Val Ser Pro Leu Pro Ser Ser Met Asp Leu Leu Ile
                85                  90                  95

Gln Asp Ser Pro Asp Ser Ser Thr Ser Pro Lys Gly Lys Gln Pro Thr
            100                 105                 110

Ser Ala Glu Asn Ser Val Ala Lys Lys Glu Asp Lys Val Pro Val Lys
        115                 120                 125

Lys Gln Lys Thr Arg Thr Val Phe Ser Ser Thr Gln Leu Cys Val Leu
    130                 135                 140

Asn Asp Arg Phe Gln Arg Gln Lys Tyr Leu Ser Leu Gln Gln Met Gln
145                 150                 155                 160

Glu Leu Ser Asn Ile Leu Asn Leu Ser Tyr Lys Gln Val Lys Thr Trp
                165                 170                 175

Phe Gln Asn Gln Arg Met Lys Ser Lys Arg Trp Gln Lys Asn Asn Trp
            180                 185                 190

Pro Lys Asn Ser Asn Gly Val Thr Gln Lys Ala Ser Ala Pro Thr Tyr
        195                 200                 205

Pro Ser Leu Tyr Ser Ser Tyr His Gln Gly Cys Leu Val Asn Pro Thr
    210                 215                 220

Gly Asn Leu Pro Met Trp Ser Asn Gln Thr Trp Asn Asn Ser Thr Trp
225                 230                 235                 240

Ser Asn Gln Thr Gln Asn Ile Gln Ser Trp Ser Asn His Ser Trp Asn
                245                 250                 255

Thr Gln Thr Trp Cys Thr Gln Ser Trp Asn Asn Gln Ala Trp Asn Ser
            260                 265                 270

Pro Phe Tyr Asn Cys Gly Glu Glu Ser Leu Gln Ser Cys Met Gln Phe
        275                 280                 285

Gln Pro Asn Ser Pro Ala Ser Asp Leu Glu Ala Ala Leu Glu Ala Ala
    290                 295                 300

Gly Glu Gly Leu Asn Val Ile Gln Gln Thr Thr Arg Tyr Phe Ser Thr
305                 310                 315                 320

Pro Gln Thr Met Asp Leu Phe Leu Asn Tyr Ser Met Asn Met Gln Pro
                325                 330                 335

Glu Asp Val Leu Val Ala Ala Leu Leu Ala Val Leu
            340                 345

<210> 41
<211> 1017
<212> DNA
<213> Artificial Sequence

<220>
<223> His-NLS-JO-86 MTD-Nanog cDNA Sequence

<400> 41
atgggcagca gccatcatca tcatcatcac agcagcggcc tggtgccgcg cggcagccat 60

atgaagaaga agaggaagct ggcggtgctg gcggcggcgc cgagtgtgga tccagcttgt 120

ccccaaagct tgccttgctt tgaagcatcc gactgtaaag aatcttcacc tatgcctgtg 180

atttgtgggc ctgaagaaaa ctatccatcc ttgcaaatgt cttctgctga gatgcctcac 240

acggagactg tctctcctct tccctcctcc atggatctgc ttattcagga cagccctgat 300

tcttccacca gtcccaaagg caaacaaccc acttctgcag agaatagtgt cgcaaaaaag 360

gaagacaagg tcccagtcaa gaaacagaag accagaactg tgttctcttc cacccagctg 420

tgtgtactca atgatagatt tcagagacag aaatacctca gcctccagca gatgcaagaa 480

ctctccaaca tcctgaacct cagctacaaa caggtgaaga cctggttcca gaaccagaga 540

atgaaatcta agaggtggca gaaaaacaac tggccgaaga atagcaatgg tgtgacgcag 600

aaggcctcag cacctaccta ccccagcctc tactcttcct accaccaggg atgcctggtg 660

aacccgactg ggaaccttcc aatgtggagc aaccagacct ggaacaattc aacctggagc 720

aaccagaccc agaacatcca gtcctggagc aaccactcct ggaacactca gacctggtgc 780

acccaatcct ggaacaatca ggcctggaac agtcccttct ataactgtgg agaggaatct 840

ctgcagtcct gcatgcagtt ccagccaaat tctcctgcca gtgacttgga ggctgctttg 900

gaagctgctg gggaaggcct taatgtaata cagcagacca ctaggtattt tagtactcca 960

caaaccatgg atttattcct aaactactcc atgaacatgc aacctgaaga cgtgtga 1017

<210> 42

<211> 338

<212> PRT

<213> Artificial Sequence

<220>

<223>    His-NLS-JO-86 MTD-Nanog Amino Acid Sequence

<400>    42
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Ser Val Asp Pro Ala Cys Pro Gln Ser Leu Pro Cys Phe Glu
        35                  40                  45

Ala Ser Asp Cys Lys Glu Ser Ser Pro Met Pro Val Ile Cys Gly Pro
    50                  55                  60

Glu Glu Asn Tyr Pro Ser Leu Gln Met Ser Ser Ala Glu Met Pro His
65                  70                  75                  80

Thr Glu Thr Val Ser Pro Leu Pro Ser Ser Met Asp Leu Leu Ile Gln
                85                  90                  95

Asp Ser Pro Asp Ser Ser Thr Ser Pro Lys Gly Lys Gln Pro Thr Ser
            100                 105                 110

Ala Glu Asn Ser Val Ala Lys Lys Glu Asp Lys Val Pro Val Lys Lys
        115                 120                 125

Gln Lys Thr Arg Thr Val Phe Ser Ser Thr Gln Leu Cys Val Leu Asn
    130                 135                 140

Asp Arg Phe Gln Arg Gln Lys Tyr Leu Ser Leu Gln Gln Met Gln Glu
145                 150                 155                 160

Leu Ser Asn Ile Leu Asn Leu Ser Tyr Lys Gln Val Lys Thr Trp Phe
                165                 170                 175

Gln Asn Gln Arg Met Lys Ser Lys Arg Trp Gln Lys Asn Asn Trp Pro
            180                 185                 190

Lys Asn Ser Asn Gly Val Thr Gln Lys Ala Ser Ala Pro Thr Tyr Pro
        195                 200                 205

Ser Leu Tyr Ser Ser Tyr His Gln Gly Cys Leu Val Asn Pro Thr Gly
    210                 215                 220

Asn Leu Pro Met Trp Ser Asn Gln Thr Trp Asn Asn Ser Thr Trp Ser
225                 230                 235                 240

Asn Gln Thr Gln Asn Ile Gln Ser Trp Ser Asn His Ser Trp Asn Thr
                245                 250                 255

Gln Thr Trp Cys Thr Gln Ser Trp Asn Asn Gln Ala Trp Asn Ser Pro
            260                 265                 270

Phe Tyr Asn Cys Gly Glu Glu Ser Leu Gln Ser Cys Met Gln Phe Gln
        275                 280                 285

Pro Asn Ser Pro Ala Ser Asp Leu Glu Ala Ala Leu Glu Ala Ala Gly
    290                 295                 300

Glu Gly Leu Asn Val Ile Gln Gln Thr Thr Arg Tyr Phe Ser Thr Pro
305                 310                 315                 320

Gln Thr Met Asp Leu Phe Leu Asn Tyr Ser Met Asn Met Gln Pro Glu
                325                 330                 335

Asp Val

<210>    43
<211>    1017
<212>    DNA
<213>    Artificial Sequence

<220>
<223>    His-NLS-Nanog-JO-86 MTD cDNA Sequence

<400>    43
atgggcagca gccatcatca tcatcatcac agcagcggcc tggtgccgcg cggcagccat        60

atgaagaaga agaggaagag tgtggatcca gcttgtcccc aaagcttgcc ttgctttgaa       120

gcatccgact gtaaagaatc ttcacctatg cctgtgattt gtgggcctga agaaaactat       180

ccatccttgc aaatgtcttc tgctgagatg cctcacacgg agactgtctc tcctcttccc       240

tcctccatgg atctgcttat tcaggacagc cctgattctt ccaccagtcc caaaggcaaa       300

caacccactt ctgcagagaa tagtgtcgca aaaaaggaag acaaggtccc agtcaagaaa       360

cagaagacca gaactgtgtt ctcttccacc cagctgtgtg tactcaatga tagatttcag       420

agacagaaat acctcagcct ccagcagatg caagaactct ccaacatcct gaacctcagc       480

tacaaacagg tgaagacctg gttccagaac cagagaatga aatctaagag gtggcagaaa       540

aacaactggc cgaagaatag caatggtgtg acgcagaagg cctcagcacc tacctacccc       600

agcctctact cttcctacca ccagggatgc ctggtgaacc cgactgggaa ccttccaatg       660

tggagcaacc agacctggaa caattcaacc tggagcaacc agacccagaa catccagtcc       720

tggagcaacc actcctggaa cactcagacc tggtgcaccc aatcctggaa caatcaggcc       780

tggaacagtc ccttctataa ctgtggagag gaatctctgc agtcctgcat gcagttccag       840

ccaaattctc ctgccagtga cttggaggct gctttggaag ctgctgggga aggccttaat       900

gtaatacagc agaccactag gtattttagt actccacaaa ccatggattt attcctaaac       960

tactccatga acatgcaacc tgaagacgtg ctggcggtgc tggcggcggc gccgtga        1017


<210>    44
<211>    338
<212>    PRT
<213>    Artificial Sequence

<220>
<223>    His-NLS-Nanog-JO-86 MTD Amino Acid Sequence

<400>    44
Met Gly Ser Ser His His His His His His Ser Ser Gly Leu Val Pro
1               5                   10                  15

Arg Gly Ser His Met Lys Lys Lys Arg Lys Ser Val Asp Pro Ala Cys
            20                  25                  30

Pro Gln Ser Leu Pro Cys Phe Glu Ala Ser Asp Cys Lys Glu Ser Ser
        35                  40                  45

Pro Met Pro Val Ile Cys Gly Pro Glu Glu Asn Tyr Pro Ser Leu Gln
    50                  55                  60

Met Ser Ser Ala Glu Met Pro His Thr Glu Thr Val Ser Pro Leu Pro
65                  70                  75                  80

Ser Ser Met Asp Leu Leu Ile Gln Asp Ser Pro Asp Ser Ser Thr Ser
                85                  90                  95

Pro Lys Gly Lys Gln Pro Thr Ser Ala Glu Asn Ser Val Ala Lys Lys
            100                 105                 110

Glu Asp Lys Val Pro Val Lys Lys Gln Lys Thr Arg Thr Val Phe Ser
        115                 120                 125

Ser Thr Gln Leu Cys Val Leu Asn Asp Arg Phe Gln Arg Gln Lys Tyr
    130                 135                 140

Leu Ser Leu Gln Gln Met Gln Glu Leu Ser Asn Ile Leu Asn Leu Ser
145                 150                 155                 160

Tyr Lys Gln Val Lys Thr Trp Phe Gln Asn Gln Arg Met Lys Ser Lys
                165                 170                 175

Arg Trp Gln Lys Asn Asn Trp Pro Lys Asn Ser Asn Gly Val Thr Gln

180                 185                 190

Lys Ala Ser Ala Pro Thr Tyr Pro Ser Leu Tyr Ser Ser Tyr His Gln
        195                 200                 205

Gly Cys Leu Val Asn Pro Thr Gly Asn Leu Pro Met Trp Ser Asn Gln
    210                 215                 220

Thr Trp Asn Asn Ser Thr Trp Ser Asn Gln Thr Gln Asn Ile Gln Ser
225                 230                 235                 240

Trp Ser Asn His Ser Trp Asn Thr Gln Thr Trp Cys Thr Gln Ser Trp
                245                 250                 255

Asn Asn Gln Ala Trp Asn Ser Pro Phe Tyr Asn Cys Gly Glu Glu Ser
            260                 265                 270

Leu Gln Ser Cys Met Gln Phe Gln Pro Asn Ser Pro Ala Ser Asp Leu
        275                 280                 285

Glu Ala Ala Leu Glu Ala Ala Gly Glu Gly Leu Asn Val Ile Gln Gln
    290                 295                 300

Thr Thr Arg Tyr Phe Ser Thr Pro Gln Thr Met Asp Leu Phe Leu Asn
305                 310                 315                 320

Tyr Ser Met Asn Met Gln Pro Glu Asp Val Leu Ala Val Leu Ala Ala
                325                 330                 335

Ala Pro

<210>   45
<211>   1041
<212>   DNA
<213>   Artificial Sequence

<220>
<223>   His-NLS-JO-86 MTD-Nanog-JO-86 MTD cDNA Sequence

<400>    45

atgggcagca gccatcatca tcatcatcac agcagcggcc tggtgccgcg cggcagccat    60

atgaagaaga agaggaagct ggcggtgctg gcggcggcgc cgagtgtgga tccagcttgt    120

ccccaaagct tgccttgctt tgaagcatcc gactgtaaag aatcttcacc tatgcctgtg    180

atttgtgggc ctgaagaaaa ctatccatcc ttgcaaatgt cttctgctga gatgcctcac    240

acggagactg tctctcctct tccctcctcc atggatctgc ttattcagga cagccctgat    300

tcttccacca gtcccaaagg caaacaaccc acttctgcag agaatagtgt cgcaaaaaag    360

gaagacaagg tcccagtcaa gaaacagaag accagaactg tgttctcttc cacccagctg    420

tgtgtactca atgatagatt tcagagacag aaatacctca gcctccagca gatgcaagaa    480

ctctccaaca tcctgaacct cagctacaaa caggtgaaga cctggttcca gaaccagaga    540

atgaaatcta agaggtggca gaaaaacaac tggccgaaga atagcaatgg tgtgacgcag    600

aaggcctcag cacctaccta ccccagcctc tactcttcct accaccaggg atgcctggtg    660

aacccgactg ggaaccttcc aatgtggagc aaccagacct ggaacaattc aacctggagc    720

aaccagaccc agaacatcca gtcctggagc aaccactcct ggaacactca gacctggtgc    780

acccaatcct ggaacaatca ggcctggaac agtcccttct ataactgtgg agaggaatct    840

ctgcagtcct gcatgcagtt ccagccaaat tctcctgcca gtgacttgga ggctgctttg    900

gaagctgctg gggaaggcct taatgtaata cagcagacca ctaggtattt tagtactcca    960

caaaccatgg atttattcct aaactactcc atgaacatgc aacctgaaga cgtgctggcg    1020

gtgctggcgg cggcgccgtg a                                              1041

<210>    46
<211>    346
<212>    PRT
<213>    Artificial Sequence

<220>
<223>    His-NLS-JO-86 MTD-Nanog-JO-86 MTD Amino Acid Sequence

<400>    46
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Ser Val Asp Pro Ala Cys Pro Gln Ser Leu Pro Cys Phe Glu
        35                  40                  45

Ala Ser Asp Cys Lys Glu Ser Ser Pro Met Pro Val Ile Cys Gly Pro
    50                  55                  60

Glu Glu Asn Tyr Pro Ser Leu Gln Met Ser Ser Ala Glu Met Pro His
65                  70                  75                  80

Thr Glu Thr Val Ser Pro Leu Pro Ser Ser Met Asp Leu Leu Ile Gln
                85                  90                  95

Asp Ser Pro Asp Ser Ser Thr Ser Pro Lys Gly Lys Gln Pro Thr Ser
            100                 105                 110

Ala Glu Asn Ser Val Ala Lys Lys Glu Asp Lys Val Pro Val Lys Lys
        115                 120                 125

Gln Lys Thr Arg Thr Val Phe Ser Ser Thr Gln Leu Cys Val Leu Asn
    130                 135                 140

Asp Arg Phe Gln Arg Gln Lys Tyr Leu Ser Leu Gln Gln Met Gln Glu
145                 150                 155                 160

Leu Ser Asn Ile Leu Asn Leu Ser Tyr Lys Gln Val Lys Thr Trp Phe
                165                 170                 175

Gln Asn Gln Arg Met Lys Ser Lys Arg Trp Gln Lys Asn Asn Trp Pro
            180                 185                 190

Lys Asn Ser Asn Gly Val Thr Gln Lys Ala Ser Ala Pro Thr Tyr Pro
        195                 200                 205

Ser Leu Tyr Ser Ser Tyr His Gln Gly Cys Leu Val Asn Pro Thr Gly
    210                 215                 220

Asn Leu Pro Met Trp Ser Asn Gln Thr Trp Asn Asn Ser Thr Trp Ser
225                 230                 235                 240

Asn Gln Thr Gln Asn Ile Gln Ser Trp Ser Asn His Ser Trp Asn Thr
                245                 250                 255

Gln Thr Trp Cys Thr Gln Ser Trp Asn Asn Gln Ala Trp Asn Ser Pro
            260                 265                 270

Phe Tyr Asn Cys Gly Glu Glu Ser Leu Gln Ser Cys Met Gln Phe Gln
        275                 280                 285

Pro Asn Ser Pro Ala Ser Asp Leu Glu Ala Ala Leu Glu Ala Ala Gly
    290                 295                 300

Glu Gly Leu Asn Val Ile Gln Gln Thr Thr Arg Tyr Phe Ser Thr Pro
305                 310                 315                 320

Gln Thr Met Asp Leu Phe Leu Asn Tyr Ser Met Asn Met Gln Pro Glu
                325                 330                 335

Asp Val Leu Ala Val Leu Ala Ala Ala Pro
            340                 345

<210> 47
<211> 1098
<212> DNA

<213>    Artificial Sequence

<220>
<223>    NLS-Oct4 cDNA Sequence

<400>    47
atgaagaaga agaggaaggc gggacacctg gcttcggatt tcgccttctc gccccctcca      60

ggtggtggag gtgatgggcc aggggggccg gagccgggct gggttgatcc tcggacctgg     120

ctaagcttcc aaggccctcc tggagggcca ggaatcgggc cgggggttgg gccaggctct     180

gaggtgtggg ggattccccc atgccccccg ccgtatgagt tctgtggggg gatggcgtac     240

tgtgggcccc aggttggagt ggggctagtg ccccaaggcg gcttggagac ctctcagcct     300

gagggcgaag caggagtcgg ggtggagagc aactccgatg gggcctcccc ggagccctgc     360

accgtcaccc ctggtgccgt gaagctggag aaggagaagc tggagcaaaa cccggaggag     420

tcccaggaca tcaaagctct gcagaaagaa ctcgagcaat ttgccaagct cctgaagcag     480

aagaggatca ccctgggata tacacaggcc gatgtggggc tcaccctggg ggttctattt     540

gggaaggtat tcagccaaac gaccatctgc cgctttgagg ctctgcagct tagcttcaag     600

aacatgtgta agctgcggcc cttgctgcag aagtgggtgg aggaagctga caacaatgaa     660

aatcttcagg agatatgcaa agcagaaacc ctcgtgcagg cccgaaagag aaagcgaacc     720

agtatcgaga accgagtgag aggcaacctg gagaatttgt tcctgcagtg cccgaaaccc     780

acactgcagc agatcagcca catcgcccag cagcttgggc tcgagaagga tgtggtccga     840

gtgtggttct gtaaccggcg ccagaagggc aagcgatcaa gcagcgacta tgcacaacga     900

gaggattttg aggctgctgg gtctcctttc tcagggggac cagtgtcctt tcctctggcc     960

ccagggcccc attttggtac cccaggctat gggagccctc acttcactgc actgtactcc    1020

tcggtccctt tccctgaggg ggaagccttt ccccctgtct ccgtcaccac tctgggctct        1080

cccatgcatt caaactga                                                    1098

<210>    48
<211>    365
<212>    PRT
<213>    Artificial Sequence

<220>
<223>    NLS-Oct4 Amino Acid Sequence

<400>    48
Met Lys Lys Lys Arg Lys Ala Gly His Leu Ala Ser Asp Phe Ala Phe
1               5                   10                  15

Ser Pro Pro Pro Gly Gly Gly Gly Asp Gly Pro Gly Gly Pro Glu Pro
            20                  25                  30

Gly Trp Val Asp Pro Arg Thr Trp Leu Ser Phe Gln Gly Pro Pro Gly
        35                  40                  45

Gly Pro Gly Ile Gly Pro Gly Val Gly Pro Gly Ser Glu Val Trp Gly
    50                  55                  60

Ile Pro Pro Cys Pro Pro Pro Tyr Glu Phe Cys Gly Gly Met Ala Tyr
65                  70                  75                  80

Cys Gly Pro Gln Val Gly Val Gly Leu Val Pro Gln Gly Gly Leu Glu
                85                  90                  95

Thr Ser Gln Pro Glu Gly Glu Ala Gly Val Gly Val Glu Ser Asn Ser
            100                 105                 110

Asp Gly Ala Ser Pro Glu Pro Cys Thr Val Thr Pro Gly Ala Val Lys
        115                 120                 125

Leu Glu Lys Glu Lys Leu Glu Gln Asn Pro Glu Glu Ser Gln Asp Ile
    130                 135                 140

Lys Ala Leu Gln Lys Glu Leu Glu Gln Phe Ala Lys Leu Leu Lys Gln
145                 150                 155                 160

Lys Arg Ile Thr Leu Gly Tyr Thr Gln Ala Asp Val Gly Leu Thr Leu
                165                 170                 175

Gly Val Leu Phe Gly Lys Val Phe Ser Gln Thr Thr Ile Cys Arg Phe
            180                 185                 190

Glu Ala Leu Gln Leu Ser Phe Lys Asn Met Cys Lys Leu Arg Pro Leu
        195                 200                 205

Leu Gln Lys Trp Val Glu Glu Ala Asp Asn Asn Glu Asn Leu Gln Glu
    210                 215                 220

Ile Cys Lys Ala Glu Thr Leu Val Gln Ala Arg Lys Arg Lys Arg Thr
225                 230                 235                 240

Ser Ile Glu Asn Arg Val Arg Gly Asn Leu Glu Asn Leu Phe Leu Gln
                245                 250                 255

Cys Pro Lys Pro Thr Leu Gln Gln Ile Ser His Ile Ala Gln Gln Leu
            260                 265                 270

Gly Leu Glu Lys Asp Val Val Arg Val Trp Phe Cys Asn Arg Arg Gln
        275                 280                 285

Lys Gly Lys Arg Ser Ser Ser Asp Tyr Ala Gln Arg Glu Asp Phe Glu
    290                 295                 300

Ala Ala Gly Ser Pro Phe Ser Gly Gly Pro Val Ser Phe Pro Leu Ala
305                 310                 315                 320

Pro Gly Pro His Phe Gly Thr Pro Gly Tyr Gly Ser Pro His Phe Thr
                325                 330                 335

Ala Leu Tyr Ser Ser Val Pro Phe Pro Glu Gly Glu Ala Phe Pro Pro

340                 345                 350

Val Ser Val Thr Thr Leu Gly Ser Pro Met His Ser Asn
        355                 360                 365

<210>   49
<211>   1125
<212>   DNA
<213>   Artificial Sequence

<220>
<223>   NLS-JO-84 MTD-Oct4 cDNA Sequence

<400>   49
atgaagaaga agaggaagct ggtggcggcg ctgctggcgg tgctggcggg acacctggct        60

tcggatttcg ccttctcgcc ccctccaggt ggtggaggtg atgggccagg ggggccggag       120

ccgggctggg ttgatcctcg gacctggcta agcttccaag gccctcctgg agggccagga       180

atcgggccgg gggttgggcc aggctctgag gtgtggggga ttcccccatg ccccccgccg       240

tatgagttct gtggggggat ggcgtactgt gggccccagg ttggagtggg gctagtgccc       300

caaggcggct tggagacctc tcagcctgag ggcgaagcag gagtcggggt ggagagcaac       360

tccgatgggg cctccccgga gccctgcacc gtcacccctg gtgccgtgaa gctggagaag       420

gagaagctgg agcaaaaccc ggaggagtcc caggacatca aagctctgca gaaagaactc       480

gagcaatttg ccaagctcct gaagcagaag aggatcaccc tgggatatac acaggccgat       540

gtggggctca ccctgggggt tctatttggg aaggtattca gccaaacgac catctgccgc       600

tttgaggctc tgcagcttag cttcaagaac atgtgtaagc tgcggccctt gctgcagaag       660

tgggtggagg aagctgacaa caatgaaaat cttcaggaga tatgcaaagc agaaaccctc       720

gtgcaggccc gaaagagaaa gcgaaccagt atcgagaacc gagtgagagg caacctggag 780

aatttgttcc tgcagtgccc gaaacccaca ctgcagcaga tcagccacat cgcccagcag 840

cttgggctcg agaaggatgt ggtccgagtg tggttctgta accggcgcca gaagggcaag 900

cgatcaagca gcgactatgc acaacgagag gattttgagg ctgctgggtc tcctttctca 960

gggggaccag tgtcctttcc tctggcccca gggccccatt ttggtacccc aggctatggg 1020

agccctcact tcactgcact gtactcctcg gtccctttcc ctgaggggga agcctttccc 1080

cctgtctccg tcaccactct gggctctccc atgcattcaa actga 1125

<210> 50
<211> 374
<212> PRT
<213> Artificial Sequence

<220>
<223> NLS-JO-84 MTD-Oct4 Amino Acid Sequence

<400> 50
Met Lys Lys Lys Arg Lys Leu Val Ala Ala Leu Leu Ala Val Leu Ala
1               5                   10                  15

Gly His Leu Ala Ser Asp Phe Ala Phe Ser Pro Pro Pro Gly Gly Gly
            20                  25                  30

Gly Asp Gly Pro Gly Gly Pro Glu Pro Gly Trp Val Asp Pro Arg Thr
        35                  40                  45

Trp Leu Ser Phe Gln Gly Pro Pro Gly Gly Pro Gly Ile Gly Pro Gly
    50                  55                  60

Val Gly Pro Gly Ser Glu Val Trp Gly Ile Pro Pro Cys Pro Pro Pro
65                  70                  75                  80

Tyr Glu Phe Cys Gly Gly Met Ala Tyr Cys Gly Pro Gln Val Gly Val
                85                  90                  95

Gly Leu Val Pro Gln Gly Gly Leu Glu Thr Ser Gln Pro Glu Gly Glu
            100                 105                 110

Ala Gly Val Gly Val Glu Ser Asn Ser Asp Gly Ala Ser Pro Glu Pro
        115                 120                 125

Cys Thr Val Thr Pro Gly Ala Val Lys Leu Glu Lys Glu Lys Leu Glu
    130                 135                 140

Gln Asn Pro Glu Glu Ser Gln Asp Ile Lys Ala Leu Gln Lys Glu Leu
145                 150                 155                 160

Glu Gln Phe Ala Lys Leu Leu Lys Gln Lys Arg Ile Thr Leu Gly Tyr
                165                 170                 175

Thr Gln Ala Asp Val Gly Leu Thr Leu Gly Val Leu Phe Gly Lys Val
            180                 185                 190

Phe Ser Gln Thr Thr Ile Cys Arg Phe Glu Ala Leu Gln Leu Ser Phe
        195                 200                 205

Lys Asn Met Cys Lys Leu Arg Pro Leu Leu Gln Lys Trp Val Glu Glu
    210                 215                 220

Ala Asp Asn Asn Glu Asn Leu Gln Glu Ile Cys Lys Ala Glu Thr Leu
225                 230                 235                 240

Val Gln Ala Arg Lys Arg Lys Arg Thr Ser Ile Glu Asn Arg Val Arg
                245                 250                 255

Gly Asn Leu Glu Asn Leu Phe Leu Gln Cys Pro Lys Pro Thr Leu Gln
            260                 265                 270

Gln Ile Ser His Ile Ala Gln Gln Leu Gly Leu Glu Lys Asp Val Val
        275                 280                 285

Arg Val Trp Phe Cys Asn Arg Arg Gln Lys Gly Lys Arg Ser Ser Ser

290                 295                 300

Asp Tyr Ala Gln Arg Glu Asp Phe Glu Ala Ala Gly Ser Pro Phe Ser
305                 310                 315                 320

Gly Gly Pro Val Ser Phe Pro Leu Ala Pro Gly Pro His Phe Gly Thr
                325                 330                 335

Pro Gly Tyr Gly Ser Pro His Phe Thr Ala Leu Tyr Ser Ser Val Pro
            340                 345                 350

Phe Pro Glu Gly Glu Ala Phe Pro Pro Val Ser Val Thr Thr Leu Gly
        355                 360                 365

Ser Pro Met His Ser Asn
    370

<210>    51
<211>    1125
<212>    DNA
<213>    Artificial Sequence

<220>
<223>    NLS-Oct4-JO-84 MTD cDNA Sequence

<400>    51
atgaagaaga agaggaaggc gggacacctg gcttcggatt tcgccttctc gccccctcca        60

ggtggtggag gtgatgggcc aggggggccg gagccgggct gggttgatcc tcggacctgg       120

ctaagcttcc aaggccctcc tggagggcca ggaatcgggc cgggggttgg gccaggctct       180

gaggtgtggg ggattccccc atgccccccg ccgtatgagt tctgtggggg gatggcgtac       240

tgtgggcccc aggttggagt ggggctagtg ccccaaggcg gcttggagac ctctcagcct       300

gagggcgaag caggagtcgg ggtggagagc aactccgatg gggcctcccc ggagccctgc       360

accgtcaccc ctggtgccgt gaagctggag aaggagaagc tggagcaaaa cccggaggag 420

tcccaggaca tcaaagctct gcagaaagaa ctcgagcaat ttgccaagct cctgaagcag 480

aagaggatca ccctgggata tacacaggcc gatgtggggc tcaccctggg ggttctattt 540

gggaaggtat tcagccaaac gaccatctgc cgctttgagg ctctgcagct tagcttcaag 600

aacatgtgta agctgcggcc cttgctgcag aagtgggtgg aggaagctga caacaatgaa 660

aatcttcagg agatatgcaa agcagaaacc ctcgtgcagg cccgaaagag aaagcgaacc 720

agtatcgaga accgagtgag aggcaacctg gagaatttgt tcctgcagtg cccgaaaccc 780

acactgcagc agatcagcca catcgcccag cagcttgggc tcgagaagga tgtggtccga 840

gtgtggttct gtaaccggcg ccagaagggc aagcgatcaa gcagcgacta tgcacaacga 900

gaggattttg aggctgctgg gtctcctttc tcagggggac cagtgtcctt tcctctggcc 960

ccagggcccc attttggtac cccaggctat gggagccctc acttcactgc actgtactcc 1020

tcggtccctt tccctgaggg ggaagccttt ccccctgtct ccgtcaccac tctgggctct 1080

cccatgcatt caaacctggt ggcggcgctg ctggcggtgc tgtga 1125

<210> 52
<211> 374
<212> PRT
<213> Artificial Sequence

<220>
<223> NLS-Oct4-JO-84 MTD Amino Acid Sequence

<400> 52
Met Lys Lys Lys Arg Lys Ala Gly His Leu Ala Ser Asp Phe Ala Phe
1               5                   10                  15

Ser Pro Pro Pro Gly Gly Gly Gly Asp Gly Pro Gly Gly Pro Glu Pro
            20                  25                  30

Gly Trp Val Asp Pro Arg Thr Trp Leu Ser Phe Gln Gly Pro Pro Gly
        35                  40                  45

Gly Pro Gly Ile Gly Pro Gly Val Gly Pro Gly Ser Glu Val Trp Gly
    50                  55                  60

Ile Pro Pro Cys Pro Pro Pro Tyr Glu Phe Cys Gly Gly Met Ala Tyr
65                  70                  75                  80

Cys Gly Pro Gln Val Gly Val Gly Leu Val Pro Gln Gly Gly Leu Glu
                85                  90                  95

Thr Ser Gln Pro Glu Gly Glu Ala Gly Val Gly Val Glu Ser Asn Ser
            100                 105                 110

Asp Gly Ala Ser Pro Glu Pro Cys Thr Val Thr Pro Gly Ala Val Lys
        115                 120                 125

Leu Glu Lys Glu Lys Leu Glu Gln Asn Pro Glu Glu Ser Gln Asp Ile
    130                 135                 140

Lys Ala Leu Gln Lys Glu Leu Glu Gln Phe Ala Lys Leu Leu Lys Gln
145                 150                 155                 160

Lys Arg Ile Thr Leu Gly Tyr Thr Gln Ala Asp Val Gly Leu Thr Leu
                165                 170                 175

Gly Val Leu Phe Gly Lys Val Phe Ser Gln Thr Thr Ile Cys Arg Phe
            180                 185                 190

Glu Ala Leu Gln Leu Ser Phe Lys Asn Met Cys Lys Leu Arg Pro Leu
        195                 200                 205

Leu Gln Lys Trp Val Glu Glu Ala Asp Asn Asn Glu Asn Leu Gln Glu
    210                 215                 220

Ile Cys Lys Ala Glu Thr Leu Val Gln Ala Arg Lys Arg Lys Arg Thr

225                 230                 235                 240

Ser Ile Glu Asn Arg Val Arg Gly Asn Leu Glu Asn Leu Phe Leu Gln
                245                 250                 255

Cys Pro Lys Pro Thr Leu Gln Gln Ile Ser His Ile Ala Gln Gln Leu
            260                 265                 270

Gly Leu Glu Lys Asp Val Val Arg Val Trp Phe Cys Asn Arg Arg Gln
        275                 280                 285

Lys Gly Lys Arg Ser Ser Ser Asp Tyr Ala Gln Arg Glu Asp Phe Glu
    290                 295                 300

Ala Ala Gly Ser Pro Phe Ser Gly Gly Pro Val Ser Phe Pro Leu Ala
305                 310                 315                 320

Pro Gly Pro His Phe Gly Thr Pro Gly Tyr Gly Ser Pro His Phe Thr
                325                 330                 335

Ala Leu Tyr Ser Ser Val Pro Phe Pro Glu Gly Glu Ala Phe Pro Pro
            340                 345                 350

Val Ser Val Thr Thr Leu Gly Ser Pro Met His Ser Asn Leu Val Ala
        355                 360                 365

Ala Leu Leu Ala Val Leu
    370

<210>   53
<211>   1152
<212>   DNA
<213>   Artificial Sequence

<220>
<223>   NLS-JO-84 MTD-Oct4-JO-84 MTD cDNA Sequence

<400>   53

atgaagaaga agaggaagct ggtggcggcg ctgctggcgg tgctggcggg acacctggct 60

tcggatttcg ccttctcgcc ccctccaggt ggtggaggtg atgggccagg ggggccggag 120

ccgggctggg ttgatcctcg gacctggcta agcttccaag gccctcctgg agggccagga 180

atcgggccgg gggttgggcc aggctctgag gtgtggggga ttcccccatg ccccccgccg 240

tatgagttct gtgggggat ggcgtactgt gggccccagg ttggagtggg gctagtgccc 300

caaggcggct tggagacctc tcagcctgag ggcgaagcag gagtcggggt ggagagcaac 360

tccgatgggg cctccccgga gccctgcacc gtcacccctg gtgccgtgaa gctggagaag 420

gagaagctgg agcaaaaccc ggaggagtcc caggacatca aagctctgca gaaagaactc 480

gagcaatttg ccaagctcct gaagcagaag aggatcaccc tgggatatac acaggccgat 540

gtggggctca ccctgggggt tctatttggg aaggtattca gccaaacgac catctgccgc 600

tttgaggctc tgcagcttag cttcaagaac atgtgtaagc tgcggccctt gctgcagaag 660

tgggtggagg aagctgacaa caatgaaaat cttcaggaga tatgcaaagc agaaaccctc 720

gtgcaggccc gaaagagaaa gcgaaccagt atcgagaacc gagtgagagg caacctggag 780

aatttgttcc tgcagtgccc gaaacccaca ctgcagcaga tcagccacat cgcccagcag 840

cttgggctcg agaaggatgt ggtccgagtg tggttctgta accggcgcca gaagggcaag 900

cgatcaagca gcgactatgc acaacgagag gattttgagg ctgctgggtc tcctttctca 960

gggggaccag tgtcctttcc tctggcccca gggccccatt ttggtacccc aggctatggg 1020

agccctcact tcactgcact gtactcctcg gtccctttcc ctgaggggga agcctttccc 1080

cctgtctccg tcaccactct gggctctccc atgcattcaa acctggtggc ggcgctgctg 1140

gcggtgctgt ga 1152

<210>    54
<211>    383
<212>    PRT
<213>    Artificial Sequence

<220>
<223>    NLS-JO-84 MTD-Oct4-JO-84 MTD Amino Acid Sequence

<400>    54
Met Lys Lys Lys Arg Lys Leu Val Ala Ala Leu Leu Ala Val Leu Ala
1               5                   10                  15

Gly His Leu Ala Ser Asp Phe Ala Phe Ser Pro Pro Pro Gly Gly Gly
            20                  25                  30

Gly Asp Gly Pro Gly Gly Pro Glu Pro Gly Trp Val Asp Pro Arg Thr
        35                  40                  45

Trp Leu Ser Phe Gln Gly Pro Pro Gly Gly Pro Gly Ile Gly Pro Gly
    50                  55                  60

Val Gly Pro Gly Ser Glu Val Trp Gly Ile Pro Pro Cys Pro Pro Pro
65                  70                  75                  80

Tyr Glu Phe Cys Gly Gly Met Ala Tyr Cys Gly Pro Gln Val Gly Val
                85                  90                  95

Gly Leu Val Pro Gln Gly Gly Leu Glu Thr Ser Gln Pro Glu Gly Glu
            100                 105                 110

Ala Gly Val Gly Val Glu Ser Asn Ser Asp Gly Ala Ser Pro Glu Pro
        115                 120                 125

Cys Thr Val Thr Pro Gly Ala Val Lys Leu Glu Lys Glu Lys Leu Glu
    130                 135                 140

Gln Asn Pro Glu Glu Ser Gln Asp Ile Lys Ala Leu Gln Lys Glu Leu
145                 150                 155                 160

Glu Gln Phe Ala Lys Leu Leu Lys Gln Lys Arg Ile Thr Leu Gly Tyr
                165                 170                 175

Thr Gln Ala Asp Val Gly Leu Thr Leu Gly Val Leu Phe Gly Lys Val
            180                 185                 190

Phe Ser Gln Thr Thr Ile Cys Arg Phe Glu Ala Leu Gln Leu Ser Phe
        195                 200                 205

Lys Asn Met Cys Lys Leu Arg Pro Leu Leu Gln Lys Trp Val Glu Glu
    210                 215                 220

Ala Asp Asn Asn Glu Asn Leu Gln Glu Ile Cys Lys Ala Glu Thr Leu
225                 230                 235                 240

Val Gln Ala Arg Lys Arg Lys Arg Thr Ser Ile Glu Asn Arg Val Arg
                245                 250                 255

Gly Asn Leu Glu Asn Leu Phe Leu Gln Cys Pro Lys Pro Thr Leu Gln
            260                 265                 270

Gln Ile Ser His Ile Ala Gln Gln Leu Gly Leu Glu Lys Asp Val Val
        275                 280                 285

Arg Val Trp Phe Cys Asn Arg Arg Gln Lys Gly Lys Arg Ser Ser Ser
    290                 295                 300

Asp Tyr Ala Gln Arg Glu Asp Phe Glu Ala Ala Gly Ser Pro Phe Ser
305                 310                 315                 320

Gly Gly Pro Val Ser Phe Pro Leu Ala Pro Gly Pro His Phe Gly Thr
                325                 330                 335

Pro Gly Tyr Gly Ser Pro His Phe Thr Ala Leu Tyr Ser Ser Val Pro
            340                 345                 350

Phe Pro Glu Gly Glu Ala Phe Pro Pro Val Ser Val Thr Thr Leu Gly
        355                 360                 365

Ser Pro Met His Ser Asn Leu Val Ala Ala Leu Leu Ala Val Leu
    370                 375                 380

<210> 55
<211> 1122
<212> DNA
<213> Artificial Sequence

<220>
<223> NLS-JO-86 MTD-Oct4 cDNA Sequence

<400> 55
atgaagaaga agaggaagct ggcggtgctg gcggcggcgc cggcgggaca cctggcttcg 60

gatttcgcct tctcgccccc tccaggtggt ggaggtgatg ggccaggggg gccggagccg 120

ggctgggttg atcctcggac ctggctaagc ttccaaggcc ctcctggagg gccaggaatc 180

gggccggggg ttgggccagg ctctgaggtg tgggggattc ccccatgccc cccgccgtat 240

gagttctgtg gggggatggc gtactgtggg ccccaggttg gagtggggct agtgccccaa 300

ggcggcttgg agacctctca gcctgagggc gaagcaggag tcggggtgga gagcaactcc 360

gatggggcct ccccggagcc ctgcaccgtc acccctggtg ccgtgaagct ggagaaggag 420

aagctggagc aaaacccgga ggagtcccag gacatcaaag ctctgcagaa agaactcgag 480

caatttgcca agctcctgaa gcagaagagg atcaccctgg gatatacaca ggccgatgtg 540

gggctcaccc tgggggttct atttgggaag gtattcagcc aaacgaccat ctgccgcttt 600

gaggctctgc agcttagctt caagaacatg tgtaagctgc ggcccttgct gcagaagtgg 660

gtggaggaag ctgacaacaa tgaaaatctt caggagatat gcaaagcaga aaccctcgtg 720

caggcccgaa agagaaagcg aaccagtatc gagaaccgag tgagaggcaa cctggagaat 780

ttgttcctgc agtgcccgaa acccacactg cagcagatca gccacatcgc ccagcagctt 840

gggctcgaga aggatgtggt ccgagtgtgg ttctgtaacc ggcgccagaa gggcaagcga 900

tcaagcagcg actatgcaca acgagaggat tttgaggctg ctgggtctcc tttctcaggg 960

ggaccagtgt cctttcctct ggccccaggg ccccattttg gtaccccagg ctatgggagc 1020

cctcacttca ctgcactgta ctcctcggtc cctttccctg agggggaagc ctttccccct 1080

gtctccgtca ccactctggg ctctcccatg cattcaaact ga 1122

<210> 56
<211> 373
<212> PRT
<213> Artificial Sequence

<220>
<223> NLS-JO-86 MTD-Oct4 Amico Acid Sequence

<400> 56
Met Lys Lys Lys Arg Lys Leu Ala Val Leu Ala Ala Ala Pro Ala Gly
1               5                   10                  15

His Leu Ala Ser Asp Phe Ala Phe Ser Pro Pro Pro Gly Gly Gly Gly
            20                  25                  30

Asp Gly Pro Gly Gly Pro Glu Pro Gly Trp Val Asp Pro Arg Thr Trp
        35                  40                  45

Leu Ser Phe Gln Gly Pro Pro Gly Gly Pro Gly Ile Gly Pro Gly Val
    50                  55                  60

Gly Pro Gly Ser Glu Val Trp Gly Ile Pro Pro Cys Pro Pro Pro Tyr
65                  70                  75                  80

Glu Phe Cys Gly Gly Met Ala Tyr Cys Gly Pro Gln Val Gly Val Gly
                85                  90                  95

Leu Val Pro Gln Gly Gly Leu Glu Thr Ser Gln Pro Glu Gly Glu Ala
            100                 105                 110

Gly Val Gly Val Glu Ser Asn Ser Asp Gly Ala Ser Pro Glu Pro Cys
        115                 120                 125

Thr Val Thr Pro Gly Ala Val Lys Leu Glu Lys Glu Lys Leu Glu Gln
    130                 135                 140

Asn Pro Glu Glu Ser Gln Asp Ile Lys Ala Leu Gln Lys Glu Leu Glu
145                 150                 155                 160

Gln Phe Ala Lys Leu Leu Lys Gln Lys Arg Ile Thr Leu Gly Tyr Thr
                165                 170                 175

Gln Ala Asp Val Gly Leu Thr Leu Gly Val Leu Phe Gly Lys Val Phe
            180                 185                 190

Ser Gln Thr Thr Ile Cys Arg Phe Glu Ala Leu Gln Leu Ser Phe Lys
        195                 200                 205

Asn Met Cys Lys Leu Arg Pro Leu Leu Gln Lys Trp Val Glu Glu Ala
    210                 215                 220

Asp Asn Asn Glu Asn Leu Gln Glu Ile Cys Lys Ala Glu Thr Leu Val
225                 230                 235                 240

Gln Ala Arg Lys Arg Lys Arg Thr Ser Ile Glu Asn Arg Val Arg Gly
                245                 250                 255

Asn Leu Glu Asn Leu Phe Leu Gln Cys Pro Lys Pro Thr Leu Gln Gln
            260                 265                 270

Ile Ser His Ile Ala Gln Gln Leu Gly Leu Glu Lys Asp Val Val Arg
        275                 280                 285

Val Trp Phe Cys Asn Arg Arg Gln Lys Gly Lys Arg Ser Ser Ser Asp

290                 295                 300

Tyr Ala Gln Arg Glu Asp Phe Glu Ala Ala Gly Ser Pro Phe Ser Gly
305                 310                 315                 320

Gly Pro Val Ser Phe Pro Leu Ala Pro Gly Pro His Phe Gly Thr Pro
                325                 330                 335

Gly Tyr Gly Ser Pro His Phe Thr Ala Leu Tyr Ser Ser Val Pro Phe
            340                 345                 350

Pro Glu Gly Glu Ala Phe Pro Pro Val Ser Val Thr Thr Leu Gly Ser
        355                 360                 365

Pro Met His Ser Asn
    370

<210>   57
<211>   1122
<212>   DNA
<213>   Artificial Sequence

<220>
<223>   NLS-Oct4-JO-86 MTD cDNA Sequence

<400>   57
atgaagaaga agaggaaggc gggacacctg gcttcggatt tcgccttctc gccccctcca        60

ggtggtggag gtgatgggcc aggggggccg gagccgggct gggttgatcc tcggacctgg       120

ctaagcttcc aaggccctcc tggagggcca ggaatcgggc cgggggttgg gccaggctct       180

gaggtgtggg ggattccccc atgccccccg ccgtatgagt tctgtggggg gatggcgtac       240

tgtgggcccc aggttggagt ggggctagtg ccccaaggcg gcttggagac ctctcagcct       300

gagggcgaag caggagtcgg ggtggagagc aactccgatg gggcctcccc ggagccctgc       360

accgtcaccc ctggtgccgt gaagctggag aaggagaagc tggagcaaaa cccggaggag 420

tcccaggaca tcaaagctct gcagaaagaa ctcgagcaat ttgccaagct cctgaagcag 480

aagaggatca ccctgggata tacacaggcc gatgtggggc tcaccctggg ggttctattt 540

gggaaggtat tcagccaaac gaccatctgc cgctttgagg ctctgcagct tagcttcaag 600

aacatgtgta agctgcggcc cttgctgcag aagtgggtgg aggaagctga caacaatgaa 660

aatcttcagg agatatgcaa agcagaaacc ctcgtgcagg cccgaaagag aaagcgaacc 720

agtatcgaga accgagtgag aggcaacctg gagaatttgt tcctgcagtg cccgaaaccc 780

acactgcagc agatcagcca catcgcccag cagcttgggc tcgagaagga tgtggtccga 840

gtgtggttct gtaaccggcg ccagaagggc aagcgatcaa gcagcgacta tgcacaacga 900

gaggattttg aggctgctgg gtctcctttc tcagggggac cagtgtcctt tcctctggcc 960

ccagggcccc attttggtac cccaggctat gggagccctc acttcactgc actgtactcc 1020

tcggtccctt tccctgaggg ggaagccttt ccccctgtct ccgtcaccac tctgggctct 1080

cccatgcatt caaacctggc ggtgctggcg gcggcgccgt ga 1122

<210> 58
<211> 373
<212> PRT
<213> Artificial Sequence

<220>
<223> NLS-Oct4-JO-86 MTD Amino Acid Sequence

<400> 58
Met Lys Lys Lys Arg Lys Ala Gly His Leu Ala Ser Asp Phe Ala Phe
1               5                   10                  15

Ser Pro Pro Pro Gly Gly Gly Gly Asp Gly Pro Gly Gly Pro Glu Pro
            20                  25                  30

Gly Trp Val Asp Pro Arg Thr Trp Leu Ser Phe Gln Gly Pro Pro Gly
        35                  40                  45

Gly Pro Gly Ile Gly Pro Gly Val Gly Pro Gly Ser Glu Val Trp Gly
    50                  55                  60

Ile Pro Pro Cys Pro Pro Pro Tyr Glu Phe Cys Gly Gly Met Ala Tyr
65                  70                  75                  80

Cys Gly Pro Gln Val Gly Val Gly Leu Val Pro Gln Gly Gly Leu Glu
                85                  90                  95

Thr Ser Gln Pro Glu Gly Glu Ala Gly Val Gly Val Glu Ser Asn Ser
            100                 105                 110

Asp Gly Ala Ser Pro Glu Pro Cys Thr Val Thr Pro Gly Ala Val Lys
        115                 120                 125

Leu Glu Lys Glu Lys Leu Glu Gln Asn Pro Glu Glu Ser Gln Asp Ile
    130                 135                 140

Lys Ala Leu Gln Lys Glu Leu Glu Gln Phe Ala Lys Leu Leu Lys Gln
145                 150                 155                 160

Lys Arg Ile Thr Leu Gly Tyr Thr Gln Ala Asp Val Gly Leu Thr Leu
                165                 170                 175

Gly Val Leu Phe Gly Lys Val Phe Ser Gln Thr Thr Ile Cys Arg Phe
            180                 185                 190

Glu Ala Leu Gln Leu Ser Phe Lys Asn Met Cys Lys Leu Arg Pro Leu
        195                 200                 205

Leu Gln Lys Trp Val Glu Glu Ala Asp Asn Asn Glu Asn Leu Gln Glu
    210                 215                 220

Ile Cys Lys Ala Glu Thr Leu Val Gln Ala Arg Lys Arg Lys Arg Thr

225                 230                 235                 240

Ser Ile Glu Asn Arg Val Arg Gly Asn Leu Glu Asn Leu Phe Leu Gln
                245                 250                 255

Cys Pro Lys Pro Thr Leu Gln Gln Ile Ser His Ile Ala Gln Gln Leu
            260                 265                 270

Gly Leu Glu Lys Asp Val Val Arg Val Trp Phe Cys Asn Arg Arg Gln
        275                 280                 285

Lys Gly Lys Arg Ser Ser Ser Asp Tyr Ala Gln Arg Glu Asp Phe Glu
    290                 295                 300

Ala Ala Gly Ser Pro Phe Ser Gly Gly Pro Val Ser Phe Pro Leu Ala
305                 310                 315                 320

Pro Gly Pro His Phe Gly Thr Pro Gly Tyr Gly Ser Pro His Phe Thr
                325                 330                 335

Ala Leu Tyr Ser Ser Val Pro Phe Pro Glu Gly Glu Ala Phe Pro Pro
            340                 345                 350

Val Ser Val Thr Thr Leu Gly Ser Pro Met His Ser Asn Leu Ala Val
        355                 360                 365

Leu Ala Ala Ala Pro
    370

<210>   59
<211>   1146
<212>   DNA
<213>   Artificial Sequence

<220>
<223>   NLS-JO-86 MTD-Oct4-JO-86 MTD cDNA Sequence

<400>   59

atgaagaaga agaggaagct ggcggtgctg gcggcggcgc cggcgggaca cctggcttcg 60

gatttcgcct tctcgccccc tccaggtggt ggaggtgatg ggccaggggg gccggagccg 120

ggctgggttg atcctcggac ctggctaagc ttccaaggcc ctcctggagg gccaggaatc 180

gggccggggg ttgggccagg ctctgaggtg tgggggattc cccatgcccc cccgccgtat 240

gagttctgtg gggggatggc gtactgtggg ccccaggttg gagtggggct agtgccccaa 300

ggcggcttgg agacctctca gcctgagggc gaagcaggag tcggggtgga gagcaactcc 360

gatggggcct ccccggagcc ctgcaccgtc acccctggtg ccgtgaagct ggagaaggag 420

aagctggagc aaaacccgga ggagtcccag gacatcaaag ctctgcagaa agaactcgag 480

caatttgcca agctcctgaa gcagaagagg atcaccctgg gatatacaca ggccgatgtg 540

gggctcaccc tgggggttct atttgggaag gtattcagcc aaacgaccat ctgccgcttt 600

gaggctctgc agcttagctt caagaacatg tgtaagctgc ggcccttgct gcagaagtgg 660

gtggaggaag ctgacaacaa tgaaaatctt caggagatat gcaaagcaga aaccctcgtg 720

caggcccgaa agagaaagcg aaccagtatc gagaaccgag tgagaggcaa cctggagaat 780

ttgttcctgc agtgcccgaa acccacactg cagcagatca gccacatcgc ccagcagctt 840

gggctcgaga aggatgtggt ccgagtgtgg ttctgtaacc ggcgccagaa gggcaagcga 900

tcaagcagcg actatgcaca acgagaggat tttgaggctg ctgggtctcc tttctcaggg 960

ggaccagtgt cctttcctct ggccccaggg ccccattttg gtaccccagg ctatgggagc 1020

cctcacttca ctgcactgta ctcctcggtc cctttccctg agggggaagc ctttccccct 1080

gtctccgtca ccactctggg ctctcccatg cattcaaacc tggcggtgct ggcggcggcg 1140

ccgtga 1146

<210>    60

<211>    381

<212>    PRT

<213>    Artificial Sequence

<220>

<223>    NLS-JO-86 MTD-Oct4-JO-86 MTD Amino Acid Sequence

<400>    60

Met Lys Lys Lys Arg Lys Leu Ala Val Leu Ala Ala Ala Pro Ala Gly
1               5                   10                  15

His Leu Ala Ser Asp Phe Ala Phe Ser Pro Pro Pro Gly Gly Gly Gly
            20                  25                  30

Asp Gly Pro Gly Gly Pro Glu Pro Gly Trp Val Asp Pro Arg Thr Trp
        35                  40                  45

Leu Ser Phe Gln Gly Pro Pro Gly Gly Pro Gly Ile Gly Pro Gly Val
    50                  55                  60

Gly Pro Gly Ser Glu Val Trp Gly Ile Pro Pro Cys Pro Pro Pro Tyr
65                  70                  75                  80

Glu Phe Cys Gly Gly Met Ala Tyr Cys Gly Pro Gln Val Gly Val Gly
                85                  90                  95

Leu Val Pro Gln Gly Gly Leu Glu Thr Ser Gln Pro Glu Gly Glu Ala
            100                 105                 110

Gly Val Gly Val Glu Ser Asn Ser Asp Gly Ala Ser Pro Glu Pro Cys
        115                 120                 125

Thr Val Thr Pro Gly Ala Val Lys Leu Glu Lys Glu Lys Leu Glu Gln
    130                 135                 140

Asn Pro Glu Glu Ser Gln Asp Ile Lys Ala Leu Gln Lys Glu Leu Glu
145                 150                 155                 160

Gln Phe Ala Lys Leu Leu Lys Gln Lys Arg Ile Thr Leu Gly Tyr Thr
                165                 170                 175

Gln Ala Asp Val Gly Leu Thr Leu Gly Val Leu Phe Gly Lys Val Phe
            180                 185                 190

Ser Gln Thr Thr Ile Cys Arg Phe Glu Ala Leu Gln Leu Ser Phe Lys
        195                 200                 205

Asn Met Cys Lys Leu Arg Pro Leu Leu Gln Lys Trp Val Glu Glu Ala
    210                 215                 220

Asp Asn Asn Glu Asn Leu Gln Glu Ile Cys Lys Ala Glu Thr Leu Val
225                 230                 235                 240

Gln Ala Arg Lys Arg Lys Arg Thr Ser Ile Glu Asn Arg Val Arg Gly
                245                 250                 255

Asn Leu Glu Asn Leu Phe Leu Gln Cys Pro Lys Pro Thr Leu Gln Gln
            260                 265                 270

Ile Ser His Ile Ala Gln Gln Leu Gly Leu Glu Lys Asp Val Val Arg
        275                 280                 285

Val Trp Phe Cys Asn Arg Arg Gln Lys Gly Lys Arg Ser Ser Ser Asp
    290                 295                 300

Tyr Ala Gln Arg Glu Asp Phe Glu Ala Ala Gly Ser Pro Phe Ser Gly
305                 310                 315                 320

Gly Pro Val Ser Phe Pro Leu Ala Pro Gly Pro His Phe Gly Thr Pro
                325                 330                 335

Gly Tyr Gly Ser Pro His Phe Thr Ala Leu Tyr Ser Ser Val Pro Phe
            340                 345                 350

Pro Glu Gly Glu Ala Phe Pro Pro Val Ser Val Thr Thr Leu Gly Ser
        355                 360                 365

Pro Met His Ser Asn Leu Ala Val Leu Ala Ala Ala Pro
    370                 375                 380

<210> 61
<211> 1158
<212> DNA
<213> Artificial Sequence

<220>
<223> His-NLS-Oct4 cDNA Sequence

<400> 61
atgggcagca gccatcatca tcatcatcac agcagcggcc tggtgccgcg cggcagccat 60

atgaagaaga agaggaaggc gggacacctg gcttcggatt tcgccttctc gccccctcca 120

ggtggtggag gtgatgggcc aggggggccg gagccgggct gggttgatcc tcggacctgg 180

ctaagcttcc aaggccctcc tggagggcca ggaatcgggc cgggggttgg gccaggctct 240

gaggtgtggg ggattccccc atgccccccg ccgtatgagt tctgtggggg gatggcgtac 300

tgtgggcccc aggttggagt ggggctagtg ccccaaggcg gcttggagac ctctcagcct 360

gagggcgaag caggagtcgg ggtggagagc aactccgatg gggcctcccc ggagccctgc 420

accgtcaccc ctggtgccgt gaagctggag aaggagaagc tggagcaaaa cccggaggag 480

tcccaggaca tcaaagctct gcagaaagaa ctcgagcaat ttgccaagct cctgaagcag 540

aagaggatca ccctgggata tacacaggcc gatgtggggc tcaccctggg ggttctattt 600

gggaaggtat tcagccaaac gaccatctgc cgctttgagg ctctgcagct tagcttcaag 660

aacatgtgta agctgcggcc cttgctgcag aagtgggtgg aggaagctga caacaatgaa 720

aatcttcagg agatatgcaa agcagaaacc ctcgtgcagg cccgaaagag aaagcgaacc 780

agtatcgaga accgagtgag aggcaacctg gagaatttgt tcctgcagtg cccgaaaccc 840

acactgcagc agatcagcca catcgcccag cagcttgggc tcgagaagga tgtggtccga 900

gtgtggttct gtaaccggcg ccagaagggc aagcgatcaa gcagcgacta tgcacaacga 960

gaggattttg aggctgctgg gtctcctttc tcagggggac cagtgtcctt tcctctggcc 1020

ccagggcccc attttggtac cccaggctat gggagccctc acttcactgc actgtactcc 1080

tcggtccctt tccctgaggg ggaagccttt ccccctgtct ccgtcaccac tctgggctct 1140

cccatgcatt caaactga 1158

<210> 62
<211> 385
<212> PRT
<213> Artificial Sequence

<220>
<223> His-NLS-Oct4 Amino Acid Sequence

<400> 62
Met Gly Ser Ser His His His His His His Ser Ser Gly Leu Val Pro
1               5                   10                  15

Arg Gly Ser His Met Lys Lys Lys Arg Lys Ala Gly His Leu Ala Ser
            20                  25                  30

Asp Phe Ala Phe Ser Pro Pro Pro Gly Gly Gly Gly Asp Gly Pro Gly
        35                  40                  45

Gly Pro Glu Pro Gly Trp Val Asp Pro Arg Thr Trp Leu Ser Phe Gln
    50                  55                  60

Gly Pro Pro Gly Gly Pro Gly Ile Gly Pro Gly Val Gly Pro Gly Ser

65                  70                  75                  80

Glu Val Trp Gly Ile Pro Pro Cys Pro Pro Pro Tyr Glu Phe Cys Gly
                85                  90                  95

Gly Met Ala Tyr Cys Gly Pro Gln Val Gly Val Gly Leu Val Pro Gln
            100                 105                 110

Gly Gly Leu Glu Thr Ser Gln Pro Glu Gly Glu Ala Gly Val Gly Val
        115                 120                 125

Glu Ser Asn Ser Asp Gly Ala Ser Pro Glu Pro Cys Thr Val Thr Pro
    130                 135                 140

Gly Ala Val Lys Leu Glu Lys Glu Lys Leu Glu Gln Asn Pro Glu Glu
145                 150                 155                 160

Ser Gln Asp Ile Lys Ala Leu Gln Lys Glu Leu Glu Gln Phe Ala Lys
                165                 170                 175

Leu Leu Lys Gln Lys Arg Ile Thr Leu Gly Tyr Thr Gln Ala Asp Val
            180                 185                 190

Gly Leu Thr Leu Gly Val Leu Phe Gly Lys Val Phe Ser Gln Thr Thr
        195                 200                 205

Ile Cys Arg Phe Glu Ala Leu Gln Leu Ser Phe Lys Asn Met Cys Lys
    210                 215                 220

Leu Arg Pro Leu Leu Gln Lys Trp Val Glu Glu Ala Asp Asn Asn Glu
225                 230                 235                 240

Asn Leu Gln Glu Ile Cys Lys Ala Glu Thr Leu Val Gln Ala Arg Lys
                245                 250                 255

Arg Lys Arg Thr Ser Ile Glu Asn Arg Val Arg Gly Asn Leu Glu Asn
            260                 265                 270

Leu Phe Leu Gln Cys Pro Lys Pro Thr Leu Gln Gln Ile Ser His Ile
        275                 280                 285

Ala Gln Gln Leu Gly Leu Glu Lys Asp Val Val Arg Val Trp Phe Cys
    290                 295                 300

Asn Arg Arg Gln Lys Gly Lys Arg Ser Ser Ser Asp Tyr Ala Gln Arg
305                 310                 315                 320

Glu Asp Phe Glu Ala Ala Gly Ser Pro Phe Ser Gly Gly Pro Val Ser
                325                 330                 335

Phe Pro Leu Ala Pro Gly Pro His Phe Gly Thr Pro Gly Tyr Gly Ser
            340                 345                 350

Pro His Phe Thr Ala Leu Tyr Ser Ser Val Pro Phe Pro Glu Gly Glu
        355                 360                 365

Ala Phe Pro Pro Val Ser Val Thr Thr Leu Gly Ser Pro Met His Ser
    370                 375                 380

Asn
385

<210>   63
<211>   1185
<212>   DNA
<213>   Artificial Sequence

<220>
<223>   His-NLS-JO-84 MTD-Oct4 cDNA Sequence

<400>   63
atgggcagca gccatcatca tcatcatcac agcagcggcc tggtgccgcg cggcagccat      60

atgaagaaga agaggaagct ggtggcggcg ctgctggcgg tgctggcggg acacctggct     120

tcggatttcg ccttctcgcc ccctccaggt ggtggaggtg atgggccagg ggggccggag     180

ccgggctggg ttgatcctcg gacctggcta agcttccaag gccctcctgg agggccagga     240

atcgggccgg gggttgggcc aggctctgag gtgtggggga ttcccccatg ccccccgccg 300

tatgagttct gtggggggat ggcgtactgt gggccccagg ttggagtggg gctagtgccc 360

caaggcggct tggagacctc tcagcctgag ggcgaagcag gagtcggggt ggagagcaac 420

tccgatgggg cctccccgga gccctgcacc gtcacccctg gtgccgtgaa gctggagaag 480

gagaagctgg agcaaaaccc ggaggagtcc caggacatca aagctctgca gaaagaactc 540

gagcaatttg ccaagctcct gaagcagaag aggatcaccc tgggatatac acaggccgat 600

gtggggctca ccctgggggt tctatttggg aaggtattca gccaaacgac catctgccgc 660

tttgaggctc tgcagcttag cttcaagaac atgtgtaagc tgcggccctt gctgcagaag 720

tgggtggagg aagctgacaa caatgaaaat cttcaggaga tatgcaaagc agaaaccctc 780

gtgcaggccc gaaagagaaa gcgaaccagt atcgagaacc gagtgagagg caacctggag 840

aatttgttcc tgcagtgccc gaaacccaca ctgcagcaga tcagccacat cgcccagcag 900

cttgggctcg agaaggatgt ggtccgagtg tggttctgta accggcgcca gaagggcaag 960

cgatcaagca gcgactatgc acaacgagag gattttgagg ctgctgggtc tcctttctca 1020

gggggaccag tgtcctttcc tctggcccca gggccccatt ttggtacccc aggctatggg 1080

agccctcact tcactgcact gtactcctcg gtccctttcc ctgaggggga agcctttccc 1140

cctgtctccg tcaccactct gggctctccc atgcattcaa actga 1185

<210> 64

<211> 394

<212> PRT

<213> Artificial Sequence

<220>

<223>    His-NLS-JO-84 MTD-Oct4 Amino Acid Sequence

<400>    64
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Ala Gly His Leu Ala Ser Asp Phe Ala Phe Ser Pro Pro
        35                  40                  45

Pro Gly Gly Gly Gly Asp Gly Pro Gly Gly Pro Glu Pro Gly Trp Val
    50                  55                  60

Asp Pro Arg Thr Trp Leu Ser Phe Gln Gly Pro Pro Gly Gly Pro Gly
65                  70                  75                  80

Ile Gly Pro Gly Val Gly Pro Gly Ser Glu Val Trp Gly Ile Pro Pro
                85                  90                  95

Cys Pro Pro Pro Tyr Glu Phe Cys Gly Gly Met Ala Tyr Cys Gly Pro
            100                 105                 110

Gln Val Gly Val Gly Leu Val Pro Gln Gly Gly Leu Glu Thr Ser Gln
        115                 120                 125

Pro Glu Gly Glu Ala Gly Val Gly Val Glu Ser Asn Ser Asp Gly Ala
    130                 135                 140

Ser Pro Glu Pro Cys Thr Val Thr Pro Gly Ala Val Lys Leu Glu Lys
145                 150                 155                 160

Glu Lys Leu Glu Gln Asn Pro Glu Glu Ser Gln Asp Ile Lys Ala Leu
                165                 170                 175

Gln Lys Glu Leu Glu Gln Phe Ala Lys Leu Leu Lys Gln Lys Arg Ile
            180                 185                 190

Thr Leu Gly Tyr Thr Gln Ala Asp Val Gly Leu Thr Leu Gly Val Leu
        195                 200                 205

Phe Gly Lys Val Phe Ser Gln Thr Thr Ile Cys Arg Phe Glu Ala Leu
    210                 215                 220

Gln Leu Ser Phe Lys Asn Met Cys Lys Leu Arg Pro Leu Leu Gln Lys
225                 230                 235                 240

Trp Val Glu Glu Ala Asp Asn Asn Glu Asn Leu Gln Glu Ile Cys Lys
                245                 250                 255

Ala Glu Thr Leu Val Gln Ala Arg Lys Arg Lys Arg Thr Ser Ile Glu
            260                 265                 270

Asn Arg Val Arg Gly Asn Leu Glu Asn Leu Phe Leu Gln Cys Pro Lys
        275                 280                 285

Pro Thr Leu Gln Gln Ile Ser His Ile Ala Gln Gln Leu Gly Leu Glu
    290                 295                 300

Lys Asp Val Val Arg Val Trp Phe Cys Asn Arg Arg Gln Lys Gly Lys
305                 310                 315                 320

Arg Ser Ser Ser Asp Tyr Ala Gln Arg Glu Asp Phe Glu Ala Ala Gly
                325                 330                 335

Ser Pro Phe Ser Gly Gly Pro Val Ser Phe Pro Leu Ala Pro Gly Pro
            340                 345                 350

His Phe Gly Thr Pro Gly Tyr Gly Ser Pro His Phe Thr Ala Leu Tyr
        355                 360                 365

Ser Ser Val Pro Phe Pro Glu Gly Glu Ala Phe Pro Pro Val Ser Val
    370                 375                 380

Thr Thr Leu Gly Ser Pro Met His Ser Asn
385                 390

<210> 65
<211> 1185
<212> DNA
<213> Artificial Sequence

<220>
<223> His-NLS-Oct4-JO-84 MTD cDNA Sequence

<400> 65
atgggcagca gccatcatca tcatcatcac agcagcggcc tggtgccgcg cggcagccat 60

atgaagaaga agaggaaggc gggacacctg gcttcggatt tcgccttctc gccccctcca 120

ggtggtggag gtgatgggcc aggggggccg gagccgggct gggttgatcc tcggacctgg 180

ctaagcttcc aaggccctcc tggagggcca ggaatcgggc cgggggttgg gccaggctct 240

gaggtgtggg ggattccccc atgccccccg ccgtatgagt tctgtggggg gatggcgtac 300

tgtgggcccc aggttggagt ggggctagtg ccccaaggcg gcttggagac ctctcagcct 360

gagggcgaag caggagtcgg ggtggagagc aactccgatg gggcctcccc ggagccctgc 420

accgtcaccc ctggtgccgt gaagctggag aaggagaagc tggagcaaaa cccggaggag 480

tcccaggaca tcaaagctct gcagaaagaa ctcgagcaat ttgccaagct cctgaagcag 540

aagaggatca ccctgggata tacacaggcc gatgtggggc tcaccctggg ggttctattt 600

gggaaggtat tcagccaaac gaccatctgc cgctttgagg ctctgcagct tagcttcaag 660

aacatgtgta agctgcggcc cttgctgcag aagtgggtgg aggaagctga caacaatgaa 720

aatcttcagg agatatgcaa agcagaaacc ctcgtgcagg cccgaaagag aaagcgaacc 780

agtatcgaga accgagtgag aggcaacctg gagaatttgt tcctgcagtg cccgaaaccc 840

acactgcagc agatcagcca catcgcccag cagcttgggc tcgagaagga tgtggtccga 900

gtgtggttct gtaaccggcg ccagaagggc aagcgatcaa gcagcgacta tgcacaacga 960

gaggattttg aggctgctgg gtctcctttc tcagggggac cagtgtcctt tcctctggcc 1020

ccagggcccc attttggtac cccaggctat gggagccctc acttcactgc actgtactcc 1080

tcggtccctt tccctgaggg ggaagccttt ccccctgtct ccgtcaccac tctgggctct 1140

cccatgcatt caaacctggt ggcggcgctg ctggcggtgc tgtga 1185

<210> 66
<211> 394
<212> PRT
<213> Artificial Sequence

<220>
<223> His-NLS-Oct4-JO-84 MTD Amino Acid Sequence

<400> 66
Met Gly Ser Ser His His His His His His Ser Ser Gly Leu Val Pro
1               5                   10                  15

Arg Gly Ser His Met Lys Lys Lys Arg Lys Ala Gly His Leu Ala Ser
            20                  25                  30

Asp Phe Ala Phe Ser Pro Pro Pro Gly Gly Gly Gly Asp Gly Pro Gly
        35                  40                  45

Gly Pro Glu Pro Gly Trp Val Asp Pro Arg Thr Trp Leu Ser Phe Gln
    50                  55                  60

Gly Pro Pro Gly Gly Pro Gly Ile Gly Pro Gly Val Gly Pro Gly Ser
65                  70                  75                  80

Glu Val Trp Gly Ile Pro Pro Cys Pro Pro Pro Tyr Glu Phe Cys Gly
                85                  90                  95

Gly Met Ala Tyr Cys Gly Pro Gln Val Gly Val Gly Leu Val Pro Gln

            100                 105                 110

Gly Gly Leu Glu Thr Ser Gln Pro Glu Gly Glu Ala Gly Val Gly Val
        115                 120                 125

Glu Ser Asn Ser Asp Gly Ala Ser Pro Glu Pro Cys Thr Val Thr Pro
    130                 135                 140

Gly Ala Val Lys Leu Glu Lys Glu Lys Leu Glu Gln Asn Pro Glu Glu
145                 150                 155                 160

Ser Gln Asp Ile Lys Ala Leu Gln Lys Glu Leu Glu Gln Phe Ala Lys
                165                 170                 175

Leu Leu Lys Gln Lys Arg Ile Thr Leu Gly Tyr Thr Gln Ala Asp Val
            180                 185                 190

Gly Leu Thr Leu Gly Val Leu Phe Gly Lys Val Phe Ser Gln Thr Thr
        195                 200                 205

Ile Cys Arg Phe Glu Ala Leu Gln Leu Ser Phe Lys Asn Met Cys Lys
    210                 215                 220

Leu Arg Pro Leu Leu Gln Lys Trp Val Glu Glu Ala Asp Asn Asn Glu
225                 230                 235                 240

Asn Leu Gln Glu Ile Cys Lys Ala Glu Thr Leu Val Gln Ala Arg Lys
                245                 250                 255

Arg Lys Arg Thr Ser Ile Glu Asn Arg Val Arg Gly Asn Leu Glu Asn
            260                 265                 270

Leu Phe Leu Gln Cys Pro Lys Pro Thr Leu Gln Gln Ile Ser His Ile
        275                 280                 285

Ala Gln Gln Leu Gly Leu Glu Lys Asp Val Val Arg Val Trp Phe Cys
    290                 295                 300

Asn Arg Arg Gln Lys Gly Lys Arg Ser Ser Ser Asp Tyr Ala Gln Arg
305                 310                 315                 320

Glu Asp Phe Glu Ala Ala Gly Ser Pro Phe Ser Gly Gly Pro Val Ser
                325                 330                 335

Phe Pro Leu Ala Pro Gly Pro His Phe Gly Thr Pro Gly Tyr Gly Ser
            340                 345                 350

Pro His Phe Thr Ala Leu Tyr Ser Ser Val Pro Phe Pro Glu Gly Glu
        355                 360                 365

Ala Phe Pro Pro Val Ser Val Thr Thr Leu Gly Ser Pro Met His Ser
    370                 375                 380

Asn Leu Val Ala Ala Leu Leu Ala Val Leu
385                 390

<210>    67
<211>    1212
<212>    DNA
<213>    Artificial Sequence

<220>
<223>    His-NLS-JO-84 MTD-Oct4-JO-84 MTD cDNA Sequence

<400>    67
atgggcagca gccatcatca tcatcatcac agcagcggcc tggtgccgcg cggcagccat      60

atgaagaaga agaggaagct ggtggcggcg ctgctggcgg tgctggcggg acacctggct     120

tcggatttcg ccttctcgcc ccctccaggt ggtggaggtg atgggccagg ggggccggag     180

ccgggctggg ttgatcctcg gacctggcta agcttccaag gccctcctgg agggccagga     240

atcgggccgg gggttgggcc aggctctgag gtgtggggga ttcccccatg ccccccgccg     300

tatgagttct gtggggggat ggcgtactgt gggccccagg ttggagtggg gctagtgccc     360

caaggcggct tggagacctc tcagcctgag ggcgaagcag gagtcggggt ggagagcaac     420

tccgatgggg cctccccgga gccctgcacc gtcacccctg gtgccgtgaa gctggagaag 480

gagaagctgg agcaaaaccc ggaggagtcc caggacatca aagctctgca gaaagaactc 540

gagcaatttg ccaagctcct gaagcagaag aggatcaccc tgggatatac acaggccgat 600

gtggggctca ccctgggggt tctatttggg aaggtattca gccaaacgac catctgccgc 660

tttgaggctc tgcagcttag cttcaagaac atgtgtaagc tgcggccctt gctgcagaag 720

tgggtggagg aagctgacaa caatgaaaat cttcaggaga tatgcaaagc agaaaccctc 780

gtgcaggccc gaaagagaaa gcgaaccagt atcgagaacc gagtgagagg caacctggag 840

aatttgttcc tgcagtgccc gaaacccaca ctgcagcaga tcagccacat cgcccagcag 900

cttgggctcg agaaggatgt ggtccgagtg tggttctgta accggcgcca gaagggcaag 960

cgatcaagca gcgactatgc acaacgagag gattttgagg ctgctgggtc tcctttctca 1020

gggggaccag tgtcctttcc tctggcccca gggccccatt ttggtacccc aggctatggg 1080

agccctcact tcactgcact gtactcctcg gtccctttcc ctgaggggga agcctttccc 1140

cctgtctccg tcaccactct gggctctccc atgcattcaa acctggtggc ggcgctgctg 1200

gcggtgctgt ga 1212

<210> 68
<211> 403
<212> PRT
<213> Artificial Sequence

<220>
<223> His-NLS-JO-84 MTD-Oct4-JO-84 MTD Amino Acid Sequence

<400> 68

Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Ala Gly His Leu Ala Ser Asp Phe Ala Phe Ser Pro Pro
        35                  40                  45

Pro Gly Gly Gly Gly Asp Gly Pro Gly Gly Pro Glu Pro Gly Trp Val
    50                  55                  60

Asp Pro Arg Thr Trp Leu Ser Phe Gln Gly Pro Pro Gly Gly Pro Gly
65                  70                  75                  80

Ile Gly Pro Gly Val Gly Pro Gly Ser Glu Val Trp Gly Ile Pro Pro
                85                  90                  95

Cys Pro Pro Pro Tyr Glu Phe Cys Gly Gly Met Ala Tyr Cys Gly Pro
            100                 105                 110

Gln Val Gly Val Gly Leu Val Pro Gln Gly Gly Leu Glu Thr Ser Gln
        115                 120                 125

Pro Glu Gly Glu Ala Gly Val Gly Val Glu Ser Asn Ser Asp Gly Ala
    130                 135                 140

Ser Pro Glu Pro Cys Thr Val Thr Pro Gly Ala Val Lys Leu Glu Lys
145                 150                 155                 160

Glu Lys Leu Glu Gln Asn Pro Glu Glu Ser Gln Asp Ile Lys Ala Leu
                165                 170                 175

Gln Lys Glu Leu Glu Gln Phe Ala Lys Leu Leu Lys Gln Lys Arg Ile
            180                 185                 190

Thr Leu Gly Tyr Thr Gln Ala Asp Val Gly Leu Thr Leu Gly Val Leu
        195                 200                 205

Phe Gly Lys Val Phe Ser Gln Thr Thr Ile Cys Arg Phe Glu Ala Leu

210                 215                 220

Gln Leu Ser Phe Lys Asn Met Cys Lys Leu Arg Pro Leu Leu Gln Lys
225                 230                 235                 240

Trp Val Glu Glu Ala Asp Asn Asn Glu Asn Leu Gln Glu Ile Cys Lys
                245                 250                 255

Ala Glu Thr Leu Val Gln Ala Arg Lys Arg Lys Arg Thr Ser Ile Glu
            260                 265                 270

Asn Arg Val Arg Gly Asn Leu Glu Asn Leu Phe Leu Gln Cys Pro Lys
        275                 280                 285

Pro Thr Leu Gln Gln Ile Ser His Ile Ala Gln Gln Leu Gly Leu Glu
    290                 295                 300

Lys Asp Val Val Arg Val Trp Phe Cys Asn Arg Arg Gln Lys Gly Lys
305                 310                 315                 320

Arg Ser Ser Ser Asp Tyr Ala Gln Arg Glu Asp Phe Glu Ala Ala Gly
                325                 330                 335

Ser Pro Phe Ser Gly Gly Pro Val Ser Phe Pro Leu Ala Pro Gly Pro
            340                 345                 350

His Phe Gly Thr Pro Gly Tyr Gly Ser Pro His Phe Thr Ala Leu Tyr
        355                 360                 365

Ser Ser Val Pro Phe Pro Glu Gly Glu Ala Phe Pro Pro Val Ser Val
    370                 375                 380

Thr Thr Leu Gly Ser Pro Met His Ser Asn Leu Val Ala Ala Leu Leu
385                 390                 395                 400

Ala Val Leu

<210>   69

<211>    1182
<212>    DNA
<213>    Artificial Sequence

<220>
<223>    His-NLS-JO-86 MTD-Oct4 cDNA Sequence

<400>    69
atgggcagca gccatcatca tcatcatcac agcagcggcc tggtgccgcg cggcagccat    60

atgaagaaga agaggaagct ggcggtgctg gcggcggcgc cggcgggaca cctggcttcg    120

gatttcgcct tctcgccccc tccaggtggt ggaggtgatg ggccaggggg gccggagccg    180

ggctgggttg atcctcggac ctggctaagc ttccaaggcc ctcctggagg gccaggaatc    240

gggccggggg ttgggccagg ctctgaggtg tgggggattc ccccatgccc cccgccgtat    300

gagttctgtg gggggatggc gtactgtggg ccccaggttg gagtggggct agtgccccaa    360

ggcggcttgg agacctctca gcctgagggc gaagcaggag tcggggtgga gagcaactcc    420

gatggggcct ccccggagcc ctgcaccgtc acccctggtg ccgtgaagct ggagaaggag    480

aagctggagc aaaacccgga ggagtcccag gacatcaaag ctctgcagaa agaactcgag    540

caatttgcca agctcctgaa gcagaagagg atcaccctgg gatatacaca ggccgatgtg    600

gggctcaccc tgggggttct atttgggaag gtattcagcc aaacgaccat ctgccgcttt    660

gaggctctgc agcttagctt caagaacatg tgtaagctgc ggcccttgct gcagaagtgg    720

gtggaggaag ctgacaacaa tgaaaatctt caggagatat gcaaagcaga aaccctcgtg    780

caggcccgaa agagaaagcg aaccagtatc gagaaccgag tgagaggcaa cctggagaat    840

ttgttcctgc agtgcccgaa acccacactg cagcagatca gccacatcgc ccagcagctt    900

gggctcgaga aggatgtggt ccgagtgtgg ttctgtaacc ggcgccagaa gggcaagcga    960

tcaagcagcg actatgcaca acgagaggat tttgaggctg ctgggtctcc tttctcaggg 1020

ggaccagtgt cctttcctct ggccccaggg ccccattttg gtaccccagg ctatgggagc 1080

cctcacttca ctgcactgta ctcctcggtc cctttccctg agggggaagc ctttccccct 1140

gtctccgtca ccactctggg ctctcccatg cattcaaact ga 1182

<210> 70
<211> 393
<212> PRT
<213> Artificial Sequence

<220>
<223> His-NLS-JO-86 MTD-Oct4 Amico Acid Sequence

<400> 70
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Ala Gly His Leu Ala Ser Asp Phe Ala Phe Ser Pro Pro Pro
        35                  40                  45

Gly Gly Gly Gly Asp Gly Pro Gly Gly Pro Glu Pro Gly Trp Val Asp
    50                  55                  60

Pro Arg Thr Trp Leu Ser Phe Gln Gly Pro Pro Gly Gly Pro Gly Ile
65                  70                  75                  80

Gly Pro Gly Val Gly Pro Gly Ser Glu Val Trp Gly Ile Pro Pro Cys
                85                  90                  95

Pro Pro Pro Tyr Glu Phe Cys Gly Gly Met Ala Tyr Cys Gly Pro Gln
            100                 105                 110

Val Gly Val Gly Leu Val Pro Gln Gly Gly Leu Glu Thr Ser Gln Pro
        115                 120                 125

Glu Gly Glu Ala Gly Val Gly Val Glu Ser Asn Ser Asp Gly Ala Ser
    130                 135                 140

Pro Glu Pro Cys Thr Val Thr Pro Gly Ala Val Lys Leu Glu Lys Glu
145                 150                 155                 160

Lys Leu Glu Gln Asn Pro Glu Glu Ser Gln Asp Ile Lys Ala Leu Gln
                165                 170                 175

Lys Glu Leu Glu Gln Phe Ala Lys Leu Leu Lys Gln Lys Arg Ile Thr
            180                 185                 190

Leu Gly Tyr Thr Gln Ala Asp Val Gly Leu Thr Leu Gly Val Leu Phe
        195                 200                 205

Gly Lys Val Phe Ser Gln Thr Thr Ile Cys Arg Phe Glu Ala Leu Gln
    210                 215                 220

Leu Ser Phe Lys Asn Met Cys Lys Leu Arg Pro Leu Leu Gln Lys Trp
225                 230                 235                 240

Val Glu Glu Ala Asp Asn Asn Glu Asn Leu Gln Glu Ile Cys Lys Ala
                245                 250                 255

Glu Thr Leu Val Gln Ala Arg Lys Arg Lys Arg Thr Ser Ile Glu Asn
            260                 265                 270

Arg Val Arg Gly Asn Leu Glu Asn Leu Phe Leu Gln Cys Pro Lys Pro
        275                 280                 285

Thr Leu Gln Gln Ile Ser His Ile Ala Gln Gln Leu Gly Leu Glu Lys
    290                 295                 300

Asp Val Val Arg Val Trp Phe Cys Asn Arg Arg Gln Lys Gly Lys Arg
305                 310                 315                 320

Ser Ser Ser Asp Tyr Ala Gln Arg Glu Asp Phe Glu Ala Ala Gly Ser
                325                 330                 335

Pro Phe Ser Gly Gly Pro Val Ser Phe Pro Leu Ala Pro Gly Pro His
            340                 345                 350

Phe Gly Thr Pro Gly Tyr Gly Ser Pro His Phe Thr Ala Leu Tyr Ser
        355                 360                 365

Ser Val Pro Phe Pro Glu Gly Glu Ala Phe Pro Pro Val Ser Val Thr
    370                 375                 380

Thr Leu Gly Ser Pro Met His Ser Asn
385                 390

<210>    71
<211>    1182
<212>    DNA
<213>    Artificial Sequence

<220>
<223>    His-NLS-Oct4-JO-86 MTD cDNA Sequence

<400>    71
atgggcagca gccatcatca tcatcatcac agcagcggcc tggtgccgcg cggcagccat        60

atgaagaaga agaggaaggc gggacacctg gcttcggatt tcgccttctc gccccctcca       120

ggtggtggag gtgatgggcc aggggggccg gagccgggct gggttgatcc tcggacctgg       180

ctaagcttcc aaggccctcc tggagggcca ggaatcgggc cgggggttgg gccaggctct       240

gaggtgtggg ggattccccc atgccccccg ccgtatgagt tctgtggggg gatggcgtac       300

tgtgggcccc aggttggagt ggggctagtg ccccaaggcg gcttggagac ctctcagcct       360

gagggcgaag caggagtcgg ggtggagagc aactccgatg gggcctcccc ggagccctgc       420

accgtcaccc ctggtgccgt gaagctggag aaggagaagc tggagcaaaa cccggaggag 480

tcccaggaca tcaaagctct gcagaaagaa ctcgagcaat ttgccaagct cctgaagcag 540

aagaggatca ccctgggata tacacaggcc gatgtggggc tcaccctggg ggttctattt 600

gggaaggtat tcagccaaac gaccatctgc cgctttgagg ctctgcagct tagcttcaag 660

aacatgtgta agctgcggcc cttgctgcag aagtgggtgg aggaagctga caacaatgaa 720

aatcttcagg agatatgcaa agcagaaacc ctcgtgcagg cccgaaagag aaagcgaacc 780

agtatcgaga accgagtgag aggcaacctg gagaatttgt tcctgcagtg cccgaaaccc 840

acactgcagc agatcagcca catcgcccag cagcttgggc tcgagaagga tgtggtccga 900

gtgtggttct gtaaccggcg ccagaagggc aagcgatcaa gcagcgacta tgcacaacga 960

gaggattttg aggctgctgg gtctcctttc tcagggggac cagtgtcctt tcctctggcc 1020

ccagggcccc attttggtac cccaggctat gggagccctc acttcactgc actgtactcc 1080

tcggtccctt tccctgaggg ggaagccttt ccccctgtct ccgtcaccac tctgggctct 1140

cccatgcatt caaacctggc ggtgctggcg gcggcgccgt ga 1182

<210> 72
<211> 393
<212> PRT
<213> Artificial Sequence

<220>
<223> His-NLS-Oct4-JO-86 MTD Amino Acid Sequence

<400> 72
Met Gly Ser Ser His His His His His His Ser Ser Gly Leu Val Pro
1               5                   10                  15

Arg Gly Ser His Met Lys Lys Lys Arg Lys Ala Gly His Leu Ala Ser
            20                  25                  30

Asp Phe Ala Phe Ser Pro Pro Pro Gly Gly Gly Gly Asp Gly Pro Gly
        35                  40                  45

Gly Pro Glu Pro Gly Trp Val Asp Pro Arg Thr Trp Leu Ser Phe Gln
    50                  55                  60

Gly Pro Pro Gly Gly Pro Gly Ile Gly Pro Gly Val Gly Pro Gly Ser
65                  70                  75                  80

Glu Val Trp Gly Ile Pro Pro Cys Pro Pro Pro Tyr Glu Phe Cys Gly
                85                  90                  95

Gly Met Ala Tyr Cys Gly Pro Gln Val Gly Val Gly Leu Val Pro Gln
            100                 105                 110

Gly Gly Leu Glu Thr Ser Gln Pro Glu Gly Glu Ala Gly Val Gly Val
        115                 120                 125

Glu Ser Asn Ser Asp Gly Ala Ser Pro Glu Pro Cys Thr Val Thr Pro
    130                 135                 140

Gly Ala Val Lys Leu Glu Lys Glu Lys Leu Glu Gln Asn Pro Glu Glu
145                 150                 155                 160

Ser Gln Asp Ile Lys Ala Leu Gln Lys Glu Leu Glu Gln Phe Ala Lys
                165                 170                 175

Leu Leu Lys Gln Lys Arg Ile Thr Leu Gly Tyr Thr Gln Ala Asp Val
            180                 185                 190

Gly Leu Thr Leu Gly Val Leu Phe Gly Lys Val Phe Ser Gln Thr Thr
        195                 200                 205

Ile Cys Arg Phe Glu Ala Leu Gln Leu Ser Phe Lys Asn Met Cys Lys
    210                 215                 220

Leu Arg Pro Leu Leu Gln Lys Trp Val Glu Glu Ala Asp Asn Asn Glu

225                 230                 235                 240

Asn Leu Gln Glu Ile Cys Lys Ala Glu Thr Leu Val Gln Ala Arg Lys
                245                 250                 255

Arg Lys Arg Thr Ser Ile Glu Asn Arg Val Arg Gly Asn Leu Glu Asn
            260                 265                 270

Leu Phe Leu Gln Cys Pro Lys Pro Thr Leu Gln Gln Ile Ser His Ile
        275                 280                 285

Ala Gln Gln Leu Gly Leu Glu Lys Asp Val Val Arg Val Trp Phe Cys
    290                 295                 300

Asn Arg Arg Gln Lys Gly Lys Arg Ser Ser Ser Asp Tyr Ala Gln Arg
305                 310                 315                 320

Glu Asp Phe Glu Ala Ala Gly Ser Pro Phe Ser Gly Gly Pro Val Ser
                325                 330                 335

Phe Pro Leu Ala Pro Gly Pro His Phe Gly Thr Pro Gly Tyr Gly Ser
            340                 345                 350

Pro His Phe Thr Ala Leu Tyr Ser Ser Val Pro Phe Pro Glu Gly Glu
        355                 360                 365

Ala Phe Pro Pro Val Ser Val Thr Thr Leu Gly Ser Pro Met His Ser
    370                 375                 380

Asn Leu Ala Val Leu Ala Ala Ala Pro
385                 390

<210>   73
<211>   1206
<212>   DNA
<213>   Artificial Sequence

<220>
<223>   His-NLS-JO-86 MTD-Oct4-JO-86 MTD cDNA Sequence

<400> 73

atgggcagca gccatcatca tcatcatcac agcagcggcc tggtgccgcg cggcagccat 60

atgaagaaga agaggaagct ggcggtgctg gcggcggcgc cggcgggaca cctggcttcg 120

gatttcgcct tctcgccccc tccaggtggt ggaggtgatg ggccaggggg gccggagccg 180

ggctgggttg atcctcggac ctggctaagc ttccaaggcc ctcctggagg gccaggaatc 240

gggccggggg ttgggccagg ctctgaggtg tgggggattc ccccatgccc cccgccgtat 300

gagttctgtg gggggatggc gtactgtggg ccccaggttg gagtggggct agtgccccaa 360

ggcggcttgg agacctctca gcctgagggc gaagcaggag tcggggtgga gagcaactcc 420

gatggggcct ccccggagcc ctgcaccgtc acccctggtg ccgtgaagct ggagaaggag 480

aagctggagc aaaacccgga ggagtcccag gacatcaaag ctctgcagaa agaactcgag 540

caatttgcca agctcctgaa gcagaagagg atcaccctgg gatatacaca ggccgatgtg 600

gggctcaccc tgggggttct atttgggaag gtattcagcc aaacgaccat ctgccgcttt 660

gaggctctgc agcttagctt caagaacatg tgtaagctgc ggcccttgct gcagaagtgg 720

gtggaggaag ctgacaacaa tgaaaatctt caggagatat gcaaagcaga aaccctcgtg 780

caggcccgaa agagaaagcg aaccagtatc gagaaccgag tgagaggcaa cctggagaat 840

ttgttcctgc agtgcccgaa acccacactg cagcagatca gccacatcgc ccagcagctt 900

gggctcgaga aggatgtggt ccgagtgtgg ttctgtaacc ggcgccagaa gggcaagcga 960

tcaagcagcg actatgcaca acgagaggat tttgaggctg ctgggtctcc tttctcaggg 1020

ggaccagtgt cctttcctct ggccccaggg ccccattttg gtaccccagg ctatgggagc 1080

cctcacttca ctgcactgta ctcctcggtc cctttccctg agggggaagc ctttccccct 1140

gtctccgtca ccactctggg ctctcccatg cattcaaacc tggcggtgct ggcggcggcg        1200

ccgtga                                                                  1206

<210>    74
<211>    401
<212>    PRT
<213>    Artificial Sequence

<220>
<223>    His-NLS-JO-86 MTD-Oct4-JO-86 MTD Amino Acid Sequence

<400>    74
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Ala Gly His Leu Ala Ser Asp Phe Ala Phe Ser Pro Pro Pro
        35                  40                  45

Gly Gly Gly Gly Asp Gly Pro Gly Gly Pro Glu Pro Gly Trp Val Asp
    50                  55                  60

Pro Arg Thr Trp Leu Ser Phe Gln Gly Pro Pro Gly Gly Pro Gly Ile
65                  70                  75                  80

Gly Pro Gly Val Gly Pro Gly Ser Glu Val Trp Gly Ile Pro Pro Cys
                85                  90                  95

Pro Pro Pro Tyr Glu Phe Cys Gly Gly Met Ala Tyr Cys Gly Pro Gln
            100                 105                 110

Val Gly Val Gly Leu Val Pro Gln Gly Gly Leu Glu Thr Ser Gln Pro
        115                 120                 125

Glu Gly Glu Ala Gly Val Gly Val Glu Ser Asn Ser Asp Gly Ala Ser
    130                 135                 140

Pro Glu Pro Cys Thr Val Thr Pro Gly Ala Val Lys Leu Glu Lys Glu
145                 150                 155                 160

Lys Leu Glu Gln Asn Pro Glu Glu Ser Gln Asp Ile Lys Ala Leu Gln
                165                 170                 175

Lys Glu Leu Glu Gln Phe Ala Lys Leu Leu Lys Gln Lys Arg Ile Thr
            180                 185                 190

Leu Gly Tyr Thr Gln Ala Asp Val Gly Leu Thr Leu Gly Val Leu Phe
        195                 200                 205

Gly Lys Val Phe Ser Gln Thr Thr Ile Cys Arg Phe Glu Ala Leu Gln
    210                 215                 220

Leu Ser Phe Lys Asn Met Cys Lys Leu Arg Pro Leu Leu Gln Lys Trp
225                 230                 235                 240

Val Glu Glu Ala Asp Asn Asn Glu Asn Leu Gln Glu Ile Cys Lys Ala
                245                 250                 255

Glu Thr Leu Val Gln Ala Arg Lys Arg Lys Arg Thr Ser Ile Glu Asn
            260                 265                 270

Arg Val Arg Gly Asn Leu Glu Asn Leu Phe Leu Gln Cys Pro Lys Pro
        275                 280                 285

Thr Leu Gln Gln Ile Ser His Ile Ala Gln Gln Leu Gly Leu Glu Lys
    290                 295                 300

Asp Val Val Arg Val Trp Phe Cys Asn Arg Arg Gln Lys Gly Lys Arg
305                 310                 315                 320

Ser Ser Ser Asp Tyr Ala Gln Arg Glu Asp Phe Glu Ala Ala Gly Ser
                325                 330                 335

Pro Phe Ser Gly Gly Pro Val Ser Phe Pro Leu Ala Pro Gly Pro His

340                 345                 350

Phe Gly Thr Pro Gly Tyr Gly Ser Pro His Phe Thr Ala Leu Tyr Ser
        355                 360                 365

Ser Val Pro Phe Pro Glu Gly Glu Ala Phe Pro Pro Val Ser Val Thr
    370                 375                 380

Thr Leu Gly Ser Pro Met His Ser Asn Leu Ala Val Leu Ala Ala Ala
385                 390                 395                 400

Pro

<210>   75
<211>   969
<212>   DNA
<213>   Artificial Sequence

<220>
<223>   NLS-Sox2 cDNA Sequence

<400>   75
atgaagaaga agaggaagta caacatgatg gagacggagc tgaagccgcc gggcccgcag      60

caaacttcgg ggggcggcgg cggcaactcc accgcggcgg cggccggcgg caaccagaaa     120

aacagcccgg accgcgtcaa gcggcccatg aatgccttca tggtgtggtc ccgcgggcag     180

cggcgcaaga tggcccagga gaaccccaag atgcacaact cggagatcag caagcgcctg     240

ggcgccgagt ggaaactttt gtcggagacg gagaagcggc cgttcatcga cgaggctaag     300

cggctgcgag cgctgcacat gaaggagcac ccggattata aataccggcc ccggcggaaa     360

accaagacgc tcatgaagaa ggataagtac acgctgcccg gcgggctgct ggcccccggc     420

ggcaatagca tggcgagcgg ggtcggggtg ggcgccggcc tgggcgcggg cgtgaaccag     480

cgcatggaca gttacgcgca catgaacggc tggagcaacg gcagctacag catgatgcag 540

gaccagctgg gctacccgca gcacccgggc ctcaatgcgc acggcgcagc gcagatgcag 600

cccatgcacc gctacgacgt gagcgccctg cagtacaact ccatgaccag ctcgcagacc 660

tacatgaacg gctcgcccac ctacagcatg tcctactcgc agcagggcac ccctggcatg 720

gctcttggct ccatgggttc ggtggtcaag tccgaggcca gctccagccc ccctgtggtt 780

acctcttcct cccactccag ggcgccctgc caggccgggg acctccggga catgatcagc 840

atgtatctcc ccggcgccga ggtgccggaa cccgccgccc ccagcagact tcacatgtcc 900

cagcactacc agagcggccc ggtgcccggc acggccatta acggcacact gcccctctca 960

cacatgtga 969

<210> 76
<211> 322
<212> PRT
<213> Artificial Sequence

<220>
<223> NLS-Sox2 Amino Acid Sequence

<400> 76
Met Lys Lys Lys Arg Lys Tyr Asn Met Met Glu Thr Glu Leu Lys Pro
1               5                   10                  15

Pro Gly Pro Gln Gln Thr Ser Gly Gly Gly Gly Gly Asn Ser Thr Ala
            20                  25                  30

Ala Ala Ala Gly Gly Asn Gln Lys Asn Ser Pro Asp Arg Val Lys Arg
        35                  40                  45

Pro Met Asn Ala Phe Met Val Trp Ser Arg Gly Gln Arg Arg Lys Met

50                  55                  60

Ala Gln Glu Asn Pro Lys Met His Asn Ser Glu Ile Ser Lys Arg Leu
65                  70                  75                  80

Gly Ala Glu Trp Lys Leu Leu Ser Glu Thr Glu Lys Arg Pro Phe Ile
                85                  90                  95

Asp Glu Ala Lys Arg Leu Arg Ala Leu His Met Lys Glu His Pro Asp
            100                 105                 110

Tyr Lys Tyr Arg Pro Arg Arg Lys Thr Lys Thr Leu Met Lys Lys Asp
        115                 120                 125

Lys Tyr Thr Leu Pro Gly Gly Leu Leu Ala Pro Gly Gly Asn Ser Met
    130                 135                 140

Ala Ser Gly Val Gly Val Gly Ala Gly Leu Gly Ala Gly Val Asn Gln
145                 150                 155                 160

Arg Met Asp Ser Tyr Ala His Met Asn Gly Trp Ser Asn Gly Ser Tyr
                165                 170                 175

Ser Met Met Gln Asp Gln Leu Gly Tyr Pro Gln His Pro Gly Leu Asn
            180                 185                 190

Ala His Gly Ala Ala Gln Met Gln Pro Met His Arg Tyr Asp Val Ser
        195                 200                 205

Ala Leu Gln Tyr Asn Ser Met Thr Ser Ser Gln Thr Tyr Met Asn Gly
    210                 215                 220

Ser Pro Thr Tyr Ser Met Ser Tyr Ser Gln Gln Gly Thr Pro Gly Met
225                 230                 235                 240

Ala Leu Gly Ser Met Gly Ser Val Val Lys Ser Glu Ala Ser Ser Ser
                245                 250                 255

Pro Pro Val Val Thr Ser Ser Ser His Ser Arg Ala Pro Cys Gln Ala
            260                 265                 270

Gly Asp Leu Arg Asp Met Ile Ser Met Tyr Leu Pro Gly Ala Glu Val
        275                 280                 285

Pro Glu Pro Ala Ala Pro Ser Arg Leu His Met Ser Gln His Tyr Gln
    290                 295                 300

Ser Gly Pro Val Pro Gly Thr Ala Ile Asn Gly Thr Leu Pro Leu Ser
305                 310                 315                 320

His Met

<210>    77
<211>    996
<212>    DNA
<213>    Artificial Sequence

<220>
<223>    NLS-JO-84 MTD-Sox2 cDNA Sequence

<400>    77
atgaagaaga agaggaagct ggtggcggcg ctgctggcgg tgctgtacaa catgatggag        60

acggagctga agccgccggg cccgcagcaa acttcggggg gcggcggcgg caactccacc       120

gcggcggcgg ccggcggcaa ccagaaaaac agcccggacc gcgtcaagcg gcccatgaat       180

gccttcatgg tgtggtcccg cgggcagcgg cgcaagatgg cccaggagaa ccccaagatg       240

cacaactcgg agatcagcaa gcgcctgggc gccgagtgga aacttttgtc ggagacggag       300

aagcggccgt tcatcgacga ggctaagcgg ctgcgagcgc tgcacatgaa ggagcacccg       360

gattataaat accggccccg gcggaaaacc aagacgctca tgaagaagga taagtacacg       420

ctgcccggcg ggctgctggc ccccggcggc aatagcatgg cgagcggggt cggggtgggc       480

gccggcctgg gcgcgggcgt gaaccagcgc atggacagtt acgcgcacat gaacggctgg 540

agcaacggca gctacagcat gatgcaggac cagctgggct acccgcagca cccgggcctc 600

aatgcgcacg gcgcagcgca gatgcagccc atgcaccgct acgacgtgag cgccctgcag 660

tacaactcca tgaccagctc gcagacctac atgaacggct cgcccaccta cagcatgtcc 720

tactcgcagc agggcacccc tggcatggct cttggctcca tgggttcggt ggtcaagtcc 780

gaggccagct ccagcccccc tgtggttacc tcttcctccc actccagggc gccctgccag 840

gccggggacc tccgggacat gatcagcatg tatctccccg gcgccgaggt gccggaaccc 900

gccgccccca gcagacttca catgtcccag cactaccaga gcggcccggt gcccggcacg 960

gccattaacg gcacactgcc cctctcacac atgtga 996

<210> 78
<211> 331
<212> PRT
<213> Artificial Sequence

<220>
<223> NLS-JO-84 MTD-Sox2 Amino Acid Sequence

<400> 78
Met Lys Lys Lys Arg Lys Leu Val Ala Ala Leu Leu Ala Val Leu Tyr
1               5                   10                  15

Asn Met Met Glu Thr Glu Leu Lys Pro Pro Gly Pro Gln Gln Thr Ser
            20                  25                  30

Gly Gly Gly Gly Gly Asn Ser Thr Ala Ala Ala Ala Gly Gly Asn Gln
        35                  40                  45

Lys Asn Ser Pro Asp Arg Val Lys Arg Pro Met Asn Ala Phe Met Val
    50                  55                  60

Trp Ser Arg Gly Gln Arg Arg Lys Met Ala Gln Glu Asn Pro Lys Met
65                  70                  75                  80

His Asn Ser Glu Ile Ser Lys Arg Leu Gly Ala Glu Trp Lys Leu Leu
                85                  90                  95

Ser Glu Thr Glu Lys Arg Pro Phe Ile Asp Glu Ala Lys Arg Leu Arg
            100                 105                 110

Ala Leu His Met Lys Glu His Pro Asp Tyr Lys Tyr Arg Pro Arg Arg
        115                 120                 125

Lys Thr Lys Thr Leu Met Lys Lys Asp Lys Tyr Thr Leu Pro Gly Gly
    130                 135                 140

Leu Leu Ala Pro Gly Gly Asn Ser Met Ala Ser Gly Val Gly Val Gly
145                 150                 155                 160

Ala Gly Leu Gly Ala Gly Val Asn Gln Arg Met Asp Ser Tyr Ala His
                165                 170                 175

Met Asn Gly Trp Ser Asn Gly Ser Tyr Ser Met Met Gln Asp Gln Leu
            180                 185                 190

Gly Tyr Pro Gln His Pro Gly Leu Asn Ala His Gly Ala Ala Gln Met
        195                 200                 205

Gln Pro Met His Arg Tyr Asp Val Ser Ala Leu Gln Tyr Asn Ser Met
    210                 215                 220

Thr Ser Ser Gln Thr Tyr Met Asn Gly Ser Pro Thr Tyr Ser Met Ser
225                 230                 235                 240

Tyr Ser Gln Gln Gly Thr Pro Gly Met Ala Leu Gly Ser Met Gly Ser
                245                 250                 255

Val Val Lys Ser Glu Ala Ser Ser Ser Pro Pro Val Val Thr Ser Ser
            260                 265                 270

Ser His Ser Arg Ala Pro Cys Gln Ala Gly Asp Leu Arg Asp Met Ile
        275                 280                 285

Ser Met Tyr Leu Pro Gly Ala Glu Val Pro Glu Pro Ala Ala Pro Ser
    290                 295                 300

Arg Leu His Met Ser Gln His Tyr Gln Ser Gly Pro Val Pro Gly Thr
305                 310                 315                 320

Ala Ile Asn Gly Thr Leu Pro Leu Ser His Met
                325                 330

<210>    79
<211>    996
<212>    DNA
<213>    Artificial Sequence

<220>
<223>    NLS-Sox2-JO-84 MTD cDNA Sequence

<400>    79
atgaagaaga agaggaagta caacatgatg gagacggagc tgaagccgcc gggcccgcag      60

caaacttcgg ggggcggcgg cggcaactcc accgcggcgg cggccggcgg caaccagaaa     120

aacagcccgg accgcgtcaa gcggcccatg aatgccttca tggtgtggtc ccgcgggcag     180

cggcgcaaga tggcccagga gaaccccaag atgcacaact cggagatcag caagcgcctg     240

ggcgccgagt ggaaactttt gtcggagacg gagaagcggc cgttcatcga cgaggctaag     300

cggctgcgag cgctgcacat gaaggagcac ccggattata aataccggcc ccggcggaaa     360

accaagacgc tcatgaagaa ggataagtac acgctgcccg gcgggctgct ggcccccggc     420

ggcaatagca tggcgagcgg ggtcggggtg ggcgccggcc tgggcgcggg cgtgaaccag     480

cgcatggaca gttacgcgca catgaacggc tggagcaacg gcagctacag catgatgcag     540

gaccagctgg gctacccgca gcacccgggc ctcaatgcgc acggcgcagc gcagatgcag 600

cccatgcacc gctacgacgt gagcgccctg cagtacaact ccatgaccag ctcgcagacc 660

tacatgaacg gctcgcccac ctacagcatg tcctactcgc agcagggcac ccctggcatg 720

gctcttggct ccatgggttc ggtggtcaag tccgaggcca gctccagccc ccctgtggtt 780

acctcttcct cccactccag ggcgccctgc caggccgggg acctccggga catgatcagc 840

atgtatctcc ccggcgccga ggtgccggaa cccgccgccc ccagcagact tcacatgtcc 900

cagcactacc agagcggccc ggtgcccggc acggccatta acggcacact gcccctctca 960

cacatgctgg tggcggcgct gctggcggtg ctgtga 996

<210> 80
<211> 331
<212> PRT
<213> Artificial Sequence

<220>
<223> NLS-Sox2-JO-84 MTD Amino Acid Sequence

<400> 80
Met Lys Lys Lys Arg Lys Tyr Asn Met Met Glu Thr Glu Leu Lys Pro
1               5                   10                  15

Pro Gly Pro Gln Gln Thr Ser Gly Gly Gly Gly Asn Ser Thr Ala
            20                  25                  30

Ala Ala Ala Gly Gly Asn Gln Lys Asn Ser Pro Asp Arg Val Lys Arg
            35                  40                  45

Pro Met Asn Ala Phe Met Val Trp Ser Arg Gly Gln Arg Arg Lys Met
        50                  55                  60

Ala Gln Glu Asn Pro Lys Met His Asn Ser Glu Ile Ser Lys Arg Leu
65                  70                  75                  80

Gly Ala Glu Trp Lys Leu Leu Ser Glu Thr Glu Lys Arg Pro Phe Ile
                85                  90                  95

Asp Glu Ala Lys Arg Leu Arg Ala Leu His Met Lys Glu His Pro Asp
            100                 105                 110

Tyr Lys Tyr Arg Pro Arg Arg Lys Thr Lys Thr Leu Met Lys Lys Asp
        115                 120                 125

Lys Tyr Thr Leu Pro Gly Gly Leu Leu Ala Pro Gly Gly Asn Ser Met
    130                 135                 140

Ala Ser Gly Val Gly Val Gly Ala Gly Leu Gly Ala Gly Val Asn Gln
145                 150                 155                 160

Arg Met Asp Ser Tyr Ala His Met Asn Gly Trp Ser Asn Gly Ser Tyr
                165                 170                 175

Ser Met Met Gln Asp Gln Leu Gly Tyr Pro Gln His Pro Gly Leu Asn
            180                 185                 190

Ala His Gly Ala Ala Gln Met Gln Pro Met His Arg Tyr Asp Val Ser
        195                 200                 205

Ala Leu Gln Tyr Asn Ser Met Thr Ser Ser Gln Thr Tyr Met Asn Gly
    210                 215                 220

Ser Pro Thr Tyr Ser Met Ser Tyr Ser Gln Gln Gly Thr Pro Gly Met
225                 230                 235                 240

Ala Leu Gly Ser Met Gly Ser Val Val Lys Ser Glu Ala Ser Ser Ser
                245                 250                 255

Pro Pro Val Val Thr Ser Ser Ser His Ser Arg Ala Pro Cys Gln Ala
            260                 265                 270

Gly Asp Leu Arg Asp Met Ile Ser Met Tyr Leu Pro Gly Ala Glu Val

275                 280                 285

Pro Glu Pro Ala Ala Pro Ser Arg Leu His Met Ser Gln His Tyr Gln
    290                 295                 300

Ser Gly Pro Val Pro Gly Thr Ala Ile Asn Gly Thr Leu Pro Leu Ser
305                 310                 315                 320

His Met Leu Val Ala Ala Leu Leu Ala Val Leu
                325                 330

<210>   81
<211>   1023
<212>   DNA
<213>   Artificial Sequence

<220>
<223>   NLS-JO-84 MTD-Sox2-JO-84 MTD cDNA Sequence

<400>   81
atgaagaaga agaggaagct ggtggcggcg ctgctggcgg tgctgtacaa catgatggag      60

acggagctga agccgccggg cccgcagcaa acttcggggg gcggcggcgg caactccacc     120

gcggcggcgg ccggcggcaa ccagaaaaac agcccggacc gcgtcaagcg gcccatgaat     180

gccttcatgg tgtggtcccg cgggcagcgg cgcaagatgg cccaggagaa ccccaagatg     240

cacaactcgg agatcagcaa gcgcctgggc gccgagtgga aacttttgtc ggagacggag     300

aagcggccgt tcatcgacga ggctaagcgg ctgcgagcgc tgcacatgaa ggagcacccg     360

gattataaat accggccccg gcggaaaacc aagacgctca tgaagaagga taagtacacg     420

ctgcccggcg ggctgctggc ccccggcggc aatagcatgg cgagcggggt cggggtgggc     480

gccggcctgg gcgcgggcgt gaaccagcgc atggacagtt acgcgcacat gaacggctgg     540

agcaacggca gctacagcat gatgcaggac cagctgggct acccgcagca cccgggcctc 600

aatgcgcacg gcgcagcgca gatgcagccc atgcaccgct acgacgtgag cgccctgcag 660

tacaactcca tgaccagctc gcagacctac atgaacggct cgcccaccta cagcatgtcc 720

tactcgcagc agggcacccc tggcatggct cttggctcca tgggttcggt ggtcaagtcc 780

gaggccagct ccagcccccc tgtggttacc tcttcctccc actccagggc gccctgccag 840

gccggggacc tccgggacat gatcagcatg tatctccccg gcgccgaggt gccggaaccc 900

gccgccccca gcagacttca catgtcccag cactaccaga gcggcccggt gcccggcacg 960

gccattaacg gcacactgcc cctctcacac atgctggtgg cggcgctgct ggcggtgctg 1020

tga 1023

<210> 82
<211> 340
<212> PRT
<213> Artificial Sequence

<220>
<223> NLS-JO-84 MTD-Sox2-JO-84 MTD Amino Acid Sequence

<400> 82
Met Lys Lys Lys Arg Lys Leu Val Ala Ala Leu Leu Ala Val Leu Tyr
1               5                   10                  15

Asn Met Met Glu Thr Glu Leu Lys Pro Pro Gly Pro Gln Gln Thr Ser
            20                  25                  30

Gly Gly Gly Gly Gly Asn Ser Thr Ala Ala Ala Ala Gly Gly Asn Gln
        35                  40                  45

Lys Asn Ser Pro Asp Arg Val Lys Arg Pro Met Asn Ala Phe Met Val
    50                  55                  60

Trp Ser Arg Gly Gln Arg Arg Lys Met Ala Gln Glu Asn Pro Lys Met
65                  70                  75                  80

His Asn Ser Glu Ile Ser Lys Arg Leu Gly Ala Glu Trp Lys Leu Leu
                85                  90                  95

Ser Glu Thr Glu Lys Arg Pro Phe Ile Asp Glu Ala Lys Arg Leu Arg
            100                 105                 110

Ala Leu His Met Lys Glu His Pro Asp Tyr Lys Tyr Arg Pro Arg Arg
        115                 120                 125

Lys Thr Lys Thr Leu Met Lys Lys Asp Lys Tyr Thr Leu Pro Gly Gly
    130                 135                 140

Leu Leu Ala Pro Gly Gly Asn Ser Met Ala Ser Gly Val Gly Val Gly
145                 150                 155                 160

Ala Gly Leu Gly Ala Gly Val Asn Gln Arg Met Asp Ser Tyr Ala His
                165                 170                 175

Met Asn Gly Trp Ser Asn Gly Ser Tyr Ser Met Met Gln Asp Gln Leu
            180                 185                 190

Gly Tyr Pro Gln His Pro Gly Leu Asn Ala His Gly Ala Ala Gln Met
        195                 200                 205

Gln Pro Met His Arg Tyr Asp Val Ser Ala Leu Gln Tyr Asn Ser Met
    210                 215                 220

Thr Ser Ser Gln Thr Tyr Met Asn Gly Ser Pro Thr Tyr Ser Met Ser
225                 230                 235                 240

Tyr Ser Gln Gln Gly Thr Pro Gly Met Ala Leu Gly Ser Met Gly Ser
                245                 250                 255

Val Val Lys Ser Glu Ala Ser Ser Ser Pro Pro Val Val Thr Ser Ser
            260                 265                 270

Ser His Ser Arg Ala Pro Cys Gln Ala Gly Asp Leu Arg Asp Met Ile
        275                 280                 285

Ser Met Tyr Leu Pro Gly Ala Glu Val Pro Glu Pro Ala Ala Pro Ser
    290                 295                 300

Arg Leu His Met Ser Gln His Tyr Gln Ser Gly Pro Val Pro Gly Thr
305                 310                 315                 320

Ala Ile Asn Gly Thr Leu Pro Leu Ser His Met Leu Val Ala Ala Leu
                325                 330                 335

Leu Ala Val Leu
            340

<210>    83
<211>    993
<212>    DNA
<213>    Artificial Sequence

<220>
<223>    NLS-JO-86 MTD-Sox2 cDNA Sequence

<400>    83
atgaagaaga agaggaagct ggcggtgctg gcggcggcgc cgtacaacat gatggagacg        60

gagctgaagc cgccgggccc gcagcaaact tcggggggcg gcggcggcaa ctccaccgcg        120

gcggcggccg gcggcaacca gaaaaacagc ccggaccgcg tcaagcggcc catgaatgcc        180

ttcatggtgt ggtcccgcgg gcagcggcgc aagatggccc aggagaaccc caagatgcac        240

aactcggaga tcagcaagcg cctgggcgcc gagtggaaac ttttgtcgga gacggagaag        300

cggccgttca tcgacgaggc taagcggctg cgagcgctgc acatgaagga gcacccggat        360

tataaatacc ggccccggcg gaaaaccaag acgctcatga agaaggataa gtacacgctg        420

cccggcgggc tgctggcccc cggcggcaat agcatggcga gcggggtcgg ggtgggcgcc 480

ggcctgggcg cgggcgtgaa ccagcgcatg gacagttacg cgcacatgaa cggctggagc 540

aacggcagct acagcatgat gcaggaccag ctgggctacc cgcagcaccc gggcctcaat 600

gcgcacggcg cagcgcagat gcagcccatg caccgctacg acgtgagcgc cctgcagtac 660

aactccatga ccagctcgca gacctacatg aacggctcgc ccacctacag catgtcctac 720

tcgcagcagg gcacccctgg catggctctt ggctccatgg gttcggtggt caagtccgag 780

gccagctcca gcccccctgt ggttacctct tcctcccact ccagggcgcc ctgccaggcc 840

ggggacctcc gggacatgat cagcatgtat ctccccggcg ccgaggtgcc ggaacccgcc 900

gcccccagca gacttcacat gtcccagcac taccagagcg gcccggtgcc cggcacggcc 960

attaacggca cactgcccct ctcacacatg tga 993

<210> 84
<211> 330
<212> PRT
<213> Artificial Sequence

<220>
<223> NLS-JO-86 MTD-Sox2 Amino Acid Sequence

<400> 84
Met Lys Lys Lys Arg Lys Leu Ala Val Leu Ala Ala Ala Pro Tyr Asn
1               5                   10                  15

Met Met Glu Thr Glu Leu Lys Pro Pro Gly Pro Gln Gln Thr Ser Gly
            20                  25                  30

Gly Gly Gly Gly Asn Ser Thr Ala Ala Ala Ala Gly Gly Asn Gln Lys
        35                  40                  45

Asn Ser Pro Asp Arg Val Lys Arg Pro Met Asn Ala Phe Met Val Trp
    50                  55                  60

Ser Arg Gly Gln Arg Arg Lys Met Ala Gln Glu Asn Pro Lys Met His
65                  70                  75                  80

Asn Ser Glu Ile Ser Lys Arg Leu Gly Ala Glu Trp Lys Leu Leu Ser
                85                  90                  95

Glu Thr Glu Lys Arg Pro Phe Ile Asp Glu Ala Lys Arg Leu Arg Ala
            100                 105                 110

Leu His Met Lys Glu His Pro Asp Tyr Lys Tyr Arg Pro Arg Arg Lys
        115                 120                 125

Thr Lys Thr Leu Met Lys Lys Asp Lys Tyr Thr Leu Pro Gly Gly Leu
    130                 135                 140

Leu Ala Pro Gly Gly Asn Ser Met Ala Ser Gly Val Gly Val Gly Ala
145                 150                 155                 160

Gly Leu Gly Ala Gly Val Asn Gln Arg Met Asp Ser Tyr Ala His Met
                165                 170                 175

Asn Gly Trp Ser Asn Gly Ser Tyr Ser Met Met Gln Asp Gln Leu Gly
            180                 185                 190

Tyr Pro Gln His Pro Gly Leu Asn Ala His Gly Ala Ala Gln Met Gln
        195                 200                 205

Pro Met His Arg Tyr Asp Val Ser Ala Leu Gln Tyr Asn Ser Met Thr
    210                 215                 220

Ser Ser Gln Thr Tyr Met Asn Gly Ser Pro Thr Tyr Ser Met Ser Tyr
225                 230                 235                 240

Ser Gln Gln Gly Thr Pro Gly Met Ala Leu Gly Ser Met Gly Ser Val
                245                 250                 255

Val Lys Ser Glu Ala Ser Ser Ser Pro Pro Val Val Thr Ser Ser Ser

            260                 265                 270

His Ser Arg Ala Pro Cys Gln Ala Gly Asp Leu Arg Asp Met Ile Ser
        275                 280                 285

Met Tyr Leu Pro Gly Ala Glu Val Pro Glu Pro Ala Ala Pro Ser Arg
    290                 295                 300

Leu His Met Ser Gln His Tyr Gln Ser Gly Pro Val Pro Gly Thr Ala
305                 310                 315                 320

Ile Asn Gly Thr Leu Pro Leu Ser His Met
                325                 330

<210> 85
<211> 993
<212> DNA
<213> Artificial Sequence

<220>
<223> NLS-Sox2-JO-86 MTD cDNA Sequence

<400> 85
atgaagaaga agaggaagta caacatgatg gagacggagc tgaagccgcc gggcccgcag 60

caaacttcgg ggggcggcgg cggcaactcc accgcggcgg cggccggcgg caaccagaaa 120

aacagcccgg accgcgtcaa gcggcccatg aatgccttca tggtgtggtc ccgcgggcag 180

cggcgcaaga tggcccagga gaaccccaag atgcacaact cggagatcag caagcgcctg 240

ggcgccgagt ggaaactttt gtcggagacg gagaagcggc cgttcatcga cgaggctaag 300

cggctgcgag cgctgcacat gaaggagcac ccggattata aataccggcc ccggcggaaa 360

accaagacgc tcatgaagaa ggataagtac acgctgcccg gcgggctgct ggcccccggc 420

ggcaatagca tggcgagcgg ggtcggggtg ggcgccggcc tgggcgcggg cgtgaaccag 480

cgcatggaca gttacgcgca catgaacggc tggagcaacg gcagctacag catgatgcag 540

gaccagctgg gctacccgca gcacccgggc ctcaatgcgc acggcgcagc gcagatgcag 600

cccatgcacc gctacgacgt gagcgccctg cagtacaact ccatgaccag ctcgcagacc 660

tacatgaacg gctcgcccac ctacagcatg tcctactcgc agcagggcac ccctggcatg 720

gctcttggct ccatgggttc ggtggtcaag tccgaggcca gctccagccc ccctgtggtt 780

acctcttcct cccactccag ggcgccctgc caggccgggg acctccggga catgatcagc 840

atgtatctcc ccggcgccga ggtgccggaa cccgccgccc ccagcagact tcacatgtcc 900

cagcactacc agagcggccc ggtgcccggc acggccatta acggcacact gcccctctca 960

cacatgctgg cggtgctggc ggcggcgccg tga 993

<210> 86
<211> 330
<212> PRT
<213> Artificial Sequence

<220>
<223> NLS-Sox2-JO-86 MTD Amino Acid Sequence

<400> 86
Met Lys Lys Lys Arg Lys Tyr Asn Met Met Glu Thr Glu Leu Lys Pro
1               5                   10                  15

Pro Gly Pro Gln Gln Thr Ser Gly Gly Gly Gly Asn Ser Thr Ala
            20                  25                  30

Ala Ala Ala Gly Gly Asn Gln Lys Asn Ser Pro Asp Arg Val Lys Arg
            35                  40                  45

Pro Met Asn Ala Phe Met Val Trp Ser Arg Gly Gln Arg Arg Lys Met

        50                  55                  60

Ala Gln Glu Asn Pro Lys Met His Asn Ser Glu Ile Ser Lys Arg Leu
65 70 75 80

Gly Ala Glu Trp Lys Leu Leu Ser Glu Thr Glu Lys Arg Pro Phe Ile
85 90 95

Asp Glu Ala Lys Arg Leu Arg Ala Leu His Met Lys Glu His Pro Asp
                100                 105                 110

Tyr Lys Tyr Arg Pro Arg Arg Lys Thr Lys Thr Leu Met Lys Lys Asp
            115                 120                 125

Lys Tyr Thr Leu Pro Gly Gly Leu Leu Ala Pro Gly Gly Asn Ser Met
        130                 135                 140

Ala Ser Gly Val Gly Val Gly Ala Gly Leu Gly Ala Gly Val Asn Gln
145 150 155 160

Arg Met Asp Ser Tyr Ala His Met Asn Gly Trp Ser Asn Gly Ser Tyr
165 170 175

Ser Met Met Gln Asp Gln Leu Gly Tyr Pro Gln His Pro Gly Leu Asn
                180                 185                 190

Ala His Gly Ala Ala Gln Met Gln Pro Met His Arg Tyr Asp Val Ser
            195                 200                 205

Ala Leu Gln Tyr Asn Ser Met Thr Ser Ser Gln Thr Tyr Met Asn Gly
        210                 215                 220

Ser Pro Thr Tyr Ser Met Ser Tyr Ser Gln Gln Gly Thr Pro Gly Met
225 230 235 240

Ala Leu Gly Ser Met Gly Ser Val Val Lys Ser Glu Ala Ser Ser Ser
245 250 255

Pro Pro Val Val Thr Ser Ser Ser His Ser Arg Ala Pro Cys Gln Ala
                260                 265                 270

Gly Asp Leu Arg Asp Met Ile Ser Met Tyr Leu Pro Gly Ala Glu Val
        275                 280                 285

Pro Glu Pro Ala Ala Pro Ser Arg Leu His Met Ser Gln His Tyr Gln
    290                 295                 300

Ser Gly Pro Val Pro Gly Thr Ala Ile Asn Gly Thr Leu Pro Leu Ser
305                 310                 315                 320

His Met Leu Ala Val Leu Ala Ala Ala Pro
                325                 330

<210>    87
<211>    1017
<212>    DNA
<213>    Artificial Sequence

<220>
<223>    NLS-JO-86 MTD-Sox2-JO-86 MTD cDNA Sequence

<400>    87
atgaagaaga agaggaagct ggcggtgctg gcggcggcgc cgtacaacat gatggagacg          60

gagctgaagc cgccgggccc gcagcaaact tcggggggcg gcggcggcaa ctccaccgcg         120

gcggcggccg gcggcaacca gaaaaacagc ccggaccgcg tcaagcggcc catgaatgcc         180

ttcatggtgt ggtcccgcgg gcagcggcgc aagatggccc aggagaaccc caagatgcac         240

aactcggaga tcagcaagcg cctgggcgcc gagtggaaac ttttgtcgga gacggagaag         300

cggccgttca tcgacgaggc taagcggctg cgagcgctgc acatgaagga gcacccggat         360

tataaatacc ggccccggcg gaaaaccaag acgctcatga agaaggataa gtacacgctg         420

cccggcgggc tgctggcccc cggcggcaat agcatggcga gcggggtcgg ggtgggcgcc         480

ggcctgggcg cgggcgtgaa ccagcgcatg gacagttacg cgcacatgaa cggctggagc 540

aacggcagct acagcatgat gcaggaccag ctgggctacc cgcagcaccc gggcctcaat 600

gcgcacggcg cagcgcagat gcagcccatg caccgctacg acgtgagcgc cctgcagtac 660

aactccatga ccagctcgca gacctacatg aacggctcgc ccacctacag catgtcctac 720

tcgcagcagg gcacccctgg catggctctt ggctccatgg gttcggtggt caagtccgag 780

gccagctcca gcccccctgt ggttacctct tcctcccact ccagggcgcc ctgccaggcc 840

ggggacctcc gggacatgat cagcatgtat ctccccggcg ccgaggtgcc ggaacccgcc 900

gcccccagca gacttcacat gtcccagcac taccagagcg gcccggtgcc cggcacggcc 960

attaacggca cactgcccct ctcacacatg ctggcggtgc tggcggcggc gccgtga 1017

<210> 88
<211> 338
<212> PRT
<213> Artificial Sequence

<220>
<223> NLS-JO-86 MTD-Sox2-JO-86 MTD Amino Acid Sequence

<400> 88
Met Lys Lys Lys Arg Lys Leu Ala Val Leu Ala Ala Ala Pro Tyr Asn
1               5                   10                  15

Met Met Glu Thr Glu Leu Lys Pro Pro Gly Pro Gln Gln Thr Ser Gly
            20                  25                  30

Gly Gly Gly Gly Asn Ser Thr Ala Ala Ala Ala Gly Gly Asn Gln Lys
        35                  40                  45

Asn Ser Pro Asp Arg Val Lys Arg Pro Met Asn Ala Phe Met Val Trp
    50                  55                  60

Ser Arg Gly Gln Arg Arg Lys Met Ala Gln Glu Asn Pro Lys Met His
65                  70                  75                  80

Asn Ser Glu Ile Ser Lys Arg Leu Gly Ala Glu Trp Lys Leu Leu Ser
                85                  90                  95

Glu Thr Glu Lys Arg Pro Phe Ile Asp Glu Ala Lys Arg Leu Arg Ala
            100                 105                 110

Leu His Met Lys Glu His Pro Asp Tyr Lys Tyr Arg Pro Arg Arg Lys
        115                 120                 125

Thr Lys Thr Leu Met Lys Lys Asp Lys Tyr Thr Leu Pro Gly Gly Leu
    130                 135                 140

Leu Ala Pro Gly Gly Asn Ser Met Ala Ser Gly Val Gly Val Gly Ala
145                 150                 155                 160

Gly Leu Gly Ala Gly Val Asn Gln Arg Met Asp Ser Tyr Ala His Met
                165                 170                 175

Asn Gly Trp Ser Asn Gly Ser Tyr Ser Met Met Gln Asp Gln Leu Gly
            180                 185                 190

Tyr Pro Gln His Pro Gly Leu Asn Ala His Gly Ala Ala Gln Met Gln
        195                 200                 205

Pro Met His Arg Tyr Asp Val Ser Ala Leu Gln Tyr Asn Ser Met Thr
    210                 215                 220

Ser Ser Gln Thr Tyr Met Asn Gly Ser Pro Thr Tyr Ser Met Ser Tyr
225                 230                 235                 240

Ser Gln Gln Gly Thr Pro Gly Met Ala Leu Gly Ser Met Gly Ser Val
                245                 250                 255

Val Lys Ser Glu Ala Ser Ser Ser Pro Pro Val Val Thr Ser Ser Ser
            260                 265                 270

His Ser Arg Ala Pro Cys Gln Ala Gly Asp Leu Arg Asp Met Ile Ser
        275                 280                 285

Met Tyr Leu Pro Gly Ala Glu Val Pro Glu Pro Ala Ala Pro Ser Arg
    290                 295                 300

Leu His Met Ser Gln His Tyr Gln Ser Gly Pro Val Pro Gly Thr Ala
305                 310                 315                 320

Ile Asn Gly Thr Leu Pro Leu Ser His Met Leu Ala Val Leu Ala Ala
                325                 330                 335

Ala Pro

<210>    89
<211>    1029
<212>    DNA
<213>    Artificial Sequence

<220>
<223>    His-NLS-Sox2 cDNA Sequence

<400>    89
atgggcagca gccatcatca tcatcatcac agcagcggcc tggtgccgcg cggcagccat        60

atgaagaaga agaggaagta caacatgatg gagacggagc tgaagccgcc gggcccgcag       120

caaacttcgg ggggcggcgg cggcaactcc accgcggcgg cggccggcgg caaccagaaa       180

aacagcccgg accgcgtcaa gcggcccatg aatgccttca tggtgtggtc ccgcgggcag       240

cggcgcaaga tggcccagga gaaccccaag atgcacaact cggagatcag caagcgcctg       300

ggcgccgagt ggaaactttt gtcggagacg gagaagcggc cgttcatcga cgaggctaag       360

cggctgcgag cgctgcacat gaaggagcac ccggattata aataccggcc ccggcggaaa       420

accaagacgc tcatgaagaa ggataagtac acgctgcccg gcgggctgct ggcccccggc 480

ggcaatagca tggcgagcgg ggtcggggtg ggcgccggcc tgggcgcggg cgtgaaccag 540

cgcatggaca gttacgcgca catgaacggc tggagcaacg gcagctacag catgatgcag 600

gaccagctgg gctacccgca gcacccgggc ctcaatgcgc acggcgcagc gcagatgcag 660

cccatgcacc gctacgacgt gagcgccctg cagtacaact ccatgaccag ctcgcagacc 720

tacatgaacg gctcgcccac ctacagcatg tcctactcgc agcagggcac ccctggcatg 780

gctcttggct ccatgggttc ggtggtcaag tccgaggcca gctccagccc ccctgtggtt 840

acctcttcct cccactccag ggcgccctgc caggccgggg acctccggga catgatcagc 900

atgtatctcc ccggcgccga ggtgccggaa cccgccgccc ccagcagact tcacatgtcc 960

cagcactacc agagcggccc ggtgcccggc acggccatta acggcacact gcccctctca 1020

cacatgtga 1029

<210> 90
<211> 342
<212> PRT
<213> Artificial Sequence

<220>
<223> His-NLS-Sox2 Amino Acid Sequence

<400> 90
Met Gly Ser Ser His His His His His His Ser Ser Gly Leu Val Pro
1               5                   10                  15

Arg Gly Ser His Met Lys Lys Lys Arg Lys Tyr Asn Met Met Glu Thr
            20                  25                  30

Glu Leu Lys Pro Pro Gly Pro Gln Gln Thr Ser Gly Gly Gly Gly Gly

35                  40                  45

Asn Ser Thr Ala Ala Ala Ala Gly Gly Asn Gln Lys Asn Ser Pro Asp
    50                  55                  60

Arg Val Lys Arg Pro Met Asn Ala Phe Met Val Trp Ser Arg Gly Gln
65                  70                  75                  80

Arg Arg Lys Met Ala Gln Glu Asn Pro Lys Met His Asn Ser Glu Ile
                85                  90                  95

Ser Lys Arg Leu Gly Ala Glu Trp Lys Leu Leu Ser Glu Thr Glu Lys
            100                 105                 110

Arg Pro Phe Ile Asp Glu Ala Lys Arg Leu Arg Ala Leu His Met Lys
        115                 120                 125

Glu His Pro Asp Tyr Lys Tyr Arg Pro Arg Arg Lys Thr Lys Thr Leu
    130                 135                 140

Met Lys Lys Asp Lys Tyr Thr Leu Pro Gly Gly Leu Leu Ala Pro Gly
145                 150                 155                 160

Gly Asn Ser Met Ala Ser Gly Val Gly Val Gly Ala Gly Leu Gly Ala
                165                 170                 175

Gly Val Asn Gln Arg Met Asp Ser Tyr Ala His Met Asn Gly Trp Ser
            180                 185                 190

Asn Gly Ser Tyr Ser Met Met Gln Asp Gln Leu Gly Tyr Pro Gln His
        195                 200                 205

Pro Gly Leu Asn Ala His Gly Ala Ala Gln Met Gln Pro Met His Arg
    210                 215                 220

Tyr Asp Val Ser Ala Leu Gln Tyr Asn Ser Met Thr Ser Ser Gln Thr
225                 230                 235                 240

Tyr Met Asn Gly Ser Pro Thr Tyr Ser Met Ser Tyr Ser Gln Gln Gly
                245                 250                 255

Thr Pro Gly Met Ala Leu Gly Ser Met Gly Ser Val Val Lys Ser Glu
            260                 265                 270

Ala Ser Ser Ser Pro Pro Val Val Thr Ser Ser Ser His Ser Arg Ala
        275                 280                 285

Pro Cys Gln Ala Gly Asp Leu Arg Asp Met Ile Ser Met Tyr Leu Pro
    290                 295                 300

Gly Ala Glu Val Pro Glu Pro Ala Ala Pro Ser Arg Leu His Met Ser
305                 310                 315                 320

Gln His Tyr Gln Ser Gly Pro Val Pro Gly Thr Ala Ile Asn Gly Thr
                325                 330                 335

Leu Pro Leu Ser His Met
            340

<210>    91
<211>    1056
<212>    DNA
<213>    Artificial Sequence

<220>
<223>    His-NLS-JO-84 MTD-Sox2 cDNA Sequence

<400>    91
atgggcagca gccatcatca tcatcatcac agcagcggcc tggtgccgcg cggcagccat        60

atgaagaaga agaggaagct ggtggcggcg ctgctggcgg tgctgtacaa catgatggag       120

acggagctga agccgccggg cccgcagcaa acttcggggg gcggcggcgg caactccacc       180

gcggcggcgg ccggcggcaa ccagaaaaac agcccggacc gcgtcaagcg gcccatgaat       240

gccttcatgg tgtggtcccg cgggcagcgg cgcaagatgg cccaggagaa ccccaagatg       300

cacaactcgg agatcagcaa gcgcctgggc gccgagtgga aacttttgtc ggagacggag 360

aagcggccgt tcatcgacga ggctaagcgg ctgcgagcgc tgcacatgaa ggagcacccg 420

gattataaat accggccccg gcggaaaacc aagacgctca tgaagaagga taagtacacg 480

ctgcccggcg ggctgctggc ccccggcggc aatagcatgg cgagcggggt cggggtgggc 540

gccggcctgg gcgcgggcgt gaaccagcgc atggacagtt acgcgcacat gaacggctgg 600

agcaacggca gctacagcat gatgcaggac cagctgggct acccgcagca cccgggcctc 660

aatgcgcacg gcgcagcgca gatgcagccc atgcaccgct acgacgtgag cgccctgcag 720

tacaactcca tgaccagctc gcagacctac atgaacggct cgcccaccta cagcatgtcc 780

tactcgcagc agggcacccc tggcatggct cttggctcca tgggttcggt ggtcaagtcc 840

gaggccagct ccagcccccc tgtggttacc tcttcctccc actccagggc gccctgccag 900

gccggggacc tccgggacat gatcagcatg tatctccccg gcgccgaggt gccggaaccc 960

gccgccccca gcagacttca catgtcccag cactaccaga gcggcccggt gcccggcacg 1020

gccattaacg gcacactgcc cctctcacac atgtga 1056

<210> 92
<211> 351
<212> PRT
<213> Artificial Sequence

<220>
<223> His-NLS-JO-84 MTD-Sox2 Amino Acid Sequence

<400> 92
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Tyr Asn Met Met Glu Thr Glu Leu Lys Pro Pro Gly Pro
        35                  40                  45

Gln Gln Thr Ser Gly Gly Gly Gly Gly Asn Ser Thr Ala Ala Ala Ala
    50                  55                  60

Gly Gly Asn Gln Lys Asn Ser Pro Asp Arg Val Lys Arg Pro Met Asn
65                  70                  75                  80

Ala Phe Met Val Trp Ser Arg Gly Gln Arg Arg Lys Met Ala Gln Glu
                85                  90                  95

Asn Pro Lys Met His Asn Ser Glu Ile Ser Lys Arg Leu Gly Ala Glu
            100                 105                 110

Trp Lys Leu Leu Ser Glu Thr Glu Lys Arg Pro Phe Ile Asp Glu Ala
        115                 120                 125

Lys Arg Leu Arg Ala Leu His Met Lys Glu His Pro Asp Tyr Lys Tyr
    130                 135                 140

Arg Pro Arg Arg Lys Thr Lys Thr Leu Met Lys Lys Asp Lys Tyr Thr
145                 150                 155                 160

Leu Pro Gly Gly Leu Leu Ala Pro Gly Gly Asn Ser Met Ala Ser Gly
                165                 170                 175

Val Gly Val Gly Ala Gly Leu Gly Ala Gly Val Asn Gln Arg Met Asp
            180                 185                 190

Ser Tyr Ala His Met Asn Gly Trp Ser Asn Gly Ser Tyr Ser Met Met
        195                 200                 205

Gln Asp Gln Leu Gly Tyr Pro Gln His Pro Gly Leu Asn Ala His Gly
    210                 215                 220

Ala Ala Gln Met Gln Pro Met His Arg Tyr Asp Val Ser Ala Leu Gln

225                 230                 235                 240

Tyr Asn Ser Met Thr Ser Ser Gln Thr Tyr Met Asn Gly Ser Pro Thr
                245                 250                 255

Tyr Ser Met Ser Tyr Ser Gln Gln Gly Thr Pro Gly Met Ala Leu Gly
            260                 265                 270

Ser Met Gly Ser Val Val Lys Ser Glu Ala Ser Ser Ser Pro Pro Val
        275                 280                 285

Val Thr Ser Ser Ser His Ser Arg Ala Pro Cys Gln Ala Gly Asp Leu
    290                 295                 300

Arg Asp Met Ile Ser Met Tyr Leu Pro Gly Ala Glu Val Pro Glu Pro
305                 310                 315                 320

Ala Ala Pro Ser Arg Leu His Met Ser Gln His Tyr Gln Ser Gly Pro
                325                 330                 335

Val Pro Gly Thr Ala Ile Asn Gly Thr Leu Pro Leu Ser His Met
            340                 345                 350

<210>   93
<211>   1056
<212>   DNA
<213>   Artificial Sequence

<220>
<223>   His-NLS-Sox2-JO-84 MTD cDNA Sequence

<400>   93
atgggcagca gccatcatca tcatcatcac agcagcggcc tggtgccgcg cggcagccat        60

atgaagaaga agaggaagta caacatgatg gagacggagc tgaagccgcc gggcccgcag       120

caaacttcgg ggggcggcgg cggcaactcc accgcggcgg cggccggcgg caaccagaaa 180

aacagcccgg accgcgtcaa gcggcccatg aatgccttca tggtgtggtc ccgcgggcag 240

cggcgcaaga tggcccagga gaaccccaag atgcacaact cggagatcag caagcgcctg 300

ggcgccgagt ggaaactttt gtcggagacg gagaagcggc cgttcatcga cgaggctaag 360

cggctgcgag cgctgcacat gaaggagcac ccggattata aataccggcc ccggcggaaa 420

accaagacgc tcatgaagaa ggataagtac acgctgcccg gcgggctgct ggcccccggc 480

ggcaatagca tggcgagcgg ggtcggggtg ggcgccggcc tgggcgcggg cgtgaaccag 540

cgcatggaca gttacgcgca catgaacggc tggagcaacg gcagctacag catgatgcag 600

gaccagctgg gctacccgca gcacccgggc ctcaatgcgc acggcgcagc gcagatgcag 660

cccatgcacc gctacgacgt gagcgccctg cagtacaact ccatgaccag ctcgcagacc 720

tacatgaacg gctcgcccac ctacagcatg tcctactcgc agcagggcac ccctggcatg 780

gctcttggct ccatgggttc ggtggtcaag tccgaggcca gctccagccc ccctgtggtt 840

acctcttcct cccactccag ggcgccctgc caggccgggg acctccggga catgatcagc 900

atgtatctcc ccggcgccga ggtgccggaa cccgccgccc ccagcagact tcacatgtcc 960

cagcactacc agagcggccc ggtgcccggc acggccatta acggcacact gcccctctca 1020

cacatgctgg tggcggcgct gctggcggtg ctgtga 1056

<210> 94
<211> 351
<212> PRT
<213> Artificial Sequence

<220>
<223> His-NLS-Sox2-JO-84 MTD Amino Acid Sequence

<400>    94

Met Gly Ser Ser His His His His His His Ser Ser Gly Leu Val Pro
1               5                   10                  15

Arg Gly Ser His Met Lys Lys Lys Arg Lys Tyr Asn Met Met Glu Thr
            20                  25                  30

Glu Leu Lys Pro Pro Gly Pro Gln Gln Thr Ser Gly Gly Gly Gly Gly
        35                  40                  45

Asn Ser Thr Ala Ala Ala Ala Gly Gly Asn Gln Lys Asn Ser Pro Asp
    50                  55                  60

Arg Val Lys Arg Pro Met Asn Ala Phe Met Val Trp Ser Arg Gly Gln
65                  70                  75                  80

Arg Arg Lys Met Ala Gln Glu Asn Pro Lys Met His Asn Ser Glu Ile
                85                  90                  95

Ser Lys Arg Leu Gly Ala Glu Trp Lys Leu Leu Ser Glu Thr Glu Lys
            100                 105                 110

Arg Pro Phe Ile Asp Glu Ala Lys Arg Leu Arg Ala Leu His Met Lys
        115                 120                 125

Glu His Pro Asp Tyr Lys Tyr Arg Pro Arg Arg Lys Thr Lys Thr Leu
    130                 135                 140

Met Lys Lys Asp Lys Tyr Thr Leu Pro Gly Gly Leu Leu Ala Pro Gly
145                 150                 155                 160

Gly Asn Ser Met Ala Ser Gly Val Gly Val Gly Ala Gly Leu Gly Ala
                165                 170                 175

Gly Val Asn Gln Arg Met Asp Ser Tyr Ala His Met Asn Gly Trp Ser
            180                 185                 190

Asn Gly Ser Tyr Ser Met Met Gln Asp Gln Leu Gly Tyr Pro Gln His

195                 200                 205

Pro Gly Leu Asn Ala His Gly Ala Ala Gln Met Gln Pro Met His Arg
    210                 215                 220

Tyr Asp Val Ser Ala Leu Gln Tyr Asn Ser Met Thr Ser Ser Gln Thr
225                 230                 235                 240

Tyr Met Asn Gly Ser Pro Thr Tyr Ser Met Ser Tyr Ser Gln Gln Gly
                245                 250                 255

Thr Pro Gly Met Ala Leu Gly Ser Met Gly Ser Val Val Lys Ser Glu
            260                 265                 270

Ala Ser Ser Ser Pro Pro Val Val Thr Ser Ser Ser His Ser Arg Ala
        275                 280                 285

Pro Cys Gln Ala Gly Asp Leu Arg Asp Met Ile Ser Met Tyr Leu Pro
    290                 295                 300

Gly Ala Glu Val Pro Glu Pro Ala Ala Pro Ser Arg Leu His Met Ser
305                 310                 315                 320

Gln His Tyr Gln Ser Gly Pro Val Pro Gly Thr Ala Ile Asn Gly Thr
                325                 330                 335

Leu Pro Leu Ser His Met Leu Val Ala Ala Leu Leu Ala Val Leu
            340                 345                 350

<210>   95
<211>   1083
<212>   DNA
<213>   Artificial Sequence

<220>
<223>   His-NLS-JO-84 MTD-Sox2-JO-84 MTD cDNA Sequence

<400>    95

atgggcagca gccatcatca tcatcatcac agcagcggcc tggtgccgcg cggcagccat        60

atgaagaaga agaggaagct ggtggcggcg ctgctggcgg tgctgtacaa catgatggag       120

acggagctga agccgccggg cccgcagcaa acttcggggg gcggcggcgg caactccacc       180

gcggcggcgg ccggcggcaa ccagaaaaac agcccggacc gcgtcaagcg gcccatgaat       240

gccttcatgg tgtggtcccg cgggcagcgg cgcaagatgg cccaggagaa ccccaagatg       300

cacaactcgg agatcagcaa gcgcctgggc gccgagtgga aacttttgtc ggagacggag       360

aagcggccgt tcatcgacga ggctaagcgg ctgcgagcgc tgcacatgaa ggagcacccg       420

gattataaat accggccccg gcggaaaacc aagacgctca tgaagaagga taagtacacg       480

ctgcccggcg ggctgctggc ccccggcggc aatagcatgg cgagcggggt cggggtgggc       540

gccggcctgg gcgcgggcgt gaaccagcgc atggacagtt acgcgcacat gaacggctgg       600

agcaacggca gctacagcat gatgcaggac cagctgggct acccgcagca cccgggcctc       660

aatgcgcacg gcgcagcgca gatgcagccc atgcaccgct acgacgtgag cgccctgcag       720

tacaactcca tgaccagctc gcagacctac atgaacggct cgcccaccta cagcatgtcc       780

tactcgcagc agggcacccc tggcatggct cttggctcca tgggttcggt ggtcaagtcc       840

gaggccagct ccagcccccc tgtggttacc tcttcctccc actccagggc gccctgccag       900

gccggggacc tccgggacat gatcagcatg tatctccccg gcgccgaggt gccggaaccc       960

gccgccccca gcagacttca catgtcccag cactaccaga gcggcccggt gcccggcacg      1020

gccattaacg gcacactgcc cctctcacac atgctggtgg cggcgctgct ggcggtgctg      1080

tga                                                                    1083

<210> 96
<211> 360
<212> PRT
<213> Artificial Sequence

<220>
<223> His-NLS-JO-84 MTD-Sox2-JO-84 MTD Amino Acid Sequence

<400> 96
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Tyr Asn Met Met Glu Thr Glu Leu Lys Pro Pro Gly Pro
        35                  40                  45

Gln Gln Thr Ser Gly Gly Gly Gly Gly Asn Ser Thr Ala Ala Ala Ala
    50                  55                  60

Gly Gly Asn Gln Lys Asn Ser Pro Asp Arg Val Lys Arg Pro Met Asn
65                  70                  75                  80

Ala Phe Met Val Trp Ser Arg Gly Gln Arg Arg Lys Met Ala Gln Glu
                85                  90                  95

Asn Pro Lys Met His Asn Ser Glu Ile Ser Lys Arg Leu Gly Ala Glu
            100                 105                 110

Trp Lys Leu Leu Ser Glu Thr Glu Lys Arg Pro Phe Ile Asp Glu Ala
        115                 120                 125

Lys Arg Leu Arg Ala Leu His Met Lys Glu His Pro Asp Tyr Lys Tyr
    130                 135                 140

Arg Pro Arg Arg Lys Thr Lys Thr Leu Met Lys Lys Asp Lys Tyr Thr
145                 150                 155                 160

Leu Pro Gly Gly Leu Leu Ala Pro Gly Gly Asn Ser Met Ala Ser Gly
                165                 170                 175

Val Gly Val Gly Ala Gly Leu Gly Ala Gly Val Asn Gln Arg Met Asp
            180                 185                 190

Ser Tyr Ala His Met Asn Gly Trp Ser Asn Gly Ser Tyr Ser Met Met
        195                 200                 205

Gln Asp Gln Leu Gly Tyr Pro Gln His Pro Gly Leu Asn Ala His Gly
    210                 215                 220

Ala Ala Gln Met Gln Pro Met His Arg Tyr Asp Val Ser Ala Leu Gln
225                 230                 235                 240

Tyr Asn Ser Met Thr Ser Ser Gln Thr Tyr Met Asn Gly Ser Pro Thr
                245                 250                 255

Tyr Ser Met Ser Tyr Ser Gln Gln Gly Thr Pro Gly Met Ala Leu Gly
            260                 265                 270

Ser Met Gly Ser Val Val Lys Ser Glu Ala Ser Ser Ser Pro Pro Val
        275                 280                 285

Val Thr Ser Ser Ser His Ser Arg Ala Pro Cys Gln Ala Gly Asp Leu
    290                 295                 300

Arg Asp Met Ile Ser Met Tyr Leu Pro Gly Ala Glu Val Pro Glu Pro
305                 310                 315                 320

Ala Ala Pro Ser Arg Leu His Met Ser Gln His Tyr Gln Ser Gly Pro
                325                 330                 335

Val Pro Gly Thr Ala Ile Asn Gly Thr Leu Pro Leu Ser His Met Leu
            340                 345                 350

Val Ala Ala Leu Leu Ala Val Leu
        355                 360

<210> 97
<211> 1053
<212> DNA
<213> Artificial Sequence

<220>
<223> His-NLS-JO-86 MTD-Sox2 cDNA Sequence

<400> 97
atgggcagca gccatcatca tcatcatcac agcagcggcc tggtgccgcg cggcagccat 60

atgaagaaga agaggaagct ggcggtgctg gcggcggcgc cgtacaacat gatggagacg 120

gagctgaagc cgccgggccc gcagcaaact tcggggggcg gcggcggcaa ctccaccgcg 180

gcggcggccg gcggcaacca gaaaaacagc ccggaccgcg tcaagcggcc catgaatgcc 240

ttcatggtgt ggtcccgcgg gcagcggcgc aagatggccc aggagaaccc caagatgcac 300

aactcggaga tcagcaagcg cctgggcgcc gagtggaaac ttttgtcgga gacggagaag 360

cggccgttca tcgacgaggc taagcggctg cgagcgctgc acatgaagga gcacccggat 420

tataaatacc ggccccggcg gaaaaccaag acgctcatga agaaggataa gtacacgctg 480

cccggcgggc tgctggcccc cggcggcaat agcatggcga gcggggtcgg ggtgggcgcc 540

ggcctgggcg cgggcgtgaa ccagcgcatg gacagttacg cgcacatgaa cggctggagc 600

aacggcagct acagcatgat gcaggaccag ctgggctacc cgcagcaccc gggcctcaat 660

gcgcacggcg cagcgcagat gcagcccatg caccgctacg acgtgagcgc cctgcagtac 720

aactccatga ccagctcgca gacctacatg aacggctcgc ccacctacag catgtcctac 780

tcgcagcagg gcacccctgg catggctctt ggctccatgg gttcggtggt caagtccgag 840

gccagctcca gcccccctgt ggttacctct tcctcccact ccagggcgcc ctgccaggcc 900

ggggacctcc gggacatgat cagcatgtat ctccccggcg ccgaggtgcc ggaacccgcc 960

gcccccagca gacttcacat gtcccagcac taccagagcg gcccggtgcc cggcacggcc 1020

attaacggca cactgcccct ctcacacatg tga 1053

<210> 98
<211> 350
<212> PRT
<213> Artificial Sequence

<220>
<223> His-NLS-JO-86 MTD-Sox2 Amino Acid Sequence

<400> 98
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Tyr Asn Met Met Glu Thr Glu Leu Lys Pro Pro Gly Pro Gln
        35                  40                  45

Gln Thr Ser Gly Gly Gly Gly Gly Asn Ser Thr Ala Ala Ala Ala Gly
    50                  55                  60

Gly Asn Gln Lys Asn Ser Pro Asp Arg Val Lys Arg Pro Met Asn Ala
65                  70                  75                  80

Phe Met Val Trp Ser Arg Gly Gln Arg Arg Lys Met Ala Gln Glu Asn
                85                  90                  95

Pro Lys Met His Asn Ser Glu Ile Ser Lys Arg Leu Gly Ala Glu Trp
            100                 105                 110

Lys Leu Leu Ser Glu Thr Glu Lys Arg Pro Phe Ile Asp Glu Ala Lys

        115                 120                 125

Arg Leu Arg Ala Leu His Met Lys Glu His Pro Asp Tyr Lys Tyr Arg
    130                 135                 140

Pro Arg Arg Lys Thr Lys Thr Leu Met Lys Lys Asp Lys Tyr Thr Leu
145                 150                 155                 160

Pro Gly Gly Leu Leu Ala Pro Gly Gly Asn Ser Met Ala Ser Gly Val
                165                 170                 175

Gly Val Gly Ala Gly Leu Gly Ala Gly Val Asn Gln Arg Met Asp Ser
            180                 185                 190

Tyr Ala His Met Asn Gly Trp Ser Asn Gly Ser Tyr Ser Met Met Gln
        195                 200                 205

Asp Gln Leu Gly Tyr Pro Gln His Pro Gly Leu Asn Ala His Gly Ala
    210                 215                 220

Ala Gln Met Gln Pro Met His Arg Tyr Asp Val Ser Ala Leu Gln Tyr
225                 230                 235                 240

Asn Ser Met Thr Ser Ser Gln Thr Tyr Met Asn Gly Ser Pro Thr Tyr
                245                 250                 255

Ser Met Ser Tyr Ser Gln Gln Gly Thr Pro Gly Met Ala Leu Gly Ser
            260                 265                 270

Met Gly Ser Val Val Lys Ser Glu Ala Ser Ser Ser Pro Pro Val Val
        275                 280                 285

Thr Ser Ser Ser His Ser Arg Ala Pro Cys Gln Ala Gly Asp Leu Arg
    290                 295                 300

Asp Met Ile Ser Met Tyr Leu Pro Gly Ala Glu Val Pro Glu Pro Ala
305                 310                 315                 320

Ala Pro Ser Arg Leu His Met Ser Gln His Tyr Gln Ser Gly Pro Val
                325                 330                 335

Pro Gly Thr Ala Ile Asn Gly Thr Leu Pro Leu Ser His Met
            340                 345                 350

<210> 99
<211> 1053
<212> DNA
<213> Artificial Sequence

<220>
<223> His-NLS-Sox2-JO-86 MTD cDNA Sequence

<400> 99
atgggcagca gccatcatca tcatcatcac agcagcggcc tggtgccgcg cggcagccat 60

atgaagaaga agaggaagta caacatgatg gagacggagc tgaagccgcc gggcccgcag 120

caaacttcgg ggggcggcgg cggcaactcc accgcggcgg cggccggcgg caaccagaaa 180

aacagcccgg accgcgtcaa gcggcccatg aatgccttca tggtgtggtc ccgcgggcag 240

cggcgcaaga tggcccagga gaaccccaag atgcacaact cggagatcag caagcgcctg 300

ggcgccgagt ggaaactttt gtcggagacg gagaagcggc cgttcatcga cgaggctaag 360

cggctgcgag cgctgcacat gaaggagcac ccggattata aataccggcc ccggcggaaa 420

accaagacgc tcatgaagaa ggataagtac acgctgcccg gcgggctgct ggcccccggc 480

ggcaatagca tggcgagcgg ggtcggggtg ggcgccggcc tgggcgcggg cgtgaaccag 540

cgcatggaca gttacgcgca catgaacggc tggagcaacg gcagctacag catgatgcag 600

gaccagctgg gctacccgca gcacccgggc ctcaatgcgc acggcgcagc gcagatgcag 660

cccatgcacc gctacgacgt gagcgccctg cagtacaact ccatgaccag ctcgcagacc 720

tacatgaacg gctcgcccac ctacagcatg tcctactcgc agcagggcac ccctggcatg 780

gctcttggct ccatgggttc ggtggtcaag tccgaggcca gctccagccc ccctgtggtt 840

acctcttcct cccactccag ggcgccctgc caggccgggg acctccggga catgatcagc 900

atgtatctcc ccggcgccga ggtgccggaa cccgccgccc ccagcagact tcacatgtcc 960

cagcactacc agagcggccc ggtgcccggc acggccatta acggcacact gcccctctca 1020

cacatgctgg cggtgctggc ggcggcgccg tga 1053

<210> 100
<211> 350
<212> PRT
<213> Artificial Sequence

<220>
<223> His-NLS-Sox2-JO-86 MTD Amino Acid Sequence

<400> 100
Met Gly Ser Ser His His His His His His Ser Ser Gly Leu Val Pro
1               5                   10                  15

Arg Gly Ser His Met Lys Lys Lys Arg Lys Tyr Asn Met Met Glu Thr
            20                  25                  30

Glu Leu Lys Pro Pro Gly Pro Gln Gln Thr Ser Gly Gly Gly Gly Gly
        35                  40                  45

Asn Ser Thr Ala Ala Ala Ala Gly Gly Asn Gln Lys Asn Ser Pro Asp
    50                  55                  60

Arg Val Lys Arg Pro Met Asn Ala Phe Met Val Trp Ser Arg Gly Gln
65                  70                  75                  80

Arg Arg Lys Met Ala Gln Glu Asn Pro Lys Met His Asn Ser Glu Ile
                85                  90                  95

Ser Lys Arg Leu Gly Ala Glu Trp Lys Leu Leu Ser Glu Thr Glu Lys
            100                 105                 110

Arg Pro Phe Ile Asp Glu Ala Lys Arg Leu Arg Ala Leu His Met Lys
        115                 120                 125

Glu His Pro Asp Tyr Lys Tyr Arg Pro Arg Arg Lys Thr Lys Thr Leu
    130                 135                 140

Met Lys Lys Asp Lys Tyr Thr Leu Pro Gly Gly Leu Leu Ala Pro Gly
145                 150                 155                 160

Gly Asn Ser Met Ala Ser Gly Val Gly Val Gly Ala Gly Leu Gly Ala
                165                 170                 175

Gly Val Asn Gln Arg Met Asp Ser Tyr Ala His Met Asn Gly Trp Ser
            180                 185                 190

Asn Gly Ser Tyr Ser Met Met Gln Asp Gln Leu Gly Tyr Pro Gln His
        195                 200                 205

Pro Gly Leu Asn Ala His Gly Ala Ala Gln Met Gln Pro Met His Arg
    210                 215                 220

Tyr Asp Val Ser Ala Leu Gln Tyr Asn Ser Met Thr Ser Ser Gln Thr
225                 230                 235                 240

Tyr Met Asn Gly Ser Pro Thr Tyr Ser Met Ser Tyr Ser Gln Gln Gly
                245                 250                 255

Thr Pro Gly Met Ala Leu Gly Ser Met Gly Ser Val Val Lys Ser Glu
            260                 265                 270

Ala Ser Ser Ser Pro Pro Val Val Thr Ser Ser Ser His Ser Arg Ala
        275                 280                 285

Pro Cys Gln Ala Gly Asp Leu Arg Asp Met Ile Ser Met Tyr Leu Pro
    290                 295                 300

Gly Ala Glu Val Pro Glu Pro Ala Ala Pro Ser Arg Leu His Met Ser

305                 310                 315                 320

Gln His Tyr Gln Ser Gly Pro Val Pro Gly Thr Ala Ile Asn Gly Thr
                325                 330                 335

Leu Pro Leu Ser His Met Leu Ala Val Leu Ala Ala Ala Pro
            340                 345                 350

<210>   101
<211>   1077
<212>   DNA
<213>   Artificial Sequence

<220>
<223>   His-NLS-JO-86 MTD-Sox2-JO-86 MTD cDNA Sequence

<400>   101
atgggcagca gccatcatca tcatcatcac agcagcggcc tggtgccgcg cggcagccat      60

atgaagaaga agaggaagct ggcggtgctg gcggcggcgc cgtacaacat gatggagacg     120

gagctgaagc cgccgggccc gcagcaaact tcggggggcg gcggcggcaa ctccaccgcg     180

gcggcggccg gcggcaacca gaaaaacagc ccggaccgcg tcaagcggcc catgaatgcc     240

ttcatggtgt ggtcccgcgg gcagcggcgc aagatggccc aggagaaccc caagatgcac     300

aactcggaga tcagcaagcg cctgggcgcc gagtggaaac ttttgtcgga gacggagaag     360

cggccgttca tcgacgaggc taagcggctg cgagcgctgc acatgaagga gcacccggat     420

tataaatacc ggccccggcg gaaaaccaag acgctcatga agaaggataa gtacacgctg     480

cccggcgggc tgctggcccc cggcggcaat agcatggcga gcggggtcgg ggtgggcgcc     540

ggcctgggcg cgggcgtgaa ccagcgcatg gacagttacg cgcacatgaa cggctggagc     600

aacggcagct acagcatgat gcaggaccag ctgggctacc cgcagcaccc gggcctcaat     660

gcgcacggcg cagcgcagat gcagcccatg caccgctacg acgtgagcgc cctgcagtac 720

aactccatga ccagctcgca gacctacatg aacggctcgc ccacctacag catgtcctac 780

tcgcagcagg gcacccctgg catggctctt ggctccatgg gttcggtggt caagtccgag 840

gccagctcca gcccccctgt ggttacctct tcctcccact ccagggcgcc ctgccaggcc 900

ggggacctcc gggacatgat cagcatgtat ctccccggcg ccgaggtgcc ggaacccgcc 960

gcccccagca gacttcacat gtcccagcac taccagagcg gcccggtgcc cggcacggcc 1020

attaacggca cactgcccct ctcacacatg ctggcggtgc tggcggcggc gccgtga 1077

<210> 102
<211> 358
<212> PRT
<213> Artificial Sequence

<220>
<223> His-NLS-JO-86 MTD-Sox2-JO-86 MTD Amino Acid Sequence

<400> 102
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Tyr Asn Met Met Glu Thr Glu Leu Lys Pro Pro Gly Pro Gln
        35                  40                  45

Gln Thr Ser Gly Gly Gly Gly Gly Asn Ser Thr Ala Ala Ala Ala Gly
    50                  55                  60

Gly Asn Gln Lys Asn Ser Pro Asp Arg Val Lys Arg Pro Met Asn Ala
65                  70                  75                  80

Phe Met Val Trp Ser Arg Gly Gln Arg Arg Lys Met Ala Gln Glu Asn
                85                  90                  95

Pro Lys Met His Asn Ser Glu Ile Ser Lys Arg Leu Gly Ala Glu Trp
            100                 105                 110

Lys Leu Leu Ser Glu Thr Glu Lys Arg Pro Phe Ile Asp Glu Ala Lys
        115                 120                 125

Arg Leu Arg Ala Leu His Met Lys Glu His Pro Asp Tyr Lys Tyr Arg
    130                 135                 140

Pro Arg Arg Lys Thr Lys Thr Leu Met Lys Lys Asp Lys Tyr Thr Leu
145                 150                 155                 160

Pro Gly Gly Leu Leu Ala Pro Gly Gly Asn Ser Met Ala Ser Gly Val
                165                 170                 175

Gly Val Gly Ala Gly Leu Gly Ala Gly Val Asn Gln Arg Met Asp Ser
            180                 185                 190

Tyr Ala His Met Asn Gly Trp Ser Asn Gly Ser Tyr Ser Met Met Gln
        195                 200                 205

Asp Gln Leu Gly Tyr Pro Gln His Pro Gly Leu Asn Ala His Gly Ala
    210                 215                 220

Ala Gln Met Gln Pro Met His Arg Tyr Asp Val Ser Ala Leu Gln Tyr
225                 230                 235                 240

Asn Ser Met Thr Ser Ser Gln Thr Tyr Met Asn Gly Ser Pro Thr Tyr
                245                 250                 255

Ser Met Ser Tyr Ser Gln Gln Gly Thr Pro Gly Met Ala Leu Gly Ser
            260                 265                 270

Met Gly Ser Val Val Lys Ser Glu Ala Ser Ser Ser Pro Pro Val Val
        275                 280                 285

Thr Ser Ser Ser His Ser Arg Ala Pro Cys Gln Ala Gly Asp Leu Arg
    290                 295                 300

Asp Met Ile Ser Met Tyr Leu Pro Gly Ala Glu Val Pro Glu Pro Ala
305                 310                 315                 320

Ala Pro Ser Arg Leu His Met Ser Gln His Tyr Gln Ser Gly Pro Val
                325                 330                 335

Pro Gly Thr Ala Ile Asn Gly Thr Leu Pro Leu Ser His Met Leu Ala
            340                 345                 350

Val Leu Ala Ala Ala Pro
        355

<210>    103
<211>    1428
<212>    DNA
<213>    Artificial Sequence

<220>
<223>    NLS-Klf4 cDNA Sequence

<400>    103
atgaagaaga agaggaaggc tgtcagcgac gcgctgctcc catctttctc cacgttcgcg        60

tctggcccgg cgggaaggga gaagacactg cgtcaagcag gtgccccgaa taaccgctgg       120

cgggaggagc tctcccacat gaagcgactt cccccagtgc ttcccggccg cccctatgac       180

ctggcggcgg cgaccgtggc cacagacctg gagagcggcg gagccggtgc ggcttgcggc       240

ggtagcaacc tggcgcccct acctcggaga gagaccgagg agttcaacga tctcctggac       300

ctggacttta ttctctccaa ttcgctgacc catcctccgg agtcagtggc cgccaccgtg       360

tcctcgtcag cgtcagcctc ctcttcgtcg tcgccgtcga gcagcggccc tgccagcgcg       420

ccctccacct gcagcttcac ctatccgatc cgggccggga acgacccggg cgtggcgccg 480

ggcggcacgg gcggaggcct cctctatggc agggagtccg ctccccctcc gacggctccc 540

ttcaacctgg cggacatcaa cgacgtgagc ccctcgggcg gcttcgtggc cgagctcctg 600

cggccagaat tggacccggt gtacattccg ccgcagcagc cgcagccgcc aggtggcggg 660

ctgatgggca agttcgtgct gaaggcgtcg ctgagcgccc ctggcagcga gtacggcagc 720

ccgtcggtca tcagcgtcag caaaggcagc cctgacggca gccacccggt ggtggtggcg 780

ccctacaacg gcgggccgcc gcgcacgtgc cccaagatca agcaggaggc ggtctcttcg 840

tgcacccact tgggcgctgg accccctctc agcaatggcc accggccggc tgcacacgac 900

ttccccctgg ggcggcagct ccccagcagg actaccccga ccctgggtct tgaggaagtg 960

ctgagcagca gggactgtca ccctgccctg ccgcttcctc ccggcttcca tccccacccg 1020

gggcccaatt acccatcctt cctgcccgat cagatgcagc cgcaagtccc gccgctccat 1080

taccaagagc tcatgccacc cggttcctgc atgccagagg agcccaagcc aaagagggga 1140

agacgatcgt ggccccggaa aaggaccgcc acccacactt gtgattacgc gggctgcggc 1200

aaaacctaca caaagagttc ccatctcaag gcacacctgc gaacccacac aggtgagaaa 1260

ccttaccact gtgactggga cggctgtgga tggaaattcg cccgctcaga tgaactgacc 1320

aggcactacc gtaaacacac ggggcaccgc ccgttccagt gccaaaaatg cgaccgagca 1380

ttttccaggt cggaccacct cgccttacac atgaagaggc atttttaa 1428

<210> 104
<211> 475
<212> PRT
<213> Artificial Sequence

<220>
<223>    NLS-Klf4 Amino Acid Sequence

<400>    104
Met Lys Lys Lys Arg Lys Ala Val Ser Asp Ala Leu Leu Pro Ser Phe
1               5                   10                  15

Ser Thr Phe Ala Ser Gly Pro Ala Gly Arg Glu Lys Thr Leu Arg Gln
            20                  25                  30

Ala Gly Ala Pro Asn Asn Arg Trp Arg Glu Glu Leu Ser His Met Lys
        35                  40                  45

Arg Leu Pro Pro Val Leu Pro Gly Arg Pro Tyr Asp Leu Ala Ala Ala
    50                  55                  60

Thr Val Ala Thr Asp Leu Glu Ser Gly Gly Ala Gly Ala Ala Cys Gly
65                  70                  75                  80

Gly Ser Asn Leu Ala Pro Leu Pro Arg Arg Glu Thr Glu Glu Phe Asn
                85                  90                  95

Asp Leu Leu Asp Leu Asp Phe Ile Leu Ser Asn Ser Leu Thr His Pro
            100                 105                 110

Pro Glu Ser Val Ala Ala Thr Val Ser Ser Ser Ala Ser Ala Ser Ser
        115                 120                 125

Ser Ser Ser Pro Ser Ser Ser Gly Pro Ala Ser Ala Pro Ser Thr Cys
    130                 135                 140

Ser Phe Thr Tyr Pro Ile Arg Ala Gly Asn Asp Pro Gly Val Ala Pro
145                 150                 155                 160

Gly Gly Thr Gly Gly Gly Leu Leu Tyr Gly Arg Glu Ser Ala Pro Pro
                165                 170                 175

Pro Thr Ala Pro Phe Asn Leu Ala Asp Ile Asn Asp Val Ser Pro Ser
            180                 185                 190

Gly Gly Phe Val Ala Glu Leu Leu Arg Pro Glu Leu Asp Pro Val Tyr
        195                 200                 205

Ile Pro Pro Gln Gln Pro Gln Pro Pro Gly Gly Gly Leu Met Gly Lys
    210                 215                 220

Phe Val Leu Lys Ala Ser Leu Ser Ala Pro Gly Ser Glu Tyr Gly Ser
225                 230                 235                 240

Pro Ser Val Ile Ser Val Ser Lys Gly Ser Pro Asp Gly Ser His Pro
                245                 250                 255

Val Val Val Ala Pro Tyr Asn Gly Gly Pro Pro Arg Thr Cys Pro Lys
            260                 265                 270

Ile Lys Gln Glu Ala Val Ser Ser Cys Thr His Leu Gly Ala Gly Pro
        275                 280                 285

Pro Leu Ser Asn Gly His Arg Pro Ala Ala His Asp Phe Pro Leu Gly
    290                 295                 300

Arg Gln Leu Pro Ser Arg Thr Thr Pro Thr Leu Gly Leu Glu Glu Val
305                 310                 315                 320

Leu Ser Ser Arg Asp Cys His Pro Ala Leu Pro Leu Pro Pro Gly Phe
                325                 330                 335

His Pro His Pro Gly Pro Asn Tyr Pro Ser Phe Leu Pro Asp Gln Met
            340                 345                 350

Gln Pro Gln Val Pro Pro Leu His Tyr Gln Glu Leu Met Pro Pro Gly
        355                 360                 365

Ser Cys Met Pro Glu Glu Pro Lys Pro Lys Arg Gly Arg Arg Ser Trp
    370                 375                 380

Pro Arg Lys Arg Thr Ala Thr His Thr Cys Asp Tyr Ala Gly Cys Gly
385                 390                 395                 400

Lys Thr Tyr Thr Lys Ser Ser His Leu Lys Ala His Leu Arg Thr His
                405                 410                 415

Thr Gly Glu Lys Pro Tyr His Cys Asp Trp Asp Gly Cys Gly Trp Lys
            420                 425                 430

Phe Ala Arg Ser Asp Glu Leu Thr Arg His Tyr Arg Lys His Thr Gly
        435                 440                 445

His Arg Pro Phe Gln Cys Gln Lys Cys Asp Arg Ala Phe Ser Arg Ser
    450                 455                 460

Asp His Leu Ala Leu His Met Lys Arg His Phe
465                 470                 475

<210> 105
<211> 1455
<212> DNA
<213> Artificial Sequence

<220>
<223> NLS-JO-84 MTD-Klf4 cDNA Sequence

<400> 105
atgaagaaga agaggaagct ggtggcggcg ctgctggcgg tgctggctgt cagcgacgcg 60

ctgctcccat ctttctccac gttcgcgtct ggcccggcgg gaagggagaa gacactgcgt 120

caagcaggtg ccccgaataa ccgctggcgg gaggagctct cccacatgaa gcgacttccc 180

ccagtgcttc ccggccgccc ctatgacctg gcggcggcga ccgtggccac agacctggag 240

agcggcggag ccggtgcggc ttgcggcggt agcaacctgg cgcccctacc tcggagagag 300

accgaggagt tcaacgatct cctggacctg gactttattc tctccaattc gctgacccat 360

cctccggagt cagtggccgc caccgtgtcc tcgtcagcgt cagcctcctc ttcgtcgtcg 420

ccgtcgagca gcggccctgc cagcgcgccc tccacctgca gcttcaccta tccgatccgg 480

gccgggaacg acccgggcgt ggcgccgggc ggcacgggcg gaggcctcct ctatggcagg 540

gagtccgctc cccctccgac ggctcccttc aacctggcgg acatcaacga cgtgagcccc 600

tcgggcggct tcgtggccga gctcctgcgg ccagaattgg acccggtgta cattccgccg 660

cagcagccgc agccgccagg tggcgggctg atgggcaagt tcgtgctgaa ggcgtcgctg 720

agcgcccctg gcagcgagta cggcagcccg tcggtcatca gcgtcagcaa aggcagccct 780

gacggcagcc acccggtggt ggtggcgccc tacaacggcg ggccgccgcg cacgtgcccc 840

aagatcaagc aggaggcggt ctcttcgtgc acccacttgg gcgctggacc ccctctcagc 900

aatggccacc ggccggctgc acacgacttc cccctggggc ggcagctccc cagcaggact 960

accccgaccc tgggtcttga ggaagtgctg agcagcaggg actgtcaccc tgccctgccg 1020

cttcctcccg gcttccatcc ccacccgggg cccaattacc catccttcct gcccgatcag 1080

atgcagccgc aagtcccgcc gctccattac caagagctca tgccacccgg ttcctgcatg 1140

ccagaggagc ccaagccaaa gaggggaaga cgatcgtggc cccggaaaag gaccgccacc 1200

cacacttgtg attacgcggg ctgcggcaaa acctacacaa agagttccca tctcaaggca 1260

cacctgcgaa cccacacagg tgagaaacct taccactgtg actgggacgg ctgtggatgg 1320

aaattcgccc gctcagatga actgaccagg cactaccgta aacacacggg gcaccgcccg 1380

ttccagtgcc aaaaatgcga ccgagcattt tccaggtcgg accacctcgc cttacacatg 1440

aagaggcatt tttaa 1455

<210> 106
<211> 484
<212> PRT

<213>    Artificial Sequence

<220>
<223>    NLS-JO-84 MTD-Klf4 Amino Acid Sequence

<400>    106
Met Lys Lys Lys Arg Lys Leu Val Ala Ala Leu Leu Ala Val Leu Ala
1               5                   10                  15

Val Ser Asp Ala Leu Leu Pro Ser Phe Ser Thr Phe Ala Ser Gly Pro
            20                  25                  30

Ala Gly Arg Glu Lys Thr Leu Arg Gln Ala Gly Ala Pro Asn Asn Arg
        35                  40                  45

Trp Arg Glu Glu Leu Ser His Met Lys Arg Leu Pro Pro Val Leu Pro
    50                  55                  60

Gly Arg Pro Tyr Asp Leu Ala Ala Ala Thr Val Ala Thr Asp Leu Glu
65                  70                  75                  80

Ser Gly Gly Ala Gly Ala Ala Cys Gly Gly Ser Asn Leu Ala Pro Leu
                85                  90                  95

Pro Arg Arg Glu Thr Glu Glu Phe Asn Asp Leu Leu Asp Leu Asp Phe
            100                 105                 110

Ile Leu Ser Asn Ser Leu Thr His Pro Pro Glu Ser Val Ala Ala Thr
        115                 120                 125

Val Ser Ser Ser Ala Ser Ala Ser Ser Ser Ser Ser Pro Ser Ser Ser
    130                 135                 140

Gly Pro Ala Ser Ala Pro Ser Thr Cys Ser Phe Thr Tyr Pro Ile Arg
145                 150                 155                 160

Ala Gly Asn Asp Pro Gly Val Ala Pro Gly Gly Thr Gly Gly Gly Leu
                165                 170                 175

Leu Tyr Gly Arg Glu Ser Ala Pro Pro Pro Thr Ala Pro Phe Asn Leu
            180                 185                 190

Ala Asp Ile Asn Asp Val Ser Pro Ser Gly Gly Phe Val Ala Glu Leu
        195                 200                 205

Leu Arg Pro Glu Leu Asp Pro Val Tyr Ile Pro Pro Gln Gln Pro Gln
    210                 215                 220

Pro Pro Gly Gly Gly Leu Met Gly Lys Phe Val Leu Lys Ala Ser Leu
225                 230                 235                 240

Ser Ala Pro Gly Ser Glu Tyr Gly Ser Pro Ser Val Ile Ser Val Ser
                245                 250                 255

Lys Gly Ser Pro Asp Gly Ser His Pro Val Val Val Ala Pro Tyr Asn
            260                 265                 270

Gly Gly Pro Pro Arg Thr Cys Pro Lys Ile Lys Gln Glu Ala Val Ser
        275                 280                 285

Ser Cys Thr His Leu Gly Ala Gly Pro Pro Leu Ser Asn Gly His Arg
    290                 295                 300

Pro Ala Ala His Asp Phe Pro Leu Gly Arg Gln Leu Pro Ser Arg Thr
305                 310                 315                 320

Thr Pro Thr Leu Gly Leu Glu Glu Val Leu Ser Ser Arg Asp Cys His
                325                 330                 335

Pro Ala Leu Pro Leu Pro Pro Gly Phe His Pro His Pro Gly Pro Asn
            340                 345                 350

Tyr Pro Ser Phe Leu Pro Asp Gln Met Gln Pro Gln Val Pro Pro Leu
        355                 360                 365

His Tyr Gln Glu Leu Met Pro Pro Gly Ser Cys Met Pro Glu Glu Pro
    370                 375                 380

Lys Pro Lys Arg Gly Arg Arg Ser Trp Pro Arg Lys Arg Thr Ala Thr

385                 390                 395                 400

His Thr Cys Asp Tyr Ala Gly Cys Gly Lys Thr Tyr Thr Lys Ser Ser
                405                 410                 415

His Leu Lys Ala His Leu Arg Thr His Thr Gly Glu Lys Pro Tyr His
            420                 425                 430

Cys Asp Trp Asp Gly Cys Gly Trp Lys Phe Ala Arg Ser Asp Glu Leu
        435                 440                 445

Thr Arg His Tyr Arg Lys His Thr Gly His Arg Pro Phe Gln Cys Gln
    450                 455                 460

Lys Cys Asp Arg Ala Phe Ser Arg Ser Asp His Leu Ala Leu His Met
465                 470                 475                 480

Lys Arg His Phe

<210>   107
<211>   1455
<212>   DNA
<213>   Artificial Sequence

<220>
<223>   NLS-Klf4-JO-84 MTD cDNA Sequence

<400>   107
atgaagaaga agaggaaggc tgtcagcgac gcgctgctcc catctttctc cacgttcgcg      60

tctggcccgg cgggaaggga gaagacactg cgtcaagcag gtgccccgaa taaccgctgg     120

cgggaggagc tctcccacat gaagcgactt cccccagtgc ttcccggccg cccctatgac     180

ctggcggcgg cgaccgtggc cacagacctg gagagcggcg gagccggtgc ggcttgcggc     240

ggtagcaacc tggcgcccct acctcggaga gagaccgagg agttcaacga tctcctggac     300

ctggacttta ttctctccaa ttcgctgacc catcctccgg agtcagtggc cgccaccgtg 360

tcctcgtcag cgtcagcctc ctcttcgtcg tcgccgtcga gcagcggccc tgccagcgcg 420

ccctccacct gcagcttcac ctatccgatc cgggccggga acgacccggg cgtggcgccg 480

ggcggcacgg gcggaggcct cctctatggc agggagtccg ctccccctcc gacggctccc 540

ttcaacctgg cggacatcaa cgacgtgagc ccctcgggcg gcttcgtggc cgagctcctg 600

cggccagaat tggacccggt gtacattccg ccgcagcagc cgcagccgcc aggtggcggg 660

ctgatgggca agttcgtgct gaaggcgtcg ctgagcgccc ctggcagcga gtacggcagc 720

ccgtcggtca tcagcgtcag caaaggcagc cctgacggca gccacccggt ggtggtggcg 780

ccctacaacg gcgggccgcc gcgcacgtgc cccaagatca agcaggaggc ggtctcttcg 840

tgcacccact tgggcgctgg accccctctc agcaatggcc accggccggc tgcacacgac 900

ttccccctgg ggcggcagct ccccagcagg actaccccga ccctgggtct tgaggaagtg 960

ctgagcagca gggactgtca ccctgccctg ccgcttcctc ccggcttcca tccccacccg 1020

gggcccaatt acccatcctt cctgcccgat cagatgcagc cgcaagtccc gccgctccat 1080

taccaagagc tcatgccacc cggttcctgc atgccagagg agcccaagcc aaagagggga 1140

agacgatcgt ggccccggaa aaggaccgcc acccacactt gtgattacgc gggctgcggc 1200

aaaacctaca caaagagttc ccatctcaag gcacacctgc gaacccacac aggtgagaaa 1260

ccttaccact gtgactggga cggctgtgga tggaaattcg cccgctcaga tgaactgacc 1320

aggcactacc gtaaacacac ggggcaccgc ccgttccagt gccaaaaatg cgaccgagca 1380

ttttccaggt cggaccacct cgccttacac atgaagaggc attttctggt ggcggcgctg 1440

ctggcggtgc tgtaa 1455

<210>    108

<211>    484

<212>    PRT

<213>    Artificial Sequence

<220>

<223>    NLS-Klf4-JO-84 MTD Amino Acid Sequence

<400>    108

Met Lys Lys Lys Arg Lys Ala Val Ser Asp Ala Leu Leu Pro Ser Phe
1               5                   10                  15

Ser Thr Phe Ala Ser Gly Pro Ala Gly Arg Glu Lys Thr Leu Arg Gln
            20                  25                  30

Ala Gly Ala Pro Asn Asn Arg Trp Arg Glu Glu Leu Ser His Met Lys
        35                  40                  45

Arg Leu Pro Pro Val Leu Pro Gly Arg Pro Tyr Asp Leu Ala Ala Ala
    50                  55                  60

Thr Val Ala Thr Asp Leu Glu Ser Gly Gly Ala Gly Ala Ala Cys Gly
65                  70                  75                  80

Gly Ser Asn Leu Ala Pro Leu Pro Arg Arg Glu Thr Glu Glu Phe Asn
                85                  90                  95

Asp Leu Leu Asp Leu Asp Phe Ile Leu Ser Asn Ser Leu Thr His Pro
            100                 105                 110

Pro Glu Ser Val Ala Ala Thr Val Ser Ser Ser Ala Ser Ala Ser Ser
        115                 120                 125

Ser Ser Ser Pro Ser Ser Ser Gly Pro Ala Ser Ala Pro Ser Thr Cys
    130                 135                 140

Ser Phe Thr Tyr Pro Ile Arg Ala Gly Asn Asp Pro Gly Val Ala Pro

145                 150                 155                 160

Gly Gly Thr Gly Gly Gly Leu Leu Tyr Gly Arg Glu Ser Ala Pro Pro
                165                 170                 175

Pro Thr Ala Pro Phe Asn Leu Ala Asp Ile Asn Asp Val Ser Pro Ser
            180                 185                 190

Gly Gly Phe Val Ala Glu Leu Leu Arg Pro Glu Leu Asp Pro Val Tyr
        195                 200                 205

Ile Pro Pro Gln Gln Pro Gln Pro Pro Gly Gly Gly Leu Met Gly Lys
    210                 215                 220

Phe Val Leu Lys Ala Ser Leu Ser Ala Pro Gly Ser Glu Tyr Gly Ser
225                 230                 235                 240

Pro Ser Val Ile Ser Val Ser Lys Gly Ser Pro Asp Gly Ser His Pro
                245                 250                 255

Val Val Val Ala Pro Tyr Asn Gly Gly Pro Pro Arg Thr Cys Pro Lys
            260                 265                 270

Ile Lys Gln Glu Ala Val Ser Ser Cys Thr His Leu Gly Ala Gly Pro
        275                 280                 285

Pro Leu Ser Asn Gly His Arg Pro Ala Ala His Asp Phe Pro Leu Gly
    290                 295                 300

Arg Gln Leu Pro Ser Arg Thr Thr Pro Thr Leu Gly Leu Glu Glu Val
305                 310                 315                 320

Leu Ser Ser Arg Asp Cys His Pro Ala Leu Pro Leu Pro Pro Gly Phe
                325                 330                 335

His Pro His Pro Gly Pro Asn Tyr Pro Ser Phe Leu Pro Asp Gln Met
            340                 345                 350

Gln Pro Gln Val Pro Pro Leu His Tyr Gln Glu Leu Met Pro Pro Gly
        355                 360                 365

Ser Cys Met Pro Glu Glu Pro Lys Pro Lys Arg Gly Arg Arg Ser Trp
370                 375                 380

Pro Arg Lys Arg Thr Ala Thr His Thr Cys Asp Tyr Ala Gly Cys Gly
385                 390                 395                 400

Lys Thr Tyr Thr Lys Ser Ser His Leu Lys Ala His Leu Arg Thr His
                405                 410                 415

Thr Gly Glu Lys Pro Tyr His Cys Asp Trp Asp Gly Cys Gly Trp Lys
            420                 425                 430

Phe Ala Arg Ser Asp Glu Leu Thr Arg His Tyr Arg Lys His Thr Gly
        435                 440                 445

His Arg Pro Phe Gln Cys Gln Lys Cys Asp Arg Ala Phe Ser Arg Ser
    450                 455                 460

Asp His Leu Ala Leu His Met Lys Arg His Phe Leu Val Ala Ala Leu
465                 470                 475                 480

Leu Ala Val Leu

<210>   109
<211>   1482
<212>   DNA
<213>   Artificial Sequence

<220>
<223>   NLS-JO-84 MTD-Klf4-JO-84 MTD cDNA Sequence

<400>   109
atgaagaaga agaggaagct ggtggcggcg ctgctggcgg tgctggctgt cagcgacgcg        60

ctgctcccat ctttctccac gttcgcgtct ggcccggcgg gaagggagaa gacactgcgt       120

caagcaggtg ccccgaataa ccgctggcgg gaggagctct cccacatgaa gcgacttccc 180

ccagtgcttc ccggccgccc ctatgacctg gcggcggcga ccgtggccac agacctggag 240

agcggcggag ccggtgcggc ttgcggcggt agcaacctgg cgcccctacc tcggagagag 300

accgaggagt tcaacgatct cctggacctg gactttattc tctccaattc gctgacccat 360

cctccggagt cagtggccgc caccgtgtcc tcgtcagcgt cagcctcctc ttcgtcgtcg 420

ccgtcgagca gcggccctgc cagcgcgccc tccacctgca gcttcaccta tccgatccgg 480

gccgggaacg acccgggcgt ggcgccgggc ggcacgggcg gaggcctcct ctatggcagg 540

gagtccgctc cccctccgac ggctcccttc aacctggcgg acatcaacga cgtgagcccc 600

tcgggcggct tcgtggccga gctcctgcgg ccagaattgg acccggtgta cattccgccg 660

cagcagccgc agccgccagg tggcgggctg atgggcaagt tcgtgctgaa ggcgtcgctg 720

agcgcccctg gcagcgagta cggcagcccg tcggtcatca gcgtcagcaa aggcagccct 780

gacggcagcc acccggtggt ggtggcgccc tacaacggcg ggccgccgcg cacgtgcccc 840

aagatcaagc aggaggcggt ctcttcgtgc acccacttgg gcgctggacc ccctctcagc 900

aatggccacc ggccggctgc acacgacttc cccctggggc ggcagctccc cagcaggact 960

accccgaccc tgggtcttga ggaagtgctg agcagcaggg actgtcaccc tgccctgccg 1020

cttcctcccg gcttccatcc ccacccgggg cccaattacc catccttcct gcccgatcag 1080

atgcagccgc aagtcccgcc gctccattac caagagctca tgccacccgg ttcctgcatg 1140

ccagaggagc ccaagccaaa gaggggaaga cgatcgtggc cccggaaaag gaccgccacc 1200

cacacttgtg attacgcggg ctgcggcaaa acctacacaa agagttccca tctcaaggca 1260

cacctgcgaa cccacacagg tgagaaacct taccactgtg actgggacgg ctgtggatgg 1320

aaattcgccc gctcagatga actgaccagg cactaccgta aacacacggg gcaccgcccg 1380

ttccagtgcc aaaaatgcga ccgagcattt tccaggtcgg accacctcgc cttacacatg 1440

aagaggcatt ttctggtggc ggcgctgctg gcggtgctgt aa 1482

<210> 110
<211> 493
<212> PRT
<213> Artificial Sequence

<220>
<223> NLS-JO-84 MTD-Klf4-JO-84 MTD Amino Acid Sequence

<400> 110
Met Lys Lys Lys Arg Lys Leu Val Ala Ala Leu Leu Ala Val Leu Ala
1               5                   10                  15

Val Ser Asp Ala Leu Leu Pro Ser Phe Ser Thr Phe Ala Ser Gly Pro
            20                  25                  30

Ala Gly Arg Glu Lys Thr Leu Arg Gln Ala Gly Ala Pro Asn Asn Arg
        35                  40                  45

Trp Arg Glu Glu Leu Ser His Met Lys Arg Leu Pro Pro Val Leu Pro
    50                  55                  60

Gly Arg Pro Tyr Asp Leu Ala Ala Ala Thr Val Ala Thr Asp Leu Glu
65                  70                  75                  80

Ser Gly Gly Ala Gly Ala Ala Cys Gly Gly Ser Asn Leu Ala Pro Leu
                85                  90                  95

Pro Arg Arg Glu Thr Glu Glu Phe Asn Asp Leu Leu Asp Leu Asp Phe
            100                 105                 110

Ile Leu Ser Asn Ser Leu Thr His Pro Pro Glu Ser Val Ala Ala Thr
        115                 120                 125

Val Ser Ser Ser Ala Ser Ala Ser Ser Ser Ser Ser Pro Ser Ser Ser
    130                 135                 140

Gly Pro Ala Ser Ala Pro Ser Thr Cys Ser Phe Thr Tyr Pro Ile Arg
145                 150                 155                 160

Ala Gly Asn Asp Pro Gly Val Ala Pro Gly Gly Thr Gly Gly Gly Leu
                165                 170                 175

Leu Tyr Gly Arg Glu Ser Ala Pro Pro Pro Thr Ala Pro Phe Asn Leu
            180                 185                 190

Ala Asp Ile Asn Asp Val Ser Pro Ser Gly Gly Phe Val Ala Glu Leu
        195                 200                 205

Leu Arg Pro Glu Leu Asp Pro Val Tyr Ile Pro Pro Gln Gln Pro Gln
    210                 215                 220

Pro Pro Gly Gly Gly Leu Met Gly Lys Phe Val Leu Lys Ala Ser Leu
225                 230                 235                 240

Ser Ala Pro Gly Ser Glu Tyr Gly Ser Pro Ser Val Ile Ser Val Ser
                245                 250                 255

Lys Gly Ser Pro Asp Gly Ser His Pro Val Val Val Ala Pro Tyr Asn
            260                 265                 270

Gly Gly Pro Pro Arg Thr Cys Pro Lys Ile Lys Gln Glu Ala Val Ser
        275                 280                 285

Ser Cys Thr His Leu Gly Ala Gly Pro Pro Leu Ser Asn Gly His Arg
    290                 295                 300

Pro Ala Ala His Asp Phe Pro Leu Gly Arg Gln Leu Pro Ser Arg Thr
305                 310                 315                 320

Thr Pro Thr Leu Gly Leu Glu Glu Val Leu Ser Ser Arg Asp Cys His
                325                 330                 335

Pro Ala Leu Pro Leu Pro Pro Gly Phe His Pro His Pro Gly Pro Asn
            340                 345                 350

Tyr Pro Ser Phe Leu Pro Asp Gln Met Gln Pro Gln Val Pro Pro Leu
        355                 360                 365

His Tyr Gln Glu Leu Met Pro Pro Gly Ser Cys Met Pro Glu Glu Pro
    370                 375                 380

Lys Pro Lys Arg Gly Arg Arg Ser Trp Pro Arg Lys Arg Thr Ala Thr
385                 390                 395                 400

His Thr Cys Asp Tyr Ala Gly Cys Gly Lys Thr Tyr Thr Lys Ser Ser
                405                 410                 415

His Leu Lys Ala His Leu Arg Thr His Thr Gly Glu Lys Pro Tyr His
            420                 425                 430

Cys Asp Trp Asp Gly Cys Gly Trp Lys Phe Ala Arg Ser Asp Glu Leu
        435                 440                 445

Thr Arg His Tyr Arg Lys His Thr Gly His Arg Pro Phe Gln Cys Gln
    450                 455                 460

Lys Cys Asp Arg Ala Phe Ser Arg Ser Asp His Leu Ala Leu His Met
465                 470                 475                 480

Lys Arg His Phe Leu Val Ala Ala Leu Leu Ala Val Leu
                485                 490

<210>   111
<211>   1452
<212>   DNA
<213>   Artificial Sequence

<220>
<223>   NLS-JO-86 MTD-Klf4 cDNA Sequence

<400>    111

atgaagaaga agaggaagct ggcggtgctg gcggcggcgc cggctgtcag cgacgcgctg        60

ctcccatctt tctccacgtt cgcgtctggc ccggcgggaa gggagaagac actgcgtcaa       120

gcaggtgccc cgaataaccg ctggcgggag gagctctccc acatgaagcg acttccccca       180

gtgcttcccg gccgccccta tgacctggcg gcggcgaccg tggccacaga cctggagagc       240

ggcggagccg gtgcggcttg cggcggtagc aacctggcgc ccctacctcg gagagagacc       300

gaggagttca acgatctcct ggacctggac tttattctct ccaattcgct gacccatcct       360

ccggagtcag tggccgccac cgtgtcctcg tcagcgtcag cctcctcttc gtcgtcgccg       420

tcgagcagcg gccctgccag cgcgccctcc acctgcagct tcacctatcc gatccgggcc       480

gggaacgacc cgggcgtggc gccgggcggc acgggcggag gcctcctcta tggcagggag       540

tccgctcccc ctccgacggc tcccttcaac ctggcggaca tcaacgacgt gagcccctcg       600

ggcggcttcg tggccgagct cctgcggcca gaattggacc cggtgtacat tccgccgcag       660

cagccgcagc cgccaggtgg cgggctgatg ggcaagttcg tgctgaaggc gtcgctgagc       720

gcccctggca gcgagtacgg cagcccgtcg gtcatcagcg tcagcaaagg cagccctgac       780

ggcagccacc cggtggtggt ggcgccctac aacggcgggc cgccgcgcac gtgccccaag       840

atcaagcagg aggcggtctc ttcgtgcacc cacttgggcg ctggaccccc tctcagcaat       900

ggccaccggc cggctgcaca cgacttcccc ctggggcggc agctccccag caggactacc       960

ccgaccctgg gtcttgagga agtgctgagc agcagggact gtcaccctgc cctgccgctt      1020

cctcccggct tccatcccca cccggggccc aattacccat ccttcctgcc cgatcagatg      1080

cagccgcaag tcccgccgct ccattaccaa gagctcatgc cacccggttc ctgcatgcca      1140

gaggagccca agccaaagag gggaagacga tcgtggcccc ggaaaaggac cgccacccac      1200

acttgtgatt acgcgggctg cggcaaaacc tacacaaaga gttcccatct caaggcacac 1260

ctgcgaaccc acacaggtga gaaaccttac cactgtgact gggacggctg tggatggaaa 1320

ttcgcccgct cagatgaact gaccaggcac taccgtaaac acacggggca ccgcccgttc 1380

cagtgccaaa aatgcgaccg agcattttcc aggtcggacc acctcgcctt acacatgaag 1440

aggcattttt aa 1452

<210> 112
<211> 483
<212> PRT
<213> Artificial Sequence

<220>
<223> NLS-JO-86 MTD-Klf4 Amino Acid Sequence

<400> 112
Met Lys Lys Lys Arg Lys Leu Ala Val Leu Ala Ala Ala Pro Ala Val
1               5                   10                  15

Ser Asp Ala Leu Leu Pro Ser Phe Ser Thr Phe Ala Ser Gly Pro Ala
            20                  25                  30

Gly Arg Glu Lys Thr Leu Arg Gln Ala Gly Ala Pro Asn Asn Arg Trp
        35                  40                  45

Arg Glu Glu Leu Ser His Met Lys Arg Leu Pro Pro Val Leu Pro Gly
    50                  55                  60

Arg Pro Tyr Asp Leu Ala Ala Ala Thr Val Ala Thr Asp Leu Glu Ser
65                  70                  75                  80

Gly Gly Ala Gly Ala Ala Cys Gly Gly Ser Asn Leu Ala Pro Leu Pro
                85                  90                  95

Arg Arg Glu Thr Glu Glu Phe Asn Asp Leu Leu Asp Leu Asp Phe Ile
            100                 105                 110

Leu Ser Asn Ser Leu Thr His Pro Pro Glu Ser Val Ala Ala Thr Val
        115                 120                 125

Ser Ser Ser Ala Ser Ala Ser Ser Ser Ser Ser Pro Ser Ser Ser Gly
    130                 135                 140

Pro Ala Ser Ala Pro Ser Thr Cys Ser Phe Thr Tyr Pro Ile Arg Ala
145                 150                 155                 160

Gly Asn Asp Pro Gly Val Ala Pro Gly Gly Thr Gly Gly Gly Leu Leu
                165                 170                 175

Tyr Gly Arg Glu Ser Ala Pro Pro Pro Thr Ala Pro Phe Asn Leu Ala
            180                 185                 190

Asp Ile Asn Asp Val Ser Pro Ser Gly Gly Phe Val Ala Glu Leu Leu
        195                 200                 205

Arg Pro Glu Leu Asp Pro Val Tyr Ile Pro Pro Gln Gln Pro Gln Pro
    210                 215                 220

Pro Gly Gly Gly Leu Met Gly Lys Phe Val Leu Lys Ala Ser Leu Ser
225                 230                 235                 240

Ala Pro Gly Ser Glu Tyr Gly Ser Pro Ser Val Ile Ser Val Ser Lys
                245                 250                 255

Gly Ser Pro Asp Gly Ser His Pro Val Val Val Ala Pro Tyr Asn Gly
            260                 265                 270

Gly Pro Pro Arg Thr Cys Pro Lys Ile Lys Gln Glu Ala Val Ser Ser
        275                 280                 285

Cys Thr His Leu Gly Ala Gly Pro Pro Leu Ser Asn Gly His Arg Pro
    290                 295                 300

Ala Ala His Asp Phe Pro Leu Gly Arg Gln Leu Pro Ser Arg Thr Thr

305                 310                 315                 320

Pro Thr Leu Gly Leu Glu Glu Val Leu Ser Ser Arg Asp Cys His Pro
                325                 330                 335

Ala Leu Pro Leu Pro Pro Gly Phe His Pro His Pro Gly Pro Asn Tyr
            340                 345                 350

Pro Ser Phe Leu Pro Asp Gln Met Gln Pro Gln Val Pro Pro Leu His
        355                 360                 365

Tyr Gln Glu Leu Met Pro Pro Gly Ser Cys Met Pro Glu Glu Pro Lys
    370                 375                 380

Pro Lys Arg Gly Arg Arg Ser Trp Pro Arg Lys Arg Thr Ala Thr His
385                 390                 395                 400

Thr Cys Asp Tyr Ala Gly Cys Gly Lys Thr Tyr Thr Lys Ser Ser His
                405                 410                 415

Leu Lys Ala His Leu Arg Thr His Thr Gly Glu Lys Pro Tyr His Cys
            420                 425                 430

Asp Trp Asp Gly Cys Gly Trp Lys Phe Ala Arg Ser Asp Glu Leu Thr
        435                 440                 445

Arg His Tyr Arg Lys His Thr Gly His Arg Pro Phe Gln Cys Gln Lys
    450                 455                 460

Cys Asp Arg Ala Phe Ser Arg Ser Asp His Leu Ala Leu His Met Lys
465                 470                 475                 480

Arg His Phe

<210>   113
<211>   1452
<212>   DNA
<213>   Artificial Sequence

<220>
<223>    NLS-Klf4-JO-86 MTD cDNA Sequence

<400>    113
atgaagaaga agaggaaggc tgtcagcgac gcgctgctcc catctttctc cacgttcgcg    60

tctggcccgg cgggaaggga gaagacactg cgtcaagcag gtgccccgaa taaccgctgg    120

cgggaggagc tctcccacat gaagcgactt cccccagtgc ttcccggccg cccctatgac    180

ctggcggcgg cgaccgtggc cacagacctg gagagcggcg gagccggtgc ggcttgcggc    240

ggtagcaacc tggcgcccct acctcggaga gagaccgagg agttcaacga tctcctggac    300

ctggacttta ttctctccaa ttcgctgacc catcctccgg agtcagtggc cgccaccgtg    360

tcctcgtcag cgtcagcctc ctcttcgtcg tcgccgtcga gcagcggccc tgccagcgcg    420

ccctccacct gcagcttcac ctatccgatc cgggccggga acgacccggg cgtggcgccg    480

ggcggcacgg gcggaggcct cctctatggc agggagtccg ctccccctcc gacggctccc    540

ttcaacctgg cggacatcaa cgacgtgagc ccctcgggcg gcttcgtggc cgagctcctg    600

cggccagaat tggacccggt gtacattccg ccgcagcagc cgcagccgcc aggtggcggg    660

ctgatgggca agttcgtgct gaaggcgtcg ctgagcgccc ctggcagcga gtacggcagc    720

ccgtcggtca tcagcgtcag caaaggcagc cctgacggca gccacccggt ggtggtggcg    780

ccctacaacg gcgggccgcc gcgcacgtgc cccaagatca agcaggaggc ggtctcttcg    840

tgcacccact tgggcgctgg accccctctc agcaatggcc accggccggc tgcacacgac    900

ttccccctgg ggcggcagct ccccagcagg actaccccga ccctgggtct tgaggaagtg    960

ctgagcagca gggactgtca ccctgccctg ccgcttcctc ccggcttcca tccccacccg    1020

gggcccaatt acccatcctt cctgcccgat cagatgcagc cgcaagtccc gccgctccat 1080

taccaagagc tcatgccacc cggttcctgc atgccagagg agcccaagcc aaagagggga 1140

agacgatcgt ggccccggaa aaggaccgcc acccacactt gtgattacgc gggctgcggc 1200

aaaacctaca caaagagttc ccatctcaag gcacacctgc gaacccacac aggtgagaaa 1260

ccttaccact gtgactggga cggctgtgga tggaaattcg cccgctcaga tgaactgacc 1320

aggcactacc gtaaacacac ggggcaccgc ccgttccagt gccaaaaatg cgaccgagca 1380

ttttccaggt cggaccacct cgccttacac atgaagaggc attttctggc ggtgctggcg 1440

gcggcgccgt aa 1452

<210> 114
<211> 483
<212> PRT
<213> Artificial Sequence

<220>
<223> NLS-Klf4-JO-86 MTD Amino Acid Sequence

<400> 114
Met Lys Lys Lys Arg Lys Ala Val Ser Asp Ala Leu Leu Pro Ser Phe
1               5                   10                  15

Ser Thr Phe Ala Ser Gly Pro Ala Gly Arg Glu Lys Thr Leu Arg Gln
            20                  25                  30

Ala Gly Ala Pro Asn Asn Arg Trp Arg Glu Glu Leu Ser His Met Lys
        35                  40                  45

Arg Leu Pro Pro Val Leu Pro Gly Arg Pro Tyr Asp Leu Ala Ala Ala
    50                  55                  60

Thr Val Ala Thr Asp Leu Glu Ser Gly Gly Ala Gly Ala Ala Cys Gly

65                  70                  75                  80

Gly Ser Asn Leu Ala Pro Leu Pro Arg Arg Glu Thr Glu Glu Phe Asn
                85                  90                  95

Asp Leu Leu Asp Leu Asp Phe Ile Leu Ser Asn Ser Leu Thr His Pro
            100                 105                 110

Pro Glu Ser Val Ala Ala Thr Val Ser Ser Ser Ala Ser Ala Ser Ser
        115                 120                 125

Ser Ser Ser Pro Ser Ser Ser Gly Pro Ala Ser Ala Pro Ser Thr Cys
    130                 135                 140

Ser Phe Thr Tyr Pro Ile Arg Ala Gly Asn Asp Pro Gly Val Ala Pro
145                 150                 155                 160

Gly Gly Thr Gly Gly Gly Leu Leu Tyr Gly Arg Glu Ser Ala Pro Pro
                165                 170                 175

Pro Thr Ala Pro Phe Asn Leu Ala Asp Ile Asn Asp Val Ser Pro Ser
            180                 185                 190

Gly Gly Phe Val Ala Glu Leu Leu Arg Pro Glu Leu Asp Pro Val Tyr
        195                 200                 205

Ile Pro Pro Gln Gln Pro Gln Pro Pro Gly Gly Gly Leu Met Gly Lys
    210                 215                 220

Phe Val Leu Lys Ala Ser Leu Ser Ala Pro Gly Ser Glu Tyr Gly Ser
225                 230                 235                 240

Pro Ser Val Ile Ser Val Ser Lys Gly Ser Pro Asp Gly Ser His Pro
                245                 250                 255

Val Val Val Ala Pro Tyr Asn Gly Gly Pro Pro Arg Thr Cys Pro Lys
            260                 265                 270

Ile Lys Gln Glu Ala Val Ser Ser Cys Thr His Leu Gly Ala Gly Pro
        275                 280                 285

Pro Leu Ser Asn Gly His Arg Pro Ala Ala His Asp Phe Pro Leu Gly
    290                 295                 300

Arg Gln Leu Pro Ser Arg Thr Thr Pro Thr Leu Gly Leu Glu Glu Val
305                 310                 315                 320

Leu Ser Ser Arg Asp Cys His Pro Ala Leu Pro Leu Pro Pro Gly Phe
                325                 330                 335

His Pro His Pro Gly Pro Asn Tyr Pro Ser Phe Leu Pro Asp Gln Met
            340                 345                 350

Gln Pro Gln Val Pro Pro Leu His Tyr Gln Glu Leu Met Pro Pro Gly
        355                 360                 365

Ser Cys Met Pro Glu Glu Pro Lys Pro Lys Arg Gly Arg Arg Ser Trp
    370                 375                 380

Pro Arg Lys Arg Thr Ala Thr His Thr Cys Asp Tyr Ala Gly Cys Gly
385                 390                 395                 400

Lys Thr Tyr Thr Lys Ser Ser His Leu Lys Ala His Leu Arg Thr His
                405                 410                 415

Thr Gly Glu Lys Pro Tyr His Cys Asp Trp Asp Gly Cys Gly Trp Lys
            420                 425                 430

Phe Ala Arg Ser Asp Glu Leu Thr Arg His Tyr Arg Lys His Thr Gly
        435                 440                 445

His Arg Pro Phe Gln Cys Gln Lys Cys Asp Arg Ala Phe Ser Arg Ser
    450                 455                 460

Asp His Leu Ala Leu His Met Lys Arg His Phe Leu Ala Val Leu Ala
465                 470                 475                 480

Ala Ala Pro

<210> 115
<211> 1476
<212> DNA
<213> Artificial Sequence

<220>
<223> NLS-JO-86 MTD-Klf4-JO-86 MTD cDNA Sequence

<400> 115
atgaagaaga agaggaagct ggcggtgctg gcggcggcgc cggctgtcag cgacgcgctg 60

ctcccatctt tctccacgtt cgcgtctggc ccggcgggaa gggagaagac actgcgtcaa 120

gcaggtgccc cgaataaccg ctggcgggag gagctctccc acatgaagcg acttccccca 180

gtgcttcccg gccgccccta tgacctggcg gcggcgaccg tggccacaga cctggagagc 240

ggcggagccg gtgcggcttg cggcggtagc aacctggcgc ccctacctcg gagagagacc 300

gaggagttca acgatctcct ggacctggac tttattctct ccaattcgct gacccatcct 360

ccggagtcag tggccgccac cgtgtcctcg tcagcgtcag cctcctcttc gtcgtcgccg 420

tcgagcagcg gccctgccag cgcgccctcc acctgcagct tcacctatcc gatccgggcc 480

gggaacgacc cgggcgtggc gccgggcggc acgggcggag gcctcctcta tggcagggag 540

tccgctcccc ctccgacggc tcccttcaac ctggcggaca tcaacgacgt gagcccctcg 600

ggcggcttcg tggccgagct cctgcggcca gaattggacc cggtgtacat tccgccgcag 660

cagccgcagc cgccaggtgg cgggctgatg ggcaagttcg tgctgaaggc gtcgctgagc 720

gcccctggca gcgagtacgg cagcccgtcg gtcatcagcg tcagcaaagg cagccctgac 780

ggcagccacc cggtggtggt ggcgccctac aacggcgggc cgccgcgcac gtgccccaag 840

atcaagcagg aggcggtctc ttcgtgcacc cacttgggcg ctggaccccc tctcagcaat 900

ggccaccggc cggctgcaca cgacttcccc ctggggcggc agctccccag caggactacc 960

ccgaccctgg gtcttgagga agtgctgagc agcagggact gtcaccctgc cctgccgctt 1020

cctcccggct tccatcccca cccggggccc aattacccat ccttcctgcc cgatcagatg 1080

cagccgcaag tcccgccgct ccattaccaa gagctcatgc cacccggttc ctgcatgcca 1140

gaggagccca agccaaagag gggaagacga tcgtggcccc ggaaaaggac cgccacccac 1200

acttgtgatt acgcgggctg cggcaaaacc tacacaaaga gttcccatct caaggcacac 1260

ctgcgaaccc acacaggtga gaaaccttac cactgtgact gggacggctg tggatggaaa 1320

ttcgcccgct cagatgaact gaccaggcac taccgtaaac acacggggca ccgcccgttc 1380

cagtgccaaa aatgcgaccg agcattttcc aggtcggacc acctcgcctt acacatgaag 1440

aggcattttc tggcggtgct ggcggcggcg ccgtaa 1476

<210> 116
<211> 491
<212> PRT
<213> Artificial Sequence

<220>
<223> NLS-JO-86 MTD-Klf4-JO-86 MTD Amino Acid Sequence

<400> 116
Met Lys Lys Lys Arg Lys Leu Ala Val Leu Ala Ala Ala Pro Ala Val
1               5                   10                  15

Ser Asp Ala Leu Leu Pro Ser Phe Ser Thr Phe Ala Ser Gly Pro Ala
            20                  25                  30

Gly Arg Glu Lys Thr Leu Arg Gln Ala Gly Ala Pro Asn Asn Arg Trp
        35                  40                  45

Arg Glu Glu Leu Ser His Met Lys Arg Leu Pro Pro Val Leu Pro Gly
50                  55                  60

Arg Pro Tyr Asp Leu Ala Ala Ala Thr Val Ala Thr Asp Leu Glu Ser
65                  70                  75                  80

Gly Gly Ala Gly Ala Ala Cys Gly Gly Ser Asn Leu Ala Pro Leu Pro
                85                  90                  95

Arg Arg Glu Thr Glu Glu Phe Asn Asp Leu Leu Asp Leu Asp Phe Ile
            100                 105                 110

Leu Ser Asn Ser Leu Thr His Pro Pro Glu Ser Val Ala Ala Thr Val
        115                 120                 125

Ser Ser Ser Ala Ser Ala Ser Ser Ser Ser Ser Pro Ser Ser Ser Gly
    130                 135                 140

Pro Ala Ser Ala Pro Ser Thr Cys Ser Phe Thr Tyr Pro Ile Arg Ala
145                 150                 155                 160

Gly Asn Asp Pro Gly Val Ala Pro Gly Gly Thr Gly Gly Gly Leu Leu
                165                 170                 175

Tyr Gly Arg Glu Ser Ala Pro Pro Pro Thr Ala Pro Phe Asn Leu Ala
            180                 185                 190

Asp Ile Asn Asp Val Ser Pro Ser Gly Gly Phe Val Ala Glu Leu Leu
        195                 200                 205

Arg Pro Glu Leu Asp Pro Val Tyr Ile Pro Pro Gln Gln Pro Gln Pro
    210                 215                 220

Pro Gly Gly Gly Leu Met Gly Lys Phe Val Leu Lys Ala Ser Leu Ser
225                 230                 235                 240

Ala Pro Gly Ser Glu Tyr Gly Ser Pro Ser Val Ile Ser Val Ser Lys
                245                 250                 255

Gly Ser Pro Asp Gly Ser His Pro Val Val Val Ala Pro Tyr Asn Gly
            260                 265                 270

Gly Pro Pro Arg Thr Cys Pro Lys Ile Lys Gln Glu Ala Val Ser Ser
        275                 280                 285

Cys Thr His Leu Gly Ala Gly Pro Pro Leu Ser Asn Gly His Arg Pro
    290                 295                 300

Ala Ala His Asp Phe Pro Leu Gly Arg Gln Leu Pro Ser Arg Thr Thr
305                 310                 315                 320

Pro Thr Leu Gly Leu Glu Glu Val Leu Ser Ser Arg Asp Cys His Pro
                325                 330                 335

Ala Leu Pro Leu Pro Pro Gly Phe His Pro His Pro Gly Pro Asn Tyr
            340                 345                 350

Pro Ser Phe Leu Pro Asp Gln Met Gln Pro Gln Val Pro Pro Leu His
        355                 360                 365

Tyr Gln Glu Leu Met Pro Pro Gly Ser Cys Met Pro Glu Glu Pro Lys
    370                 375                 380

Pro Lys Arg Gly Arg Arg Ser Trp Pro Arg Lys Arg Thr Ala Thr His
385                 390                 395                 400

Thr Cys Asp Tyr Ala Gly Cys Gly Lys Thr Tyr Thr Lys Ser Ser His
                405                 410                 415

Leu Lys Ala His Leu Arg Thr His Thr Gly Glu Lys Pro Tyr His Cys
            420                 425                 430

Asp Trp Asp Gly Cys Gly Trp Lys Phe Ala Arg Ser Asp Glu Leu Thr
        435                 440                 445

Arg His Tyr Arg Lys His Thr Gly His Arg Pro Phe Gln Cys Gln Lys
    450                 455                 460

Cys Asp Arg Ala Phe Ser Arg Ser Asp His Leu Ala Leu His Met Lys

465                 470                 475                 480

Arg His Phe Leu Ala Val Leu Ala Ala Ala Pro
                485                 490

<210>   117
<211>   1488
<212>   DNA
<213>   Artificial Sequence

<220>
<223>   His-NLS-Klf4 cDNA Sequence

<400>   117
atgggcagca gccatcatca tcatcatcac agcagcggcc tggtgccgcg cggcagccat      60

atgaagaaga agaggaaggc tgtcagcgac gcgctgctcc catctttctc cacgttcgcg     120

tctggcccgg cgggaaggga gaagacactg cgtcaagcag gtgccccgaa taaccgctgg     180

cgggaggagc tctcccacat gaagcgactt cccccagtgc ttcccggccg cccctatgac     240

ctggcggcgg cgaccgtggc cacagacctg gagagcggcg gagccggtgc ggcttgcggc     300

ggtagcaacc tggcgcccct acctcggaga gagaccgagg agttcaacga tctcctggac     360

ctggacttta ttctctccaa ttcgctgacc catcctccgg agtcagtggc cgccaccgtg     420

tcctcgtcag cgtcagcctc ctcttcgtcg tcgccgtcga gcagcggccc tgccagcgcg     480

ccctccacct gcagcttcac ctatccgatc cgggccggga acgacccggg cgtggcgccg     540

ggcggcacgg gcggaggcct cctctatggc agggagtccg ctccccctcc gacggctccc     600

ttcaacctgg cggacatcaa cgacgtgagc ccctcgggcg gcttcgtggc cgagctcctg     660

cggccagaat tggacccggt gtacattccg ccgcagcagc cgcagccgcc aggtggcggg     720

ctgatgggca agttcgtgct gaaggcgtcg ctgagcgccc ctggcagcga gtacggcagc 780

ccgtcggtca tcagcgtcag caaaggcagc cctgacggca gccacccggt ggtggtggcg 840

ccctacaacg gcgggccgcc gcgcacgtgc cccaagatca agcaggaggc ggtctcttcg 900

tgcacccact tgggcgctgg accccctctc agcaatggcc accggccggc tgcacacgac 960

ttccccctgg ggcggcagct ccccagcagg actaccccga ccctgggtct tgaggaagtg 1020

ctgagcagca gggactgtca ccctgccctg ccgcttcctc ccggcttcca tccccacccg 1080

gggcccaatt acccatcctt cctgcccgat cagatgcagc cgcaagtccc gccgctccat 1140

taccaagagc tcatgccacc cggttcctgc atgccagagg agcccaagcc aaagagggga 1200

agacgatcgt ggccccggaa aaggaccgcc acccacactt gtgattacgc gggctgcggc 1260

aaaacctaca caaagagttc ccatctcaag gcacacctgc gaacccacac aggtgagaaa 1320

ccttaccact gtgactggga cggctgtgga tggaaattcg cccgctcaga tgaactgacc 1380

aggcactacc gtaaacacac ggggcaccgc ccgttccagt gccaaaaatg cgaccgagca 1440

ttttccaggt cggaccacct cgccttacac atgaagaggc atttttaa 1488

<210> 118
<211> 495
<212> PRT
<213> Artificial Sequence

<220>
<223> His-NLS-Klf4 Amino Acid Sequence

<400> 118
Met Gly Ser Ser His His His His His His Ser Ser Gly Leu Val Pro
1               5                   10                  15

Arg Gly Ser His Met Lys Lys Lys Arg Lys Ala Val Ser Asp Ala Leu
            20                  25                  30

Leu Pro Ser Phe Ser Thr Phe Ala Ser Gly Pro Ala Gly Arg Glu Lys
        35                  40                  45

Thr Leu Arg Gln Ala Gly Ala Pro Asn Asn Arg Trp Arg Glu Glu Leu
    50                  55                  60

Ser His Met Lys Arg Leu Pro Pro Val Leu Pro Gly Arg Pro Tyr Asp
65                  70                  75                  80

Leu Ala Ala Ala Thr Val Ala Thr Asp Leu Glu Ser Gly Gly Ala Gly
                85                  90                  95

Ala Ala Cys Gly Gly Ser Asn Leu Ala Pro Leu Pro Arg Arg Glu Thr
            100                 105                 110

Glu Glu Phe Asn Asp Leu Leu Asp Leu Asp Phe Ile Leu Ser Asn Ser
        115                 120                 125

Leu Thr His Pro Pro Glu Ser Val Ala Ala Thr Val Ser Ser Ser Ala
    130                 135                 140

Ser Ala Ser Ser Ser Ser Ser Pro Ser Ser Ser Gly Pro Ala Ser Ala
145                 150                 155                 160

Pro Ser Thr Cys Ser Phe Thr Tyr Pro Ile Arg Ala Gly Asn Asp Pro
                165                 170                 175

Gly Val Ala Pro Gly Gly Thr Gly Gly Gly Leu Leu Tyr Gly Arg Glu
            180                 185                 190

Ser Ala Pro Pro Pro Thr Ala Pro Phe Asn Leu Ala Asp Ile Asn Asp
        195                 200                 205

Val Ser Pro Ser Gly Gly Phe Val Ala Glu Leu Leu Arg Pro Glu Leu
    210                 215                 220

Asp Pro Val Tyr Ile Pro Pro Gln Gln Pro Gln Pro Pro Gly Gly Gly

225                 230                 235                 240

Leu Met Gly Lys Phe Val Leu Lys Ala Ser Leu Ser Ala Pro Gly Ser
                245                 250                 255

Glu Tyr Gly Ser Pro Ser Val Ile Ser Val Ser Lys Gly Ser Pro Asp
            260                 265                 270

Gly Ser His Pro Val Val Val Ala Pro Tyr Asn Gly Gly Pro Pro Arg
        275                 280                 285

Thr Cys Pro Lys Ile Lys Gln Glu Ala Val Ser Ser Cys Thr His Leu
    290                 295                 300

Gly Ala Gly Pro Pro Leu Ser Asn Gly His Arg Pro Ala Ala His Asp
305                 310                 315                 320

Phe Pro Leu Gly Arg Gln Leu Pro Ser Arg Thr Thr Pro Thr Leu Gly
                325                 330                 335

Leu Glu Glu Val Leu Ser Ser Arg Asp Cys His Pro Ala Leu Pro Leu
            340                 345                 350

Pro Pro Gly Phe His Pro His Pro Gly Pro Asn Tyr Pro Ser Phe Leu
        355                 360                 365

Pro Asp Gln Met Gln Pro Gln Val Pro Pro Leu His Tyr Gln Glu Leu
    370                 375                 380

Met Pro Pro Gly Ser Cys Met Pro Glu Glu Pro Lys Pro Lys Arg Gly
385                 390                 395                 400

Arg Arg Ser Trp Pro Arg Lys Arg Thr Ala Thr His Thr Cys Asp Tyr
                405                 410                 415

Ala Gly Cys Gly Lys Thr Tyr Thr Lys Ser Ser His Leu Lys Ala His
            420                 425                 430

Leu Arg Thr His Thr Gly Glu Lys Pro Tyr His Cys Asp Trp Asp Gly
        435                 440                 445

Cys Gly Trp Lys Phe Ala Arg Ser Asp Glu Leu Thr Arg His Tyr Arg
    450                 455                 460

Lys His Thr Gly His Arg Pro Phe Gln Cys Gln Lys Cys Asp Arg Ala
465                 470                 475                 480

Phe Ser Arg Ser Asp His Leu Ala Leu His Met Lys Arg His Phe
                485                 490                 495

<210>    119
<211>    1515
<212>    DNA
<213>    Artificial Sequence

<220>
<223>    His-NLS-JO-84 MTD-Klf4 cDNA Sequence

<400>    119
atgggcagca gccatcatca tcatcatcac agcagcggcc tggtgccgcg cggcagccat    60

atgaagaaga agaggaagct ggtggcggcg ctgctggcgg tgctggctgt cagcgacgcg    120

ctgctcccat ctttctccac gttcgcgtct ggcccggcgg gaagggagaa gacactgcgt    180

caagcaggtg ccccgaataa ccgctggcgg gaggagctct cccacatgaa gcgacttccc    240

ccagtgcttc ccggccgccc ctatgacctg gcggcggcga ccgtggccac agacctggag    300

agcggcggag ccggtgcggc ttgcggcggt agcaacctgg cgcccctacc tcggagagag    360

accgaggagt tcaacgatct cctggacctg gactttattc tctccaattc gctgacccat    420

cctccggagt cagtggccgc caccgtgtcc tcgtcagcgt cagcctcctc ttcgtcgtcg    480

ccgtcgagca gcggccctgc cagcgcgccc tccacctgca gcttcaccta tccgatccgg    540

gccgggaacg acccgggcgt ggcgccgggc ggcacgggcg gaggcctcct ctatggcagg    600

gagtccgctc cccctccgac ggctcccttc aacctggcgg acatcaacga cgtgagcccc 660

tcgggcggct tcgtggccga gctcctgcgg ccagaattgg acccggtgta cattccgccg 720

cagcagccgc agccgccagg tggcgggctg atgggcaagt tcgtgctgaa ggcgtcgctg 780

agcgcccctg gcagcgagta cggcagcccg tcggtcatca gcgtcagcaa aggcagccct 840

gacggcagcc acccggtggt ggtggcgccc tacaacggcg ggccgccgcg cacgtgcccc 900

aagatcaagc aggaggcggt ctcttcgtgc acccacttgg gcgctggacc ccctctcagc 960

aatggccacc ggccggctgc acacgacttc cccctggggc ggcagctccc cagcaggact 1020

accccgaccc tgggtcttga ggaagtgctg agcagcaggg actgtcaccc tgccctgccg 1080

cttcctcccg gcttccatcc ccacccgggg cccaattacc catccttcct gcccgatcag 1140

atgcagccgc aagtcccgcc gctccattac caagagctca tgccacccgg ttcctgcatg 1200

ccagaggagc ccaagccaaa gaggggaaga cgatcgtggc cccggaaaag gaccgccacc 1260

cacacttgtg attacgcggg ctgcggcaaa acctacacaa agagttccca tctcaaggca 1320

cacctgcgaa cccacacagg tgagaaacct taccactgtg actgggacgg ctgtggatgg 1380

aaattcgccc gctcagatga actgaccagg cactaccgta aacacacggg gcaccgcccg 1440

ttccagtgcc aaaaatgcga ccgagcattt tccaggtcgg accacctcgc cttacacatg 1500

aagaggcatt tttaa 1515

<210> 120
<211> 504
<212> PRT
<213> Artificial Sequence

<220>

<223>    His-NLS-JO-84 MTD-Klf4 Amino Acid Sequence

<400>    120
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Ala Val Ser Asp Ala Leu Leu Pro Ser Phe Ser Thr Phe
        35                  40                  45

Ala Ser Gly Pro Ala Gly Arg Glu Lys Thr Leu Arg Gln Ala Gly Ala
    50                  55                  60

Pro Asn Asn Arg Trp Arg Glu Glu Leu Ser His Met Lys Arg Leu Pro
65                  70                  75                  80

Pro Val Leu Pro Gly Arg Pro Tyr Asp Leu Ala Ala Ala Thr Val Ala
                85                  90                  95

Thr Asp Leu Glu Ser Gly Gly Ala Gly Ala Ala Cys Gly Gly Ser Asn
            100                 105                 110

Leu Ala Pro Leu Pro Arg Arg Glu Thr Glu Glu Phe Asn Asp Leu Leu
        115                 120                 125

Asp Leu Asp Phe Ile Leu Ser Asn Ser Leu Thr His Pro Pro Glu Ser
    130                 135                 140

Val Ala Ala Thr Val Ser Ser Ser Ala Ser Ala Ser Ser Ser Ser Ser
145                 150                 155                 160

Pro Ser Ser Ser Gly Pro Ala Ser Ala Pro Ser Thr Cys Ser Phe Thr
                165                 170                 175

Tyr Pro Ile Arg Ala Gly Asn Asp Pro Gly Val Ala Pro Gly Gly Thr
            180                 185                 190

Gly Gly Gly Leu Leu Tyr Gly Arg Glu Ser Ala Pro Pro Pro Thr Ala
        195                 200                 205

Pro Phe Asn Leu Ala Asp Ile Asn Asp Val Ser Pro Ser Gly Gly Phe
    210                 215                 220

Val Ala Glu Leu Leu Arg Pro Glu Leu Asp Pro Val Tyr Ile Pro Pro
225                 230                 235                 240

Gln Gln Pro Gln Pro Pro Gly Gly Gly Leu Met Gly Lys Phe Val Leu
                245                 250                 255

Lys Ala Ser Leu Ser Ala Pro Gly Ser Glu Tyr Gly Ser Pro Ser Val
            260                 265                 270

Ile Ser Val Ser Lys Gly Ser Pro Asp Gly Ser His Pro Val Val Val
        275                 280                 285

Ala Pro Tyr Asn Gly Gly Pro Pro Arg Thr Cys Pro Lys Ile Lys Gln
    290                 295                 300

Glu Ala Val Ser Ser Cys Thr His Leu Gly Ala Gly Pro Pro Leu Ser
305                 310                 315                 320

Asn Gly His Arg Pro Ala Ala His Asp Phe Pro Leu Gly Arg Gln Leu
                325                 330                 335

Pro Ser Arg Thr Thr Pro Thr Leu Gly Leu Glu Glu Val Leu Ser Ser
            340                 345                 350

Arg Asp Cys His Pro Ala Leu Pro Leu Pro Pro Gly Phe His Pro His
        355                 360                 365

Pro Gly Pro Asn Tyr Pro Ser Phe Leu Pro Asp Gln Met Gln Pro Gln
    370                 375                 380

Val Pro Pro Leu His Tyr Gln Glu Leu Met Pro Pro Gly Ser Cys Met
385                 390                 395                 400

Pro Glu Glu Pro Lys Pro Lys Arg Gly Arg Arg Ser Trp Pro Arg Lys

405                 410                 415

Arg Thr Ala Thr His Thr Cys Asp Tyr Ala Gly Cys Gly Lys Thr Tyr
            420                 425                 430

Thr Lys Ser Ser His Leu Lys Ala His Leu Arg Thr His Thr Gly Glu
        435                 440                 445

Lys Pro Tyr His Cys Asp Trp Asp Gly Cys Gly Trp Lys Phe Ala Arg
    450                 455                 460

Ser Asp Glu Leu Thr Arg His Tyr Arg Lys His Thr Gly His Arg Pro
465                 470                 475                 480

Phe Gln Cys Gln Lys Cys Asp Arg Ala Phe Ser Arg Ser Asp His Leu
                485                 490                 495

Ala Leu His Met Lys Arg His Phe
            500

<210>    121
<211>    1515
<212>    DNA
<213>    Artificial Sequence

<220>
<223>    His-NLS-Klf4-JO-84 MTD cDNA Sequence

<400>    121
atgggcagca gccatcatca tcatcatcac agcagcggcc tggtgccgcg cggcagccat        60

atgaagaaga agaggaaggc tgtcagcgac gcgctgctcc catctttctc cacgttcgcg       120

tctggcccgg cgggaaggga gaagacactg cgtcaagcag gtgccccgaa taaccgctgg       180

cgggaggagc tctcccacat gaagcgactt cccccagtgc ttcccggccg cccctatgac       240

ctggcggcgg cgaccgtggc cacagacctg gagagcggcg gagccggtgc ggcttgcggc       300

ggtagcaacc tggcgcccct acctcggaga gagaccgagg agttcaacga tctcctggac 360

ctggacttta ttctctccaa ttcgctgacc catcctccgg agtcagtggc cgccaccgtg 420

tcctcgtcag cgtcagcctc ctcttcgtcg tcgccgtcga gcagcggccc tgccagcgcg 480

ccctccacct gcagcttcac ctatccgatc cgggccggga acgacccggg cgtggcgccg 540

ggcggcacgg gcggaggcct cctctatggc agggagtccg ctccccctcc gacggctccc 600

ttcaacctgg cggacatcaa cgacgtgagc ccctcgggcg gcttcgtggc cgagctcctg 660

cggccagaat tggacccggt gtacattccg ccgcagcagc cgcagccgcc aggtggcggg 720

ctgatgggca agttcgtgct gaaggcgtcg ctgagcgccc ctggcagcga gtacggcagc 780

ccgtcggtca tcagcgtcag caaaggcagc cctgacggca gccacccggt ggtggtggcg 840

ccctacaacg gcgggccgcc gcgcacgtgc cccaagatca agcaggaggc ggtctcttcg 900

tgcacccact tgggcgctgg accccctctc agcaatggcc accggccggc tgcacacgac 960

ttccccctgg ggcggcagct ccccagcagg actaccccga ccctgggtct tgaggaagtg 1020

ctgagcagca gggactgtca ccctgccctg ccgcttcctc ccggcttcca tccccacccg 1080

gggcccaatt acccatcctt cctgcccgat cagatgcagc cgcaagtccc gccgctccat 1140

taccaagagc tcatgccacc cggttcctgc atgccagagg agcccaagcc aaagagggga 1200

agacgatcgt ggccccggaa aaggaccgcc acccacactt gtgattacgc gggctgcggc 1260

aaaacctaca caaagagttc ccatctcaag gcacacctgc gaacccacac aggtgagaaa 1320

ccttaccact gtgactggga cggctgtgga tggaaattcg cccgctcaga tgaactgacc 1380

aggcactacc gtaaacacac ggggcaccgc ccgttccagt gccaaaaatg cgaccgagca 1440

ttttccaggt cggaccacct cgccttacac atgaagaggc attttctggt ggcggcgctg 1500

ctggcggtgc tgtaa                                                    1515

<210>  122
<211>  504
<212>  PRT
<213>  Artificial Sequence

<220>
<223>  His-NLS-Klf4-JO-84 MTD Amino Acid Sequence

<400>  122
Met Gly Ser Ser His His His His His His Ser Ser Gly Leu Val Pro
1               5                   10                  15

Arg Gly Ser His Met Lys Lys Lys Arg Lys Ala Val Ser Asp Ala Leu
            20                  25                  30

Leu Pro Ser Phe Ser Thr Phe Ala Ser Gly Pro Ala Gly Arg Glu Lys
        35                  40                  45

Thr Leu Arg Gln Ala Gly Ala Pro Asn Asn Arg Trp Arg Glu Glu Leu
    50                  55                  60

Ser His Met Lys Arg Leu Pro Pro Val Leu Pro Gly Arg Pro Tyr Asp
65                  70                  75                  80

Leu Ala Ala Ala Thr Val Ala Thr Asp Leu Glu Ser Gly Gly Ala Gly
                85                  90                  95

Ala Ala Cys Gly Gly Ser Asn Leu Ala Pro Leu Pro Arg Arg Glu Thr
            100                 105                 110

Glu Glu Phe Asn Asp Leu Leu Asp Leu Asp Phe Ile Leu Ser Asn Ser
        115                 120                 125

Leu Thr His Pro Pro Glu Ser Val Ala Ala Thr Val Ser Ser Ser Ala
    130                 135                 140

Ser Ala Ser Ser Ser Ser Ser Pro Ser Ser Ser Gly Pro Ala Ser Ala
145                 150                 155                 160

Pro Ser Thr Cys Ser Phe Thr Tyr Pro Ile Arg Ala Gly Asn Asp Pro
                165                 170                 175

Gly Val Ala Pro Gly Gly Thr Gly Gly Gly Leu Leu Tyr Gly Arg Glu
            180                 185                 190

Ser Ala Pro Pro Pro Thr Ala Pro Phe Asn Leu Ala Asp Ile Asn Asp
        195                 200                 205

Val Ser Pro Ser Gly Gly Phe Val Ala Glu Leu Leu Arg Pro Glu Leu
    210                 215                 220

Asp Pro Val Tyr Ile Pro Pro Gln Gln Pro Gln Pro Pro Gly Gly Gly
225                 230                 235                 240

Leu Met Gly Lys Phe Val Leu Lys Ala Ser Leu Ser Ala Pro Gly Ser
                245                 250                 255

Glu Tyr Gly Ser Pro Ser Val Ile Ser Val Ser Lys Gly Ser Pro Asp
            260                 265                 270

Gly Ser His Pro Val Val Val Ala Pro Tyr Asn Gly Gly Pro Pro Arg
        275                 280                 285

Thr Cys Pro Lys Ile Lys Gln Glu Ala Val Ser Ser Cys Thr His Leu
    290                 295                 300

Gly Ala Gly Pro Pro Leu Ser Asn Gly His Arg Pro Ala Ala His Asp
305                 310                 315                 320

Phe Pro Leu Gly Arg Gln Leu Pro Ser Arg Thr Thr Pro Thr Leu Gly
                325                 330                 335

Leu Glu Glu Val Leu Ser Ser Arg Asp Cys His Pro Ala Leu Pro Leu
            340                 345                 350

Pro Pro Gly Phe His Pro His Pro Gly Pro Asn Tyr Pro Ser Phe Leu
        355                 360                 365

Pro Asp Gln Met Gln Pro Gln Val Pro Pro Leu His Tyr Gln Glu Leu
    370                 375                 380

Met Pro Pro Gly Ser Cys Met Pro Glu Glu Pro Lys Pro Lys Arg Gly
385                 390                 395                 400

Arg Arg Ser Trp Pro Arg Lys Arg Thr Ala Thr His Thr Cys Asp Tyr
                405                 410                 415

Ala Gly Cys Gly Lys Thr Tyr Thr Lys Ser Ser His Leu Lys Ala His
            420                 425                 430

Leu Arg Thr His Thr Gly Glu Lys Pro Tyr His Cys Asp Trp Asp Gly
        435                 440                 445

Cys Gly Trp Lys Phe Ala Arg Ser Asp Glu Leu Thr Arg His Tyr Arg
    450                 455                 460

Lys His Thr Gly His Arg Pro Phe Gln Cys Gln Lys Cys Asp Arg Ala
465                 470                 475                 480

Phe Ser Arg Ser Asp His Leu Ala Leu His Met Lys Arg His Phe Leu
                485                 490                 495

Val Ala Ala Leu Leu Ala Val Leu
            500

<210>   123
<211>   1542
<212>   DNA
<213>   Artificial Sequence

<220>
<223>   His-NLS-JO-84 MTD-Klf4-JO-84 MTD cDNA Sequence

<400> 123

atgggcagca gccatcatca tcatcatcac agcagcggcc tggtgccgcg cggcagccat 60

atgaagaaga agaggaagct ggtggcggcg ctgctggcgg tgctggctgt cagcgacgcg 120

ctgctcccat ctttctccac gttcgcgtct ggcccggcgg gaagggagaa gacactgcgt 180

caagcaggtg ccccgaataa ccgctggcgg gaggagctct cccacatgaa gcgacttccc 240

ccagtgcttc ccggccgccc ctatgacctg gcggcggcga ccgtggccac agacctggag 300

agcggcggag ccggtgcggc ttgcggcggt agcaacctgg cgcccctacc tcggagagag 360

accgaggagt tcaacgatct cctggacctg gactttattc tctccaattc gctgacccat 420

cctccggagt cagtggccgc caccgtgtcc tcgtcagcgt cagcctcctc ttcgtcgtcg 480

ccgtcgagca gcggccctgc cagcgcgccc tccacctgca gcttcaccta tccgatccgg 540

gccgggaacg acccgggcgt ggcgccgggc ggcacgggcg gaggcctcct ctatggcagg 600

gagtccgctc cccctccgac ggctcccttc aacctggcgg acatcaacga cgtgagcccc 660

tcgggcggct tcgtggccga gctcctgcgg ccagaattgg acccggtgta cattccgccg 720

cagcagccgc agccgccagg tggcgggctg atgggcaagt tcgtgctgaa ggcgtcgctg 780

agcgcccctg gcagcgagta cggcagcccg tcggtcatca gcgtcagcaa aggcagccct 840

gacggcagcc acccggtggt ggtggcgccc tacaacggcg ggccgccgcg cacgtgcccc 900

aagatcaagc aggaggcggt ctcttcgtgc acccacttgg gcgctggacc ccctctcagc 960

aatggccacc ggccggctgc acacgacttc cccctggggc ggcagctccc cagcaggact 1020

accccgaccc tgggtcttga ggaagtgctg agcagcaggg actgtcaccc tgccctgccg 1080

cttcctcccg gcttccatcc ccacccgggg cccaattacc catccttcct gcccgatcag 1140

atgcagccgc aagtcccgcc gctccattac caagagctca tgccacccgg ttcctgcatg 1200

ccagaggagc ccaagccaaa gaggggaaga cgatcgtggc cccggaaaag gaccgccacc 1260

cacacttgtg attacgcggg ctgcggcaaa acctacacaa agagttccca tctcaaggca 1320

cacctgcgaa cccacacagg tgagaaacct taccactgtg actgggacgg ctgtggatgg 1380

aaattcgccc gctcagatga actgaccagg cactaccgta aacacacggg gcaccgcccg 1440

ttccagtgcc aaaaatgcga ccgagcattt tccaggtcgg accacctcgc cttacacatg 1500

aagaggcatt ttctggtggc ggcgctgctg gcggtgctgt aa 1542

<210> 124
<211> 513
<212> PRT
<213> Artificial Sequence

<220>
<223> His-NLS-JO-84 MTD-Klf4-JO-84 MTD Amino Acid Sequence

<400> 124
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Ala Val Ser Asp Ala Leu Leu Pro Ser Phe Ser Thr Phe
        35                  40                  45

Ala Ser Gly Pro Ala Gly Arg Glu Lys Thr Leu Arg Gln Ala Gly Ala
    50                  55                  60

Pro Asn Asn Arg Trp Arg Glu Glu Leu Ser His Met Lys Arg Leu Pro
65                  70                  75                  80

Pro Val Leu Pro Gly Arg Pro Tyr Asp Leu Ala Ala Ala Thr Val Ala

                85                  90                  95

Thr Asp Leu Glu Ser Gly Gly Ala Gly Ala Ala Cys Gly Gly Ser Asn
            100                 105                 110

Leu Ala Pro Leu Pro Arg Arg Glu Thr Glu Glu Phe Asn Asp Leu Leu
        115                 120                 125

Asp Leu Asp Phe Ile Leu Ser Asn Ser Leu Thr His Pro Pro Glu Ser
    130                 135                 140

Val Ala Ala Thr Val Ser Ser Ser Ala Ser Ala Ser Ser Ser Ser Ser
145                 150                 155                 160

Pro Ser Ser Ser Gly Pro Ala Ser Ala Pro Ser Thr Cys Ser Phe Thr
                165                 170                 175

Tyr Pro Ile Arg Ala Gly Asn Asp Pro Gly Val Ala Pro Gly Gly Thr
            180                 185                 190

Gly Gly Gly Leu Leu Tyr Gly Arg Glu Ser Ala Pro Pro Pro Thr Ala
        195                 200                 205

Pro Phe Asn Leu Ala Asp Ile Asn Asp Val Ser Pro Ser Gly Gly Phe
    210                 215                 220

Val Ala Glu Leu Leu Arg Pro Glu Leu Asp Pro Val Tyr Ile Pro Pro
225                 230                 235                 240

Gln Gln Pro Gln Pro Pro Gly Gly Gly Leu Met Gly Lys Phe Val Leu
                245                 250                 255

Lys Ala Ser Leu Ser Ala Pro Gly Ser Glu Tyr Gly Ser Pro Ser Val
            260                 265                 270

Ile Ser Val Ser Lys Gly Ser Pro Asp Gly Ser His Pro Val Val Val
        275                 280                 285

Ala Pro Tyr Asn Gly Gly Pro Pro Arg Thr Cys Pro Lys Ile Lys Gln
    290                 295                 300

Glu Ala Val Ser Ser Cys Thr His Leu Gly Ala Gly Pro Pro Leu Ser
305                 310                 315                 320

Asn Gly His Arg Pro Ala Ala His Asp Phe Pro Leu Gly Arg Gln Leu
                325                 330                 335

Pro Ser Arg Thr Thr Pro Thr Leu Gly Leu Glu Glu Val Leu Ser Ser
            340                 345                 350

Arg Asp Cys His Pro Ala Leu Pro Leu Pro Pro Gly Phe His Pro His
        355                 360                 365

Pro Gly Pro Asn Tyr Pro Ser Phe Leu Pro Asp Gln Met Gln Pro Gln
    370                 375                 380

Val Pro Pro Leu His Tyr Gln Glu Leu Met Pro Pro Gly Ser Cys Met
385                 390                 395                 400

Pro Glu Glu Pro Lys Pro Lys Arg Gly Arg Arg Ser Trp Pro Arg Lys
                405                 410                 415

Arg Thr Ala Thr His Thr Cys Asp Tyr Ala Gly Cys Gly Lys Thr Tyr
            420                 425                 430

Thr Lys Ser Ser His Leu Lys Ala His Leu Arg Thr His Thr Gly Glu
        435                 440                 445

Lys Pro Tyr His Cys Asp Trp Asp Gly Cys Gly Trp Lys Phe Ala Arg
    450                 455                 460

Ser Asp Glu Leu Thr Arg His Tyr Arg Lys His Thr Gly His Arg Pro
465                 470                 475                 480

Phe Gln Cys Gln Lys Cys Asp Arg Ala Phe Ser Arg Ser Asp His Leu
                485                 490                 495

Ala Leu His Met Lys Arg His Phe Leu Val Ala Ala Leu Leu Ala Val
            500                 505                 510

Leu

<210> 125
<211> 1512
<212> DNA
<213> Artificial Sequence

<220>
<223> His-NLS-JO-86 MTD-Klf4 cDNA Sequence

<400> 125
atgggcagca gccatcatca tcatcatcac agcagcggcc tggtgccgcg cggcagccat 60

atgaagaaga agaggaagct ggcggtgctg gcggcggcgc cggctgtcag cgacgcgctg 120

ctcccatctt tctccacgtt cgcgtctggc ccggcgggaa gggagaagac actgcgtcaa 180

gcaggtgccc cgaataaccg ctggcgggag gagctctccc acatgaagcg acttccccca 240

gtgcttcccg gccgccccta tgacctggcg gcggcgaccg tggccacaga cctggagagc 300

ggcggagccg gtgcggcttg cggcggtagc aacctggcgc ccctacctcg gagagagacc 360

gaggagttca acgatctcct ggacctggac tttattctct ccaattcgct gacccatcct 420

ccggagtcag tggccgccac cgtgtcctcg tcagcgtcag cctcctcttc gtcgtcgccg 480

tcgagcagcg gccctgccag cgcgccctcc acctgcagct tcacctatcc gatccgggcc 540

gggaacgacc cgggcgtggc gccgggcggc acgggcggag gcctcctcta tggcagggag 600

tccgctcccc ctccgacggc tcccttcaac ctggcggaca tcaacgacgt gagcccctcg 660

ggcggcttcg tggccgagct cctgcggcca gaattggacc cggtgtacat tccgccgcag 720

cagccgcagc cgccaggtgg cgggctgatg ggcaagttcg tgctgaaggc gtcgctgagc 780

gcccctggca gcgagtacgg cagcccgtcg gtcatcagcg tcagcaaagg cagccctgac 840

ggcagccacc cggtggtggt ggcgccctac aacggcgggc cgccgcgcac gtgccccaag 900

atcaagcagg aggcggtctc ttcgtgcacc cacttgggcg ctggaccccc tctcagcaat 960

ggccaccggc cggctgcaca cgacttcccc ctggggcggc agctccccag caggactacc 1020

ccgaccctgg gtcttgagga agtgctgagc agcagggact gtcaccctgc cctgccgctt 1080

cctcccggct tccatcccca cccggggccc aattacccat ccttcctgcc cgatcagatg 1140

cagccgcaag tcccgccgct ccattaccaa gagctcatgc cacccggttc ctgcatgcca 1200

gaggagccca agccaaagag gggaagacga tcgtggcccc ggaaaaggac cgccacccac 1260

acttgtgatt acgcgggctg cggcaaaacc tacacaaaga gttcccatct caaggcacac 1320

ctgcgaaccc acacaggtga gaaaccttac cactgtgact gggacggctg tggatggaaa 1380

ttcgcccgct cagatgaact gaccaggcac taccgtaaac acacggggca ccgcccgttc 1440

cagtgccaaa aatgcgaccg agcattttcc aggtcggacc acctcgcctt acacatgaag 1500

aggcattttt aa 1512

<210> 126
<211> 503
<212> PRT
<213> Artificial Sequence

<220>
<223> His-NLS-JO-86 MTD-Klf4 Amino Acid Sequence

<400> 126
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Ala Val Ser Asp Ala Leu Leu Pro Ser Phe Ser Thr Phe Ala
        35                  40                  45

Ser Gly Pro Ala Gly Arg Glu Lys Thr Leu Arg Gln Ala Gly Ala Pro
    50                  55                  60

Asn Asn Arg Trp Arg Glu Glu Leu Ser His Met Lys Arg Leu Pro Pro
65                  70                  75                  80

Val Leu Pro Gly Arg Pro Tyr Asp Leu Ala Ala Ala Thr Val Ala Thr
                85                  90                  95

Asp Leu Glu Ser Gly Gly Ala Gly Ala Ala Cys Gly Gly Ser Asn Leu
            100                 105                 110

Ala Pro Leu Pro Arg Arg Glu Thr Glu Glu Phe Asn Asp Leu Leu Asp
        115                 120                 125

Leu Asp Phe Ile Leu Ser Asn Ser Leu Thr His Pro Pro Glu Ser Val
    130                 135                 140

Ala Ala Thr Val Ser Ser Ser Ala Ser Ala Ser Ser Ser Ser Ser Pro
145                 150                 155                 160

Ser Ser Ser Gly Pro Ala Ser Ala Pro Ser Thr Cys Ser Phe Thr Tyr
                165                 170                 175

Pro Ile Arg Ala Gly Asn Asp Pro Gly Val Ala Pro Gly Gly Thr Gly
            180                 185                 190

Gly Gly Leu Leu Tyr Gly Arg Glu Ser Ala Pro Pro Pro Thr Ala Pro
        195                 200                 205

Phe Asn Leu Ala Asp Ile Asn Asp Val Ser Pro Ser Gly Gly Phe Val
    210                 215                 220

Ala Glu Leu Leu Arg Pro Glu Leu Asp Pro Val Tyr Ile Pro Pro Gln

225                 230                 235                 240

Gln Pro Gln Pro Pro Gly Gly Gly Leu Met Gly Lys Phe Val Leu Lys
                245                 250                 255

Ala Ser Leu Ser Ala Pro Gly Ser Glu Tyr Gly Ser Pro Ser Val Ile
            260                 265                 270

Ser Val Ser Lys Gly Ser Pro Asp Gly Ser His Pro Val Val Val Ala
        275                 280                 285

Pro Tyr Asn Gly Gly Pro Pro Arg Thr Cys Pro Lys Ile Lys Gln Glu
    290                 295                 300

Ala Val Ser Ser Cys Thr His Leu Gly Ala Gly Pro Pro Leu Ser Asn
305                 310                 315                 320

Gly His Arg Pro Ala Ala His Asp Phe Pro Leu Gly Arg Gln Leu Pro
                325                 330                 335

Ser Arg Thr Thr Pro Thr Leu Gly Leu Glu Glu Val Leu Ser Ser Arg
            340                 345                 350

Asp Cys His Pro Ala Leu Pro Leu Pro Pro Gly Phe His Pro His Pro
        355                 360                 365

Gly Pro Asn Tyr Pro Ser Phe Leu Pro Asp Gln Met Gln Pro Gln Val
    370                 375                 380

Pro Pro Leu His Tyr Gln Glu Leu Met Pro Pro Gly Ser Cys Met Pro
385                 390                 395                 400

Glu Glu Pro Lys Pro Lys Arg Gly Arg Arg Ser Trp Pro Arg Lys Arg
                405                 410                 415

Thr Ala Thr His Thr Cys Asp Tyr Ala Gly Cys Gly Lys Thr Tyr Thr
            420                 425                 430

Lys Ser Ser His Leu Lys Ala His Leu Arg Thr His Thr Gly Glu Lys
        435                 440                 445

Pro Tyr His Cys Asp Trp Asp Gly Cys Gly Trp Lys Phe Ala Arg Ser
    450                 455                 460

Asp Glu Leu Thr Arg His Tyr Arg Lys His Thr Gly His Arg Pro Phe
465                 470                 475                 480

Gln Cys Gln Lys Cys Asp Arg Ala Phe Ser Arg Ser Asp His Leu Ala
                485                 490                 495

Leu His Met Lys Arg His Phe
            500

<210> 127
<211> 1512
<212> DNA
<213> Artificial Sequence

<220>
<223> His-NLS-Klf4-JO-86 MTD cDNA Sequence

<400> 127
atgggcagca gccatcatca tcatcatcac agcagcggcc tggtgccgcg cggcagccat 60

atgaagaaga agaggaaggc tgtcagcgac gcgctgctcc catctttctc cacgttcgcg 120

tctggcccgg cgggaaggga gaagacactg cgtcaagcag gtgccccgaa taaccgctgg 180

cgggaggagc tctcccacat gaagcgactt cccccagtgc ttcccggccg cccctatgac 240

ctggcggcgg cgaccgtggc cacagacctg gagagcggcg gagccggtgc ggcttgcggc 300

ggtagcaacc tggcgcccct acctcggaga gagaccgagg agttcaacga tctcctggac 360

ctggacttta ttctctccaa ttcgctgacc catcctccgg agtcagtggc cgccaccgtg 420

tcctcgtcag cgtcagcctc ctcttcgtcg tcgccgtcga gcagcggccc tgccagcgcg 480

ccctccacct gcagcttcac ctatccgatc cgggccggga acgacccggg cgtggcgccg 540

ggcggcacgg gcggaggcct cctctatggc agggagtccg ctccccctcc gacggctccc 600

ttcaacctgg cggacatcaa cgacgtgagc ccctcgggcg gcttcgtggc cgagctcctg 660

cggccagaat tggacccggt gtacattccg ccgcagcagc cgcagccgcc aggtggcggg 720

ctgatgggca agttcgtgct gaaggcgtcg ctgagcgccc ctggcagcga gtacggcagc 780

ccgtcggtca tcagcgtcag caaaggcagc cctgacggca gccacccggt ggtggtggcg 840

ccctacaacg gcgggccgcc gcgcacgtgc cccaagatca agcaggaggc ggtctcttcg 900

tgcacccact tgggcgctgg accccctctc agcaatggcc accggccggc tgcacacgac 960

ttccccctgg ggcggcagct ccccagcagg actaccccga ccctgggtct tgaggaagtg 1020

ctgagcagca gggactgtca ccctgccctg ccgcttcctc ccggcttcca tccccacccg 1080

gggcccaatt acccatcctt cctgcccgat cagatgcagc cgcaagtccc gccgctccat 1140

taccaagagc tcatgccacc cggttcctgc atgccagagg agcccaagcc aaagagggga 1200

agacgatcgt ggccccggaa aaggaccgcc acccacactt gtgattacgc gggctgcggc 1260

aaaacctaca caaagagttc ccatctcaag gcacacctgc gaacccacac aggtgagaaa 1320

ccttaccact gtgactggga cggctgtgga tggaaattcg cccgctcaga tgaactgacc 1380

aggcactacc gtaaacacac ggggcaccgc ccgttccagt gccaaaaatg cgaccgagca 1440

ttttccaggt cggaccacct cgccttacac atgaagaggc attttctggc ggtgctggcg 1500

gcggcgccgt aa 1512

<210> 128
<211> 503
<212> PRT

<213>    Artificial Sequence

<220>
<223>    His-NLS-Klf4-JO-86 MTD Amino Acid Sequence

<400>    128
Met Gly Ser Ser His His His His His His Ser Ser Gly Leu Val Pro
1               5                   10                  15

Arg Gly Ser His Met Lys Lys Lys Arg Lys Ala Val Ser Asp Ala Leu
            20                  25                  30

Leu Pro Ser Phe Ser Thr Phe Ala Ser Gly Pro Ala Gly Arg Glu Lys
        35                  40                  45

Thr Leu Arg Gln Ala Gly Ala Pro Asn Asn Arg Trp Arg Glu Glu Leu
    50                  55                  60

Ser His Met Lys Arg Leu Pro Pro Val Leu Pro Gly Arg Pro Tyr Asp
65                  70                  75                  80

Leu Ala Ala Ala Thr Val Ala Thr Asp Leu Glu Ser Gly Gly Ala Gly
                85                  90                  95

Ala Ala Cys Gly Gly Ser Asn Leu Ala Pro Leu Pro Arg Arg Glu Thr
            100                 105                 110

Glu Glu Phe Asn Asp Leu Leu Asp Leu Asp Phe Ile Leu Ser Asn Ser
        115                 120                 125

Leu Thr His Pro Pro Glu Ser Val Ala Ala Thr Val Ser Ser Ser Ala
    130                 135                 140

Ser Ala Ser Ser Ser Ser Ser Pro Ser Ser Ser Gly Pro Ala Ser Ala
145                 150                 155                 160

Pro Ser Thr Cys Ser Phe Thr Tyr Pro Ile Arg Ala Gly Asn Asp Pro
                165                 170                 175

Gly Val Ala Pro Gly Gly Thr Gly Gly Gly Leu Leu Tyr Gly Arg Glu
            180                 185                 190

Ser Ala Pro Pro Pro Thr Ala Pro Phe Asn Leu Ala Asp Ile Asn Asp
        195                 200                 205

Val Ser Pro Ser Gly Gly Phe Val Ala Glu Leu Leu Arg Pro Glu Leu
    210                 215                 220

Asp Pro Val Tyr Ile Pro Pro Gln Gln Pro Gln Pro Pro Gly Gly Gly
225                 230                 235                 240

Leu Met Gly Lys Phe Val Leu Lys Ala Ser Leu Ser Ala Pro Gly Ser
                245                 250                 255

Glu Tyr Gly Ser Pro Ser Val Ile Ser Val Ser Lys Gly Ser Pro Asp
            260                 265                 270

Gly Ser His Pro Val Val Val Ala Pro Tyr Asn Gly Gly Pro Pro Arg
        275                 280                 285

Thr Cys Pro Lys Ile Lys Gln Glu Ala Val Ser Ser Cys Thr His Leu
    290                 295                 300

Gly Ala Gly Pro Pro Leu Ser Asn Gly His Arg Pro Ala Ala His Asp
305                 310                 315                 320

Phe Pro Leu Gly Arg Gln Leu Pro Ser Arg Thr Thr Pro Thr Leu Gly
                325                 330                 335

Leu Glu Glu Val Leu Ser Ser Arg Asp Cys His Pro Ala Leu Pro Leu
            340                 345                 350

Pro Pro Gly Phe His Pro His Pro Gly Pro Asn Tyr Pro Ser Phe Leu
        355                 360                 365

Pro Asp Gln Met Gln Pro Gln Val Pro Pro Leu His Tyr Gln Glu Leu
    370                 375                 380

Met Pro Pro Gly Ser Cys Met Pro Glu Glu Pro Lys Pro Lys Arg Gly

385                 390                 395                 400

Arg Arg Ser Trp Pro Arg Lys Arg Thr Ala Thr His Thr Cys Asp Tyr
                405                 410                 415

Ala Gly Cys Gly Lys Thr Tyr Thr Lys Ser Ser His Leu Lys Ala His
            420                 425                 430

Leu Arg Thr His Thr Gly Glu Lys Pro Tyr His Cys Asp Trp Asp Gly
        435                 440                 445

Cys Gly Trp Lys Phe Ala Arg Ser Asp Glu Leu Thr Arg His Tyr Arg
    450                 455                 460

Lys His Thr Gly His Arg Pro Phe Gln Cys Gln Lys Cys Asp Arg Ala
465                 470                 475                 480

Phe Ser Arg Ser Asp His Leu Ala Leu His Met Lys Arg His Phe Leu
                485                 490                 495

Ala Val Leu Ala Ala Ala Pro
            500

<210>   129
<211>   1536
<212>   DNA
<213>   Artificial Sequence

<220>
<223>   His-NLS-JO-86 MTD-Klf4-JO-86 MTD cDNA Sequence

<400>   129
atgggcagca gccatcatca tcatcatcac agcagcggcc tggtgccgcg cggcagccat      60

atgaagaaga agaggaagct ggcggtgctg gcggcggcgc cggctgtcag cgacgcgctg     120

ctcccatctt tctccacgtt cgcgtctggc ccggcgggaa gggagaagac actgcgtcaa     180

gcaggtgccc cgaataaccg ctggcgggag gagctctccc acatgaagcg acttccccca 240

gtgcttcccg gccgccccta tgacctggcg gcggcgaccg tggccacaga cctggagagc 300

ggcggagccg gtgcggcttg cggcggtagc aacctggcgc ccctacctcg gagagagacc 360

gaggagttca acgatctcct ggacctggac tttattctct ccaattcgct gacccatcct 420

ccggagtcag tggccgccac cgtgtcctcg tcagcgtcag cctcctcttc gtcgtcgccg 480

tcgagcagcg gccctgccag cgcgccctcc acctgcagct tcacctatcc gatccgggcc 540

gggaacgacc cgggcgtggc gccgggcggc acgggcggag gcctcctcta tggcagggag 600

tccgctcccc ctccgacggc tcccttcaac ctggcggaca tcaacgacgt gagcccctcg 660

ggcggcttcg tggccgagct cctgcggcca gaattggacc cggtgtacat tccgccgcag 720

cagccgcagc cgccaggtgg cgggctgatg ggcaagttcg tgctgaaggc gtcgctgagc 780

gcccctggca gcgagtacgg cagcccgtcg gtcatcagcg tcagcaaagg cagccctgac 840

ggcagccacc cggtggtggt ggcgccctac aacggcgggc cgccgcgcac gtgccccaag 900

atcaagcagg aggcggtctc ttcgtgcacc cacttgggcg ctggaccccc tctcagcaat 960

ggccaccggc cggctgcaca cgacttcccc ctggggcggc agctccccag caggactacc 1020

ccgaccctgg gtcttgagga agtgctgagc agcagggact gtcaccctgc cctgccgctt 1080

cctcccggct tccatcccca cccggggccc aattacccat ccttcctgcc cgatcagatg 1140

cagccgcaag tcccgccgct ccattaccaa gagctcatgc cacccggttc ctgcatgcca 1200

gaggagccca agccaaagag gggaagacga tcgtggcccc ggaaaaggac cgccacccac 1260

acttgtgatt acgcgggctg cggcaaaacc tacacaaaga gttcccatct caaggcacac 1320

ctgcgaaccc acacaggtga gaaaccttac cactgtgact gggacggctg tggatggaaa 1380

ttcgcccgct cagatgaact gaccaggcac taccgtaaac acacggggca ccgcccgttc 1440

cagtgccaaa aatgcgaccg agcattttcc aggtcggacc acctcgcctt acacatgaag 1500

aggcattttc tggcggtgct ggcggcggcg ccgtaa 1536

<210> 130
<211> 511
<212> PRT
<213> Artificial Sequence

<220>
<223> His-NLS-JO-86 MTD-Klf4-JO-86 MTD Amino Acid Sequence

<400> 130
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Ala Val Ser Asp Ala Leu Leu Pro Ser Phe Ser Thr Phe Ala
        35                  40                  45

Ser Gly Pro Ala Gly Arg Glu Lys Thr Leu Arg Gln Ala Gly Ala Pro
    50                  55                  60

Asn Asn Arg Trp Arg Glu Glu Leu Ser His Met Lys Arg Leu Pro Pro
65                  70                  75                  80

Val Leu Pro Gly Arg Pro Tyr Asp Leu Ala Ala Ala Thr Val Ala Thr
                85                  90                  95

Asp Leu Glu Ser Gly Gly Ala Gly Ala Ala Cys Gly Gly Ser Asn Leu
            100                 105                 110

Ala Pro Leu Pro Arg Arg Glu Thr Glu Glu Phe Asn Asp Leu Leu Asp
        115                 120                 125

Leu Asp Phe Ile Leu Ser Asn Ser Leu Thr His Pro Pro Glu Ser Val
    130                 135                 140

Ala Ala Thr Val Ser Ser Ser Ala Ser Ala Ser Ser Ser Ser Ser Pro
145                 150                 155                 160

Ser Ser Ser Gly Pro Ala Ser Ala Pro Ser Thr Cys Ser Phe Thr Tyr
                165                 170                 175

Pro Ile Arg Ala Gly Asn Asp Pro Gly Val Ala Pro Gly Gly Thr Gly
            180                 185                 190

Gly Gly Leu Leu Tyr Gly Arg Glu Ser Ala Pro Pro Pro Thr Ala Pro
        195                 200                 205

Phe Asn Leu Ala Asp Ile Asn Asp Val Ser Pro Ser Gly Gly Phe Val
    210                 215                 220

Ala Glu Leu Leu Arg Pro Glu Leu Asp Pro Val Tyr Ile Pro Pro Gln
225                 230                 235                 240

Gln Pro Gln Pro Pro Gly Gly Gly Leu Met Gly Lys Phe Val Leu Lys
                245                 250                 255

Ala Ser Leu Ser Ala Pro Gly Ser Glu Tyr Gly Ser Pro Ser Val Ile
            260                 265                 270

Ser Val Ser Lys Gly Ser Pro Asp Gly Ser His Pro Val Val Val Ala
        275                 280                 285

Pro Tyr Asn Gly Gly Pro Pro Arg Thr Cys Pro Lys Ile Lys Gln Glu
    290                 295                 300

Ala Val Ser Ser Cys Thr His Leu Gly Ala Gly Pro Pro Leu Ser Asn
305                 310                 315                 320

Gly His Arg Pro Ala Ala His Asp Phe Pro Leu Gly Arg Gln Leu Pro
                325                 330                 335

Ser Arg Thr Thr Pro Thr Leu Gly Leu Glu Glu Val Leu Ser Ser Arg
            340                 345                 350

Asp Cys His Pro Ala Leu Pro Leu Pro Pro Gly Phe His Pro His Pro
        355                 360                 365

Gly Pro Asn Tyr Pro Ser Phe Leu Pro Asp Gln Met Gln Pro Gln Val
    370                 375                 380

Pro Pro Leu His Tyr Gln Glu Leu Met Pro Pro Gly Ser Cys Met Pro
385                 390                 395                 400

Glu Glu Pro Lys Pro Lys Arg Gly Arg Arg Ser Trp Pro Arg Lys Arg
                405                 410                 415

Thr Ala Thr His Thr Cys Asp Tyr Ala Gly Cys Gly Lys Thr Tyr Thr
            420                 425                 430

Lys Ser Ser His Leu Lys Ala His Leu Arg Thr His Thr Gly Glu Lys
        435                 440                 445

Pro Tyr His Cys Asp Trp Asp Gly Cys Gly Trp Lys Phe Ala Arg Ser
    450                 455                 460

Asp Glu Leu Thr Arg His Tyr Arg Lys His Thr Gly His Arg Pro Phe
465                 470                 475                 480

Gln Cys Gln Lys Cys Asp Arg Ala Phe Ser Arg Ser Asp His Leu Ala
                485                 490                 495

Leu His Met Lys Arg His Phe Leu Ala Val Leu Ala Ala Ala Pro
            500                 505                 510

<210>   131
<211>   1383
<212>   DNA
<213>   Artificial Sequence

<220>
<223>    NLS-cMyc cDNA Sequence

<400>    131
atgaagaaga agaggaagct ggattttttt cgggtagtgg aaaaccagca gcctcccgcg    60

acgatgcccc tcaacgttag cttcaccaac aggaactatg acctcgacta cgactcggtg    120

cagccgtatt tctactgcga cgaggaggag aacttctacc agcagcagca gcagagcgag    180

ctgcagcccc cggcgcccag cgaggatatc tggaagaaat tcgagctgct gcccaccccg    240

cccctgtccc ctagccgccg ctccgggctc tgctcgccct cctacgttgc ggtcacaccc    300

ttctcccttc ggggagacaa cgacggcggt ggcgggagct tctccacggc cgaccagctg    360

gagatggtga ccgagctgct gggaggagac atggtgaacc agagtttcat ctgcgacccg    420

gacgacgaga ccttcatcaa aaacatcatc atccaggact gtatgtggag cggcttctcg    480

gccgccgcca agctcgtctc agagaagctg gcctcctacc aggctgcgcg caaagacagc    540

ggcagcccga accccgcccg cggccacagc gtctgctcca cctccagctt gtacctgcag    600

gatctgagcg ccgccgcctc agagtgcatc gacccctcgg tggtcttccc ctaccctctc    660

aacgacagca gctcgcccaa gtcctgcgcc tcgcaagact ccagcgcctt ctctccgtcc    720

tcggattctc tgctctcctc gacggagtcc tccccgcagg gcagccccga gcccctggtg    780

ctccatgagg agacaccgcc caccaccagc agcgactctg aggaggaaca agaagatgag    840

gaagaaatcg atgttgtttc tgtggaaaag aggcaggctc ctggcaaaag gtcagagtct    900

ggatcacctt ctgctggagg ccacagcaaa cctcctcaca gcccactggt cctcaagagg    960

tgccacgtct ccacacatca gcacaactac gcagcgcctc cctccactcg gaaggactat    1020

cctgctgcca agagggtcaa gttggacagt gtcagagtcc tgagacagat cagcaacaac 1080

cgaaaatgca ccagccccag gtcctcggac accgaggaga atgtcaagag gcgaacacac 1140

aacgtcttgg agcgccagag gaggaacgag ctaaaacgga gcttttttgc cctgcgtgac 1200

cagatcccgg agttggaaaa caatgaaaag gcccccaagg tagttatcct taaaaaagcc 1260

acagcataca tcctgtccgt ccaagcagag gagcaaaagc tcatttctga agaggacttg 1320

ttgcggaaac gacgagaaca gttgaaacac aaacttgaac agctacggaa ctcttgtgcg 1380

taa 1383

<210> 132
<211> 460
<212> PRT
<213> Artificial Sequence

<220>
<223> NLS-cMyc Amino Acid Sequence

<400> 132
Met Lys Lys Lys Arg Lys Leu Asp Phe Phe Arg Val Val Glu Asn Gln
1               5                   10                  15

Gln Pro Pro Ala Thr Met Pro Leu Asn Val Ser Phe Thr Asn Arg Asn
            20                  25                  30

Tyr Asp Leu Asp Tyr Asp Ser Val Gln Pro Tyr Phe Tyr Cys Asp Glu
        35                  40                  45

Glu Glu Asn Phe Tyr Gln Gln Gln Gln Gln Ser Glu Leu Gln Pro Pro
    50                  55                  60

Ala Pro Ser Glu Asp Ile Trp Lys Lys Phe Glu Leu Leu Pro Thr Pro
65                  70                  75                  80

Pro Leu Ser Pro Ser Arg Arg Ser Gly Leu Cys Ser Pro Ser Tyr Val
                85                  90                  95

Ala Val Thr Pro Phe Ser Leu Arg Gly Asp Asn Asp Gly Gly Gly Gly
            100                 105                 110

Ser Phe Ser Thr Ala Asp Gln Leu Glu Met Val Thr Glu Leu Leu Gly
        115                 120                 125

Gly Asp Met Val Asn Gln Ser Phe Ile Cys Asp Pro Asp Asp Glu Thr
    130                 135                 140

Phe Ile Lys Asn Ile Ile Ile Gln Asp Cys Met Trp Ser Gly Phe Ser
145                 150                 155                 160

Ala Ala Ala Lys Leu Val Ser Glu Lys Leu Ala Ser Tyr Gln Ala Ala
                165                 170                 175

Arg Lys Asp Ser Gly Ser Pro Asn Pro Ala Arg Gly His Ser Val Cys
            180                 185                 190

Ser Thr Ser Ser Leu Tyr Leu Gln Asp Leu Ser Ala Ala Ala Ser Glu
        195                 200                 205

Cys Ile Asp Pro Ser Val Val Phe Pro Tyr Pro Leu Asn Asp Ser Ser
    210                 215                 220

Ser Pro Lys Ser Cys Ala Ser Gln Asp Ser Ser Ala Phe Ser Pro Ser
225                 230                 235                 240

Ser Asp Ser Leu Leu Ser Ser Thr Glu Ser Ser Pro Gln Gly Ser Pro
                245                 250                 255

Glu Pro Leu Val Leu His Glu Glu Thr Pro Pro Thr Thr Ser Ser Asp
            260                 265                 270

Ser Glu Glu Glu Gln Glu Asp Glu Glu Glu Ile Asp Val Val Ser Val
        275                 280                 285

Glu Lys Arg Gln Ala Pro Gly Lys Arg Ser Glu Ser Gly Ser Pro Ser

290                 295                 300

Ala Gly Gly His Ser Lys Pro Pro His Ser Pro Leu Val Leu Lys Arg
305                 310                 315                 320

Cys His Val Ser Thr His Gln His Asn Tyr Ala Ala Pro Pro Ser Thr
                325                 330                 335

Arg Lys Asp Tyr Pro Ala Ala Lys Arg Val Lys Leu Asp Ser Val Arg
            340                 345                 350

Val Leu Arg Gln Ile Ser Asn Asn Arg Lys Cys Thr Ser Pro Arg Ser
        355                 360                 365

Ser Asp Thr Glu Glu Asn Val Lys Arg Arg Thr His Asn Val Leu Glu
    370                 375                 380

Arg Gln Arg Arg Asn Glu Leu Lys Arg Ser Phe Phe Ala Leu Arg Asp
385                 390                 395                 400

Gln Ile Pro Glu Leu Glu Asn Asn Glu Lys Ala Pro Lys Val Val Ile
                405                 410                 415

Leu Lys Lys Ala Thr Ala Tyr Ile Leu Ser Val Gln Ala Glu Glu Gln
            420                 425                 430

Lys Leu Ile Ser Glu Glu Asp Leu Leu Arg Lys Arg Arg Glu Gln Leu
        435                 440                 445

Lys His Lys Leu Glu Gln Leu Arg Asn Ser Cys Ala
    450                 455                 460

<210>   133
<211>   1410
<212>   DNA
<213>   Artificial Sequence

<220>
<223>   NLS-JO-84 MTD-cMyc cDNA Sequence

<400> 133

atgaagaaga agaggaagct ggtggcggcg ctgctggcgg tgctgctgga tttttttcgg 60

gtagtggaaa accagcagcc tcccgcgacg atgcccctca acgttagctt caccaacagg 120

aactatgacc tcgactacga ctcggtgcag ccgtatttct actgcgacga ggaggagaac 180

ttctaccagc agcagcagca gagcgagctg cagcccccgg cgcccagcga ggatatctgg 240

aagaaattcg agctgctgcc caccccgccc ctgtccccta gccgccgctc cgggctctgc 300

tcgccctcct acgttgcggt cacacccttc tcccttcggg gagacaacga cggcggtggc 360

gggagcttct ccacggccga ccagctggag atggtgaccg agctgctggg aggagacatg 420

gtgaaccaga gtttcatctg cgacccggac gacgagacct tcatcaaaaa catcatcatc 480

caggactgta tgtggagcgg cttctcggcc gccgccaagc tcgtctcaga gaagctggcc 540

tcctaccagg ctgcgcgcaa agacagcggc agcccgaacc ccgcccgcgg ccacagcgtc 600

tgctccacct ccagcttgta cctgcaggat ctgagcgccg ccgcctcaga gtgcatcgac 660

ccctcggtgg tcttccccta ccctctcaac gacagcagct cgcccaagtc ctgcgcctcg 720

caagactcca gcgccttctc tccgtcctcg gattctctgc tctcctcgac ggagtcctcc 780

ccgcagggca gccccgagcc cctggtgctc catgaggaga caccgcccac caccagcagc 840

gactctgagg aggaacaaga agatgaggaa gaaatcgatg ttgtttctgt ggaaaagagg 900

caggctcctg gcaaaaggtc agagtctgga tcaccttctg ctggaggcca cagcaaacct 960

cctcacagcc cactggtcct caagaggtgc cacgtctcca cacatcagca caactacgca 1020

gcgcctccct ccactcggaa ggactatcct gctgccaaga gggtcaagtt ggacagtgtc 1080

agagtcctga gacagatcag caacaaccga aaatgcacca gccccaggtc ctcggacacc 1140

gaggagaatg tcaagaggcg aacacacaac gtcttggagc gccagaggag gaacgagcta 1200

aaacggagct tttttgccct gcgtgaccag atcccggagt tggaaaacaa tgaaaaggcc 1260

cccaaggtag ttatccttaa aaaagccaca gcatacatcc tgtccgtcca agcagaggag 1320

caaaagctca tttctgaaga ggacttgttg cggaaacgac gagaacagtt gaaacacaaa 1380

cttgaacagc tacggaactc ttgtgcgtaa 1410

<210> 134
<211> 469
<212> PRT
<213> Artificial Sequence

<220>
<223> NLS-JO-84 MTD-cMyc Amino Acid Sequence

<400> 134
Met Lys Lys Lys Arg Lys Leu Val Ala Ala Leu Leu Ala Val Leu Leu
1               5                   10                  15

Asp Phe Phe Arg Val Val Glu Asn Gln Gln Pro Pro Ala Thr Met Pro
            20                  25                  30

Leu Asn Val Ser Phe Thr Asn Arg Asn Tyr Asp Leu Asp Tyr Asp Ser
        35                  40                  45

Val Gln Pro Tyr Phe Tyr Cys Asp Glu Glu Glu Asn Phe Tyr Gln Gln
    50                  55                  60

Gln Gln Gln Ser Glu Leu Gln Pro Pro Ala Pro Ser Glu Asp Ile Trp
65                  70                  75                  80

Lys Lys Phe Glu Leu Leu Pro Thr Pro Pro Leu Ser Pro Ser Arg Arg
                85                  90                  95

Ser Gly Leu Cys Ser Pro Ser Tyr Val Ala Val Thr Pro Phe Ser Leu
            100                 105                 110

Arg Gly Asp Asn Asp Gly Gly Gly Gly Ser Phe Ser Thr Ala Asp Gln
        115                 120                 125

Leu Glu Met Val Thr Glu Leu Leu Gly Gly Asp Met Val Asn Gln Ser
    130                 135                 140

Phe Ile Cys Asp Pro Asp Asp Glu Thr Phe Ile Lys Asn Ile Ile Ile
145                 150                 155                 160

Gln Asp Cys Met Trp Ser Gly Phe Ser Ala Ala Ala Lys Leu Val Ser
                165                 170                 175

Glu Lys Leu Ala Ser Tyr Gln Ala Ala Arg Lys Asp Ser Gly Ser Pro
            180                 185                 190

Asn Pro Ala Arg Gly His Ser Val Cys Ser Thr Ser Ser Leu Tyr Leu
        195                 200                 205

Gln Asp Leu Ser Ala Ala Ala Ser Glu Cys Ile Asp Pro Ser Val Val
    210                 215                 220

Phe Pro Tyr Pro Leu Asn Asp Ser Ser Ser Pro Lys Ser Cys Ala Ser
225                 230                 235                 240

Gln Asp Ser Ser Ala Phe Ser Pro Ser Ser Asp Ser Leu Leu Ser Ser
                245                 250                 255

Thr Glu Ser Ser Pro Gln Gly Ser Pro Glu Pro Leu Val Leu His Glu
            260                 265                 270

Glu Thr Pro Pro Thr Thr Ser Ser Asp Ser Glu Glu Glu Gln Glu Asp
        275                 280                 285

Glu Glu Glu Ile Asp Val Val Ser Val Glu Lys Arg Gln Ala Pro Gly
    290                 295                 300

Lys Arg Ser Glu Ser Gly Ser Pro Ser Ala Gly Gly His Ser Lys Pro

305                 310                 315                 320

Pro His Ser Pro Leu Val Leu Lys Arg Cys His Val Ser Thr His Gln
                325                 330                 335

His Asn Tyr Ala Ala Pro Pro Ser Thr Arg Lys Asp Tyr Pro Ala Ala
            340                 345                 350

Lys Arg Val Lys Leu Asp Ser Val Arg Val Leu Arg Gln Ile Ser Asn
        355                 360                 365

Asn Arg Lys Cys Thr Ser Pro Arg Ser Ser Asp Thr Glu Glu Asn Val
    370                 375                 380

Lys Arg Arg Thr His Asn Val Leu Glu Arg Gln Arg Arg Asn Glu Leu
385                 390                 395                 400

Lys Arg Ser Phe Phe Ala Leu Arg Asp Gln Ile Pro Glu Leu Glu Asn
                405                 410                 415

Asn Glu Lys Ala Pro Lys Val Val Ile Leu Lys Lys Ala Thr Ala Tyr
            420                 425                 430

Ile Leu Ser Val Gln Ala Glu Glu Gln Lys Leu Ile Ser Glu Glu Asp
        435                 440                 445

Leu Leu Arg Lys Arg Arg Glu Gln Leu Lys His Lys Leu Glu Gln Leu
    450                 455                 460

Arg Asn Ser Cys Ala
465

<210>   135
<211>   1410
<212>   DNA
<213>   Artificial Sequence

<220>
<223>   NLS-cMyc-JO-84 MTD cDNA Sequence

<400> 135

atgaagaaga agaggaagct ggattttttt cgggtagtgg aaaaccagca gcctcccgcg 60

acgatgcccc tcaacgttag cttcaccaac aggaactatg acctcgacta cgactcggtg 120

cagccgtatt tctactgcga cgaggaggag aacttctacc agcagcagca gcagagcgag 180

ctgcagcccc cggcgcccag cgaggatatc tggaagaaat tcgagctgct gcccaccccg 240

cccctgtccc ctagccgccg ctccgggctc tgctcgccct cctacgttgc ggtcacaccc 300

ttctcccttc ggggagacaa cgacggcggt ggcgggagct tctccacggc cgaccagctg 360

gagatggtga ccgagctgct gggaggagac atggtgaacc agagtttcat ctgcgacccg 420

gacgacgaga ccttcatcaa aaacatcatc atccaggact gtatgtggag cggcttctcg 480

gccgccgcca agctcgtctc agagaagctg gcctcctacc aggctgcgcg caaagacagc 540

ggcagcccga accccgcccg cggccacagc gtctgctcca cctccagctt gtacctgcag 600

gatctgagcg ccgccgcctc agagtgcatc gacccctcgg tggtcttccc ctaccctctc 660

aacgacagca gctcgcccaa gtcctgcgcc tcgcaagact ccagcgcctt ctctccgtcc 720

tcggattctc tgctctcctc gacggagtcc tccccgcagg gcagccccga gcccctggtg 780

ctccatgagg agacaccgcc caccaccagc agcgactctg aggaggaaca agaagatgag 840

gaagaaatcg atgttgtttc tgtggaaaag aggcaggctc ctggcaaaag gtcagagtct 900

ggatcacctt ctgctggagg ccacagcaaa cctcctcaca gcccactggt cctcaagagg 960

tgccacgtct ccacacatca gcacaactac gcagcgcctc cctccactcg gaaggactat 1020

cctgctgcca agagggtcaa gttggacagt gtcagagtcc tgagacagat cagcaacaac 1080

cgaaaatgca ccagccccag gtcctcggac accgaggaga atgtcaagag gcgaacacac 1140

aacgtcttgg agcgccagag gaggaacgag ctaaaacgga gcttttttgc cctgcgtgac 1200

cagatcccgg agttggaaaa caatgaaaag gcccccaagg tagttatcct taaaaaagcc 1260

acagcataca tcctgtccgt ccaagcagag gagcaaaagc tcatttctga agaggacttg 1320

ttgcggaaac gacgagaaca gttgaaacac aaacttgaac agctacggaa ctcttgtgcg 1380

ctggtggcgg cgctgctggc ggtgctgtaa 1410

<210> 136
<211> 469
<212> PRT
<213> Artificial Sequence

<220>
<223> NLS-cMyc-JO-84 MTD Amino Acid Sequence

<400> 136
Met Lys Lys Lys Arg Lys Leu Asp Phe Phe Arg Val Val Glu Asn Gln
1               5                   10                  15

Gln Pro Pro Ala Thr Met Pro Leu Asn Val Ser Phe Thr Asn Arg Asn
            20                  25                  30

Tyr Asp Leu Asp Tyr Asp Ser Val Gln Pro Tyr Phe Tyr Cys Asp Glu
        35                  40                  45

Glu Glu Asn Phe Tyr Gln Gln Gln Gln Gln Ser Glu Leu Gln Pro Pro
    50                  55                  60

Ala Pro Ser Glu Asp Ile Trp Lys Lys Phe Glu Leu Leu Pro Thr Pro
65                  70                  75                  80

Pro Leu Ser Pro Ser Arg Arg Ser Gly Leu Cys Ser Pro Ser Tyr Val
                85                  90                  95

Ala Val Thr Pro Phe Ser Leu Arg Gly Asp Asn Asp Gly Gly Gly Gly
            100                 105                 110

Ser Phe Ser Thr Ala Asp Gln Leu Glu Met Val Thr Glu Leu Leu Gly
        115                 120                 125

Gly Asp Met Val Asn Gln Ser Phe Ile Cys Asp Pro Asp Asp Glu Thr
    130                 135                 140

Phe Ile Lys Asn Ile Ile Ile Gln Asp Cys Met Trp Ser Gly Phe Ser
145                 150                 155                 160

Ala Ala Ala Lys Leu Val Ser Glu Lys Leu Ala Ser Tyr Gln Ala Ala
                165                 170                 175

Arg Lys Asp Ser Gly Ser Pro Asn Pro Ala Arg Gly His Ser Val Cys
            180                 185                 190

Ser Thr Ser Ser Leu Tyr Leu Gln Asp Leu Ser Ala Ala Ala Ser Glu
        195                 200                 205

Cys Ile Asp Pro Ser Val Val Phe Pro Tyr Pro Leu Asn Asp Ser Ser
    210                 215                 220

Ser Pro Lys Ser Cys Ala Ser Gln Asp Ser Ser Ala Phe Ser Pro Ser
225                 230                 235                 240

Ser Asp Ser Leu Leu Ser Ser Thr Glu Ser Ser Pro Gln Gly Ser Pro
                245                 250                 255

Glu Pro Leu Val Leu His Glu Glu Thr Pro Pro Thr Thr Ser Ser Asp
            260                 265                 270

Ser Glu Glu Glu Gln Glu Asp Glu Glu Glu Ile Asp Val Val Ser Val
        275                 280                 285

Glu Lys Arg Gln Ala Pro Gly Lys Arg Ser Glu Ser Gly Ser Pro Ser
    290                 295                 300

Ala Gly Gly His Ser Lys Pro Pro His Ser Pro Leu Val Leu Lys Arg

305                 310                 315                 320

Cys His Val Ser Thr His Gln His Asn Tyr Ala Ala Pro Pro Ser Thr
                325                 330                 335

Arg Lys Asp Tyr Pro Ala Ala Lys Arg Val Lys Leu Asp Ser Val Arg
            340                 345                 350

Val Leu Arg Gln Ile Ser Asn Asn Arg Lys Cys Thr Ser Pro Arg Ser
        355                 360                 365

Ser Asp Thr Glu Glu Asn Val Lys Arg Arg Thr His Asn Val Leu Glu
    370                 375                 380

Arg Gln Arg Arg Asn Glu Leu Lys Arg Ser Phe Phe Ala Leu Arg Asp
385                 390                 395                 400

Gln Ile Pro Glu Leu Glu Asn Asn Glu Lys Ala Pro Lys Val Val Ile
                405                 410                 415

Leu Lys Lys Ala Thr Ala Tyr Ile Leu Ser Val Gln Ala Glu Glu Gln
            420                 425                 430

Lys Leu Ile Ser Glu Glu Asp Leu Leu Arg Lys Arg Arg Glu Gln Leu
        435                 440                 445

Lys His Lys Leu Glu Gln Leu Arg Asn Ser Cys Ala Leu Val Ala Ala
    450                 455                 460

Leu Leu Ala Val Leu
465

<210>    137
<211>    1437
<212>    DNA
<213>    Artificial Sequence

<220>
<223>    NLS-JO-84 MTD-cMyc-JO-84 MTD cDNA Sequence

<400> 137

atgaagaaga agaggaagct ggtggcggcg ctgctggcgg tgctgctgga tttttttcgg 60

gtagtggaaa accagcagcc tcccgcgacg atgcccctca acgttagctt caccaacagg 120

aactatgacc tcgactacga ctcggtgcag ccgtatttct actgcgacga ggaggagaac 180

ttctaccagc agcagcagca gagcgagctg cagcccccgg cgcccagcga ggatatctgg 240

aagaaattcg agctgctgcc caccccgccc ctgtccccta gccgccgctc cgggctctgc 300

tcgccctcct acgttgcggt cacacccttc tcccttcggg gagacaacga cggcggtggc 360

gggagcttct ccacggccga ccagctggag atggtgaccg agctgctggg aggagacatg 420

gtgaaccaga gtttcatctg cgacccggac gacgagacct tcatcaaaaa catcatcatc 480

caggactgta tgtggagcgg cttctcggcc gccgccaagc tcgtctcaga gaagctggcc 540

tcctaccagg ctgcgcgcaa agacagcggc agcccgaacc ccgcccgcgg ccacagcgtc 600

tgctccacct ccagcttgta cctgcaggat ctgagcgccg ccgcctcaga gtgcatcgac 660

ccctcggtgg tcttccccta ccctctcaac gacagcagct cgcccaagtc ctgcgcctcg 720

caagactcca gcgccttctc tccgtcctcg gattctctgc tctcctcgac ggagtcctcc 780

ccgcagggca gccccgagcc cctggtgctc catgaggaga caccgcccac caccagcagc 840

gactctgagg aggaacaaga agatgaggaa gaaatcgatg ttgtttctgt ggaaaagagg 900

caggctcctg gcaaaaggtc agagtctgga tcaccttctg ctggaggcca cagcaaacct 960

cctcacagcc cactggtcct caagaggtgc cacgtctcca cacatcagca caactacgca 1020

gcgcctccct ccactcggaa ggactatcct gctgccaaga gggtcaagtt ggacagtgtc 1080

agagtcctga gacagatcag caacaaccga aaatgcacca gccccaggtc ctcggacacc 1140

gaggagaatg tcaagaggcg aacacacaac gtcttggagc gccagaggag gaacgagcta 1200

aaacggagct tttttgccct gcgtgaccag atcccggagt tggaaaacaa tgaaaaggcc 1260

cccaaggtag ttatccttaa aaaagccaca gcatacatcc tgtccgtcca agcagaggag 1320

caaaagctca tttctgaaga ggacttgttg cggaaacgac gagaacagtt gaaacacaaa 1380

cttgaacagc tacggaactc ttgtgcgctg gtggcggcgc tgctggcggt gctgtaa 1437

<210> 138
<211> 478
<212> PRT
<213> Artificial Sequence

<220>
<223> NLS-JO-84 MTD-cMyc-JO-84 MTD Amino Acid Sequence

<400> 138
Met Lys Lys Lys Arg Lys Leu Val Ala Ala Leu Leu Ala Val Leu Leu
1               5                   10                  15

Asp Phe Phe Arg Val Val Glu Asn Gln Gln Pro Pro Ala Thr Met Pro
            20                  25                  30

Leu Asn Val Ser Phe Thr Asn Arg Asn Tyr Asp Leu Asp Tyr Asp Ser
        35                  40                  45

Val Gln Pro Tyr Phe Tyr Cys Asp Glu Glu Glu Asn Phe Tyr Gln Gln
    50                  55                  60

Gln Gln Gln Ser Glu Leu Gln Pro Pro Ala Pro Ser Glu Asp Ile Trp
65                  70                  75                  80

Lys Lys Phe Glu Leu Leu Pro Thr Pro Pro Leu Ser Pro Ser Arg Arg
                85                  90                  95

Ser Gly Leu Cys Ser Pro Ser Tyr Val Ala Val Thr Pro Phe Ser Leu
            100                 105                 110

Arg Gly Asp Asn Asp Gly Gly Gly Gly Ser Phe Ser Thr Ala Asp Gln
        115                 120                 125

Leu Glu Met Val Thr Glu Leu Leu Gly Gly Asp Met Val Asn Gln Ser
    130                 135                 140

Phe Ile Cys Asp Pro Asp Asp Glu Thr Phe Ile Lys Asn Ile Ile Ile
145                 150                 155                 160

Gln Asp Cys Met Trp Ser Gly Phe Ser Ala Ala Ala Lys Leu Val Ser
                165                 170                 175

Glu Lys Leu Ala Ser Tyr Gln Ala Ala Arg Lys Asp Ser Gly Ser Pro
            180                 185                 190

Asn Pro Ala Arg Gly His Ser Val Cys Ser Thr Ser Ser Leu Tyr Leu
        195                 200                 205

Gln Asp Leu Ser Ala Ala Ala Ser Glu Cys Ile Asp Pro Ser Val Val
    210                 215                 220

Phe Pro Tyr Pro Leu Asn Asp Ser Ser Ser Pro Lys Ser Cys Ala Ser
225                 230                 235                 240

Gln Asp Ser Ser Ala Phe Ser Pro Ser Ser Asp Ser Leu Leu Ser Ser
                245                 250                 255

Thr Glu Ser Ser Pro Gln Gly Ser Pro Glu Pro Leu Val Leu His Glu
            260                 265                 270

Glu Thr Pro Pro Thr Thr Ser Ser Asp Ser Glu Glu Glu Gln Glu Asp
        275                 280                 285

Glu Glu Glu Ile Asp Val Val Ser Val Glu Lys Arg Gln Ala Pro Gly
    290                 295                 300

Lys Arg Ser Glu Ser Gly Ser Pro Ser Ala Gly Gly His Ser Lys Pro

305                 310                 315                 320

Pro His Ser Pro Leu Val Leu Lys Arg Cys His Val Ser Thr His Gln
                325                 330                 335

His Asn Tyr Ala Ala Pro Pro Ser Thr Arg Lys Asp Tyr Pro Ala Ala
            340                 345                 350

Lys Arg Val Lys Leu Asp Ser Val Arg Val Leu Arg Gln Ile Ser Asn
        355                 360                 365

Asn Arg Lys Cys Thr Ser Pro Arg Ser Ser Asp Thr Glu Glu Asn Val
    370                 375                 380

Lys Arg Arg Thr His Asn Val Leu Glu Arg Gln Arg Arg Asn Glu Leu
385                 390                 395                 400

Lys Arg Ser Phe Phe Ala Leu Arg Asp Gln Ile Pro Glu Leu Glu Asn
                405                 410                 415

Asn Glu Lys Ala Pro Lys Val Val Ile Leu Lys Lys Ala Thr Ala Tyr
            420                 425                 430

Ile Leu Ser Val Gln Ala Glu Glu Gln Lys Leu Ile Ser Glu Glu Asp
        435                 440                 445

Leu Leu Arg Lys Arg Arg Glu Gln Leu Lys His Lys Leu Glu Gln Leu
    450                 455                 460

Arg Asn Ser Cys Ala Leu Val Ala Ala Leu Leu Ala Val Leu
465                 470                 475

<210>   139
<211>   1407
<212>   DNA
<213>   Artificial Sequence

<220>
<223>   NLS-JO-86 MTD-cMyc cDNA Sequence

<400>    139

atgaagaaga agaggaagct ggcggtgctg gcggcggcgc cgctggattt ttttcgggta        60

gtggaaaacc agcagcctcc cgcgacgatg cccctcaacg ttagcttcac caacaggaac       120

tatgacctcg actacgactc ggtgcagccg tatttctact gcgacgagga ggagaacttc       180

taccagcagc agcagcagag cgagctgcag cccccggcgc ccagcgagga tatctggaag       240

aaattcgagc tgctgcccac cccgcccctg tcccctagcc gccgctccgg gctctgctcg       300

ccctcctacg ttgcggtcac acccttctcc cttcggggag acaacgacgg cggtggcggg       360

agcttctcca cggccgacca gctggagatg gtgaccgagc tgctgggagg agacatggtg       420

aaccagagtt tcatctgcga cccggacgac gagaccttca tcaaaaacat catcatccag       480

gactgtatgt ggagcggctt ctcggccgcc gccaagctcg tctcagagaa gctggcctcc       540

taccaggctg cgcgcaaaga cagcggcagc ccgaaccccg cccgcggcca cagcgtctgc       600

tccacctcca gcttgtacct gcaggatctg agcgccgccg cctcagagtg catcgacccc       660

tcggtggtct tcccctaccc tctcaacgac agcagctcgc ccaagtcctg cgcctcgcaa       720

gactccagcg ccttctctcc gtcctcggat tctctgctct cctcgacgga gtcctccccg       780

cagggcagcc ccgagcccct ggtgctccat gaggagacac cgcccaccac cagcagcgac       840

tctgaggagg aacaagaaga tgaggaagaa atcgatgttg tttctgtgga aaagaggcag       900

gctcctggca aaaggtcaga gtctggatca ccttctgctg gaggccacag caaacctcct       960

cacagcccac tggtcctcaa gaggtgccac gtctccacac atcagcacaa ctacgcagcg      1020

cctccctcca ctcggaagga ctatcctgct gccaagaggg tcaagttgga cagtgtcaga      1080

gtcctgagac agatcagcaa caaccgaaaa tgcaccagcc ccaggtcctc ggacaccgag      1140

gagaatgtca agaggcgaac acacaacgtc ttggagcgcc agaggaggaa cgagctaaaa 1200

cggagctttt ttgccctgcg tgaccagatc ccggagttgg aaaacaatga aaaggccccc 1260

aaggtagtta tccttaaaaa agccacagca tacatcctgt ccgtccaagc agaggagcaa 1320

aagctcattt ctgaagagga cttgttgcgg aaacgacgag aacagttgaa acacaaactt 1380

gaacagctac ggaactcttg tgcgtaa 1407

<210> 140
<211> 468
<212> PRT
<213> Artificial Sequence

<220>
<223> NLS-JO-86 MTD-cMyc Amino Acid Sequence

<400> 140
Met Lys Lys Lys Arg Lys Leu Ala Val Leu Ala Ala Ala Pro Leu Asp
1               5                   10                  15

Phe Phe Arg Val Val Glu Asn Gln Gln Pro Pro Ala Thr Met Pro Leu
            20                  25                  30

Asn Val Ser Phe Thr Asn Arg Asn Tyr Asp Leu Asp Tyr Asp Ser Val
        35                  40                  45

Gln Pro Tyr Phe Tyr Cys Asp Glu Glu Glu Asn Phe Tyr Gln Gln Gln
    50                  55                  60

Gln Gln Ser Glu Leu Gln Pro Pro Ala Pro Ser Glu Asp Ile Trp Lys
65                  70                  75                  80

Lys Phe Glu Leu Leu Pro Thr Pro Pro Leu Ser Pro Ser Arg Arg Ser
                85                  90                  95

Gly Leu Cys Ser Pro Ser Tyr Val Ala Val Thr Pro Phe Ser Leu Arg
            100                 105                 110

Gly Asp Asn Asp Gly Gly Gly Gly Ser Phe Ser Thr Ala Asp Gln Leu
        115                 120                 125

Glu Met Val Thr Glu Leu Leu Gly Gly Asp Met Val Asn Gln Ser Phe
    130                 135                 140

Ile Cys Asp Pro Asp Asp Glu Thr Phe Ile Lys Asn Ile Ile Ile Gln
145                 150                 155                 160

Asp Cys Met Trp Ser Gly Phe Ser Ala Ala Ala Lys Leu Val Ser Glu
                165                 170                 175

Lys Leu Ala Ser Tyr Gln Ala Ala Arg Lys Asp Ser Gly Ser Pro Asn
            180                 185                 190

Pro Ala Arg Gly His Ser Val Cys Ser Thr Ser Ser Leu Tyr Leu Gln
        195                 200                 205

Asp Leu Ser Ala Ala Ala Ser Glu Cys Ile Asp Pro Ser Val Val Phe
    210                 215                 220

Pro Tyr Pro Leu Asn Asp Ser Ser Ser Pro Lys Ser Cys Ala Ser Gln
225                 230                 235                 240

Asp Ser Ser Ala Phe Ser Pro Ser Ser Asp Ser Leu Leu Ser Ser Thr
                245                 250                 255

Glu Ser Ser Pro Gln Gly Ser Pro Glu Pro Leu Val Leu His Glu Glu
            260                 265                 270

Thr Pro Pro Thr Thr Ser Ser Asp Ser Glu Glu Glu Gln Glu Asp Glu
        275                 280                 285

Glu Glu Ile Asp Val Val Ser Val Glu Lys Arg Gln Ala Pro Gly Lys
    290                 295                 300

Arg Ser Glu Ser Gly Ser Pro Ser Ala Gly Gly His Ser Lys Pro Pro

305                 310                 315                 320

His Ser Pro Leu Val Leu Lys Arg Cys His Val Ser Thr His Gln His
                325                 330                 335

Asn Tyr Ala Ala Pro Pro Ser Thr Arg Lys Asp Tyr Pro Ala Ala Lys
            340                 345                 350

Arg Val Lys Leu Asp Ser Val Arg Val Leu Arg Gln Ile Ser Asn Asn
        355                 360                 365

Arg Lys Cys Thr Ser Pro Arg Ser Ser Asp Thr Glu Glu Asn Val Lys
    370                 375                 380

Arg Arg Thr His Asn Val Leu Glu Arg Gln Arg Arg Asn Glu Leu Lys
385                 390                 395                 400

Arg Ser Phe Phe Ala Leu Arg Asp Gln Ile Pro Glu Leu Glu Asn Asn
                405                 410                 415

Glu Lys Ala Pro Lys Val Val Ile Leu Lys Lys Ala Thr Ala Tyr Ile
            420                 425                 430

Leu Ser Val Gln Ala Glu Glu Gln Lys Leu Ile Ser Glu Glu Asp Leu
        435                 440                 445

Leu Arg Lys Arg Arg Glu Gln Leu Lys His Lys Leu Glu Gln Leu Arg
    450                 455                 460

Asn Ser Cys Ala
465

<210>   141
<211>   1407
<212>   DNA
<213>   Artificial Sequence

<220>
<223>   NLS-cMyc-JO-86 MTD cDNA Sequence

<400> 141

atgaagaaga agaggaagct ggattttttt cgggtagtgg aaaaccagca gcctcccgcg 60

acgatgcccc tcaacgttag cttcaccaac aggaactatg acctcgacta cgactcggtg 120

cagccgtatt tctactgcga cgaggaggag aacttctacc agcagcagca gcagagcgag 180

ctgcagcccc cggcgcccag cgaggatatc tggaagaaat tcgagctgct gcccaccccg 240

cccctgtccc ctagccgccg ctccgggctc tgctcgccct cctacgttgc ggtcacaccc 300

ttctcccttc ggggagacaa cgacggcggt ggcgggagct tctccacggc cgaccagctg 360

gagatggtga ccgagctgct gggaggagac atggtgaacc agagtttcat ctgcgacccg 420

gacgacgaga ccttcatcaa aaacatcatc atccaggact gtatgtggag cggcttctcg 480

gccgccgcca agctcgtctc agagaagctg gcctcctacc aggctgcgcg caaagacagc 540

ggcagcccga accccgcccg cggccacagc gtctgctcca cctccagctt gtacctgcag 600

gatctgagcg ccgccgcctc agagtgcatc gacccctcgg tggtcttccc ctaccctctc 660

aacgacagca gctcgcccaa gtcctgcgcc tcgcaagact ccagcgcctt ctctccgtcc 720

tcggattctc tgctctcctc gacggagtcc tccccgcagg gcagccccga gcccctggtg 780

ctccatgagg agacaccgcc caccaccagc agcgactctg aggaggaaca agaagatgag 840

gaagaaatcg atgttgtttc tgtggaaaag aggcaggctc ctggcaaaag gtcagagtct 900

ggatcacctt ctgctggagg ccacagcaaa cctcctcaca gcccactggt cctcaagagg 960

tgccacgtct ccacacatca gcacaactac gcagcgcctc cctccactcg gaaggactat 1020

cctgctgcca agagggtcaa gttggacagt gtcagagtcc tgagacagat cagcaacaac 1080

cgaaaatgca ccagccccag gtcctcggac accgaggaga atgtcaagag gcgaacacac 1140

aacgtcttgg agcgccagag gaggaacgag ctaaaacgga gcttttttgc cctgcgtgac 1200

cagatcccgg agttggaaaa caatgaaaag gcccccaagg tagttatcct taaaaaagcc 1260

acagcataca tcctgtccgt ccaagcagag gagcaaaagc tcatttctga agaggacttg 1320

ttgcggaaac gacgagaaca gttgaaacac aaacttgaac agctacggaa ctcttgtgcg 1380

ctggcggtgc tggcggcggc gccgtaa 1407

<210> 142
<211> 468
<212> PRT
<213> Artificial Sequence

<220>
<223> NLS-cMyc-JO-86 MTD Amino Acid Sequence

<400> 142
Met Lys Lys Lys Arg Lys Leu Asp Phe Phe Arg Val Val Glu Asn Gln
1               5                   10                  15

Gln Pro Pro Ala Thr Met Pro Leu Asn Val Ser Phe Thr Asn Arg Asn
            20                  25                  30

Tyr Asp Leu Asp Tyr Asp Ser Val Gln Pro Tyr Phe Tyr Cys Asp Glu
        35                  40                  45

Glu Glu Asn Phe Tyr Gln Gln Gln Gln Gln Ser Glu Leu Gln Pro Pro
    50                  55                  60

Ala Pro Ser Glu Asp Ile Trp Lys Lys Phe Glu Leu Leu Pro Thr Pro
65                  70                  75                  80

Pro Leu Ser Pro Ser Arg Arg Ser Gly Leu Cys Ser Pro Ser Tyr Val
                85                  90                  95

Ala Val Thr Pro Phe Ser Leu Arg Gly Asp Asn Asp Gly Gly Gly Gly
            100                 105                 110

Ser Phe Ser Thr Ala Asp Gln Leu Glu Met Val Thr Glu Leu Leu Gly
        115                 120                 125

Gly Asp Met Val Asn Gln Ser Phe Ile Cys Asp Pro Asp Asp Glu Thr
    130                 135                 140

Phe Ile Lys Asn Ile Ile Ile Gln Asp Cys Met Trp Ser Gly Phe Ser
145                 150                 155                 160

Ala Ala Ala Lys Leu Val Ser Glu Lys Leu Ala Ser Tyr Gln Ala Ala
                165                 170                 175

Arg Lys Asp Ser Gly Ser Pro Asn Pro Ala Arg Gly His Ser Val Cys
            180                 185                 190

Ser Thr Ser Ser Leu Tyr Leu Gln Asp Leu Ser Ala Ala Ala Ser Glu
        195                 200                 205

Cys Ile Asp Pro Ser Val Val Phe Pro Tyr Pro Leu Asn Asp Ser Ser
    210                 215                 220

Ser Pro Lys Ser Cys Ala Ser Gln Asp Ser Ser Ala Phe Ser Pro Ser
225                 230                 235                 240

Ser Asp Ser Leu Leu Ser Ser Thr Glu Ser Ser Pro Gln Gly Ser Pro
                245                 250                 255

Glu Pro Leu Val Leu His Glu Glu Thr Pro Pro Thr Thr Ser Ser Asp
            260                 265                 270

Ser Glu Glu Glu Gln Glu Asp Glu Glu Glu Ile Asp Val Val Ser Val
        275                 280                 285

Glu Lys Arg Gln Ala Pro Gly Lys Arg Ser Glu Ser Gly Ser Pro Ser
    290                 295                 300

Ala Gly Gly His Ser Lys Pro Pro His Ser Pro Leu Val Leu Lys Arg

305                 310                 315                 320

Cys His Val Ser Thr His Gln His Asn Tyr Ala Ala Pro Pro Ser Thr
                325                 330                 335

Arg Lys Asp Tyr Pro Ala Ala Lys Arg Val Lys Leu Asp Ser Val Arg
            340                 345                 350

Val Leu Arg Gln Ile Ser Asn Asn Arg Lys Cys Thr Ser Pro Arg Ser
        355                 360                 365

Ser Asp Thr Glu Glu Asn Val Lys Arg Arg Thr His Asn Val Leu Glu
    370                 375                 380

Arg Gln Arg Arg Asn Glu Leu Lys Arg Ser Phe Phe Ala Leu Arg Asp
385                 390                 395                 400

Gln Ile Pro Glu Leu Glu Asn Asn Glu Lys Ala Pro Lys Val Val Ile
                405                 410                 415

Leu Lys Lys Ala Thr Ala Tyr Ile Leu Ser Val Gln Ala Glu Glu Gln
            420                 425                 430

Lys Leu Ile Ser Glu Glu Asp Leu Leu Arg Lys Arg Arg Glu Gln Leu
        435                 440                 445

Lys His Lys Leu Glu Gln Leu Arg Asn Ser Cys Ala Leu Ala Val Leu
    450                 455                 460

Ala Ala Ala Pro
465

<210>   143
<211>   1431
<212>   DNA
<213>   Artificial Sequence

<220>
<223>   NLS-JO-86 MTD-cMyc-JO-86 MTD cDNA Sequence

<400> 143

atgaagaaga agaggaagct ggcggtgctg gcggcggcgc cgctggattt ttttcgggta 60

gtggaaaacc agcagcctcc cgcgacgatg cccctcaacg ttagcttcac caacaggaac 120

tatgacctcg actacgactc ggtgcagccg tatttctact gcgacgagga ggagaacttc 180

taccagcagc agcagcagag cgagctgcag cccccggcgc ccagcgagga tatctggaag 240

aaattcgagc tgctgcccac cccgcccctg tcccctagcc gccgctccgg gctctgctcg 300

ccctcctacg ttgcggtcac acccttctcc cttcggggag acaacgacgg cggtggcggg 360

agcttctcca cggccgacca gctggagatg gtgaccgagc tgctgggagg agacatggtg 420

aaccagagtt tcatctgcga cccggacgac gagaccttca tcaaaaacat catcatccag 480

gactgtatgt ggagcggctt ctcggccgcc gccaagctcg tctcagagaa gctggcctcc 540

taccaggctg cgcgcaaaga cagcggcagc ccgaaccccg cccgcggcca cagcgtctgc 600

tccacctcca gcttgtacct gcaggatctg agcgccgccg cctcagagtg catcgacccc 660

tcggtggtct tcccctaccc tctcaacgac agcagctcgc ccaagtcctg cgcctcgcaa 720

gactccagcg ccttctctcc gtcctcggat tctctgctct cctcgacgga gtcctccccg 780

cagggcagcc ccgagcccct ggtgctccat gaggagacac cgcccaccac cagcagcgac 840

tctgaggagg aacaagaaga tgaggaagaa atcgatgttg tttctgtgga aaagaggcag 900

gctcctggca aaaggtcaga gtctggatca ccttctgctg gaggccacag caaacctcct 960

cacagcccac tggtcctcaa gaggtgccac gtctccacac atcagcacaa ctacgcagcg 1020

cctccctcca ctcggaagga ctatcctgct gccaagaggg tcaagttgga cagtgtcaga 1080

gtcctgagac agatcagcaa caaccgaaaa tgcaccagcc ccaggtcctc ggacaccgag 1140

gagaatgtca agaggcgaac acacaacgtc ttggagcgcc agaggaggaa cgagctaaaa 1200

cggagctttt ttgccctgcg tgaccagatc ccggagttgg aaaacaatga aaaggccccc 1260

aaggtagtta tccttaaaaa agccacagca tacatcctgt ccgtccaagc agaggagcaa 1320

aagctcattt ctgaagagga cttgttgcgg aaacgacgag aacagttgaa acacaaactt 1380

gaacagctac ggaactcttg tgcgctggcg gtgctggcgg cggcgccgta a 1431

<210> 144
<211> 476
<212> PRT
<213> Artificial Sequence

<220>
<223> NLS-JO-86 MTD-cMyc-JO-86 MTD Amino Acid Sequence

<400> 144
Met Lys Lys Lys Arg Lys Leu Ala Val Leu Ala Ala Ala Pro Leu Asp
1               5                   10                  15

Phe Phe Arg Val Val Glu Asn Gln Gln Pro Pro Ala Thr Met Pro Leu
            20                  25                  30

Asn Val Ser Phe Thr Asn Arg Asn Tyr Asp Leu Asp Tyr Asp Ser Val
        35                  40                  45

Gln Pro Tyr Phe Tyr Cys Asp Glu Glu Glu Asn Phe Tyr Gln Gln Gln
    50                  55                  60

Gln Gln Ser Glu Leu Gln Pro Pro Ala Pro Ser Glu Asp Ile Trp Lys
65                  70                  75                  80

Lys Phe Glu Leu Leu Pro Thr Pro Pro Leu Ser Pro Ser Arg Arg Ser
                85                  90                  95

Gly Leu Cys Ser Pro Ser Tyr Val Ala Val Thr Pro Phe Ser Leu Arg
            100                 105                 110

Gly Asp Asn Asp Gly Gly Gly Gly Ser Phe Ser Thr Ala Asp Gln Leu
        115                 120                 125

Glu Met Val Thr Glu Leu Leu Gly Gly Asp Met Val Asn Gln Ser Phe
    130                 135                 140

Ile Cys Asp Pro Asp Asp Glu Thr Phe Ile Lys Asn Ile Ile Ile Gln
145                 150                 155                 160

Asp Cys Met Trp Ser Gly Phe Ser Ala Ala Ala Lys Leu Val Ser Glu
                165                 170                 175

Lys Leu Ala Ser Tyr Gln Ala Ala Arg Lys Asp Ser Gly Ser Pro Asn
            180                 185                 190

Pro Ala Arg Gly His Ser Val Cys Ser Thr Ser Ser Leu Tyr Leu Gln
        195                 200                 205

Asp Leu Ser Ala Ala Ala Ser Glu Cys Ile Asp Pro Ser Val Val Phe
    210                 215                 220

Pro Tyr Pro Leu Asn Asp Ser Ser Ser Pro Lys Ser Cys Ala Ser Gln
225                 230                 235                 240

Asp Ser Ser Ala Phe Ser Pro Ser Ser Asp Ser Leu Leu Ser Ser Thr
                245                 250                 255

Glu Ser Ser Pro Gln Gly Ser Pro Glu Pro Leu Val Leu His Glu Glu
            260                 265                 270

Thr Pro Pro Thr Thr Ser Ser Asp Ser Glu Glu Glu Gln Glu Asp Glu
        275                 280                 285

Glu Glu Ile Asp Val Val Ser Val Glu Lys Arg Gln Ala Pro Gly Lys
    290                 295                 300

Arg Ser Glu Ser Gly Ser Pro Ser Ala Gly Gly His Ser Lys Pro Pro

305                 310                 315                 320

His Ser Pro Leu Val Leu Lys Arg Cys His Val Ser Thr His Gln His
                325                 330                 335

Asn Tyr Ala Ala Pro Pro Ser Thr Arg Lys Asp Tyr Pro Ala Ala Lys
            340                 345                 350

Arg Val Lys Leu Asp Ser Val Arg Val Leu Arg Gln Ile Ser Asn Asn
        355                 360                 365

Arg Lys Cys Thr Ser Pro Arg Ser Ser Asp Thr Glu Glu Asn Val Lys
    370                 375                 380

Arg Arg Thr His Asn Val Leu Glu Arg Gln Arg Arg Asn Glu Leu Lys
385                 390                 395                 400

Arg Ser Phe Phe Ala Leu Arg Asp Gln Ile Pro Glu Leu Glu Asn Asn
                405                 410                 415

Glu Lys Ala Pro Lys Val Val Ile Leu Lys Lys Ala Thr Ala Tyr Ile
            420                 425                 430

Leu Ser Val Gln Ala Glu Glu Gln Lys Leu Ile Ser Glu Glu Asp Leu
        435                 440                 445

Leu Arg Lys Arg Arg Glu Gln Leu Lys His Lys Leu Glu Gln Leu Arg
    450                 455                 460

Asn Ser Cys Ala Leu Ala Val Leu Ala Ala Ala Pro
465                 470                 475

<210>   145
<211>   1443
<212>   DNA
<213>   Artificial Sequence

<220>
<223>   His-NLS-cMyc cDNA Sequence

<400>    145

atgggcagca gccatcatca tcatcatcac agcagcggcc tggtgccgcg cggcagccat        60

atgaagaaga agaggaagct ggattttttt cgggtagtgg aaaaccagca gcctcccgcg       120

acgatgcccc tcaacgttag cttcaccaac aggaactatg acctcgacta cgactcggtg       180

cagccgtatt tctactgcga cgaggaggag aacttctacc agcagcagca gcagagcgag       240

ctgcagcccc cggcgcccag cgaggatatc tggaagaaat tcgagctgct gcccaccccg       300

cccctgtccc ctagccgccg ctccgggctc tgctcgccct cctacgttgc ggtcacaccc       360

ttctcccttc ggggagacaa cgacggcggt ggcgggagct tctccacggc cgaccagctg       420

gagatggtga ccgagctgct gggaggagac atggtgaacc agagtttcat ctgcgacccg       480

gacgacgaga ccttcatcaa aaacatcatc atccaggact gtatgtggag cggcttctcg       540

gccgccgcca agctcgtctc agagaagctg gcctcctacc aggctgcgcg caaagacagc       600

ggcagcccga accccgcccg cggccacagc gtctgctcca cctccagctt gtacctgcag       660

gatctgagcg ccgccgcctc agagtgcatc gacccctcgg tggtcttccc ctaccctctc       720

aacgacagca gctcgcccaa gtcctgcgcc tcgcaagact ccagcgcctt ctctccgtcc       780

tcggattctc tgctctcctc gacggagtcc tccccgcagg gcagccccga gcccctggtg       840

ctccatgagg agacaccgcc caccaccagc agcgactctg aggaggaaca agaagatgag       900

gaagaaatcg atgttgtttc tgtggaaaag aggcaggctc ctggcaaaag gtcagagtct       960

ggatcacctt ctgctggagg ccacagcaaa cctcctcaca gcccactggt cctcaagagg      1020

tgccacgtct ccacacatca gcacaactac gcagcgcctc cctccactcg gaaggactat      1080

cctgctgcca agagggtcaa gttggacagt gtcagagtcc tgagacagat cagcaacaac      1140

cgaaaatgca ccagccccag gtcctcggac accgaggaga atgtcaagag gcgaacacac 1200

aacgtcttgg agcgccagag gaggaacgag ctaaaacgga gcttttttgc cctgcgtgac 1260

cagatcccgg agttggaaaa caatgaaaag gcccccaagg tagttatcct taaaaaagcc 1320

acagcataca tcctgtccgt ccaagcagag gagcaaaagc tcatttctga agaggacttg 1380

ttgcggaaac gacgagaaca gttgaaacac aaacttgaac agctacggaa ctcttgtgcg 1440

taa 1443

<210> 146
<211> 480
<212> PRT
<213> Artificial Sequence

<220>
<223> His-NLS-cMyc Amino Acid Sequence

<400> 146
Met Gly Ser Ser His His His His His His Ser Ser Gly Leu Val Pro
1               5                   10                  15

Arg Gly Ser His Met Lys Lys Lys Arg Lys Leu Asp Phe Phe Arg Val
            20                  25                  30

Val Glu Asn Gln Gln Pro Pro Ala Thr Met Pro Leu Asn Val Ser Phe
        35                  40                  45

Thr Asn Arg Asn Tyr Asp Leu Asp Tyr Asp Ser Val Gln Pro Tyr Phe
    50                  55                  60

Tyr Cys Asp Glu Glu Glu Asn Phe Tyr Gln Gln Gln Gln Gln Ser Glu
65                  70                  75                  80

Leu Gln Pro Pro Ala Pro Ser Glu Asp Ile Trp Lys Lys Phe Glu Leu

                85                  90                  95

Leu Pro Thr Pro Pro Leu Ser Pro Ser Arg Arg Ser Gly Leu Cys Ser
            100                 105                 110

Pro Ser Tyr Val Ala Val Thr Pro Phe Ser Leu Arg Gly Asp Asn Asp
        115                 120                 125

Gly Gly Gly Gly Ser Phe Ser Thr Ala Asp Gln Leu Glu Met Val Thr
    130                 135                 140

Glu Leu Leu Gly Gly Asp Met Val Asn Gln Ser Phe Ile Cys Asp Pro
145                 150                 155                 160

Asp Asp Glu Thr Phe Ile Lys Asn Ile Ile Ile Gln Asp Cys Met Trp
                165                 170                 175

Ser Gly Phe Ser Ala Ala Ala Lys Leu Val Ser Glu Lys Leu Ala Ser
            180                 185                 190

Tyr Gln Ala Ala Arg Lys Asp Ser Gly Ser Pro Asn Pro Ala Arg Gly
        195                 200                 205

His Ser Val Cys Ser Thr Ser Ser Leu Tyr Leu Gln Asp Leu Ser Ala
    210                 215                 220

Ala Ala Ser Glu Cys Ile Asp Pro Ser Val Val Phe Pro Tyr Pro Leu
225                 230                 235                 240

Asn Asp Ser Ser Ser Pro Lys Ser Cys Ala Ser Gln Asp Ser Ser Ala
                245                 250                 255

Phe Ser Pro Ser Ser Asp Ser Leu Leu Ser Ser Thr Glu Ser Ser Pro
            260                 265                 270

Gln Gly Ser Pro Glu Pro Leu Val Leu His Glu Glu Thr Pro Pro Thr
        275                 280                 285

Thr Ser Ser Asp Ser Glu Glu Glu Gln Glu Asp Glu Glu Glu Ile Asp
    290                 295                 300

Val Val Ser Val Glu Lys Arg Gln Ala Pro Gly Lys Arg Ser Glu Ser
305                 310                 315                 320

Gly Ser Pro Ser Ala Gly Gly His Ser Lys Pro Pro His Ser Pro Leu
                325                 330                 335

Val Leu Lys Arg Cys His Val Ser Thr His Gln His Asn Tyr Ala Ala
            340                 345                 350

Pro Pro Ser Thr Arg Lys Asp Tyr Pro Ala Ala Lys Arg Val Lys Leu
        355                 360                 365

Asp Ser Val Arg Val Leu Arg Gln Ile Ser Asn Asn Arg Lys Cys Thr
    370                 375                 380

Ser Pro Arg Ser Ser Asp Thr Glu Glu Asn Val Lys Arg Arg Thr His
385                 390                 395                 400

Asn Val Leu Glu Arg Gln Arg Arg Asn Glu Leu Lys Arg Ser Phe Phe
                405                 410                 415

Ala Leu Arg Asp Gln Ile Pro Glu Leu Glu Asn Asn Glu Lys Ala Pro
            420                 425                 430

Lys Val Val Ile Leu Lys Lys Ala Thr Ala Tyr Ile Leu Ser Val Gln
        435                 440                 445

Ala Glu Glu Gln Lys Leu Ile Ser Glu Glu Asp Leu Leu Arg Lys Arg
    450                 455                 460

Arg Glu Gln Leu Lys His Lys Leu Glu Gln Leu Arg Asn Ser Cys Ala
465                 470                 475                 480

<210>    147
<211>    1470
<212>    DNA
<213>    Artificial Sequence

<220>
<223>    His-NLS-JO-84 MTD-cMyc cDNA Sequence

<400>    147
atgggcagca gccatcatca tcatcatcac agcagcggcc tggtgccgcg cggcagccat    60

atgaagaaga agaggaagct ggtggcggcg ctgctggcgg tgctgctgga tttttttcgg    120

gtagtggaaa accagcagcc tcccgcgacg atgcccctca acgttagctt caccaacagg    180

aactatgacc tcgactacga ctcggtgcag ccgtatttct actgcgacga ggaggagaac    240

ttctaccagc agcagcagca gagcgagctg cagcccccgg cgcccagcga ggatatctgg    300

aagaaattcg agctgctgcc caccccgccc ctgtccccta gccgccgctc cgggctctgc    360

tcgccctcct acgttgcggt cacacccttc tcccttcggg gagacaacga cggcggtggc    420

gggagcttct ccacggccga ccagctggag atggtgaccg agctgctggg aggagacatg    480

gtgaaccaga gtttcatctg cgacccggac gacgagacct tcatcaaaaa catcatcatc    540

caggactgta tgtggagcgg cttctcggcc gccgccaagc tcgtctcaga gaagctggcc    600

tcctaccagg ctgcgcgcaa agacagcggc agcccgaacc ccgcccgcgg ccacagcgtc    660

tgctccacct ccagcttgta cctgcaggat ctgagcgccg ccgcctcaga gtgcatcgac    720

ccctcggtgg tcttccccta ccctctcaac gacagcagct cgcccaagtc ctgcgcctcg    780

caagactcca gcgccttctc tccgtcctcg gattctctgc tctcctcgac ggagtcctcc    840

ccgcagggca gccccgagcc cctggtgctc catgaggaga caccgcccac caccagcagc    900

gactctgagg aggaacaaga agatgaggaa gaaatcgatg ttgtttctgt ggaaaagagg    960

caggctcctg gcaaaaggtc agagtctgga tcaccttctg ctggaggcca cagcaaacct    1020

cctcacagcc cactggtcct caagaggtgc cacgtctcca cacatcagca caactacgca    1080

gcgcctccct ccactcggaa ggactatcct gctgccaaga gggtcaagtt ggacagtgtc 1140

agagtcctga gacagatcag caacaaccga aaatgcacca gccccaggtc ctcggacacc 1200

gaggagaatg tcaagaggcg aacacacaac gtcttggagc gccagaggag gaacgagcta 1260

aaacggagct tttttgccct gcgtgaccag atcccggagt tggaaaacaa tgaaaaggcc 1320

cccaaggtag ttatccttaa aaaagccaca gcatacatcc tgtccgtcca agcagaggag 1380

caaaagctca tttctgaaga ggacttgttg cggaaacgac gagaacagtt gaaacacaaa 1440

cttgaacagc tacggaactc ttgtgcgtaa 1470

<210> 148
<211> 489
<212> PRT
<213> Artificial Sequence

<220>
<223> His-NLS-JO-84 MTD-cMyc Amino Acid Sequence

<400> 148
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Leu Asp Phe Phe Arg Val Val Glu Asn Gln Gln Pro Pro
        35                  40                  45

Ala Thr Met Pro Leu Asn Val Ser Phe Thr Asn Arg Asn Tyr Asp Leu
    50                  55                  60

Asp Tyr Asp Ser Val Gln Pro Tyr Phe Tyr Cys Asp Glu Glu Glu Asn
65                  70                  75                  80

Phe Tyr Gln Gln Gln Gln Gln Ser Glu Leu Gln Pro Pro Ala Pro Ser
                85                  90                  95

Glu Asp Ile Trp Lys Lys Phe Glu Leu Leu Pro Thr Pro Pro Leu Ser
            100                 105                 110

Pro Ser Arg Arg Ser Gly Leu Cys Ser Pro Ser Tyr Val Ala Val Thr
        115                 120                 125

Pro Phe Ser Leu Arg Gly Asp Asn Asp Gly Gly Gly Gly Ser Phe Ser
    130                 135                 140

Thr Ala Asp Gln Leu Glu Met Val Thr Glu Leu Leu Gly Gly Asp Met
145                 150                 155                 160

Val Asn Gln Ser Phe Ile Cys Asp Pro Asp Asp Glu Thr Phe Ile Lys
                165                 170                 175

Asn Ile Ile Ile Gln Asp Cys Met Trp Ser Gly Phe Ser Ala Ala Ala
            180                 185                 190

Lys Leu Val Ser Glu Lys Leu Ala Ser Tyr Gln Ala Ala Arg Lys Asp
        195                 200                 205

Ser Gly Ser Pro Asn Pro Ala Arg Gly His Ser Val Cys Ser Thr Ser
    210                 215                 220

Ser Leu Tyr Leu Gln Asp Leu Ser Ala Ala Ala Ser Glu Cys Ile Asp
225                 230                 235                 240

Pro Ser Val Val Phe Pro Tyr Pro Leu Asn Asp Ser Ser Ser Pro Lys
                245                 250                 255

Ser Cys Ala Ser Gln Asp Ser Ser Ala Phe Ser Pro Ser Ser Asp Ser
            260                 265                 270

Leu Leu Ser Ser Thr Glu Ser Ser Pro Gln Gly Ser Pro Glu Pro Leu
        275                 280                 285

Val Leu His Glu Glu Thr Pro Pro Thr Thr Ser Ser Asp Ser Glu Glu
    290                 295                 300

Glu Gln Glu Asp Glu Glu Glu Ile Asp Val Val Ser Val Glu Lys Arg
305                 310                 315                 320

Gln Ala Pro Gly Lys Arg Ser Glu Ser Gly Ser Pro Ser Ala Gly Gly
                325                 330                 335

His Ser Lys Pro Pro His Ser Pro Leu Val Leu Lys Arg Cys His Val
            340                 345                 350

Ser Thr His Gln His Asn Tyr Ala Ala Pro Pro Ser Thr Arg Lys Asp
        355                 360                 365

Tyr Pro Ala Ala Lys Arg Val Lys Leu Asp Ser Val Arg Val Leu Arg
    370                 375                 380

Gln Ile Ser Asn Asn Arg Lys Cys Thr Ser Pro Arg Ser Ser Asp Thr
385                 390                 395                 400

Glu Glu Asn Val Lys Arg Arg Thr His Asn Val Leu Glu Arg Gln Arg
                405                 410                 415

Arg Asn Glu Leu Lys Arg Ser Phe Phe Ala Leu Arg Asp Gln Ile Pro
            420                 425                 430

Glu Leu Glu Asn Asn Glu Lys Ala Pro Lys Val Val Ile Leu Lys Lys
        435                 440                 445

Ala Thr Ala Tyr Ile Leu Ser Val Gln Ala Glu Glu Gln Lys Leu Ile
    450                 455                 460

Ser Glu Glu Asp Leu Leu Arg Lys Arg Arg Glu Gln Leu Lys His Lys
465                 470                 475                 480

Leu Glu Gln Leu Arg Asn Ser Cys Ala
                485

<210>    149

<211>    1470

<212>    DNA

<213>    Artificial Sequence

<220>

<223>    His-NLS-cMyc-JO-84 MTD cDNA Sequence

<400>    149

atgggcagca gccatcatca tcatcatcac agcagcggcc tggtgccgcg cggcagccat    60

atgaagaaga agaggaagct ggattttttt cgggtagtgg aaaaccagca gcctcccgcg    120

acgatgcccc tcaacgttag cttcaccaac aggaactatg acctcgacta cgactcggtg    180

cagccgtatt tctactgcga cgaggaggag aacttctacc agcagcagca gcagagcgag    240

ctgcagcccc cggcgcccag cgaggatatc tggaagaaat tcgagctgct gcccaccccg    300

cccctgtccc ctagccgccg ctccgggctc tgctcgccct cctacgttgc ggtcacaccc    360

ttctcccttc ggggagacaa cgacggcggt ggcgggagct tctccacggc cgaccagctg    420

gagatggtga ccgagctgct gggaggagac atggtgaacc agagtttcat ctgcgacccg    480

gacgacgaga ccttcatcaa aaacatcatc atccaggact gtatgtggag cggcttctcg    540

gccgccgcca agctcgtctc agagaagctg gcctcctacc aggctgcgcg caaagacagc    600

ggcagcccga accccgcccg cggccacagc gtctgctcca cctccagctt gtacctgcag    660

gatctgagcg ccgccgcctc agagtgcatc gacccctcgg tggtcttccc ctaccctctc    720

aacgacagca gctcgcccaa gtcctgcgcc tcgcaagact ccagcgcctt ctctccgtcc    780

tcggattctc tgctctcctc gacggagtcc tccccgcagg gcagccccga gcccctggtg    840

ctccatgagg agacaccgcc caccaccagc agcgactctg aggaggaaca agaagatgag    900

gaagaaatcg atgttgtttc tgtggaaaag aggcaggctc ctggcaaaag gtcagagtct 960

ggatcacctt ctgctggagg ccacagcaaa cctcctcaca gcccactggt cctcaagagg 1020

tgccacgtct ccacacatca gcacaactac gcagcgcctc cctccactcg gaaggactat 1080

cctgctgcca agagggtcaa gttggacagt gtcagagtcc tgagacagat cagcaacaac 1140

cgaaaatgca ccagccccag gtcctcggac accgaggaga atgtcaagag gcgaacacac 1200

aacgtcttgg agcgccagag gaggaacgag ctaaaacgga gcttttttgc cctgcgtgac 1260

cagatcccgg agttggaaaa caatgaaaag gcccccaagg tagttatcct taaaaaagcc 1320

acagcataca tcctgtccgt ccaagcagag gagcaaaagc tcatttctga agaggacttg 1380

ttgcggaaac gacgagaaca gttgaaacac aaacttgaac agctacggaa ctcttgtgcg 1440

ctggtggcgg cgctgctggc ggtgctgtaa 1470

<210> 150
<211> 489
<212> PRT
<213> Artificial Sequence

<220>
<223> His-NLS-cMyc-JO-84 MTD Amino Acid Sequence

<400> 150
Met Gly Ser Ser His His His His His His Ser Ser Gly Leu Val Pro
1               5                   10                  15

Arg Gly Ser His Met Lys Lys Lys Arg Lys Leu Asp Phe Phe Arg Val
            20                  25                  30

Val Glu Asn Gln Gln Pro Pro Ala Thr Met Pro Leu Asn Val Ser Phe
        35                  40                  45

Thr Asn Arg Asn Tyr Asp Leu Asp Tyr Asp Ser Val Gln Pro Tyr Phe
    50                  55                  60

Tyr Cys Asp Glu Glu Glu Asn Phe Tyr Gln Gln Gln Gln Gln Ser Glu
65                  70                  75                  80

Leu Gln Pro Pro Ala Pro Ser Glu Asp Ile Trp Lys Lys Phe Glu Leu
                85                  90                  95

Leu Pro Thr Pro Pro Leu Ser Pro Ser Arg Arg Ser Gly Leu Cys Ser
            100                 105                 110

Pro Ser Tyr Val Ala Val Thr Pro Phe Ser Leu Arg Gly Asp Asn Asp
        115                 120                 125

Gly Gly Gly Gly Ser Phe Ser Thr Ala Asp Gln Leu Glu Met Val Thr
    130                 135                 140

Glu Leu Leu Gly Gly Asp Met Val Asn Gln Ser Phe Ile Cys Asp Pro
145                 150                 155                 160

Asp Asp Glu Thr Phe Ile Lys Asn Ile Ile Ile Gln Asp Cys Met Trp
                165                 170                 175

Ser Gly Phe Ser Ala Ala Ala Lys Leu Val Ser Glu Lys Leu Ala Ser
            180                 185                 190

Tyr Gln Ala Ala Arg Lys Asp Ser Gly Ser Pro Asn Pro Ala Arg Gly
        195                 200                 205

His Ser Val Cys Ser Thr Ser Ser Leu Tyr Leu Gln Asp Leu Ser Ala
    210                 215                 220

Ala Ala Ser Glu Cys Ile Asp Pro Ser Val Val Phe Pro Tyr Pro Leu
225                 230                 235                 240

Asn Asp Ser Ser Ser Pro Lys Ser Cys Ala Ser Gln Asp Ser Ser Ala
                245                 250                 255

Phe Ser Pro Ser Ser Asp Ser Leu Leu Ser Ser Thr Glu Ser Ser Pro

            260                 265                 270

Gln Gly Ser Pro Glu Pro Leu Val Leu His Glu Glu Thr Pro Pro Thr
        275                 280                 285

Thr Ser Ser Asp Ser Glu Glu Glu Gln Glu Asp Glu Glu Glu Ile Asp
    290                 295                 300

Val Val Ser Val Glu Lys Arg Gln Ala Pro Gly Lys Arg Ser Glu Ser
305                 310                 315                 320

Gly Ser Pro Ser Ala Gly Gly His Ser Lys Pro Pro His Ser Pro Leu
                325                 330                 335

Val Leu Lys Arg Cys His Val Ser Thr His Gln His Asn Tyr Ala Ala
            340                 345                 350

Pro Pro Ser Thr Arg Lys Asp Tyr Pro Ala Ala Lys Arg Val Lys Leu
        355                 360                 365

Asp Ser Val Arg Val Leu Arg Gln Ile Ser Asn Asn Arg Lys Cys Thr
    370                 375                 380

Ser Pro Arg Ser Ser Asp Thr Glu Glu Asn Val Lys Arg Arg Thr His
385                 390                 395                 400

Asn Val Leu Glu Arg Gln Arg Arg Asn Glu Leu Lys Arg Ser Phe Phe
                405                 410                 415

Ala Leu Arg Asp Gln Ile Pro Glu Leu Glu Asn Asn Glu Lys Ala Pro
            420                 425                 430

Lys Val Val Ile Leu Lys Lys Ala Thr Ala Tyr Ile Leu Ser Val Gln
        435                 440                 445

Ala Glu Glu Gln Lys Leu Ile Ser Glu Glu Asp Leu Leu Arg Lys Arg
    450                 455                 460

Arg Glu Gln Leu Lys His Lys Leu Glu Gln Leu Arg Asn Ser Cys Ala
465                 470                 475                 480

Leu Val Ala Ala Leu Leu Ala Val Leu
                485

<210> 151
<211> 1497
<212> DNA
<213> Artificial Sequence

<220>
<223> His-NLS-JO-84 MTD-cMyc-JO-84 MTD cDNA Sequence

<400> 151
atgggcagca gccatcatca tcatcatcac agcagcggcc tggtgccgcg cggcagccat 60

atgaagaaga agaggaagct ggtggcggcg ctgctggcgg tgctgctgga tttttttcgg 120

gtagtggaaa accagcagcc tcccgcgacg atgcccctca acgttagctt caccaacagg 180

aactatgacc tcgactacga ctcggtgcag ccgtatttct actgcgacga ggaggagaac 240

ttctaccagc agcagcagca gagcgagctg cagcccccgg cgcccagcga ggatatctgg 300

aagaaattcg agctgctgcc caccccgccc ctgtccccta gccgccgctc cgggctctgc 360

tcgccctcct acgttgcggt cacacccttc tcccttcggg gagacaacga cggcggtggc 420

gggagcttct ccacggccga ccagctggag atggtgaccg agctgctggg aggagacatg 480

gtgaaccaga gtttcatctg cgacccggac gacgagacct tcatcaaaaa catcatcatc 540

caggactgta tgtggagcgg cttctcggcc gccgccaagc tcgtctcaga gaagctggcc 600

tcctaccagg ctgcgcgcaa agacagcggc agcccgaacc ccgcccgcgg ccacagcgtc 660

tgctccacct ccagcttgta cctgcaggat ctgagcgccg ccgcctcaga gtgcatcgac 720

ccctcggtgg tcttccccta ccctctcaac gacagcagct cgcccaagtc ctgcgcctcg 780

caagactcca gcgccttctc tccgtcctcg gattctctgc tctcctcgac ggagtcctcc 840

ccgcagggca gccccgagcc cctggtgctc catgaggaga caccgcccac caccagcagc 900

gactctgagg aggaacaaga agatgaggaa gaaatcgatg ttgtttctgt ggaaaagagg 960

caggctcctg gcaaaaggtc agagtctgga tcaccttctg ctggaggcca cagcaaacct 1020

cctcacagcc cactggtcct caagaggtgc cacgtctcca cacatcagca caactacgca 1080

gcgcctccct ccactcggaa ggactatcct gctgccaaga gggtcaagtt ggacagtgtc 1140

agagtcctga gacagatcag caacaaccga aaatgcacca gccccaggtc ctcggacacc 1200

gaggagaatg tcaagaggcg aacacacaac gtcttggagc gccagaggag gaacgagcta 1260

aaacggagct tttttgccct gcgtgaccag atcccggagt tggaaaacaa tgaaaaggcc 1320

cccaaggtag ttatccttaa aaaagccaca gcatacatcc tgtccgtcca agcagaggag 1380

caaaagctca tttctgaaga ggacttgttg cggaaacgac gagaacagtt gaaacacaaa 1440

cttgaacagc tacggaactc ttgtgcgctg gtggcggcgc tgctggcggt gctgtaa 1497

<210> 152
<211> 498
<212> PRT
<213> Artificial Sequence

<220>
<223> His-NLS-JO-84 MTD-cMyc-JO-84 MTD Amino Acid Sequence

<400> 152
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu

20                  25                  30

Ala Val Leu Leu Asp Phe Phe Arg Val Val Glu Asn Gln Gln Pro Pro
        35                  40                  45

Ala Thr Met Pro Leu Asn Val Ser Phe Thr Asn Arg Asn Tyr Asp Leu
    50                  55                  60

Asp Tyr Asp Ser Val Gln Pro Tyr Phe Tyr Cys Asp Glu Glu Glu Asn
65                  70                  75                  80

Phe Tyr Gln Gln Gln Gln Gln Ser Glu Leu Gln Pro Pro Ala Pro Ser
                85                  90                  95

Glu Asp Ile Trp Lys Lys Phe Glu Leu Leu Pro Thr Pro Pro Leu Ser
            100                 105                 110

Pro Ser Arg Arg Ser Gly Leu Cys Ser Pro Ser Tyr Val Ala Val Thr
        115                 120                 125

Pro Phe Ser Leu Arg Gly Asp Asn Asp Gly Gly Gly Gly Ser Phe Ser
    130                 135                 140

Thr Ala Asp Gln Leu Glu Met Val Thr Glu Leu Leu Gly Gly Asp Met
145                 150                 155                 160

Val Asn Gln Ser Phe Ile Cys Asp Pro Asp Asp Glu Thr Phe Ile Lys
                165                 170                 175

Asn Ile Ile Ile Gln Asp Cys Met Trp Ser Gly Phe Ser Ala Ala Ala
            180                 185                 190

Lys Leu Val Ser Glu Lys Leu Ala Ser Tyr Gln Ala Ala Arg Lys Asp
        195                 200                 205

Ser Gly Ser Pro Asn Pro Ala Arg Gly His Ser Val Cys Ser Thr Ser
    210                 215                 220

Ser Leu Tyr Leu Gln Asp Leu Ser Ala Ala Ala Ser Glu Cys Ile Asp
225                 230                 235                 240

Pro Ser Val Val Phe Pro Tyr Pro Leu Asn Asp Ser Ser Ser Pro Lys
                245                 250                 255

Ser Cys Ala Ser Gln Asp Ser Ser Ala Phe Ser Pro Ser Ser Asp Ser
            260                 265                 270

Leu Leu Ser Ser Thr Glu Ser Ser Pro Gln Gly Ser Pro Glu Pro Leu
        275                 280                 285

Val Leu His Glu Glu Thr Pro Pro Thr Thr Ser Ser Asp Ser Glu Glu
    290                 295                 300

Glu Gln Glu Asp Glu Glu Glu Ile Asp Val Val Ser Val Glu Lys Arg
305                 310                 315                 320

Gln Ala Pro Gly Lys Arg Ser Glu Ser Gly Ser Pro Ser Ala Gly Gly
                325                 330                 335

His Ser Lys Pro Pro His Ser Pro Leu Val Leu Lys Arg Cys His Val
            340                 345                 350

Ser Thr His Gln His Asn Tyr Ala Ala Pro Pro Ser Thr Arg Lys Asp
        355                 360                 365

Tyr Pro Ala Ala Lys Arg Val Lys Leu Asp Ser Val Arg Val Leu Arg
    370                 375                 380

Gln Ile Ser Asn Asn Arg Lys Cys Thr Ser Pro Arg Ser Ser Asp Thr
385                 390                 395                 400

Glu Glu Asn Val Lys Arg Arg Thr His Asn Val Leu Glu Arg Gln Arg
                405                 410                 415

Arg Asn Glu Leu Lys Arg Ser Phe Phe Ala Leu Arg Asp Gln Ile Pro
            420                 425                 430

Glu Leu Glu Asn Asn Glu Lys Ala Pro Lys Val Val Ile Leu Lys Lys
        435                 440                 445

Ala Thr Ala Tyr Ile Leu Ser Val Gln Ala Glu Glu Gln Lys Leu Ile
    450                 455                 460

Ser Glu Glu Asp Leu Leu Arg Lys Arg Arg Glu Gln Leu Lys His Lys
465                 470                 475                 480

Leu Glu Gln Leu Arg Asn Ser Cys Ala Leu Val Ala Ala Leu Leu Ala
                485                 490                 495

Val Leu

<210>    153
<211>    1467
<212>    DNA
<213>    Artificial Sequence

<220>
<223>    His-NLS-JO-86 MTD-cMyc cDNA Sequence

<400>    153
atgggcagca gccatcatca tcatcatcac agcagcggcc tggtgccgcg cggcagccat    60

atgaagaaga agaggaagct ggcggtgctg gcggcggcgc cgctggattt ttttcgggta    120

gtggaaaacc agcagcctcc cgcgacgatg cccctcaacg ttagcttcac caacaggaac    180

tatgacctcg actacgactc ggtgcagccg tatttctact gcgacgagga ggagaacttc    240

taccagcagc agcagcagag cgagctgcag cccccggcgc ccagcgagga tatctggaag    300

aaattcgagc tgctgcccac cccgcccctg tcccctagcc gccgctccgg gctctgctcg    360

ccctcctacg ttgcggtcac acccttctcc cttcggggag acaacgacgg cggtggcggg    420

agcttctcca cggccgacca gctggagatg gtgaccgagc tgctgggagg agacatggtg    480

aaccagagtt tcatctgcga cccggacgac gagaccttca tcaaaaacat catcatccag    540

gactgtatgt ggagcggctt ctcggccgcc gccaagctcg tctcagagaa gctggcctcc 600

taccaggctg cgcgcaaaga cagcggcagc ccgaaccccg cccgcggcca cagcgtctgc 660

tccacctcca gcttgtacct gcaggatctg agcgccgccg cctcagagtg catcgacccc 720

tcggtggtct tcccctaccc tctcaacgac agcagctcgc ccaagtcctg cgcctcgcaa 780

gactccagcg ccttctctcc gtcctcggat tctctgctct cctcgacgga gtcctccccg 840

cagggcagcc ccgagcccct ggtgctccat gaggagacac cgcccaccac cagcagcgac 900

tctgaggagg aacaagaaga tgaggaagaa atcgatgttg tttctgtgga aaagaggcag 960

gctcctggca aaaggtcaga gtctggatca ccttctgctg gaggccacag caaacctcct 1020

cacagcccac tggtcctcaa gaggtgccac gtctccacac atcagcacaa ctacgcagcg 1080

cctccctcca ctcggaagga ctatcctgct gccaagaggg tcaagttgga cagtgtcaga 1140

gtcctgagac agatcagcaa caaccgaaaa tgcaccagcc ccaggtcctc ggacaccgag 1200

gagaatgtca agaggcgaac acacaacgtc ttggagcgcc agaggaggaa cgagctaaaa 1260

cggagctttt ttgccctgcg tgaccagatc ccggagttgg aaaacaatga aaaggccccc 1320

aaggtagtta tccttaaaaa agccacagca tacatcctgt ccgtccaagc agaggagcaa 1380

aagctcattt ctgaagagga cttgttgcgg aaacgacgag aacagttgaa acacaaactt 1440

gaacagctac ggaactcttg tgcgtaa 1467

<210> 154
<211> 488
<212> PRT
<213> Artificial Sequence

<220>

<223>    His-NLS-JO-86 MTD-cMyc Amino Acid Sequence

<400>    154
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Leu Asp Phe Phe Arg Val Val Glu Asn Gln Gln Pro Pro Ala
        35                  40                  45

Thr Met Pro Leu Asn Val Ser Phe Thr Asn Arg Asn Tyr Asp Leu Asp
    50                  55                  60

Tyr Asp Ser Val Gln Pro Tyr Phe Tyr Cys Asp Glu Glu Glu Asn Phe
65                  70                  75                  80

Tyr Gln Gln Gln Gln Gln Ser Glu Leu Gln Pro Pro Ala Pro Ser Glu
                85                  90                  95

Asp Ile Trp Lys Lys Phe Glu Leu Leu Pro Thr Pro Pro Leu Ser Pro
            100                 105                 110

Ser Arg Arg Ser Gly Leu Cys Ser Pro Ser Tyr Val Ala Val Thr Pro
        115                 120                 125

Phe Ser Leu Arg Gly Asp Asn Asp Gly Gly Gly Gly Ser Phe Ser Thr
    130                 135                 140

Ala Asp Gln Leu Glu Met Val Thr Glu Leu Leu Gly Gly Asp Met Val
145                 150                 155                 160

Asn Gln Ser Phe Ile Cys Asp Pro Asp Asp Glu Thr Phe Ile Lys Asn
                165                 170                 175

Ile Ile Ile Gln Asp Cys Met Trp Ser Gly Phe Ser Ala Ala Ala Lys
            180                 185                 190

Leu Val Ser Glu Lys Leu Ala Ser Tyr Gln Ala Ala Arg Lys Asp Ser
        195                 200                 205

Gly Ser Pro Asn Pro Ala Arg Gly His Ser Val Cys Ser Thr Ser Ser
    210                 215                 220

Leu Tyr Leu Gln Asp Leu Ser Ala Ala Ala Ser Glu Cys Ile Asp Pro
225                 230                 235                 240

Ser Val Val Phe Pro Tyr Pro Leu Asn Asp Ser Ser Ser Pro Lys Ser
                245                 250                 255

Cys Ala Ser Gln Asp Ser Ser Ala Phe Ser Pro Ser Ser Asp Ser Leu
            260                 265                 270

Leu Ser Ser Thr Glu Ser Ser Pro Gln Gly Ser Pro Glu Pro Leu Val
        275                 280                 285

Leu His Glu Glu Thr Pro Pro Thr Thr Ser Ser Asp Ser Glu Glu Glu
    290                 295                 300

Gln Glu Asp Glu Glu Glu Ile Asp Val Val Ser Val Glu Lys Arg Gln
305                 310                 315                 320

Ala Pro Gly Lys Arg Ser Glu Ser Gly Ser Pro Ser Ala Gly Gly His
                325                 330                 335

Ser Lys Pro Pro His Ser Pro Leu Val Leu Lys Arg Cys His Val Ser
            340                 345                 350

Thr His Gln His Asn Tyr Ala Ala Pro Pro Ser Thr Arg Lys Asp Tyr
        355                 360                 365

Pro Ala Ala Lys Arg Val Lys Leu Asp Ser Val Arg Val Leu Arg Gln
    370                 375                 380

Ile Ser Asn Asn Arg Lys Cys Thr Ser Pro Arg Ser Ser Asp Thr Glu
385                 390                 395                 400

Glu Asn Val Lys Arg Arg Thr His Asn Val Leu Glu Arg Gln Arg Arg

405                 410                 415

Asn Glu Leu Lys Arg Ser Phe Phe Ala Leu Arg Asp Gln Ile Pro Glu
            420                 425                 430

Leu Glu Asn Asn Glu Lys Ala Pro Lys Val Val Ile Leu Lys Lys Ala
        435                 440                 445

Thr Ala Tyr Ile Leu Ser Val Gln Ala Glu Glu Gln Lys Leu Ile Ser
    450                 455                 460

Glu Glu Asp Leu Leu Arg Lys Arg Arg Glu Gln Leu Lys His Lys Leu
465                 470                 475                 480

Glu Gln Leu Arg Asn Ser Cys Ala
                485

<210>    155
<211>    1467
<212>    DNA
<213>    Artificial Sequence

<220>
<223>    His-NLS-cMyc-JO-86 MTD cDNA Sequence

<400>    155
atgggcagca gccatcatca tcatcatcac agcagcggcc tggtgccgcg cggcagccat      60

atgaagaaga agaggaagct ggattttttt cgggtagtgg aaaaccagca gcctcccgcg     120

acgatgcccc tcaacgttag cttcaccaac aggaactatg acctcgacta cgactcggtg     180

cagccgtatt tctactgcga cgaggaggag aacttctacc agcagcagca gcagagcgag     240

ctgcagcccc cggcgcccag cgaggatatc tggaagaaat tcgagctgct gcccaccccg     300

cccctgtccc ctagccgccg ctccgggctc tgctcgccct cctacgttgc ggtcacaccc     360

ttctcccttc ggggagacaa cgacggcggt ggcgggagct tctccacggc cgaccagctg 420

gagatggtga ccgagctgct gggaggagac atggtgaacc agagtttcat ctgcgacccg 480

gacgacgaga ccttcatcaa aaacatcatc atccaggact gtatgtggag cggcttctcg 540

gccgccgcca agctcgtctc agagaagctg gcctcctacc aggctgcgcg caaagacagc 600

ggcagcccga accccgcccg cggccacagc gtctgctcca cctccagctt gtacctgcag 660

gatctgagcg ccgccgcctc agagtgcatc gacccctcgg tggtcttccc ctaccctctc 720

aacgacagca gctcgcccaa gtcctgcgcc tcgcaagact ccagcgcctt ctctccgtcc 780

tcggattctc tgctctcctc gacggagtcc tccccgcagg gcagccccga gcccctggtg 840

ctccatgagg agacaccgcc caccaccagc agcgactctg aggaggaaca agaagatgag 900

gaagaaatcg atgttgtttc tgtggaaaag aggcaggctc ctggcaaaag gtcagagtct 960

ggatcacctt ctgctggagg ccacagcaaa cctcctcaca gcccactggt cctcaagagg 1020

tgccacgtct ccacacatca gcacaactac gcagcgcctc cctccactcg gaaggactat 1080

cctgctgcca agagggtcaa gttggacagt gtcagagtcc tgagacagat cagcaacaac 1140

cgaaaatgca ccagccccag gtcctcggac accgaggaga atgtcaagag gcgaacacac 1200

aacgtcttgg agcgccagag gaggaacgag ctaaaacgga gcttttttgc cctgcgtgac 1260

cagatcccgg agttggaaaa caatgaaaag gcccccaagg tagttatcct taaaaaagcc 1320

acagcataca tcctgtccgt ccaagcagag gagcaaaagc tcatttctga agaggacttg 1380

ttgcggaaac gacgagaaca gttgaaacac aaacttgaac agctacggaa ctcttgtgcg 1440

ctggcggtgc tggcggcggc gccgtaa 1467

<210> 156

<211>    488
<212>    PRT
<213>    Artificial Sequence

<220>
<223>    His-NLS-cMyc-JO-86 MTD Amino Acid Sequence

<400>    156
Met Gly Ser Ser His His His His His His Ser Ser Gly Leu Val Pro
1               5                   10                  15

Arg Gly Ser His Met Lys Lys Lys Arg Lys Leu Asp Phe Phe Arg Val
            20                  25                  30

Val Glu Asn Gln Gln Pro Pro Ala Thr Met Pro Leu Asn Val Ser Phe
        35                  40                  45

Thr Asn Arg Asn Tyr Asp Leu Asp Tyr Asp Ser Val Gln Pro Tyr Phe
    50                  55                  60

Tyr Cys Asp Glu Glu Glu Asn Phe Tyr Gln Gln Gln Gln Gln Ser Glu
65                  70                  75                  80

Leu Gln Pro Pro Ala Pro Ser Glu Asp Ile Trp Lys Lys Phe Glu Leu
                85                  90                  95

Leu Pro Thr Pro Pro Leu Ser Pro Ser Arg Arg Ser Gly Leu Cys Ser
            100                 105                 110

Pro Ser Tyr Val Ala Val Thr Pro Phe Ser Leu Arg Gly Asp Asn Asp
        115                 120                 125

Gly Gly Gly Gly Ser Phe Ser Thr Ala Asp Gln Leu Glu Met Val Thr
    130                 135                 140

Glu Leu Leu Gly Gly Asp Met Val Asn Gln Ser Phe Ile Cys Asp Pro
145                 150                 155                 160

Asp Asp Glu Thr Phe Ile Lys Asn Ile Ile Ile Gln Asp Cys Met Trp

                165                 170                 175

Ser Gly Phe Ser Ala Ala Ala Lys Leu Val Ser Glu Lys Leu Ala Ser
            180                 185                 190

Tyr Gln Ala Ala Arg Lys Asp Ser Gly Ser Pro Asn Pro Ala Arg Gly
        195                 200                 205

His Ser Val Cys Ser Thr Ser Ser Leu Tyr Leu Gln Asp Leu Ser Ala
    210                 215                 220

Ala Ala Ser Glu Cys Ile Asp Pro Ser Val Val Phe Pro Tyr Pro Leu
225                 230                 235                 240

Asn Asp Ser Ser Ser Pro Lys Ser Cys Ala Ser Gln Asp Ser Ser Ala
                245                 250                 255

Phe Ser Pro Ser Ser Asp Ser Leu Leu Ser Ser Thr Glu Ser Ser Pro
            260                 265                 270

Gln Gly Ser Pro Glu Pro Leu Val Leu His Glu Glu Thr Pro Pro Thr
        275                 280                 285

Thr Ser Ser Asp Ser Glu Glu Glu Gln Glu Asp Glu Glu Glu Ile Asp
    290                 295                 300

Val Val Ser Val Glu Lys Arg Gln Ala Pro Gly Lys Arg Ser Glu Ser
305                 310                 315                 320

Gly Ser Pro Ser Ala Gly Gly His Ser Lys Pro Pro His Ser Pro Leu
                325                 330                 335

Val Leu Lys Arg Cys His Val Ser Thr His Gln His Asn Tyr Ala Ala
            340                 345                 350

Pro Pro Ser Thr Arg Lys Asp Tyr Pro Ala Ala Lys Arg Val Lys Leu
        355                 360                 365

Asp Ser Val Arg Val Leu Arg Gln Ile Ser Asn Asn Arg Lys Cys Thr
    370                 375                 380

Ser Pro Arg Ser Ser Asp Thr Glu Glu Asn Val Lys Arg Arg Thr His
385                 390                 395                 400

Asn Val Leu Glu Arg Gln Arg Arg Asn Glu Leu Lys Arg Ser Phe Phe
                405                 410                 415

Ala Leu Arg Asp Gln Ile Pro Glu Leu Glu Asn Asn Glu Lys Ala Pro
            420                 425                 430

Lys Val Val Ile Leu Lys Lys Ala Thr Ala Tyr Ile Leu Ser Val Gln
        435                 440                 445

Ala Glu Glu Gln Lys Leu Ile Ser Glu Glu Asp Leu Leu Arg Lys Arg
    450                 455                 460

Arg Glu Gln Leu Lys His Lys Leu Glu Gln Leu Arg Asn Ser Cys Ala
465                 470                 475                 480

Leu Ala Val Leu Ala Ala Ala Pro
                485

<210>    157
<211>    1491
<212>    DNA
<213>    Artificial Sequence

<220>
<223>    His-NLS-JO-86 MTD-cMyc-JO-86 MTD cDNA Sequence

<400>    157
atgggcagca gccatcatca tcatcatcac agcagcggcc tggtgccgcg cggcagccat      60

atgaagaaga agaggaagct ggcggtgctg gcggcggcgc cgctggattt ttttcgggta     120

gtggaaaacc agcagcctcc cgcgacgatg cccctcaacg ttagcttcac caacaggaac     180

tatgacctcg actacgactc ggtgcagccg tatttctact gcgacgagga ggagaacttc     240

taccagcagc agcagcagag cgagctgcag cccccggcgc ccagcgagga tatctggaag 300

aaattcgagc tgctgcccac cccgcccctg tcccctagcc gccgctccgg gctctgctcg 360

ccctcctacg ttgcggtcac acccttctcc cttcggggag acaacgacgg cggtggcggg 420

agcttctcca cggccgacca gctggagatg gtgaccgagc tgctgggagg agacatggtg 480

aaccagagtt tcatctgcga cccggacgac gagaccttca tcaaaaacat catcatccag 540

gactgtatgt ggagcggctt ctcggccgcc gccaagctcg tctcagagaa gctggcctcc 600

taccaggctg cgcgcaaaga cagcggcagc ccgaaccccg cccgcggcca cagcgtctgc 660

tccacctcca gcttgtacct gcaggatctg agcgccgccg cctcagagtg catcgacccc 720

tcggtggtct tcccctaccc tctcaacgac agcagctcgc ccaagtcctg cgcctcgcaa 780

gactccagcg ccttctctcc gtcctcggat tctctgctct cctcgacgga gtcctccccg 840

cagggcagcc ccgagcccct ggtgctccat gaggagacac cgcccaccac cagcagcgac 900

tctgaggagg aacaagaaga tgaggaagaa atcgatgttg tttctgtgga aaagaggcag 960

gctcctggca aaaggtcaga gtctggatca ccttctgctg gaggccacag caaacctcct 1020

cacagcccac tggtcctcaa gaggtgccac gtctccacac atcagcacaa ctacgcagcg 1080

cctccctcca ctcggaagga ctatcctgct gccaagaggg tcaagttgga cagtgtcaga 1140

gtcctgagac agatcagcaa caaccgaaaa tgcaccagcc ccaggtcctc ggacaccgag 1200

gagaatgtca agaggcgaac acacaacgtc ttggagcgcc agaggaggaa cgagctaaaa 1260

cggagctttt ttgccctgcg tgaccagatc ccggagttgg aaaacaatga aaaggccccc 1320

aaggtagtta tccttaaaaa agccacagca tacatcctgt ccgtccaagc agaggagcaa 1380

aagctcattt ctgaagagga cttgttgcgg aaacgacgag aacagttgaa acacaaactt 1440

gaacagctac ggaactcttg tgcgctggcg gtgctggcgg cggcgccgta a 1491

<210> 158
<211> 496
<212> PRT
<213> Artificial Sequence

<220>
<223> His-NLS-JO-86 MTD-cMyc-JO-86 MTD Amino Acid Sequence

<400> 158
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Leu Asp Phe Phe Arg Val Val Glu Asn Gln Gln Pro Pro Ala
        35                  40                  45

Thr Met Pro Leu Asn Val Ser Phe Thr Asn Arg Asn Tyr Asp Leu Asp
    50                  55                  60

Tyr Asp Ser Val Gln Pro Tyr Phe Tyr Cys Asp Glu Glu Glu Asn Phe
65                  70                  75                  80

Tyr Gln Gln Gln Gln Gln Ser Glu Leu Gln Pro Pro Ala Pro Ser Glu
                85                  90                  95

Asp Ile Trp Lys Lys Phe Glu Leu Leu Pro Thr Pro Pro Leu Ser Pro
            100                 105                 110

Ser Arg Arg Ser Gly Leu Cys Ser Pro Ser Tyr Val Ala Val Thr Pro
        115                 120                 125

Phe Ser Leu Arg Gly Asp Asn Asp Gly Gly Gly Gly Ser Phe Ser Thr
    130                 135                 140

Ala Asp Gln Leu Glu Met Val Thr Glu Leu Leu Gly Gly Asp Met Val
145                 150                 155                 160

Asn Gln Ser Phe Ile Cys Asp Pro Asp Asp Glu Thr Phe Ile Lys Asn
                165                 170                 175

Ile Ile Ile Gln Asp Cys Met Trp Ser Gly Phe Ser Ala Ala Ala Lys
            180                 185                 190

Leu Val Ser Glu Lys Leu Ala Ser Tyr Gln Ala Ala Arg Lys Asp Ser
        195                 200                 205

Gly Ser Pro Asn Pro Ala Arg Gly His Ser Val Cys Ser Thr Ser Ser
    210                 215                 220

Leu Tyr Leu Gln Asp Leu Ser Ala Ala Ala Ser Glu Cys Ile Asp Pro
225                 230                 235                 240

Ser Val Val Phe Pro Tyr Pro Leu Asn Asp Ser Ser Ser Pro Lys Ser
                245                 250                 255

Cys Ala Ser Gln Asp Ser Ser Ala Phe Ser Pro Ser Ser Asp Ser Leu
            260                 265                 270

Leu Ser Ser Thr Glu Ser Ser Pro Gln Gly Ser Pro Glu Pro Leu Val
        275                 280                 285

Leu His Glu Glu Thr Pro Pro Thr Thr Ser Ser Asp Ser Glu Glu Glu
    290                 295                 300

Gln Glu Asp Glu Glu Glu Ile Asp Val Val Ser Val Glu Lys Arg Gln
305                 310                 315                 320

Ala Pro Gly Lys Arg Ser Glu Ser Gly Ser Pro Ser Ala Gly Gly His
                325                 330                 335

Ser Lys Pro Pro His Ser Pro Leu Val Leu Lys Arg Cys His Val Ser
            340                 345                 350

Thr His Gln His Asn Tyr Ala Ala Pro Pro Ser Thr Arg Lys Asp Tyr
        355                 360                 365

Pro Ala Ala Lys Arg Val Lys Leu Asp Ser Val Arg Val Leu Arg Gln
    370                 375                 380

Ile Ser Asn Asn Arg Lys Cys Thr Ser Pro Arg Ser Ser Asp Thr Glu
385                 390                 395                 400

Glu Asn Val Lys Arg Arg Thr His Asn Val Leu Glu Arg Gln Arg Arg
                405                 410                 415

Asn Glu Leu Lys Arg Ser Phe Phe Ala Leu Arg Asp Gln Ile Pro Glu
            420                 425                 430

Leu Glu Asn Asn Glu Lys Ala Pro Lys Val Val Ile Leu Lys Lys Ala
        435                 440                 445

Thr Ala Tyr Ile Leu Ser Val Gln Ala Glu Glu Gln Lys Leu Ile Ser
    450                 455                 460

Glu Glu Asp Leu Leu Arg Lys Arg Arg Glu Gln Leu Lys His Lys Leu
465                 470                 475                 480

Glu Gln Leu Arg Asn Ser Cys Ala Leu Ala Val Leu Ala Ala Ala Pro
                485                 490                 495

<210>    159
<211>    645
<212>    DNA
<213>    Artificial Sequence

<220>
<223>    NLS-Lin28 cDNA Sequence

<400>    159

atgaagaaga agaggaaggg ctccgtgtcc aaccagcagt ttgcaggtgg ctgcgccaag 60

gcggcagaag aggcgcccga ggaggcgccg gaggacgcgg cccgggcggc ggacgagcct 120

cagctgctgc acggtgcggg catctgtaag tggttcaacg tgcgcatggg gttcggcttc 180

ctgtccatga ccgcccgcgc cggggtcgcg ctcgaccccc cagtggatgt ctttgtgcac 240

cagagtaagc tgcacatgga agggttccgg agcttgaagg agggtgaggc agtggagttc 300

acctttaaga agtcagccaa gggtctggaa tccatccgtg tcaccggacc tggtggagta 360

ttctgtattg ggagtgagag gcggccaaaa ggaaagagca tgcagaagcg cagatcaaaa 420

ggagacaggt gctacaactg tggaggtcta gatcatcatg ccaaggaatg caagctgcca 480

ccccagccca agaagtgcca cttctgccag agcatcagcc atatggtagc ctcatgtccg 540

ctgaaggccc agcagggccc tagtgcacag ggaaagccaa cctactttcg agaggaagaa 600

gaagaaatcc acagccctac cctgctcccg gaggcacaga attga 645

<210> 160
<211> 214
<212> PRT
<213> Artificial Sequence

<220>
<223> NLS-Lin28 Amino Acid Sequence

<400> 160
Met Lys Lys Lys Arg Lys Gly Ser Val Ser Asn Gln Gln Phe Ala Gly
1               5                   10                  15

Gly Cys Ala Lys Ala Ala Glu Glu Ala Pro Glu Glu Ala Pro Glu Asp
            20                  25                  30

Ala Ala Arg Ala Ala Asp Glu Pro Gln Leu Leu His Gly Ala Gly Ile

35                  40                  45

Cys Lys Trp Phe Asn Val Arg Met Gly Phe Gly Phe Leu Ser Met Thr
    50                  55                  60

Ala Arg Ala Gly Val Ala Leu Asp Pro Pro Val Asp Val Phe Val His
65                  70                  75                  80

Gln Ser Lys Leu His Met Glu Gly Phe Arg Ser Leu Lys Glu Gly Glu
                85                  90                  95

Ala Val Glu Phe Thr Phe Lys Lys Ser Ala Lys Gly Leu Glu Ser Ile
            100                 105                 110

Arg Val Thr Gly Pro Gly Gly Val Phe Cys Ile Gly Ser Glu Arg Arg
        115                 120                 125

Pro Lys Gly Lys Ser Met Gln Lys Arg Arg Ser Lys Gly Asp Arg Cys
    130                 135                 140

Tyr Asn Cys Gly Gly Leu Asp His His Ala Lys Glu Cys Lys Leu Pro
145                 150                 155                 160

Pro Gln Pro Lys Lys Cys His Phe Cys Gln Ser Ile Ser His Met Val
                165                 170                 175

Ala Ser Cys Pro Leu Lys Ala Gln Gln Gly Pro Ser Ala Gln Gly Lys
            180                 185                 190

Pro Thr Tyr Phe Arg Glu Glu Glu Glu Glu Ile His Ser Pro Thr Leu
        195                 200                 205

Leu Pro Glu Ala Gln Asn
    210

<210>   161
<211>   672
<212>   DNA
<213>   Artificial Sequence

<220>

<223>    NLS-JO-84 MTD-Lin28 cDNA Sequence

<400>    161
atgaagaaga agaggaagct ggtggcggcg ctgctggcgg tgctgggctc cgtgtccaac        60

cagcagtttg caggtggctg cgccaaggcg gcagaagagg cgcccgagga ggcgccggag       120

gacgcggccc gggcggcgga cgagcctcag ctgctgcacg gtgcgggcat ctgtaagtgg       180

ttcaacgtgc gcatggggtt cggcttcctg tccatgaccg cccgcgccgg ggtcgcgctc       240

gaccccccag tggatgtctt tgtgcaccag agtaagctgc acatggaagg gttccggagc       300

ttgaaggagg gtgaggcagt ggagttcacc tttaagaagt cagccaaggg tctggaatcc       360

atccgtgtca ccggacctgg tggagtattc tgtattggga gtgagaggcg gccaaaagga       420

aagagcatgc agaagcgcag atcaaaagga gacaggtgct acaactgtgg aggtctagat       480

catcatgcca aggaatgcaa gctgccaccc cagcccaaga agtgccactt ctgccagagc       540

atcagccata tggtagcctc atgtccgctg aaggcccagc agggccctag tgcacaggga       600

aagccaacct actttcgaga ggaagaagaa gaaatccaca gccctaccct gctcccggag       660

gcacagaatt ga                                                            672

<210>    162
<211>    223
<212>    PRT
<213>    Artificial Sequence

<220>
<223>    NLS-JO-84 MTD-Lin28 Amino Acid Sequence

<400> 162

Met Lys Lys Lys Arg Lys Leu Val Ala Ala Leu Leu Ala Val Leu Gly
1               5                   10                  15

Ser Val Ser Asn Gln Gln Phe Ala Gly Gly Cys Ala Lys Ala Ala Glu
            20                  25                  30

Glu Ala Pro Glu Glu Ala Pro Glu Asp Ala Ala Arg Ala Ala Asp Glu
        35                  40                  45

Pro Gln Leu Leu His Gly Ala Gly Ile Cys Lys Trp Phe Asn Val Arg
    50                  55                  60

Met Gly Phe Gly Phe Leu Ser Met Thr Ala Arg Ala Gly Val Ala Leu
65                  70                  75                  80

Asp Pro Pro Val Asp Val Phe Val His Gln Ser Lys Leu His Met Glu
                85                  90                  95

Gly Phe Arg Ser Leu Lys Glu Gly Glu Ala Val Glu Phe Thr Phe Lys
            100                 105                 110

Lys Ser Ala Lys Gly Leu Glu Ser Ile Arg Val Thr Gly Pro Gly Gly
        115                 120                 125

Val Phe Cys Ile Gly Ser Glu Arg Arg Pro Lys Gly Lys Ser Met Gln
    130                 135                 140

Lys Arg Arg Ser Lys Gly Asp Arg Cys Tyr Asn Cys Gly Gly Leu Asp
145                 150                 155                 160

His His Ala Lys Glu Cys Lys Leu Pro Pro Gln Pro Lys Lys Cys His
                165                 170                 175

Phe Cys Gln Ser Ile Ser His Met Val Ala Ser Cys Pro Leu Lys Ala
            180                 185                 190

Gln Gln Gly Pro Ser Ala Gln Gly Lys Pro Thr Tyr Phe Arg Glu Glu
        195                 200                 205

Glu Glu Glu Ile His Ser Pro Thr Leu Leu Pro Glu Ala Gln Asn
210                 215                 220

<210>    163
<211>    672
<212>    DNA
<213>    Artificial Sequence

<220>
<223>    NLS-Lin28-JO-84 MTD cDNA Sequence

<400>    163
atgaagaaga agaggaaggg ctccgtgtcc aaccagcagt ttgcaggtgg ctgcgccaag    60

gcggcagaag aggcgcccga ggaggcgccg gaggacgcgg cccgggcggc ggacgagcct    120

cagctgctgc acggtgcggg catctgtaag tggttcaacg tgcgcatggg gttcggcttc    180

ctgtccatga ccgcccgcgc cggggtcgcg ctcgaccccc cagtggatgt ctttgtgcac    240

cagagtaagc tgcacatgga agggttccgg agcttgaagg agggtgaggc agtggagttc    300

acctttaaga agtcagccaa gggtctggaa tccatccgtg tcaccggacc tggtggagta    360

ttctgtattg ggagtgagag gcggccaaaa ggaaagagca tgcagaagcg cagatcaaaa    420

ggagacaggt gctacaactg tggaggtcta gatcatcatg ccaaggaatg caagctgcca    480

ccccagccca agaagtgcca cttctgccag agcatcagcc atatggtagc ctcatgtccg    540

ctgaaggccc agcagggccc tagtgcacag ggaaagccaa cctactttcg agaggaagaa    600

gaagaaatcc acagccctac cctgctcccg gaggcacaga atctggtggc ggcgctgctg    660

gcggtgctgt ga                                                        672

<210>    164
<211>    223
<212>    PRT
<213>    Artificial Sequence

<220>
<223>    NLS-Lin28-JO-84 MTD Amino Acid Sequence

<400>    164
Met Lys Lys Lys Arg Lys Gly Ser Val Ser Asn Gln Gln Phe Ala Gly
1               5                   10                  15

Gly Cys Ala Lys Ala Ala Glu Glu Ala Pro Glu Glu Ala Pro Glu Asp
            20                  25                  30

Ala Ala Arg Ala Ala Asp Glu Pro Gln Leu Leu His Gly Ala Gly Ile
        35                  40                  45

Cys Lys Trp Phe Asn Val Arg Met Gly Phe Gly Phe Leu Ser Met Thr
    50                  55                  60

Ala Arg Ala Gly Val Ala Leu Asp Pro Pro Val Asp Val Phe Val His
65                  70                  75                  80

Gln Ser Lys Leu His Met Glu Gly Phe Arg Ser Leu Lys Glu Gly Glu
                85                  90                  95

Ala Val Glu Phe Thr Phe Lys Lys Ser Ala Lys Gly Leu Glu Ser Ile
            100                 105                 110

Arg Val Thr Gly Pro Gly Gly Val Phe Cys Ile Gly Ser Glu Arg Arg
        115                 120                 125

Pro Lys Gly Lys Ser Met Gln Lys Arg Arg Ser Lys Gly Asp Arg Cys
    130                 135                 140

Tyr Asn Cys Gly Gly Leu Asp His His Ala Lys Glu Cys Lys Leu Pro
145                 150                 155                 160

Pro Gln Pro Lys Lys Cys His Phe Cys Gln Ser Ile Ser His Met Val
                165                 170                 175

Ala Ser Cys Pro Leu Lys Ala Gln Gln Gly Pro Ser Ala Gln Gly Lys
            180                 185                 190

Pro Thr Tyr Phe Arg Glu Glu Glu Glu Glu Ile His Ser Pro Thr Leu
        195                 200                 205

Leu Pro Glu Ala Gln Asn Leu Val Ala Ala Leu Leu Ala Val Leu
    210                 215                 220

<210>   165
<211>   699
<212>   DNA
<213>   Artificial Sequence

<220>
<223>   NLS-JO-84 MTD-Lin28-JO-84 MTD cDNA Sequence

<400>   165
atgaagaaga agaggaagct ggtggcggcg ctgctggcgg tgctgggctc cgtgtccaac        60

cagcagtttg caggtggctg cgccaaggcg gcagaagagg cgcccgagga ggcgccggag       120

gacgcggccc gggcggcgga cgagcctcag ctgctgcacg gtgcgggcat ctgtaagtgg       180

ttcaacgtgc gcatggggtt cggcttcctg tccatgaccg cccgcgccgg ggtcgcgctc       240

gaccccccag tggatgtctt tgtgcaccag agtaagctgc acatggaagg gttccggagc       300

ttgaaggagg gtgaggcagt ggagttcacc tttaagaagt cagccaaggg tctggaatcc       360

atccgtgtca ccggacctgg tggagtattc tgtattggga gtgagaggcg gccaaaagga       420

aagagcatgc agaagcgcag atcaaaagga gacaggtgct acaactgtgg aggtctagat 480

catcatgcca aggaatgcaa gctgccaccc cagcccaaga agtgccactt ctgccagagc 540

atcagccata tggtagcctc atgtccgctg aaggcccagc agggccctag tgcacaggga 600

aagccaacct actttcgaga ggaagaagaa gaaatccaca gccctaccct gctcccggag 660

gcacagaatc tggtggcggc gctgctggcg gtgctgtga 699

<210> 166
<211> 232
<212> PRT
<213> Artificial Sequence

<220>
<223> NLS-JO-84 MTD-Lin28-JO-84 MTD Amino Acid Sequence

<400> 166
Met Lys Lys Lys Arg Lys Leu Val Ala Ala Leu Leu Ala Val Leu Gly
1               5                   10                  15

Ser Val Ser Asn Gln Gln Phe Ala Gly Gly Cys Ala Lys Ala Ala Glu
            20                  25                  30

Glu Ala Pro Glu Glu Ala Pro Glu Asp Ala Ala Arg Ala Ala Asp Glu
        35                  40                  45

Pro Gln Leu Leu His Gly Ala Gly Ile Cys Lys Trp Phe Asn Val Arg
    50                  55                  60

Met Gly Phe Gly Phe Leu Ser Met Thr Ala Arg Ala Gly Val Ala Leu
65                  70                  75                  80

Asp Pro Pro Val Asp Val Phe Val His Gln Ser Lys Leu His Met Glu
                85                  90                  95

Gly Phe Arg Ser Leu Lys Glu Gly Glu Ala Val Glu Phe Thr Phe Lys

100                 105                 110

Lys Ser Ala Lys Gly Leu Glu Ser Ile Arg Val Thr Gly Pro Gly Gly
        115                 120                 125

Val Phe Cys Ile Gly Ser Glu Arg Arg Pro Lys Gly Lys Ser Met Gln
    130                 135                 140

Lys Arg Arg Ser Lys Gly Asp Arg Cys Tyr Asn Cys Gly Gly Leu Asp
145                 150                 155                 160

His His Ala Lys Glu Cys Lys Leu Pro Pro Gln Pro Lys Lys Cys His
                165                 170                 175

Phe Cys Gln Ser Ile Ser His Met Val Ala Ser Cys Pro Leu Lys Ala
            180                 185                 190

Gln Gln Gly Pro Ser Ala Gln Gly Lys Pro Thr Tyr Phe Arg Glu Glu
        195                 200                 205

Glu Glu Glu Ile His Ser Pro Thr Leu Leu Pro Glu Ala Gln Asn Leu
    210                 215                 220

Val Ala Ala Leu Leu Ala Val Leu
225                 230

<210>   167
<211>   669
<212>   DNA
<213>   Artificial Sequence

<220>
<223>   NLS-JO-86 MTD-Lin28 cDNA Sequence

<400>   167
atgaagaaga agaggaagct ggcggtgctg gcggcggcgc cgggctccgt gtccaaccag          60

cagtttgcag gtggctgcgc caaggcggca gaagaggcgc ccgaggaggc gccggaggac         120

gcggcccggg cggcggacga gcctcagctg ctgcacggtg cgggcatctg taagtggttc 180

aacgtgcgca tggggttcgg cttcctgtcc atgaccgccc gcgccggggt cgcgctcgac 240

cccccagtgg atgtctttgt gcaccagagt aagctgcaca tggaagggtt ccggagcttg 300

aaggagggtg aggcagtgga gttcaccttt aagaagtcag ccaagggtct ggaatccatc 360

cgtgtcaccg gacctggtgg agtattctgt attgggagtg agaggcggcc aaaaggaaag 420

agcatgcaga agcgcagatc aaaaggagac aggtgctaca actgtggagg tctagatcat 480

catgccaagg aatgcaagct gccaccccag cccaagaagt gccacttctg ccagagcatc 540

agccatatgg tagcctcatg tccgctgaag gcccagcagg gccctagtgc acagggaaag 600

ccaacctact ttcgagagga agaagaagaa atccacagcc ctaccctgct cccggaggca 660

cagaattga 669

<210> 168
<211> 222
<212> PRT
<213> Artificial Sequence

<220>
<223> NLS-JO-86 MTD-Lin28 Amino Acid Sequence

<400> 168
Met Lys Lys Lys Arg Lys Leu Ala Val Leu Ala Ala Ala Pro Gly Ser
1               5                   10                  15

Val Ser Asn Gln Gln Phe Ala Gly Gly Cys Ala Lys Ala Ala Glu Glu
            20                  25                  30

Ala Pro Glu Glu Ala Pro Glu Asp Ala Ala Arg Ala Ala Asp Glu Pro
        35                  40                  45

Gln Leu Leu His Gly Ala Gly Ile Cys Lys Trp Phe Asn Val Arg Met
50                  55                  60

Gly Phe Gly Phe Leu Ser Met Thr Ala Arg Ala Gly Val Ala Leu Asp
65                  70                  75                  80

Pro Pro Val Asp Val Phe Val His Gln Ser Lys Leu His Met Glu Gly
                85                  90                  95

Phe Arg Ser Leu Lys Glu Gly Glu Ala Val Glu Phe Thr Phe Lys Lys
            100                 105                 110

Ser Ala Lys Gly Leu Glu Ser Ile Arg Val Thr Gly Pro Gly Gly Val
        115                 120                 125

Phe Cys Ile Gly Ser Glu Arg Arg Pro Lys Gly Lys Ser Met Gln Lys
    130                 135                 140

Arg Arg Ser Lys Gly Asp Arg Cys Tyr Asn Cys Gly Gly Leu Asp His
145                 150                 155                 160

His Ala Lys Glu Cys Lys Leu Pro Pro Gln Pro Lys Lys Cys His Phe
                165                 170                 175

Cys Gln Ser Ile Ser His Met Val Ala Ser Cys Pro Leu Lys Ala Gln
            180                 185                 190

Gln Gly Pro Ser Ala Gln Gly Lys Pro Thr Tyr Phe Arg Glu Glu Glu
        195                 200                 205

Glu Glu Ile His Ser Pro Thr Leu Leu Pro Glu Ala Gln Asn
    210                 215                 220

<210>   169
<211>   669
<212>   DNA

<213>    Artificial Sequence

<220>
<223>    NLS-Lin28-JO-86 MTD cDNA Sequence

<400>    169
atgaagaaga agaggaaggg ctccgtgtcc aaccagcagt ttgcaggtgg ctgcgccaag        60

gcggcagaag aggcgcccga ggaggcgccg gaggacgcgg cccgggcggc ggacgagcct       120

cagctgctgc acggtgcggg catctgtaag tggttcaacg tgcgcatggg gttcggcttc       180

ctgtccatga ccgcccgcgc cggggtcgcg ctcgaccccc cagtggatgt ctttgtgcac       240

cagagtaagc tgcacatgga agggttccgg agcttgaagg agggtgaggc agtggagttc       300

acctttaaga agtcagccaa gggtctggaa tccatccgtg tcaccggacc tggtggagta       360

ttctgtattg ggagtgagag gcggccaaaa ggaaagagca tgcagaagcg cagatcaaaa       420

ggagacaggt gctacaactg tggaggtcta gatcatcatg ccaaggaatg caagctgcca       480

ccccagccca agaagtgcca cttctgccag agcatcagcc atatggtagc ctcatgtccg       540

ctgaaggccc agcagggccc tagtgcacag ggaaagccaa cctactttcg agaggaagaa       600

gaagaaatcc acagccctac cctgctcccg gaggcacaga atctggcggt gctggcggcg       660

gcgccgtga                                                               669

<210>    170
<211>    222
<212>    PRT
<213>    Artificial Sequence

<220>
<223>    NLS-Lin28-JO-86 MTD Amino Acid Sequence

<400>    170

Met Lys Lys Lys Arg Lys Gly Ser Val Ser Asn Gln Gln Phe Ala Gly
1               5                   10                  15

Gly Cys Ala Lys Ala Ala Glu Glu Ala Pro Glu Glu Ala Pro Glu Asp
            20                  25                  30

Ala Ala Arg Ala Ala Asp Glu Pro Gln Leu Leu His Gly Ala Gly Ile
        35                  40                  45

Cys Lys Trp Phe Asn Val Arg Met Gly Phe Gly Phe Leu Ser Met Thr
    50                  55                  60

Ala Arg Ala Gly Val Ala Leu Asp Pro Pro Val Asp Val Phe Val His
65                  70                  75                  80

Gln Ser Lys Leu His Met Glu Gly Phe Arg Ser Leu Lys Glu Gly Glu
                85                  90                  95

Ala Val Glu Phe Thr Phe Lys Lys Ser Ala Lys Gly Leu Glu Ser Ile
            100                 105                 110

Arg Val Thr Gly Pro Gly Gly Val Phe Cys Ile Gly Ser Glu Arg Arg
        115                 120                 125

Pro Lys Gly Lys Ser Met Gln Lys Arg Arg Ser Lys Gly Asp Arg Cys
    130                 135                 140

Tyr Asn Cys Gly Gly Leu Asp His His Ala Lys Glu Cys Lys Leu Pro
145                 150                 155                 160

Pro Gln Pro Lys Lys Cys His Phe Cys Gln Ser Ile Ser His Met Val
                165                 170                 175

Ala Ser Cys Pro Leu Lys Ala Gln Gln Gly Pro Ser Ala Gln Gly Lys
            180                 185                 190

Pro Thr Tyr Phe Arg Glu Glu Glu Glu Glu Ile His Ser Pro Thr Leu
        195                 200                 205

Leu Pro Glu Ala Gln Asn Leu Ala Val Leu Ala Ala Ala Pro
    210                 215                 220

<210> 171
<211> 693
<212> DNA
<213> Artificial Sequence

<220>
<223> NLS-JO-86 MTD-Lin28-JO-86 MTD cDNA Sequence

<400> 171
atgaagaaga agaggaagct ggcggtgctg gcggcggcgc cgggctccgt gtccaaccag 60

cagtttgcag gtggctgcgc caaggcggca gaagaggcgc ccgaggaggc gccggaggac 120

gcggcccggg cggcggacga gcctcagctg ctgcacggtg cgggcatctg taagtggttc 180

aacgtgcgca tggggttcgg cttcctgtcc atgaccgccc gcgccggggt cgcgctcgac 240

cccccagtgg atgtctttgt gcaccagagt aagctgcaca tggaagggtt ccggagcttg 300

aaggagggtg aggcagtgga gttcaccttt aagaagtcag ccaagggtct ggaatccatc 360

cgtgtcaccg gacctggtgg agtattctgt attgggagtg agaggcggcc aaaaggaaag 420

agcatgcaga agcgcagatc aaaaggagac aggtgctaca actgtggagg tctagatcat 480

catgccaagg aatgcaagct gccaccccag cccaagaagt gccacttctg ccagagcatc 540

agccatatgg tagcctcatg tccgctgaag gcccagcagg gccctagtgc acagggaaag 600

ccaacctact ttcgagagga agaagaagaa atccacagcc ctaccctgct cccggaggca 660

cagaatctgg cggtgctggc ggcggcgccg tga 693

<210>    172
<211>    230
<212>    PRT
<213>    Artificial Sequence

<220>
<223>    NLS-JO-86 MTD-Lin28-JO-86 MTD Amino Acid Sequence

<400>    172
Met Lys Lys Lys Arg Lys Leu Ala Val Leu Ala Ala Ala Pro Gly Ser
1               5                   10                  15

Val Ser Asn Gln Gln Phe Ala Gly Gly Cys Ala Lys Ala Ala Glu Glu
            20                  25                  30

Ala Pro Glu Glu Ala Pro Glu Asp Ala Ala Arg Ala Ala Asp Glu Pro
        35                  40                  45

Gln Leu Leu His Gly Ala Gly Ile Cys Lys Trp Phe Asn Val Arg Met
    50                  55                  60

Gly Phe Gly Phe Leu Ser Met Thr Ala Arg Ala Gly Val Ala Leu Asp
65                  70                  75                  80

Pro Pro Val Asp Val Phe Val His Gln Ser Lys Leu His Met Glu Gly
                85                  90                  95

Phe Arg Ser Leu Lys Glu Gly Glu Ala Val Glu Phe Thr Phe Lys Lys
            100                 105                 110

Ser Ala Lys Gly Leu Glu Ser Ile Arg Val Thr Gly Pro Gly Gly Val
        115                 120                 125

Phe Cys Ile Gly Ser Glu Arg Arg Pro Lys Gly Lys Ser Met Gln Lys
    130                 135                 140

Arg Arg Ser Lys Gly Asp Arg Cys Tyr Asn Cys Gly Gly Leu Asp His

145                 150                 155                 160

His Ala Lys Glu Cys Lys Leu Pro Pro Gln Pro Lys Lys Cys His Phe
            165                 170                 175

Cys Gln Ser Ile Ser His Met Val Ala Ser Cys Pro Leu Lys Ala Gln
        180                 185                 190

Gln Gly Pro Ser Ala Gln Gly Lys Pro Thr Tyr Phe Arg Glu Glu Glu
    195                 200                 205

Glu Glu Ile His Ser Pro Thr Leu Leu Pro Glu Ala Gln Asn Leu Ala
    210                 215                 220

Val Leu Ala Ala Ala Pro
225                 230

<210>    173
<211>    705
<212>    DNA
<213>    Artificial Sequence

<220>
<223>    His-NLS-Lin28 cDNA Sequence

<400>    173
atgggcagca gccatcatca tcatcatcac agcagcggcc tggtgccgcg cggcagccat        60

atgaagaaga agaggaaggg ctccgtgtcc aaccagcagt ttgcaggtgg ctgcgccaag       120

gcggcagaag aggcgcccga ggaggcgccg gaggacgcgg cccgggcggc ggacgagcct       180

cagctgctgc acggtgcggg catctgtaag tggttcaacg tgcgcatggg gttcggcttc       240

ctgtccatga ccgcccgcgc cggggtcgcg ctcgaccccc cagtggatgt ctttgtgcac       300

cagagtaagc tgcacatgga agggttccgg agcttgaagg agggtgaggc agtggagttc       360

acctttaaga agtcagccaa gggtctggaa tccatccgtg tcaccggacc tggtggagta 420

ttctgtattg ggagtgagag gcggccaaaa ggaaagagca tgcagaagcg cagatcaaaa 480

ggagacaggt gctacaactg tggaggtcta gatcatcatg ccaaggaatg caagctgcca 540

ccccagccca agaagtgcca cttctgccag agcatcagcc atatggtagc ctcatgtccg 600

ctgaaggccc agcagggccc tagtgcacag ggaaagccaa cctactttcg agaggaagaa 660

gaagaaatcc acagccctac cctgctcccg gaggcacaga attga 705

<210> 174
<211> 234
<212> PRT
<213> Artificial Sequence

<220>
<223> His-NLS-Lin28 Amino Acid Sequence

<400> 174
Met Gly Ser Ser His His His His His His Ser Ser Gly Leu Val Pro
1               5                   10                  15

Arg Gly Ser His Met Lys Lys Lys Arg Lys Gly Ser Val Ser Asn Gln
            20                  25                  30

Gln Phe Ala Gly Gly Cys Ala Lys Ala Ala Glu Glu Ala Pro Glu Glu
        35                  40                  45

Ala Pro Glu Asp Ala Ala Arg Ala Ala Asp Glu Pro Gln Leu Leu His
    50                  55                  60

Gly Ala Gly Ile Cys Lys Trp Phe Asn Val Arg Met Gly Phe Gly Phe
65                  70                  75                  80

Leu Ser Met Thr Ala Arg Ala Gly Val Ala Leu Asp Pro Pro Val Asp
                85                  90                  95

Val Phe Val His Gln Ser Lys Leu His Met Glu Gly Phe Arg Ser Leu
            100                 105                 110

Lys Glu Gly Glu Ala Val Glu Phe Thr Phe Lys Lys Ser Ala Lys Gly
        115                 120                 125

Leu Glu Ser Ile Arg Val Thr Gly Pro Gly Gly Val Phe Cys Ile Gly
    130                 135                 140

Ser Glu Arg Arg Pro Lys Gly Lys Ser Met Gln Lys Arg Arg Ser Lys
145                 150                 155                 160

Gly Asp Arg Cys Tyr Asn Cys Gly Gly Leu Asp His His Ala Lys Glu
                165                 170                 175

Cys Lys Leu Pro Pro Gln Pro Lys Lys Cys His Phe Cys Gln Ser Ile
            180                 185                 190

Ser His Met Val Ala Ser Cys Pro Leu Lys Ala Gln Gln Gly Pro Ser
        195                 200                 205

Ala Gln Gly Lys Pro Thr Tyr Phe Arg Glu Glu Glu Glu Glu Ile His
    210                 215                 220

Ser Pro Thr Leu Leu Pro Glu Ala Gln Asn
225                 230

<210>   175
<211>   732
<212>   DNA
<213>   Artificial Sequence

<220>
<223>   His-NLS-JO-84 MTD-Lin28 cDNA Sequence

<400>   175
atgggcagca gccatcatca tcatcatcac agcagcggcc tggtgccgcg cggcagccat        60

atgaagaaga agaggaagct ggtggcggcg ctgctggcgg tgctgggctc cgtgtccaac 120

cagcagtttg caggtggctg cgccaaggcg gcagaagagg cgcccgagga ggcgccggag 180

gacgcggccc gggcggcgga cgagcctcag ctgctgcacg gtgcgggcat ctgtaagtgg 240

ttcaacgtgc gcatggggtt cggcttcctg tccatgaccg cccgcgccgg ggtcgcgctc 300

gaccccccag tggatgtctt tgtgcaccag agtaagctgc acatggaagg gttccggagc 360

ttgaaggagg gtgaggcagt ggagttcacc tttaagaagt cagccaaggg tctggaatcc 420

atccgtgtca ccggacctgg tggagtattc tgtattggga gtgagaggcg gccaaaagga 480

aagagcatgc agaagcgcag atcaaaagga gacaggtgct acaactgtgg aggtctagat 540

catcatgcca aggaatgcaa gctgccaccc cagcccaaga agtgccactt ctgccagagc 600

atcagccata tggtagcctc atgtccgctg aaggcccagc agggccctag tgcacaggga 660

aagccaacct actttcgaga ggaagaagaa gaaatccaca gccctaccct gctcccggag 720

gcacagaatt ga 732

<210> 176
<211> 243
<212> PRT
<213> Artificial Sequence

<220>
<223> His-NLS-JO-84 MTD-Lin28 Amino Acid Sequence

<400> 176
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu

20                  25                  30

Ala Val Leu Gly Ser Val Ser Asn Gln Gln Phe Ala Gly Gly Cys Ala
        35                  40                  45

Lys Ala Ala Glu Glu Ala Pro Glu Glu Ala Pro Glu Asp Ala Ala Arg
    50                  55                  60

Ala Ala Asp Glu Pro Gln Leu Leu His Gly Ala Gly Ile Cys Lys Trp
65                  70                  75                  80

Phe Asn Val Arg Met Gly Phe Gly Phe Leu Ser Met Thr Ala Arg Ala
                85                  90                  95

Gly Val Ala Leu Asp Pro Pro Val Asp Val Phe Val His Gln Ser Lys
            100                 105                 110

Leu His Met Glu Gly Phe Arg Ser Leu Lys Glu Gly Glu Ala Val Glu
        115                 120                 125

Phe Thr Phe Lys Lys Ser Ala Lys Gly Leu Glu Ser Ile Arg Val Thr
    130                 135                 140

Gly Pro Gly Gly Val Phe Cys Ile Gly Ser Glu Arg Arg Pro Lys Gly
145                 150                 155                 160

Lys Ser Met Gln Lys Arg Arg Ser Lys Gly Asp Arg Cys Tyr Asn Cys
                165                 170                 175

Gly Gly Leu Asp His His Ala Lys Glu Cys Lys Leu Pro Pro Gln Pro
            180                 185                 190

Lys Lys Cys His Phe Cys Gln Ser Ile Ser His Met Val Ala Ser Cys
        195                 200                 205

Pro Leu Lys Ala Gln Gln Gly Pro Ser Ala Gln Gly Lys Pro Thr Tyr
    210                 215                 220

Phe Arg Glu Glu Glu Glu Glu Ile His Ser Pro Thr Leu Leu Pro Glu
225                 230                 235                 240

Ala Gln Asn

<210> 177
<211> 732
<212> DNA
<213> Artificial Sequence

<220>
<223> His-NLS-Lin28-JO-84 MTD cDNA Sequence

<400> 177
atgggcagca gccatcatca tcatcatcac agcagcggcc tggtgccgcg cggcagccat 60

atgaagaaga agaggaaggg ctccgtgtcc aaccagcagt ttgcaggtgg ctgcgccaag 120

gcggcagaag aggcgcccga ggaggcgccg gaggacgcgg cccgggcggc ggacgagcct 180

cagctgctgc acggtgcggg catctgtaag tggttcaacg tgcgcatggg gttcggcttc 240

ctgtccatga ccgcccgcgc cggggtcgcg ctcgaccccc cagtggatgt ctttgtgcac 300

cagagtaagc tgcacatgga agggttccgg agcttgaagg agggtgaggc agtggagttc 360

acctttaaga agtcagccaa gggtctggaa tccatccgtg tcaccggacc tggtggagta 420

ttctgtattg ggagtgagag gcggccaaaa ggaaagagca tgcagaagcg cagatcaaaa 480

ggagacaggt gctacaactg tggaggtcta gatcatcatg ccaaggaatg caagctgcca 540

ccccagccca agaagtgcca cttctgccag agcatcagcc atatggtagc ctcatgtccg 600

ctgaaggccc agcagggccc tagtgcacag ggaaagccaa cctactttcg agaggaagaa 660

gaagaaatcc acagccctac cctgctcccg gaggcacaga atctggtggc ggcgctgctg 720

gcggtgctgt ga 732

<210>    178
<211>    243
<212>    PRT
<213>    Artificial Sequence

<220>
<223>    His-NLS-Lin28-JO-84 MTD Amino Acid Sequence

<400>    178
Met Gly Ser Ser His His His His His His Ser Ser Gly Leu Val Pro
1               5                   10                  15

Arg Gly Ser His Met Lys Lys Lys Arg Lys Gly Ser Val Ser Asn Gln
            20                  25                  30

Gln Phe Ala Gly Gly Cys Ala Lys Ala Ala Glu Glu Ala Pro Glu Glu
        35                  40                  45

Ala Pro Glu Asp Ala Ala Arg Ala Ala Asp Glu Pro Gln Leu Leu His
    50                  55                  60

Gly Ala Gly Ile Cys Lys Trp Phe Asn Val Arg Met Gly Phe Gly Phe
65                  70                  75                  80

Leu Ser Met Thr Ala Arg Ala Gly Val Ala Leu Asp Pro Pro Val Asp
                85                  90                  95

Val Phe Val His Gln Ser Lys Leu His Met Glu Gly Phe Arg Ser Leu
            100                 105                 110

Lys Glu Gly Glu Ala Val Glu Phe Thr Phe Lys Lys Ser Ala Lys Gly
        115                 120                 125

Leu Glu Ser Ile Arg Val Thr Gly Pro Gly Gly Val Phe Cys Ile Gly
    130                 135                 140

Ser Glu Arg Arg Pro Lys Gly Lys Ser Met Gln Lys Arg Arg Ser Lys

145                 150                 155                 160

Gly Asp Arg Cys Tyr Asn Cys Gly Gly Leu Asp His His Ala Lys Glu
                165                 170                 175

Cys Lys Leu Pro Pro Gln Pro Lys Lys Cys His Phe Cys Gln Ser Ile
            180                 185                 190

Ser His Met Val Ala Ser Cys Pro Leu Lys Ala Gln Gln Gly Pro Ser
        195                 200                 205

Ala Gln Gly Lys Pro Thr Tyr Phe Arg Glu Glu Glu Glu Glu Ile His
    210                 215                 220

Ser Pro Thr Leu Leu Pro Glu Ala Gln Asn Leu Val Ala Ala Leu Leu
225                 230                 235                 240

Ala Val Leu

<210>   179
<211>   759
<212>   DNA
<213>   Artificial Sequence

<220>
<223>   His-NLS-JO-84 MTD-Lin28-JO-84 MTD cDNA Sequence

<400>   179
atgggcagca gccatcatca tcatcatcac agcagcggcc tggtgccgcg cggcagccat      60

atgaagaaga agaggaagct ggtggcggcg ctgctggcgg tgctgggctc cgtgtccaac     120

cagcagtttg caggtggctg cgccaaggcg gcagaagagg cgcccgagga ggcgccggag     180

gacgcggccc gggcggcgga cgagcctcag ctgctgcacg gtgcgggcat ctgtaagtgg     240

ttcaacgtgc gcatggggtt cggcttcctg tccatgaccg cccgcgccgg ggtcgcgctc     300

gaccccccag tggatgtctt tgtgcaccag agtaagctgc acatggaagg gttccggagc 360

ttgaaggagg gtgaggcagt ggagttcacc tttaagaagt cagccaaggg tctggaatcc 420

atccgtgtca ccggacctgg tggagtattc tgtattggga gtgagaggcg gccaaaagga 480

aagagcatgc agaagcgcag atcaaaagga gacaggtgct acaactgtgg aggtctagat 540

catcatgcca aggaatgcaa gctgccaccc cagcccaaga agtgccactt ctgccagagc 600

atcagccata tggtagcctc atgtccgctg aaggcccagc agggccctag tgcacaggga 660

aagccaacct actttcgaga ggaagaagaa gaaatccaca gccctaccct gctcccggag 720

gcacagaatc tggtggcggc gctgctggcg gtgctgtga 759

<210> 180
<211> 252
<212> PRT
<213> Artificial Sequence

<220>
<223> His-NLS-JO-84 MTD-Lin28-JO-84 MTD Amino Acid Sequence

<400> 180
Met Gly Ser Ser His His His His His His Ser Ser Gly Leu Val Pro
1               5                   10                  15

Arg Gly Ser His Met Lys Lys Lys Arg Lys Leu Val Ala Ala Leu Leu
            20                  25                  30

Ala Val Leu Gly Ser Val Ser Asn Gln Gln Phe Ala Gly Gly Cys Ala
        35                  40                  45

Lys Ala Ala Glu Glu Ala Pro Glu Glu Ala Pro Glu Asp Ala Ala Arg
    50                  55                  60

Ala Ala Asp Glu Pro Gln Leu Leu His Gly Ala Gly Ile Cys Lys Trp
65                  70                  75                  80

Phe Asn Val Arg Met Gly Phe Gly Phe Leu Ser Met Thr Ala Arg Ala
                85                  90                  95

Gly Val Ala Leu Asp Pro Pro Val Asp Val Phe Val His Gln Ser Lys
            100                 105                 110

Leu His Met Glu Gly Phe Arg Ser Leu Lys Glu Gly Glu Ala Val Glu
        115                 120                 125

Phe Thr Phe Lys Lys Ser Ala Lys Gly Leu Glu Ser Ile Arg Val Thr
    130                 135                 140

Gly Pro Gly Gly Val Phe Cys Ile Gly Ser Glu Arg Arg Pro Lys Gly
145                 150                 155                 160

Lys Ser Met Gln Lys Arg Arg Ser Lys Gly Asp Arg Cys Tyr Asn Cys
                165                 170                 175

Gly Gly Leu Asp His His Ala Lys Glu Cys Lys Leu Pro Pro Gln Pro
            180                 185                 190

Lys Lys Cys His Phe Cys Gln Ser Ile Ser His Met Val Ala Ser Cys
        195                 200                 205

Pro Leu Lys Ala Gln Gln Gly Pro Ser Ala Gln Gly Lys Pro Thr Tyr
    210                 215                 220

Phe Arg Glu Glu Glu Glu Glu Ile His Ser Pro Thr Leu Leu Pro Glu
225                 230                 235                 240

Ala Gln Asn Leu Val Ala Ala Leu Leu Ala Val Leu
                245                 250

<210>   181
<211>   729
<212>   DNA

<213>    Artificial Sequence

<220>
<223>    His-NLS-JO-86 MTD-Lin28 cDNA Sequence

<400>    181
atgggcagca gccatcatca tcatcatcac agcagcggcc tggtgccgcg cggcagccat      60

atgaagaaga agaggaagct ggcggtgctg gcggcggcgc cgggctccgt gtccaaccag      120

cagtttgcag gtggctgcgc caaggcggca gaagaggcgc ccgaggaggc gccggaggac      180

gcggcccggg cggcggacga gcctcagctg ctgcacggtg cgggcatctg taagtggttc      240

aacgtgcgca tggggttcgg cttcctgtcc atgaccgccc gcgccggggt cgcgctcgac      300

cccccagtgg atgtctttgt gcaccagagt aagctgcaca tggaagggtt ccggagcttg      360

aaggagggtg aggcagtgga gttcaccttt aagaagtcag ccaagggtct ggaatccatc      420

cgtgtcaccg gacctggtgg agtattctgt attgggagtg agaggcggcc aaaaggaaag      480

agcatgcaga agcgcagatc aaaaggagac aggtgctaca actgtggagg tctagatcat      540

catgccaagg aatgcaagct gccaccccag cccaagaagt gccacttctg ccagagcatc      600

agccatatgg tagcctcatg tccgctgaag gcccagcagg gccctagtgc acagggaaag      660

ccaacctact ttcgagagga agaagaagaa atccacagcc ctaccctgct cccggaggca      720

cagaattga                                                              729

<210>    182
<211>    242
<212>    PRT
<213>    Artificial Sequence

<220>

<223>    His-NLS-JO-86 MTD-Lin28 Amino Acid Sequence

<400>    182
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala
            20                  25                  30

Ala Pro Gly Ser Val Ser Asn Gln Gln Phe Ala Gly Gly Cys Ala Lys
        35                  40                  45

Ala Ala Glu Glu Ala Pro Glu Glu Ala Pro Glu Asp Ala Ala Arg Ala
    50                  55                  60

Ala Asp Glu Pro Gln Leu Leu His Gly Ala Gly Ile Cys Lys Trp Phe
65                  70                  75                  80

Asn Val Arg Met Gly Phe Gly Phe Leu Ser Met Thr Ala Arg Ala Gly
                85                  90                  95

Val Ala Leu Asp Pro Pro Val Asp Val Phe Val His Gln Ser Lys Leu
            100                 105                 110

His Met Glu Gly Phe Arg Ser Leu Lys Glu Gly Glu Ala Val Glu Phe
        115                 120                 125

Thr Phe Lys Lys Ser Ala Lys Gly Leu Glu Ser Ile Arg Val Thr Gly
    130                 135                 140

Pro Gly Gly Val Phe Cys Ile Gly Ser Glu Arg Arg Pro Lys Gly Lys
145                 150                 155                 160

Ser Met Gln Lys Arg Arg Ser Lys Gly Asp Arg Cys Tyr Asn Cys Gly
                165                 170                 175

Gly Leu Asp His His Ala Lys Glu Cys Lys Leu Pro Pro Gln Pro Lys
            180                 185                 190

Lys Cys His Phe Cys Gln Ser Ile Ser His Met Val Ala Ser Cys Pro
        195                 200                 205

Leu Lys Ala Gln Gln Gly Pro Ser Ala Gln Gly Lys Pro Thr Tyr Phe
    210                 215                 220

Arg Glu Glu Glu Glu Glu Ile His Ser Pro Thr Leu Leu Pro Glu Ala
225                 230                 235                 240

Gln Asn

<210>    183
<211>    729
<212>    DNA
<213>    Artificial Sequence

<220>
<223>    His-NLS-Lin28-JO-86 MTD cDNA Sequence

<400>    183
atgggcagca gccatcatca tcatcatcac agcagcggcc tggtgccgcg cggcagccat      60

atgaagaaga agaggaaggg ctccgtgtcc aaccagcagt ttgcaggtgg ctgcgccaag     120

gcggcagaag aggcgcccga ggaggcgccg gaggacgcgg cccgggcggc ggacgagcct     180

cagctgctgc acggtgcggg catctgtaag tggttcaacg tgcgcatggg gttcggcttc     240

ctgtccatga ccgcccgcgc cggggtcgcg ctcgaccccc cagtggatgt ctttgtgcac     300

cagagtaagc tgcacatgga agggttccgg agcttgaagg agggtgaggc agtggagttc     360

acctttaaga agtcagccaa gggtctggaa tccatccgtg tcaccggacc tggtggagta     420

ttctgtattg ggagtgagag gcggccaaaa ggaaagagca tgcagaagcg cagatcaaaa     480

ggagacaggt gctacaactg tggaggtcta gatcatcatg ccaaggaatg caagctgcca     540

ccccagccca agaagtgcca cttctgccag agcatcagcc atatggtagc ctcatgtccg 600

ctgaaggccc agcagggccc tagtgcacag ggaaagccaa cctactttcg agaggaagaa 660

gaagaaatcc acagccctac cctgctcccg gaggcacaga atctggcggt gctggcggcg 720

gcgccgtga 729

<210> 184
<211> 242
<212> PRT
<213> Artificial Sequence

<220>
<223> His-NLS-Lin28-JO-86 MTD Amino Acid Sequence

<400> 184
Met Gly Ser Ser His His His His His His Ser Ser Gly Leu Val Pro
1               5                   10                  15

Arg Gly Ser His Met Lys Lys Lys Arg Lys Gly Ser Val Ser Asn Gln
            20                  25                  30

Gln Phe Ala Gly Gly Cys Ala Lys Ala Ala Glu Glu Ala Pro Glu Glu
        35                  40                  45

Ala Pro Glu Asp Ala Ala Arg Ala Ala Asp Glu Pro Gln Leu Leu His
    50                  55                  60

Gly Ala Gly Ile Cys Lys Trp Phe Asn Val Arg Met Gly Phe Gly Phe
65                  70                  75                  80

Leu Ser Met Thr Ala Arg Ala Gly Val Ala Leu Asp Pro Pro Val Asp
                85                  90                  95

Val Phe Val His Gln Ser Lys Leu His Met Glu Gly Phe Arg Ser Leu
            100                 105                 110

Lys Glu Gly Glu Ala Val Glu Phe Thr Phe Lys Lys Ser Ala Lys Gly
        115                 120                 125

Leu Glu Ser Ile Arg Val Thr Gly Pro Gly Gly Val Phe Cys Ile Gly
    130                 135                 140

Ser Glu Arg Arg Pro Lys Gly Lys Ser Met Gln Lys Arg Arg Ser Lys
145                 150                 155                 160

Gly Asp Arg Cys Tyr Asn Cys Gly Gly Leu Asp His His Ala Lys Glu
                165                 170                 175

Cys Lys Leu Pro Pro Gln Pro Lys Lys Cys His Phe Cys Gln Ser Ile
            180                 185                 190

Ser His Met Val Ala Ser Cys Pro Leu Lys Ala Gln Gln Gly Pro Ser
        195                 200                 205

Ala Gln Gly Lys Pro Thr Tyr Phe Arg Glu Glu Glu Glu Glu Ile His
    210                 215                 220

Ser Pro Thr Leu Leu Pro Glu Ala Gln Asn Leu Ala Val Leu Ala Ala
225                 230                 235                 240

Ala Pro

<210>   185
<211>   753
<212>   DNA
<213>   Artificial Sequence

<220>
<223>   His-NLS-JO-86 MTD-Lin28-JO-86 MTD cDNA Sequence

<400>   185
atgggcagca gccatcatca tcatcatcac agcagcggcc tggtgccgcg cggcagccat        60

atgaagaaga agaggaagct ggcggtgctg gcggcggcgc cgggctccgt gtccaaccag 120

cagtttgcag gtggctgcgc caaggcggca gaagaggcgc ccgaggaggc gccggaggac 180

gcggcccggg cggcggacga gcctcagctg ctgcacggtg cgggcatctg taagtggttc 240

aacgtgcgca tggggttcgg cttcctgtcc atgaccgccc gcgccggggt cgcgctcgac 300

cccccagtgg atgtctttgt gcaccagagt aagctgcaca tggaagggtt ccggagcttg 360

aaggagggtg aggcagtgga gttcaccttt aagaagtcag ccaagggtct ggaatccatc 420

cgtgtcaccg gacctggtgg agtattctgt attgggagtg agaggcggcc aaaaggaaag 480

agcatgcaga agcgcagatc aaaaggagac aggtgctaca actgtggagg tctagatcat 540

catgccaagg aatgcaagct gccaccccag cccaagaagt gccacttctg ccagagcatc 600

agccatatgg tagcctcatg tccgctgaag gcccagcagg gccctagtgc acagggaaag 660

ccaacctact ttcgagagga agaagaagaa atccacagcc ctaccctgct cccggaggca 720

cagaatctgg cggtgctggc ggcggcgccg tga 753

<210> 186
<211> 250
<212> PRT
<213> Artificial Sequence

<220>
<223> His-NLS-JO-86 MTD-Lin28-JO-86 MTD Amino Acid Sequence

<400> 186
Met Gly Ser Ser His His His His His His Ser Ser Gly Leu Val Pro
1               5                   10                  15

Arg Gly Ser His Met Lys Lys Lys Arg Lys Leu Ala Val Leu Ala Ala

20                  25                  30

Ala Pro Gly Ser Val Ser Asn Gln Gln Phe Ala Gly Gly Cys Ala Lys
        35                  40                  45

Ala Ala Glu Glu Ala Pro Glu Glu Ala Pro Glu Asp Ala Ala Arg Ala
    50                  55                  60

Ala Asp Glu Pro Gln Leu Leu His Gly Ala Gly Ile Cys Lys Trp Phe
65                  70                  75                  80

Asn Val Arg Met Gly Phe Gly Phe Leu Ser Met Thr Ala Arg Ala Gly
                85                  90                  95

Val Ala Leu Asp Pro Pro Val Asp Val Phe Val His Gln Ser Lys Leu
            100                 105                 110

His Met Glu Gly Phe Arg Ser Leu Lys Glu Gly Glu Ala Val Glu Phe
        115                 120                 125

Thr Phe Lys Lys Ser Ala Lys Gly Leu Glu Ser Ile Arg Val Thr Gly
    130                 135                 140

Pro Gly Gly Val Phe Cys Ile Gly Ser Glu Arg Arg Pro Lys Gly Lys
145                 150                 155                 160

Ser Met Gln Lys Arg Arg Ser Lys Gly Asp Arg Cys Tyr Asn Cys Gly
                165                 170                 175

Gly Leu Asp His His Ala Lys Glu Cys Lys Leu Pro Pro Gln Pro Lys
            180                 185                 190

Lys Cys His Phe Cys Gln Ser Ile Ser His Met Val Ala Ser Cys Pro
        195                 200                 205

Leu Lys Ala Gln Gln Gly Pro Ser Ala Gln Gly Lys Pro Thr Tyr Phe
    210                 215                 220

Arg Glu Glu Glu Glu Glu Ile His Ser Pro Thr Leu Leu Pro Glu Ala
225                 230                 235                 240

Gln Asn Leu Ala Val Leu Ala Ala Ala Pro
                245                 250

<210>   187
<211>   45
<212>   DNA
<213>   Artificial Sequence

<220>
<223>   HhN-5'

<400>   187
ccgcatatga agaagaagag gaagagtgtg gatccagctt gtccc                       45

<210>   188
<211>   36
<212>   DNA
<213>   Artificial Sequence

<220>
<223>   HhN-3'

<400>   188
ccgcatatgt cacacgtctt caggttgcat gttcat                                 36

<210>   189
<211>   75
<212>   DNA
<213>   Artificial Sequence

<220>
<223>   HM1hN-5'

<400>    189
ccgcatatga agaagaagag gaagctggtg gcggcgctgc tggcggtgct gagtgtggat    60

ccagcttgtc cccaa    75

<210>    190
<211>    63
<212>    DNA
<213>    Artificial Sequence

<220>
<223>    HhNM1-3'

<400>    190
ccgcatatgt cacagcaccg ccagcagcgc cgccaccagc acgtcttcag gttgcatgtt    60

cat    63

<210>    191
<211>    72
<212>    DNA
<213>    Artificial Sequence

<220>
<223>    HM2hN-5'

<400>    191
ccgcatatga agaagaagag gaagctggcg gtgctggcgg cggcgccgag tgtggatcca    60

gcttgtcccc aa    72

<210>    192
<211>    60
<212>    DNA
<213>    Artificial Sequence

<220>
<223>    HhNM2-3'

<400>    192
ccgcatatgt cacggcgccg ccgccagcac cgccagcacg tcttcaggtt gcatgttcat          60

                                                                            60

<210>    193
<211>    45
<212>    DNA
<213>    Artificial Sequence

<220>
<223>    HhO-5'

<400>    193
ccgcatatga agaagaagag gaaggcggga cacctggctt cggat                          45

<210>    194
<211>    36
<212>    DNA
<213>    Artificial Sequence

<220>
<223>    HhO-3'

<400>    194
ccgcatatgt cagtttgaat gcatgggaga gcccag                                    36

<210>    195
<211>    75
<212>    DNA

<213>    Artificial Sequence

<220>
<223>    HM1hO-5'

<400>    195
ccgcatatga agaagaagag gaagctggtg gcggcgctgc tggcggtgct ggcgggacac    60

ctggcttcgg atttc                                                      75

<210>    196
<211>    63
<212>    DNA
<213>    Artificial Sequence

<220>
<223>    HhOM1-3'

<400>    196
ccgcatatgt cacagcaccg ccagcagcgc cgccaccagg tttgaatgca tgggagagcc    60

cag                                                                   63

<210>    197
<211>    72
<212>    DNA
<213>    Artificial Sequence

<220>
<223>    HM2hO-5'

<400>    197
ccgcatatga agaagaagag gaagctggcg gtgctggcgg cggcgccggc gggacacctg    60

gcttcggatt tc                                                         72

<210>    198
<211>    60
<212>    DNA
<213>    Artificial Sequence

<220>
<223>    HhOM2-3'

<400>    198
ccgcatatgt cacggcgccg ccgccagcac cgccaggttt gaatgcatgg gagagcccag          60

                                                                           60

<210>    199
<211>    45
<212>    DNA
<213>    Artificial Sequence

<220>
<223>    HhS-5'

<400>    199
ccgcatatga agaagaagag gaagtacaac atgatggaga cggag                          45

<210>    200
<211>    36
<212>    DNA
<213>    Artificial Sequence

<220>
<223>    HhS-3'

<400>    200

ccgcatatgt cacatgtgtg agaggggcag tgtgcc 36

<210> 201
<211> 75
<212> DNA
<213> Artificial Sequence

<220>
<223> HM1hS-5'

<400> 201
ccgcatatga agaagaagag gaagctggtg gcggcgctgc tggcggtgct gtacaacatg 60

atggagacgg agctg 75

<210> 202
<211> 63
<212> DNA
<213> Artificial Sequence

<220>
<223> HhSM1-3'

<400> 202
ccgcatatgt cacagcaccg ccagcagcgc cgccaccagc atgtgtgaga ggggcagtgt 60

gcc 63

<210> 203
<211> 72
<212> DNA
<213> Artificial Sequence

<220>
<223> HM2hS-5'

<400> 203
ccgcatatga agaagaagag gaagctggcg gtgctggcgg cggcgccgta caacatgatg 60

gagacggagc tg 72

<210> 204
<211> 60
<212> DNA
<213> Artificial Sequence

<220>
<223> HhSM2-3'

<400> 204
ccgcatatgt cacggcgccg ccgccagcac cgccagcatg tgtgagaggg gcagtgtgcc 60

60

<210> 205
<211> 45
<212> DNA
<213> Artificial Sequence

<220>
<223> HhK-5'

<400> 205
ccgcatatga agaagaagag gaaggctgtc agcgacgcgc tgctc 45

<210> 206
<211> 36
<212> DNA
<213> Artificial Sequence

<220>

<223>    HhK-3'

<400>    206
ccgcatatgt taaaaatgcc tcttcatgtg taaggc                                36

<210>    207
<211>    75
<212>    DNA
<213>    Artificial Sequence

<220>

<223>    HM1hK-5'

<400>    207
ccgcatatga agaagaagag gaagctggtg gcggcgctgc tggcggtgct ggctgtcagc      60

gacgcgctgc tccca                                                       75

<210>    208
<211>    63
<212>    DNA
<213>    Artificial Sequence

<220>

<223>    HhKM1-3'

<400>    208
ccgcatatgt tacagcaccg ccagcagcgc cgccaccaga aaatgcctct tcatgtgtaa      60

ggc                                                                    63

<210>    209

<211>    72
<212>    DNA
<213>    Artificial Sequence

<220>
<223>    HM2hK-5'

<400>    209
ccgcatatga agaagaagag gaagctggcg gtgctggcgg cggcgccggc tgtcagcgac        60

gcgctgctcc ca                                                            72

<210>    210
<211>    60
<212>    DNA
<213>    Artificial Sequence

<220>
<223>    HhKM2-3'

<400>    210
ccgcatatgt tacggcgccg ccgccagcac cgccagaaaa tgcctcttca tgtgtaaggc        60

                                                                         60

<210>    211
<211>    45
<212>    DNA
<213>    Artificial Sequence

<220>
<223>    HhM-5'

<400>    211
ccgcatatga agaagaagag gaaggatttt tttcgggtag tggaa                        45

<210> 212
<211> 36
<212> DNA
<213> Artificial Sequence

<220>
<223> HhM-3'

<400> 212
ccgcatatgt tacgcacaag agttccgtag ctgttc 36

<210> 213
<211> 75
<212> DNA
<213> Artificial Sequence

<220>
<223> HM1hM-5'

<400> 213
ccgcatatga agaagaagag gaagctggtg gcggcgctgc tggcggtgct ggattttttt 60

cgggtagtgg aaaac 75

<210> 214
<211> 63
<212> DNA
<213> Artificial Sequence

<220>
<223> HhMM1-3'

<400> 214

ccgcatatgt tacagcaccg ccagcagcgc cgccaccagc gcacaagagt tccgtagctg 60

ttc 63

<210> 215
<211> 72
<212> DNA
<213> Artificial Sequence

<220>
<223> HM2hM-5'

<400> 215
ccgcatatga agaagaagag gaagctggcg gtgctggcgg cggcgccgga tttttttcgg 60

gtagtggaaa ac 72

<210> 216
<211> 60
<212> DNA
<213> Artificial Sequence

<220>
<223> HhMM2-3'

<400> 216
ccgcatatgt tacggcgccg ccgccagcac cgccagcgca caagagttcc gtagctgttc 60

60

<210> 217
<211> 45
<212> DNA
<213> Artificial Sequence

<220>
<223>    HhL-5'

<400>    217
ccgggatcca agaagaagag gaagggctcc gtgtccaacc agcag                45

<210>    218
<211>    36
<212>    DNA
<213>    Artificial Sequence

<220>
<223>    HhL-3'

<400>    218
ccgggatcct caattctgag cctccgggag cagggt                          36

<210>    219
<211>    75
<212>    DNA
<213>    Artificial Sequence

<220>
<223>    HM1hL-5'

<400>    219
ccgggatcca agaagaagag gaagctggtg gcggcgctgc tggcggtgct gggctccgtg    60

tccaaccagc agttt                                                 75

<210>    220
<211>    63
<212>    DNA
<213>    Artificial Sequence

<220>
<223>    HhLM1-3'

<400>    220
ccgggatcct tacagcaccg ccagcagcgc cgccaccagt caattctgag cctccgggag    60

cag                                                                  63

<210>    221
<211>    72
<212>    DNA
<213>    Artificial Sequence

<220>
<223>    HM2hL-5'

<400>    221
ccgggatcca agaagaagag gaagctggcg gtgctggcgg cggcgccggg ctccgtgtcc    60

aaccagcagt tt                                                        72

<210>    222
<211>    60
<212>    DNA
<213>    Artificial Sequence

<220>
<223>    HhLM2-3'

<400>    222
ccgggatcct tacggcgccg ccgccagcac cgccagtcaa ttctgagcct ccgggagcag    60

                                                                     60

<210> 223
<211> 48
<212> DNA
<213> Artificial Sequence

<220>
<223> JO-10 MTD cDNA Sequence

<400> 223
ctgggcggcg cggtggtggc ggcgccggtg gcggcggcgg tggcgccg 48

<210> 224
<211> 16
<212> PRT
<213> Artificial Sequence

<220>
<223> JO-10 MTD Amino Acid Sequence Sequence

<400> 224
Leu Gly Gly Ala Val Val Ala Ala Pro Val Ala Ala Ala Val Ala Pro
1               5                   10                  15

<210> 225
<211> 27
<212> DNA
<213> Artificial Sequence

<220>
<223> JO-52 MTD cDNA Sequence

<400> 225

ccgctgctgc tgctgctgcc ggcgctg                                          27

<210>    226
<211>    9
<212>    PRT
<213>    Artificial Sequence

<220>
<223>    JO-52 MTD Amino Acid Sequence Sequence

<400>    226
Pro Leu Leu Leu Leu Leu Pro Ala Leu
1               5

<210>    227
<211>    36
<212>    DNA
<213>    Artificial Sequence

<220>
<223>    JO-132 MTD cDNA Sequence

<400>    227
gcggtggtgg tgccggcgat tgtgctggcg gcgccg                               36

<210>    228
<211>    12
<212>    PRT
<213>    Artificial Sequence

<220>
<223>    JO-132 MTD Amino Acid Sequence Sequence

<400>    228

Ala Val Val Val Pro Ala Ile Val Leu Ala Ala Pro
1               5                   10

<210>    229
<211>    30
<212>    DNA
<213>    Artificial Sequence

<220>
<223>    JO-145 MTD cDNA Sequence

<400>    229
gcggcggcgc cggtgctgct gctgctgctg                                        30

<210>    230
<211>    10
<212>    PRT
<213>    Artificial Sequence

<220>
<223>    JO-145 MTD Amino Acid Sequence Sequence

<400>    230
Ala Ala Ala Pro Val Leu Leu Leu Leu Leu
1               5                   10

<210>    231
<211>    27
<212>    DNA
<213>    Artificial Sequence

<220>
<223>    JO-173 MTD cDNA Sequence

<400>    231
gcggtgattc cgattctggc ggtgccg                                          27

<210>    232
<211>    9
<212>    PRT
<213>    Artificial Sequence

<220>
<223>    JO-173 MTD Amino Acid Sequence Sequence

<400>    232
Ala Val Ile Pro Ile Leu Ala Val Pro
1               5

<210>    233
<211>    27
<212>    DNA
<213>    Artificial Sequence

<220>
<223>    JO-181 MTD cDNA Sequence

<400>    233
gcggtgctgc tgctgccggc ggcggcg                                          27

<210>    234
<211>    9
<212>    PRT
<213>    Artificial Sequence

<220>
<223>    JO-181 MTD Amino Acid Sequence Sequence

<400>    234
Ala Val Leu Leu Leu Pro Ala Ala Ala
1               5

<210>    235
<211>    960
<212>    DNA
<213>    Artificial Sequence

<220>
<223>    Codon optimized His-NLS-Nanog cDNA Sequence

<400>    235
atgggttcaa gtcatcatca tcatcatcat aaaaaaaaac gcaaaagtgt ggatccggca      60

tgcccgcagt cactgccgtg ttttgaagcg tcggactgca aagaaagctc tccgatgccg     120

gttatttgtg gcccggaaga aaactatccg tcactgcaga tgagttccgc cgaaatgccg     180

cataccgaaa cggtcagccc gctgccgtca tcgatggatc tgctgatcca agattcaccg     240

gacagctcta cgtcgccgaa aggtaaacag ccgaccagtg cagaaaactc cgttgctaaa     300

aaagaagata aagtgccggt taaaaaacaa aaaacccgta cggtctttag ttccacgcag     360

ctgtgcgtgc tgaatgaccg tttccagcgc caaaaatatc tgagcctgca gcaaatgcaa     420

gaactgagca acattctgaa tctgtcttac aaacaggtga aaacctggtt ccagaaccaa     480

cgtatgaaaa gtaaacgctg gcagaaaaac aattggccga aaaactccaa tggcgttacg     540

cagaaagcga gtgccccgac ctacccgtcc ctgtattcat cgtaccatca gggctgtctg     600

gtcaacccga ccggtaatct gccgatgtgg tctaatcaga cctggaacaa tagtacgtgg     660

tccaaccaga cccaaaatat tcagagctgg tctaaccaca gctggaatac ccagacgtgg     720

tgcacccaaa gctggaacaa tcaggcatgg aactctccgt tttataattg tggtgaagaa     780

tcactgcagt cgtgcatgca gttccaaccg aacagcccgg cttctgatct ggaagcggcc 840

ctggaagcag ctggcgaagg tctgaatgtt atccagcaaa ccacgcgcta ctttagcacc 900

ccgcaaacga tggacctgtt tctgaattac tcgatgaata tgcaaccgga agatgtgtaa 960

960

<210> 236
<211> 1008
<212> DNA
<213> Artificial Sequence

<220>
<223> Codon optimized His-NLS-JO-10 MTD Nanog cDNA Sequence

<400> 236
atgggttcaa gtcatcatca tcatcatcat aaaaaaaaac gcaaactggg cggcgcggtc 60

gtggcggctc cggtggcagc agcggtggca ccgagtgtgg atccggcatg cccgcagtca 120

ctgccgtgtt ttgaagcgtc ggactgcaaa gaaagctctc cgatgccggt tatttgtggc 180

ccggaagaaa actatccgtc actgcagatg agttccgccg aaatgccgca taccgaaacg 240

gtcagcccgc tgccgtcatc gatggatctg ctgatccaag attcaccgga cagctctacg 300

tcgccgaaag gtaaacagcc gaccagtgca gaaaactccg ttgctaaaaa agaagataaa 360

gtgccggtta aaaaacaaaa aacccgtacg gtctttagtt ccacgcagct gtgcgtgctg 420

aatgaccgtt tccagcgcca aaaatatctg agcctgcagc aaatgcaaga actgagcaac 480

attctgaatc tgtcttacaa acaggtgaaa acctggttcc agaaccaacg tatgaaaagt 540

aaacgctggc agaaaaacaa ttggccgaaa aactccaatg gcgttacgca gaaagcgagt 600

gccccgacct acccgtccct gtattcatcg taccatcagg gctgtctggt caacccgacc 660

ggtaatctgc cgatgtggtc taatcagacc tggaacaata gtacgtggtc caaccagacc 720

caaaatattc agagctggtc taaccacagc tggaataccc agacgtggtg cacccaaagc 780

tggaacaatc aggcatggaa ctctccgttt tataattgtg gtgaagaatc actgcagtcg 840

tgcatgcagt tccaaccgaa cagcccggct tctgatctgg aagcggccct ggaagcagct 900

ggcgaaggtc tgaatgttat ccagcaaacc acgcgctact ttagcacccc gcaaacgatg 960

gacctgtttc tgaattactc gatgaatatg caaccggaag atgtgtaa 1008

<210> 237
<211> 335
<212> PRT
<213> Artificial Sequence

<220>
<223> Codon optimized His-NLS-JO-10 MTD Nanog Amino Acid Sequence

<400> 237
Met Gly Ser Ser His His His His His His Lys Lys Lys Arg Lys Leu
1               5                   10                  15

Gly Gly Ala Val Val Ala Ala Pro Val Ala Ala Ala Val Ala Pro Ser
            20                  25                  30

Val Asp Pro Ala Cys Pro Gln Ser Leu Pro Cys Phe Glu Ala Ser Asp
        35                  40                  45

Cys Lys Glu Ser Ser Pro Met Pro Val Ile Cys Gly Pro Glu Glu Asn
    50                  55                  60

Tyr Pro Ser Leu Gln Met Ser Ser Ala Glu Met Pro His Thr Glu Thr
65                  70                  75                  80

Val Ser Pro Leu Pro Ser Ser Met Asp Leu Leu Ile Gln Asp Ser Pro
                85                  90                  95

Asp Ser Ser Thr Ser Pro Lys Gly Lys Gln Pro Thr Ser Ala Glu Asn
            100                 105                 110

Ser Val Ala Lys Lys Glu Asp Lys Val Pro Val Lys Lys Gln Lys Thr
        115                 120                 125

Arg Thr Val Phe Ser Ser Thr Gln Leu Cys Val Leu Asn Asp Arg Phe
    130                 135                 140

Gln Arg Gln Lys Tyr Leu Ser Leu Gln Gln Met Gln Glu Leu Ser Asn
145                 150                 155                 160

Ile Leu Asn Leu Ser Tyr Lys Gln Val Lys Thr Trp Phe Gln Asn Gln
                165                 170                 175

Arg Met Lys Ser Lys Arg Trp Gln Lys Asn Asn Trp Pro Lys Asn Ser
            180                 185                 190

Asn Gly Val Thr Gln Lys Ala Ser Ala Pro Thr Tyr Pro Ser Leu Tyr
        195                 200                 205

Ser Ser Tyr His Gln Gly Cys Leu Val Asn Pro Thr Gly Asn Leu Pro
    210                 215                 220

Met Trp Ser Asn Gln Thr Trp Asn Asn Ser Thr Trp Ser Asn Gln Thr
225                 230                 235                 240

Gln Asn Ile Gln Ser Trp Ser Asn His Ser Trp Asn Thr Gln Thr Trp
                245                 250                 255

Cys Thr Gln Ser Trp Asn Asn Gln Ala Trp Asn Ser Pro Phe Tyr Asn
            260                 265                 270

Cys Gly Glu Glu Ser Leu Gln Ser Cys Met Gln Phe Gln Pro Asn Ser
        275                 280                 285

Pro Ala Ser Asp Leu Glu Ala Ala Leu Glu Ala Ala Gly Glu Gly Leu

290                 295                 300

Asn Val Ile Gln Gln Thr Thr Arg Tyr Phe Ser Thr Pro Gln Thr Met
305                 310                 315                 320

Asp Leu Phe Leu Asn Tyr Ser Met Asn Met Gln Pro Glu Asp Val
                325                 330                 335

<210>    238
<211>    1125
<212>    DNA
<213>    Artificial Sequence

<220>
<223>    Codon optimized His-NLS-Oct4 cDNA Sequence

<400>    238
atgggttcgt cgcatcatca tcatcatcat aaaaaaaaac gcaaagctgg tcacctggct      60

tccgattttg ctttctcacc gccgccgggc ggtggcggtg atggtccggg cggtccggaa     120

ccgggttggg tggacccgcg tacctggctg tccttccagg gtccgccggg cggtccgggt     180

attggtccgg gtgttggtcc gggctcagaa gtctggggta tcccgccgtg cccgccgccg     240

tatgaatttt gcggtggtat ggcatactgt ggtccgcagg tcggtgtggg tctggttccg     300

cagggcggtc tggaaacgtc gcaaccggaa ggcgaagcag gtgttggcgt cgaatcgaac     360

agcgatggcg ctagcccgga accgtgtacc gtgacgccgg gtgcggttaa actggaaaaa     420

gaaaaactgg aacagaatcc ggaagaaagc caagatatta aagcgctgca gaaagaactg     480

gaacaattcg ccaaactgct gaaacagaaa cgcatcaccc tgggttatac gcaagcggac     540

gttggtctga ccctgggcgt cctgtttggt aaagtgttct cgcagaccac gatttgccgc     600

tttgaagctc tgcaactgag cttcaaaaac atgtgtaaac tgcgtccgct gctgcagaaa     660

tgggtcgaag aagcggataa caatgaaaat ctgcaggaaa tttgcaaagc agaaaccctg 720

gtgcaagctc gtaaacgcaa acgtacgtct atcgaaaacc gcgttcgtgg caacctggaa 780

aatctgttcc tgcagtgccc gaaaccgacc ctgcagcaaa ttagccatat cgcccagcaa 840

ctgggcctgg aaaaagacgt ggttcgtgtg tggttttgta atcgtcgcca gaaaggtaaa 900

cgcagctcta gtgattacgc acagcgtgaa gactttgaag cagcaggttc tccgttcagt 960

ggcggtccgg tctcttttcc gctggcaccg ggtccgcatt ttggtacccc gggttatggc 1020

agtccgcact tcacggcact gtactcctca gtgccgtttc cggaaggcga agcgttcccg 1080

ccggtctccg tcaccaccct gggtagtccg atgcacagta attaa 1125

<210> 239
<211> 1152
<212> DNA
<213> Artificial Sequence

<220>
<223> Codon optimized His-NLS-JO-52 MTD Oct4 cDNA Sequence

<400> 239
atgggttcgt cgcatcatca tcatcatcat aaaaaaaaac gcaaaccgct gctgctgctg 60

ctgccggccc tggctggtca cctggcttcc gattttgctt tctcaccgcc gccgggcggt 120

ggcggtgatg gtccgggcgg tccggaaccg ggttgggtgg acccgcgtac ctggctgtcc 180

ttccagggtc cgccgggcgg tccgggtatt ggtccgggtg ttggtccggg ctcagaagtc 240

tggggtatcc cgccgtgccc gccgccgtat gaattttgcg gtggtatggc atactgtggt 300

ccgcaggtcg gtgtgggtct ggttccgcag ggcggtctgg aaacgtcgca accggaaggc 360

gaagcaggtg ttggcgtcga atcgaacagc gatggcgcta gcccggaacc gtgtaccgtg 420

acgccgggtg cggttaaact ggaaaaagaa aaactggaac agaatccgga agaaagccaa 480

gatattaaag cgctgcagaa agaactggaa caattcgcca aactgctgaa acagaaacgc 540

atcaccctgg gttatacgca agcggacgtt ggtctgaccc tgggcgtcct gtttggtaaa 600

gtgttctcgc agaccacgat ttgccgcttt gaagctctgc aactgagctt caaaaacatg 660

tgtaaactgc gtccgctgct gcagaaatgg gtcgaagaag cggataacaa tgaaaatctg 720

caggaaattt gcaaagcaga aaccctggtg caagctcgta aacgcaaacg tacgtctatc 780

gaaaaccgcg ttcgtggcaa cctggaaaat ctgttcctgc agtgcccgaa accgaccctg 840

cagcaaatta gccatatcgc ccagcaactg ggcctggaaa aagacgtggt tcgtgtgtgg 900

ttttgtaatc gtcgccagaa aggtaaacgc agctctagtg attacgcaca gcgtgaagac 960

tttgaagcag caggttctcc gttcagtggc ggtccggtct cttttccgct ggcaccgggt 1020

ccgcattttg gtaccccggg ttatggcagt ccgcacttca cggcactgta ctcctcagtg 1080

ccgtttccgg aaggcgaagc gttcccgccg gtctccgtca ccaccctggg tagtccgatg 1140

cacagtaatt aa 1152

<210> 240
<211> 383
<212> PRT
<213> Artificial Sequence

<220>
<223> Codon optimized His-NLS-JO-52 MTD Oct4 Amino Acid Sequence

<400> 240
Met Gly Ser Ser His His His His His His Lys Lys Lys Arg Lys Pro

1                   5                        10                       15

Leu Leu Leu Leu Leu Pro Ala Leu Ala Gly His Leu Ala Ser Asp Phe
                20                       25                       30

Ala Phe Ser Pro Pro Pro Gly Gly Gly Gly Asp Gly Pro Gly Gly Pro
            35                       40                       45

Glu Pro Gly Trp Val Asp Pro Arg Thr Trp Leu Ser Phe Gln Gly Pro
    50                       55                       60

Pro Gly Gly Pro Gly Ile Gly Pro Gly Val Gly Pro Gly Ser Glu Val
65                       70                       75                       80

Trp Gly Ile Pro Pro Cys Pro Pro Pro Tyr Glu Phe Cys Gly Gly Met
                    85                       90                       95

Ala Tyr Cys Gly Pro Gln Val Gly Val Gly Leu Val Pro Gln Gly Gly
                100                      105                      110

Leu Glu Thr Ser Gln Pro Glu Gly Glu Ala Gly Val Gly Val Glu Ser
            115                      120                      125

Asn Ser Asp Gly Ala Ser Pro Glu Pro Cys Thr Val Thr Pro Gly Ala
    130                      135                      140

Val Lys Leu Glu Lys Glu Lys Leu Glu Gln Asn Pro Glu Glu Ser Gln
145                      150                      155                      160

Asp Ile Lys Ala Leu Gln Lys Glu Leu Glu Gln Phe Ala Lys Leu Leu
                    165                      170                      175

Lys Gln Lys Arg Ile Thr Leu Gly Tyr Thr Gln Ala Asp Val Gly Leu
                180                      185                      190

Thr Leu Gly Val Leu Phe Gly Lys Val Phe Ser Gln Thr Thr Ile Cys
            195                      200                      205

Arg Phe Glu Ala Leu Gln Leu Ser Phe Lys Asn Met Cys Lys Leu Arg
    210                      215                      220

Pro Leu Leu Gln Lys Trp Val Glu Glu Ala Asp Asn Asn Glu Asn Leu
225                 230                 235                 240

Gln Glu Ile Cys Lys Ala Glu Thr Leu Val Gln Ala Arg Lys Arg Lys
                245                 250                 255

Arg Thr Ser Ile Glu Asn Arg Val Arg Gly Asn Leu Glu Asn Leu Phe
            260                 265                 270

Leu Gln Cys Pro Lys Pro Thr Leu Gln Gln Ile Ser His Ile Ala Gln
        275                 280                 285

Gln Leu Gly Leu Glu Lys Asp Val Val Arg Val Trp Phe Cys Asn Arg
    290                 295                 300

Arg Gln Lys Gly Lys Arg Ser Ser Ser Asp Tyr Ala Gln Arg Glu Asp
305                 310                 315                 320

Phe Glu Ala Ala Gly Ser Pro Phe Ser Gly Gly Pro Val Ser Phe Pro
                325                 330                 335

Leu Ala Pro Gly Pro His Phe Gly Thr Pro Gly Tyr Gly Ser Pro His
            340                 345                 350

Phe Thr Ala Leu Tyr Ser Ser Val Pro Phe Pro Glu Gly Glu Ala Phe
        355                 360                 365

Pro Pro Val Ser Val Thr Thr Leu Gly Ser Pro Met His Ser Asn
    370                 375                 380

<210>   241
<211>   996
<212>   DNA
<213>   Artificial Sequence

<220>

<223>    Codon optimized His-NLS-Sox2 cDNA Sequence

<400>    241
atgggctcgt cgcaccacca ccaccaccac aaaaaaaaac gcaaatataa catgatggaa    60

accgaactga aaccgccggg tccgcagcaa acctcgggcg gtggcggtgg caacagcacg    120

gcggcggcgg cgggtggcaa ccagaaaaat tcaccggatc gtgtgaaacg cccgatgaat    180

gcatttatgg tttggagtcg tggtcagcgt cgcaaaatgg ctcaagaaaa cccgaaaatg    240

cataactctg aaatcagtaa acgtctgggc gcggaatgga aactgctgag cgaaaccgaa    300

aaacgcccgt tcatcgatga agcgaaacgt ctgcgcgccc tgcatatgaa agaacacccg    360

gactataaat accgcccgcg tcgcaaaacg aaaacgctga tgaaaaaaga taaatatacg    420

ctgccgggtg gcctgctggc accgggtggc aacagcatgg cgtcaggtgt tggtgtcggt    480

gcaggtctgg gtgcaggtgt caatcagcgt atggattctt acgcccatat gaacggttgg    540

agtaatggca gttattccat gatgcaggac caactgggtt acccgcagca tccgggtctg    600

aacgcacacg gcgcagcaca gatgcaaccg atgcaccgct atgacgtttc cgccctgcag    660

tacaactcaa tgaccagctc tcaaacgtat atgaatggct ctccgaccta ttcaatgtcg    720

tacagccagc agggtacgcc gggtatggca ctgggttcga tgggcagcgt ggttaaaagc    780

gaagccagtt cctcaccgcc ggtcgtgacc tcgagctctc actcccgtgc accgtgccag    840

gctggtgatc tgcgcgacat gattagcatg tatctgccgg gtgcagaagt gccggaaccg    900

gcagctccgt ctcgtctgca tatgagtcag cactatcaat cgggtccggt tccgggtacg    960

gctatcaacg gtacgctgcc gctgtcgcac atgtaa                              996

<210>    242

<211> 1023
<212> DNA
<213> Artificial Sequence

<220>
<223> Codon optimized His-NLS-JO-181 MTD Sox2 cDNA Sequence

<400> 242
atgggctcgt cgcaccacca ccaccaccac aaaaaaaaac gcaaagcggt cctgctgctg 60

ccggctgctg cctataacat gatggaaacc gaactgaaac cgccgggtcc gcagcaaacc 120

tcgggcggtg gcggtggcaa cagcacggcg gcggcggcgg gtggcaacca gaaaaattca 180

ccggatcgtg tgaaacgccc gatgaatgca tttatggttt ggagtcgtgg tcagcgtcgc 240

aaaatggctc aagaaaaccc gaaaatgcat aactctgaaa tcagtaaacg tctgggcgcg 300

gaatggaaac tgctgagcga aaccgaaaaa cgcccgttca tcgatgaagc gaaacgtctg 360

cgcgccctgc atatgaaaga acacccggac tataaatacc gcccgcgtcg caaaacgaaa 420

acgctgatga aaaaagataa atatacgctg ccgggtggcc tgctggcacc gggtggcaac 480

agcatggcgt caggtgttgg tgtcggtgca ggtctgggtg caggtgtcaa tcagcgtatg 540

gattcttacg cccatatgaa cggttggagt aatggcagtt attccatgat gcaggaccaa 600

ctgggttacc cgcagcatcc gggtctgaac gcacacggcg cagcacagat gcaaccgatg 660

caccgctatg acgtttccgc cctgcagtac aactcaatga ccagctctca aacgtatatg 720

aatggctctc cgacctattc aatgtcgtac agccagcagg gtacgccggg tatggcactg 780

ggttcgatgg gcagcgtggt taaaagcgaa gccagttcct caccgccggt cgtgacctcg 840

agctctcact cccgtgcacc gtgccaggct ggtgatctgc gcgacatgat tagcatgtat 900

ctgccgggtg cagaagtgcc ggaaccggca gctccgtctc gtctgcatat gagtcagcac 960

tatcaatcgg gtccggttcc gggtacggct atcaacggta cgctgccgct gtcgcacatg 1020

taa 1023

<210> 243
<211> 340
<212> PRT
<213> Artificial Sequence

<220>
<223> Codon optimized His-NLS-JO-181 MTD Sox2 Amino Acid Sequence

<400> 243
Met Gly Ser Ser His His His His His His Lys Lys Lys Arg Lys Ala
1               5                   10                  15

Val Leu Leu Leu Pro Ala Ala Ala Tyr Asn Met Met Glu Thr Glu Leu
            20                  25                  30

Lys Pro Pro Gly Pro Gln Gln Thr Ser Gly Gly Gly Gly Gly Asn Ser
        35                  40                  45

Thr Ala Ala Ala Ala Gly Gly Asn Gln Lys Asn Ser Pro Asp Arg Val
    50                  55                  60

Lys Arg Pro Met Asn Ala Phe Met Val Trp Ser Arg Gly Gln Arg Arg
65                  70                  75                  80

Lys Met Ala Gln Glu Asn Pro Lys Met His Asn Ser Glu Ile Ser Lys
                85                  90                  95

Arg Leu Gly Ala Glu Trp Lys Leu Leu Ser Glu Thr Glu Lys Arg Pro
            100                 105                 110

Phe Ile Asp Glu Ala Lys Arg Leu Arg Ala Leu His Met Lys Glu His
        115                 120                 125

Pro Asp Tyr Lys Tyr Arg Pro Arg Arg Lys Thr Lys Thr Leu Met Lys
    130                 135                 140

Lys Asp Lys Tyr Thr Leu Pro Gly Gly Leu Leu Ala Pro Gly Gly Asn
145                 150                 155                 160

Ser Met Ala Ser Gly Val Gly Val Gly Ala Gly Leu Gly Ala Gly Val
                165                 170                 175

Asn Gln Arg Met Asp Ser Tyr Ala His Met Asn Gly Trp Ser Asn Gly
            180                 185                 190

Ser Tyr Ser Met Met Gln Asp Gln Leu Gly Tyr Pro Gln His Pro Gly
        195                 200                 205

Leu Asn Ala His Gly Ala Ala Gln Met Gln Pro Met His Arg Tyr Asp
    210                 215                 220

Val Ser Ala Leu Gln Tyr Asn Ser Met Thr Ser Ser Gln Thr Tyr Met
225                 230                 235                 240

Asn Gly Ser Pro Thr Tyr Ser Met Ser Tyr Ser Gln Gln Gly Thr Pro
                245                 250                 255

Gly Met Ala Leu Gly Ser Met Gly Ser Val Val Lys Ser Glu Ala Ser
            260                 265                 270

Ser Ser Pro Pro Val Val Thr Ser Ser Ser His Ser Arg Ala Pro Cys
        275                 280                 285

Gln Ala Gly Asp Leu Arg Asp Met Ile Ser Met Tyr Leu Pro Gly Ala
    290                 295                 300

Glu Val Pro Glu Pro Ala Ala Pro Ser Arg Leu His Met Ser Gln His
305                 310                 315                 320

Tyr Gln Ser Gly Pro Val Pro Gly Thr Ala Ile Asn Gly Thr Leu Pro
                325                 330                 335

Leu Ser His Met

            340

<210> 244
<211> 1455
<212> DNA
<213> Artificial Sequence

<220>
<223> Codon optimized His-NLS-Klf4 cDNA Sequence

<400> 244
atgggttcga gtcatcatca tcatcatcat aaaaaaaaac gtaaagcagt gagcgacgca 60

ctgctgccgt cgtttagcac gttcgctagt ggtccggcag gtcgtgaaaa aaccctgcgt 120

caggcaggtg caccgaacaa tcgttggcgc gaagaactgt cccacatgaa acgtctgccg 180

ccggtgctgc cgggtcgtcc gtatgatctg gcggcggcaa cggttgcaac cgacctggaa 240

tcaggcggtg ctggtgctgc atgcggtggt tcgaacctgg caccgctgcc gcgtcgcgaa 300

accgaagaat ttaacgatct gctggatctg gacttcattc tgtctaatag tctgacgcat 360

ccgccggaaa gcgtcgcagc aaccgtgagc tctagtgctt ctgcgtcctc atcgagctct 420

ccgagttcct caggtccggc atcggcaccg agcacgtgtt cttttaccta tccgatccgt 480

gcaggcaatg atccgggtgt tgctccgggc ggtaccggcg gtggcctgct gtacggtcgt 540

gaaagcgctc cgccgccgac cgcaccgttt aacctggccg atattaatga cgtttccccg 600

tcaggtggct tcgtcgcaga actgctgcgt ccggaactgg acccggtcta tatcccgccg 660

cagcaaccgc aaccgccggg tggcggtctg atgggtaaat ttgtgctgaa agcctcgctg 720

agcgcaccgg gcagcgaata tggttctccg agtgtgattt ccgtttcaaa aggcagtccg 780

gatggttccc acccggtggt tgtcgcaccg tacaacggtg gtccgccgcg tacgtgcccg 840

aaaatcaaac aagaagccgt gtcgagctgt acccatctgg gtgcaggtcc gccgctgtca 900

aatggtcacc gtccggctgc acatgatttc ccgctgggtc gtcagctgcc gtctcgtacc 960

acgccgaccc tgggtctgga agaagttctg tctagtcgtg actgccatcc ggcactgccg 1020

ctgccgccgg gctttcatcc gcacccgggt ccgaactatc cgtctttcct gccggatcag 1080

atgcaaccgc aggtcccgcc gctgcactac caggaactga tgccgccggg cagttgtatg 1140

ccggaagaac cgaaaccgaa acgtggtcgt cgctcctggc cgcgtaaacg taccgcaacg 1200

catacctgcg actatgccgg ctgtggtaaa acgtacacca aatcctcaca tctgaaagcg 1260

cacctgcgca cgcataccgg cgaaaaaccg tatcactgcg attgggacgg ctgtggttgg 1320

aaatttgccc gtagcgatga actgacgcgt cattaccgca aacataccgg tcaccgcccg 1380

ttccaatgcc agaaatgcga ccgcgccttc tcccgttccg accacctggc actgcacatg 1440

aaacgtcact tctaa 1455

<210> 245
<211> 1482
<212> DNA
<213> Artificial Sequence

<220>
<223> Codon optimized His-NLS-JO-173 MTD Klf4 cDNA Sequence

<400> 245
atgggttcga gtcatcatca tcatcatcat aaaaaaaaac gtaaagcagt catcccgatt 60

ctggcggtcc cggcagtgag cgacgcactg ctgccgtcgt ttagcacgtt cgctagtggt 120

ccggcaggtc gtgaaaaaac cctgcgtcag gcaggtgcac cgaacaatcg ttggcgcgaa 180

gaactgtccc acatgaaacg tctgccgccg gtgctgccgg gtcgtccgta tgatctggcg 240

gcggcaacgg ttgcaaccga cctggaatca ggcggtgctg gtgctgcatg cggtggttcg 300

aacctggcac cgctgccgcg tcgcgaaacc gaagaattta acgatctgct ggatctggac 360

ttcattctgt ctaatagtct gacgcatccg ccggaaagcg tcgcagcaac cgtgagctct 420

agtgcttctg cgtcctcatc gagctctccg agttcctcag gtccggcatc ggcaccgagc 480

acgtgttctt ttacctatcc gatccgtgca ggcaatgatc cgggtgttgc tccgggcggt 540

accggcggtg gcctgctgta cggtcgtgaa agcgctccgc cgccgaccgc accgtttaac 600

ctggccgata ttaatgacgt ttccccgtca ggtggcttcg tcgcagaact gctgcgtccg 660

gaactggacc cggtctatat cccgccgcag caaccgcaac cgccgggtgg cggtctgatg 720

ggtaaatttg tgctgaaagc ctcgctgagc gcaccgggca gcgaatatgg ttctccgagt 780

gtgatttccg tttcaaaagg cagtccggat ggttcccacc cggtggttgt cgcaccgtac 840

aacggtggtc cgccgcgtac gtgcccgaaa atcaaacaag aagccgtgtc gagctgtacc 900

catctgggtg caggtccgcc gctgtcaaat ggtcaccgtc cggctgcaca tgatttcccg 960

ctgggtcgtc agctgccgtc tcgtaccacg ccgaccctgg gtctggaaga agttctgtct 1020

agtcgtgact gccatccggc actgccgctg ccgccgggct ttcatccgca cccgggtccg 1080

aactatccgt ctttcctgcc ggatcagatg caaccgcagg tcccgccgct gcactaccag 1140

gaactgatgc cgccgggcag ttgtatgccg gaagaaccga aaccgaaacg tggtcgtcgc 1200

tcctggccgc gtaaacgtac cgcaacgcat acctgcgact atgccggctg tggtaaaacg 1260

tacaccaaat cctcacatct gaaagcgcac ctgcgcacgc ataccggcga aaaaccgtat 1320

cactgcgatt gggacggctg tggttggaaa tttgcccgta gcgatgaact gacgcgtcat 1380

taccgcaaac ataccggtca ccgcccgttc caatgccaga aatgcgaccg cgccttctcc 1440

cgttccgacc acctggcact gcacatgaaa cgtcacttct aa 1482

<210> 246
<211> 493
<212> PRT
<213> Artificial Sequence

<220>
<223> Codon optimized His-NLS-JO-173 MTD Klf4 Amino Acid Sequence

<400> 246
Met Gly Ser Ser His His His His His His Lys Lys Lys Arg Lys Ala
1               5                   10                  15

Val Ile Pro Ile Leu Ala Val Pro Ala Val Ser Asp Ala Leu Leu Pro
            20                  25                  30

Ser Phe Ser Thr Phe Ala Ser Gly Pro Ala Gly Arg Glu Lys Thr Leu
        35                  40                  45

Arg Gln Ala Gly Ala Pro Asn Asn Arg Trp Arg Glu Glu Leu Ser His
    50                  55                  60

Met Lys Arg Leu Pro Pro Val Leu Pro Gly Arg Pro Tyr Asp Leu Ala
65                  70                  75                  80

Ala Ala Thr Val Ala Thr Asp Leu Glu Ser Gly Gly Ala Gly Ala Ala
                85                  90                  95

Cys Gly Gly Ser Asn Leu Ala Pro Leu Pro Arg Arg Glu Thr Glu Glu
            100                 105                 110

Phe Asn Asp Leu Leu Asp Leu Asp Phe Ile Leu Ser Asn Ser Leu Thr
        115                 120                 125

His Pro Pro Glu Ser Val Ala Ala Thr Val Ser Ser Ser Ala Ser Ala

130                 135                 140

Ser Ser Ser Ser Ser Pro Ser Ser Ser Gly Pro Ala Ser Ala Pro Ser
145                 150                 155                 160

Thr Cys Ser Phe Thr Tyr Pro Ile Arg Ala Gly Asn Asp Pro Gly Val
                165                 170                 175

Ala Pro Gly Gly Thr Gly Gly Gly Leu Leu Tyr Gly Arg Glu Ser Ala
            180                 185                 190

Pro Pro Pro Thr Ala Pro Phe Asn Leu Ala Asp Ile Asn Asp Val Ser
        195                 200                 205

Pro Ser Gly Gly Phe Val Ala Glu Leu Leu Arg Pro Glu Leu Asp Pro
    210                 215                 220

Val Tyr Ile Pro Pro Gln Gln Pro Gln Pro Pro Gly Gly Gly Leu Met
225                 230                 235                 240

Gly Lys Phe Val Leu Lys Ala Ser Leu Ser Ala Pro Gly Ser Glu Tyr
                245                 250                 255

Gly Ser Pro Ser Val Ile Ser Val Ser Lys Gly Ser Pro Asp Gly Ser
            260                 265                 270

His Pro Val Val Val Ala Pro Tyr Asn Gly Gly Pro Pro Arg Thr Cys
        275                 280                 285

Pro Lys Ile Lys Gln Glu Ala Val Ser Ser Cys Thr His Leu Gly Ala
    290                 295                 300

Gly Pro Pro Leu Ser Asn Gly His Arg Pro Ala Ala His Asp Phe Pro
305                 310                 315                 320

Leu Gly Arg Gln Leu Pro Ser Arg Thr Thr Pro Thr Leu Gly Leu Glu
                325                 330                 335

Glu Val Leu Ser Ser Arg Asp Cys His Pro Ala Leu Pro Leu Pro Pro
            340                 345                 350

Gly Phe His Pro His Pro Gly Pro Asn Tyr Pro Ser Phe Leu Pro Asp
        355                 360                 365

Gln Met Gln Pro Gln Val Pro Pro Leu His Tyr Gln Glu Leu Met Pro
    370                 375                 380

Pro Gly Ser Cys Met Pro Glu Glu Pro Lys Pro Lys Arg Gly Arg Arg
385                 390                 395                 400

Ser Trp Pro Arg Lys Arg Thr Ala Thr His Thr Cys Asp Tyr Ala Gly
                405                 410                 415

Cys Gly Lys Thr Tyr Thr Lys Ser Ser His Leu Lys Ala His Leu Arg
            420         .       425                 430

Thr His Thr Gly Glu Lys Pro Tyr His Cys Asp Trp Asp Gly Cys Gly
        435                 440                 445

Trp Lys Phe Ala Arg Ser Asp Glu Leu Thr Arg His Tyr Arg Lys His
    450                 455                 460

Thr Gly His Arg Pro Phe Gln Cys Gln Lys Cys Asp Arg Ala Phe Ser
465                 470                 475                 480

Arg Ser Asp His Leu Ala Leu His Met Lys Arg His Phe
                485                 490

<210>   247
<211>   1410
<212>   DNA
<213>   Artificial Sequence

<220>
<223>   Codon optimized His-NLS-cMyc cDNA Sequence

<400>   247
atgggttcgt cgcaccacca ccaccatcat aaaaaaaaac gcaaactgga cttcttccgt      60

gtggttgaaa accagcaacc gccggcgacc atgccgctga atgtgtcctt tacgaaccgc 120

aattatgatc tggactacga ttcagttcaa ccgtattttt actgcgacga agaagaaaac 180

ttctatcagc aacagcaaca gtccgaactg cagccgccgg caccgtcaga agatatctgg 240

aaaaaattcg aactgctgcc gaccccgccg ctgtcgccga gccgtcgctc cggtctgtgt 300

tctccgagtt acgtcgccgt gacgccgttt agtctgcgtg gtgacaatga tggcggtggc 360

ggttccttct caaccgcaga tcaactggaa atggttacgg aactgctggg cggtgacatg 420

gtcaaccaga gctttatctg cgatccggat gacgaaacct tcatcaaaaa catcatcatt 480

caggactgta tgtggtcggg ctttagcgcg gcggcaaaac tggtctctga aaaactggca 540

agttatcagg ctgcgcgtaa agattcgggt agcccgaacc cggcacgtgg tcattctgtt 600

tgcagtacca gctctctgta tctgcaggac ctgtcggccg cagctagcga atgtatcgat 660

ccgagcgtcg tgtttccgta cccgctgaat gatagttcct caccgaaatc ctgcgcatca 720

caagactcga gcgctttctc cccgtctagt gattcactgc tgtcctcaac cgaatcgagc 780

ccgcagggtt ctccggaacc gctggtgctg cacgaagaaa cgccgccgac cacgtctagt 840

gattctgaag aagaacaaga agacgaagaa gaaattgatg ttgtcagcgt tgaaaaacgt 900

caggcgccgg gcaaacgctc tgaaagtggt tccccgtcag ccggcggtca ttcgaaaccg 960

ccgcacagcc cgctggtgct gaaacgttgc catgtttcta cccatcagca caactatgcg 1020

gccccgccga gtacgcgtaa agactacccg gcagctaaac gcgtgaaact ggatagcgtt 1080

cgtgtcctgc gccagatttc taacaatcgt aaatgtacca gtccgcgctc ctcagatacg 1140

gaagaaaacg tcaaacgtcg cacccacaat gtgctggaac gccaacgtcg caatgaactg 1200

aaacgtagct ttttcgcact gcgcgatcag atcccggaac tggaaaacaa tgaaaaagct 1260

ccgaaagtgg ttatcctgaa aaaagcgacc gcctatattc tgtcggtgca agcggaagaa 1320

cagaaactga ttagcgaaga agatctgctg cgtaaacgtc gtgaacaact gaaacacaaa 1380

ctggaacaac tgcgtaattc ttgcgcttaa 1410

<210> 248
<211> 1440
<212> DNA
<213> Artificial Sequence

<220>
<223> Codon optimized His-NLS-JO-145 MTD cMyc cDNA Sequence

<400> 248
atgggttcgt cgcaccacca ccaccatcat aaaaaaaaac gcaaagcggc tgccccggtt 60

ctgctgctgc tgctgctgga cttcttccgt gtggttgaaa accagcaacc gccggcgacc 120

atgccgctga atgtgtcctt tacgaaccgc aattatgatc tggactacga ttcagttcaa 180

ccgtattttt actgcgacga agaagaaaac ttctatcagc aacagcaaca gtccgaactg 240

cagccgccgg caccgtcaga agatatctgg aaaaaattcg aactgctgcc gaccccgccg 300

ctgtcgccga gccgtcgctc cggtctgtgt tctccgagtt acgtcgccgt gacgccgttt 360

agtctgcgtg gtgacaatga tggcggtggc ggttccttct caaccgcaga tcaactggaa 420

atggttacgg aactgctggg cggtgacatg gtcaaccaga gctttatctg cgatccggat 480

gacgaaacct tcatcaaaaa catcatcatt caggactgta tgtggtcggg ctttagcgcg 540

gcggcaaaac tggtctctga aaaactggca agttatcagg ctgcgcgtaa agattcgggt 600

agcccgaacc cggcacgtgg tcattctgtt tgcagtacca gctctctgta tctgcaggac 660

ctgtcggccg cagctagcga atgtatcgat ccgagcgtcg tgtttccgta cccgctgaat 720

gatagttcct caccgaaatc ctgcgcatca caagactcga gcgctttctc cccgtctagt 780

gattcactgc tgtcctcaac cgaatcgagc ccgcagggtt ctccggaacc gctggtgctg 840

cacgaagaaa cgccgccgac cacgtctagt gattctgaag aagaacaaga agacgaagaa 900

gaaattgatg ttgtcagcgt tgaaaaacgt caggcgccgg gcaaacgctc tgaaagtggt 960

tccccgtcag ccggcggtca ttcgaaaccg ccgcacagcc cgctggtgct gaaacgttgc 1020

catgtttcta cccatcagca caactatgcg gccccgccga gtacgcgtaa agactacccg 1080

gcagctaaac gcgtgaaact ggatagcgtt cgtgtcctgc gccagatttc taacaatcgt 1140

aaatgtacca gtccgcgctc ctcagatacg gaagaaaacg tcaaacgtcg cacccacaat 1200

gtgctggaac gccaacgtcg caatgaactg aaacgtagct ttttcgcact gcgcgatcag 1260

atcccggaac tggaaaacaa tgaaaaagct ccgaaagtgg ttatcctgaa aaaagcgacc 1320

gcctatattc tgtcggtgca agcggaagaa cagaaactga ttagcgaaga agatctgctg 1380

cgtaaacgtc gtgaacaact gaaacacaaa ctggaacaac tgcgtaattc ttgcgcttaa 1440

1440

<210> 249
<211> 479
<212> PRT
<213> Artificial Sequence

<220>
<223> Codon optimized His-NLS-JO-145 MTD cMyc Amino Acid Sequence

<400> 249
Met Gly Ser Ser His His His His His His Lys Lys Lys Arg Lys Ala

1               5                   10                  15

Ala Ala Pro Val Leu Leu Leu Leu Leu Leu Asp Phe Phe Arg Val Val
            20                  25                  30

Glu Asn Gln Gln Pro Pro Ala Thr Met Pro Leu Asn Val Ser Phe Thr
        35                  40                  45

Asn Arg Asn Tyr Asp Leu Asp Tyr Asp Ser Val Gln Pro Tyr Phe Tyr
    50                  55                  60

Cys Asp Glu Glu Glu Asn Phe Tyr Gln Gln Gln Gln Gln Ser Glu Leu
65                  70                  75                  80

Gln Pro Pro Ala Pro Ser Glu Asp Ile Trp Lys Lys Phe Glu Leu Leu
                85                  90                  95

Pro Thr Pro Pro Leu Ser Pro Ser Arg Arg Ser Gly Leu Cys Ser Pro
            100                 105                 110

Ser Tyr Val Ala Val Thr Pro Phe Ser Leu Arg Gly Asp Asn Asp Gly
        115                 120                 125

Gly Gly Gly Ser Phe Ser Thr Ala Asp Gln Leu Glu Met Val Thr Glu
    130                 135                 140

Leu Leu Gly Gly Asp Met Val Asn Gln Ser Phe Ile Cys Asp Pro Asp
145                 150                 155                 160

Asp Glu Thr Phe Ile Lys Asn Ile Ile Ile Gln Asp Cys Met Trp Ser
                165                 170                 175

Gly Phe Ser Ala Ala Ala Lys Leu Val Ser Glu Lys Leu Ala Ser Tyr
            180                 185                 190

Gln Ala Ala Arg Lys Asp Ser Gly Ser Pro Asn Pro Ala Arg Gly His
        195                 200                 205

Ser Val Cys Ser Thr Ser Ser Leu Tyr Leu Gln Asp Leu Ser Ala Ala
    210                 215                 220

Ala Ser Glu Cys Ile Asp Pro Ser Val Val Phe Pro Tyr Pro Leu Asn
225                 230                 235                 240

Asp Ser Ser Ser Pro Lys Ser Cys Ala Ser Gln Asp Ser Ser Ala Phe
                245                 250                 255

Ser Pro Ser Ser Asp Ser Leu Leu Ser Ser Thr Glu Ser Ser Pro Gln
            260                 265                 270

Gly Ser Pro Glu Pro Leu Val Leu His Glu Glu Thr Pro Pro Thr Thr
        275                 280                 285

Ser Ser Asp Ser Glu Glu Glu Gln Glu Asp Glu Glu Glu Ile Asp Val
    290                 295                 300

Val Ser Val Glu Lys Arg Gln Ala Pro Gly Lys Arg Ser Glu Ser Gly
305                 310                 315                 320

Ser Pro Ser Ala Gly Gly His Ser Lys Pro Pro His Ser Pro Leu Val
                325                 330                 335

Leu Lys Arg Cys His Val Ser Thr His Gln His Asn Tyr Ala Ala Pro
            340                 345                 350

Pro Ser Thr Arg Lys Asp Tyr Pro Ala Ala Lys Arg Val Lys Leu Asp
        355                 360                 365

Ser Val Arg Val Leu Arg Gln Ile Ser Asn Asn Arg Lys Cys Thr Ser
    370                 375                 380

Pro Arg Ser Ser Asp Thr Glu Glu Asn Val Lys Arg Arg Thr His Asn
385                 390                 395                 400

Val Leu Glu Arg Gln Arg Arg Asn Glu Leu Lys Arg Ser Phe Phe Ala
                405                 410                 415

Leu Arg Asp Gln Ile Pro Glu Leu Glu Asn Asn Glu Lys Ala Pro Lys
            420                 425                 430

Val Val Ile Leu Lys Lys Ala Thr Ala Tyr Ile Leu Ser Val Gln Ala
        435                 440                 445

Glu Glu Gln Lys Leu Ile Ser Glu Glu Asp Leu Leu Arg Lys Arg Arg
    450                 455                 460

Glu Gln Leu Lys His Lys Leu Glu Gln Leu Arg Asn Ser Cys Ala
465                 470                 475

<210>    250
<211>    672
<212>    DNA
<213>    Artificial Sequence

<220>
<223>    Codon optimized His-NLS-Lin28 cDNA Sequence

<400>    250
atgggctcgt cgcatcatca tcatcatcat aaaaaaaaac gcaaaggttc ggttagcaac      60

cagcaatttg cgggcggttg cgccaaagcg gccgaagaag caccggaaga agctccggaa     120

gatgcagctc gtgcagcaga cgaaccgcag ctgctgcatg gcgcaggtat ttgtaaatgg     180

ttcaatgtcc gtatgggctt tggtttcctg tctatgaccg cacgtgctgg tgtggcactg     240

gatccgccgg tggacgtttt tgtccatcaa agcaaactgc acatggaagg cttccgttct     300

ctgaaagaag gtgaagctgt tgaatttacc ttcaaaaaat ctgccaaagg cctggaatcc     360

attcgcgtga cgggtccggg cggtgtgttt tgcatcggca gcgaacgtcg cccgaagggt     420

aaatcaatgc agaaacgtcg ctcgaaaggt gatcgctgct ataactgtgg cggtctggac     480

catcacgcca aagaatgcaa actgccgccg cagccgaaaa aatgccattt ctgtcaaagc     540

atctctcaca tggtcgcgag ttgtccgctg aaagcccagc aaggcccgtc cgcacagggt     600

aaaccgacgt actttcgtga agaagaagaa gaaatccact ccccgacgct gctgccggaa 660

gcccagaact aa 672

<210> 251
<211> 708
<212> DNA
<213> Artificial Sequence

<220>
<223> Codon optimized His-NLS-JO-132 MTD Lin28 cDNA Sequence

<400> 251
atgggctcgt cgcatcatca tcatcatcat aaaaaaaaac gcaaagcagt cgtcgtcccg 60

gcaatcgtcc tggcggcacc gggttcggtt agcaaccagc aatttgcggg cggttgcgcc 120

aaagcggccg aagaagcacc ggaagaagct ccggaagatg cagctcgtgc agcagacgaa 180

ccgcagctgc tgcatggcgc aggtatttgt aaatggttca atgtccgtat gggctttggt 240

ttcctgtcta tgaccgcacg tgctggtgtg gcactggatc cgccggtgga cgtttttgtc 300

catcaaagca aactgcacat ggaaggcttc cgttctctga aagaaggtga agctgttgaa 360

tttaccttca aaaaatctgc caaaggcctg gaatccattc gcgtgacggg tccgggcggt 420

gtgttttgca tcggcagcga acgtcgcccg aagggtaaat caatgcagaa acgtcgctcg 480

aaaggtgatc gctgctataa ctgtggcggt ctggaccatc acgccaaaga atgcaaactg 540

ccgccgcagc cgaaaaaatg ccatttctgt caaagcatct ctcacatggt cgcgagttgt 600

ccgctgaaag cccagcaagg cccgtccgca cagggtaaac cgacgtactt tcgtgaagaa 660

gaagaagaaa tccactcccc gacgctgctg ccggaagccc agaactaa 708

<210>    252

<211>    235

<212>    PRT

<213>    Artificial Sequence

<220>

<223>    Codon optimized His-NLS-JO-132 MTD Lin28 Amino Acid Sequence

<400>    252

Met Gly Ser Ser His His His His His His Lys Lys Lys Arg Lys Ala
1               5                   10                  15

Val Val Val Pro Ala Ile Val Leu Ala Ala Pro Gly Ser Val Ser Asn
            20                  25                  30

Gln Gln Phe Ala Gly Gly Cys Ala Lys Ala Ala Glu Glu Ala Pro Glu
        35                  40                  45

Glu Ala Pro Glu Asp Ala Ala Arg Ala Ala Asp Glu Pro Gln Leu Leu
    50                  55                  60

His Gly Ala Gly Ile Cys Lys Trp Phe Asn Val Arg Met Gly Phe Gly
65                  70                  75                  80

Phe Leu Ser Met Thr Ala Arg Ala Gly Val Ala Leu Asp Pro Pro Val
                85                  90                  95

Asp Val Phe Val His Gln Ser Lys Leu His Met Glu Gly Phe Arg Ser
            100                 105                 110

Leu Lys Glu Gly Glu Ala Val Glu Phe Thr Phe Lys Lys Ser Ala Lys
        115                 120                 125

Gly Leu Glu Ser Ile Arg Val Thr Gly Pro Gly Gly Val Phe Cys Ile
    130                 135                 140

Gly Ser Glu Arg Arg Pro Lys Gly Lys Ser Met Gln Lys Arg Arg Ser
145                 150                 155                 160

Lys Gly Asp Arg Cys Tyr Asn Cys Gly Gly Leu Asp His His Ala Lys
                165                 170                 175

Glu Cys Lys Leu Pro Pro Gln Pro Lys Lys Cys His Phe Cys Gln Ser
            180                 185                 190

Ile Ser His Met Val Ala Ser Cys Pro Leu Lys Ala Gln Gln Gly Pro
        195                 200                 205

Ser Ala Gln Gly Lys Pro Thr Tyr Phe Arg Glu Glu Glu Glu Glu Ile
    210                 215                 220

His Ser Pro Thr Leu Leu Pro Glu Ala Gln Asn
225                 230                 235

## Description

### FIELD OF THE INVENTION

The present invention relates to a reprogramming transcription factor recombinant protein in which a macromolecule transduction domain (MTD) is fused to a reprogramming transcription factor to obtain cell permeability. The present invention also relates to a polynucleotide which encodes said cell-permeable reprogramming transcription factor recombinant protein and an expression vector for said cell-permeable reprogramming transcription factor recombinant protein. Said cell-permeable reprogramming transcription factor recombinant protein can effectively increase the ability of reprogramming in a somatic cell, and thus can be useful in the establishment of an induced pluripotent stem cell (iPS cell).

### BACKGROUND

Embryonic stem (ES) cells are "pluripotent stem cells" derived from the inner cell mass (ICM) of blastocyst stage embryos. They can differentiate into three primary germ layers, i.e., endoderm, mesoderm, and ectoderm, which develop into the human body. The endoderm gives rise to cells of the thyroid gland, cells of the lung, and pancreatic cells; the mesoderm develops into cardiac muscle cells, muscle cells, kidney cells, and blood cells; and the ectoderm gives rise to not only skin cells, nervous cells, and pigment cells but also reproductive cells, i.e., ovum and sperm. Since embryonic stem cells can differentiate into such various types of cells, they are expected to play an important role as a tool for treating incurable diseases.

Embryonic stem cells can be established by using cells from frozen human embryos or by a somatic cell nuclear transfer technique in which the nucleus of a somatic cell is transferred to a denucleated egg cell. However, these two methods have ethical issues in that cells derived from human embryos are used and a large number of human eggs are required. In addition, low efficiency of the nuclear transfer and potential risk of fatal genetic defects caused by cloning are also problematic.

Another method of establishing embryonic stem cells that avoids the use of human embryos and eggs is to reprogram human somatic cells with reprogramming transcription factors. In this regard, it was reported that 24 candidate genes which were involved in the maintenance of embryonic stem cells were selected, and among them, four genes (Oct4, Sox2, c-Myc, and Klf4) were found to induce reprogramming of cells when transferred into the cell via viral vectors (Yamanaka, Cell 126: 633-678, 2006). It was shown that these four retrovirally-delivered factors were expressed in mouse fibroblast cells and induced reprogramming of the somatic cells to generate induced pluripotent stem cells. In addition, another study showed that human somatic cells were successfully reprogrammed by delivering Oct4 and Sox2 from the four reprogramming transcription factors in combination with new factors, i.e., Lin28 and Nanog (Yu J, Science 318(5858): 1917-20, 2007). Thus, it can be appreciated that among the six transcription factors experimented above (Oct4, Sox2, c-Myc, Klf4, Nanog, and Lin28), Oct4 and Sox2 are indispensible for induction of reprogramming, while c-Myc, Klf4, Nanog, and Lin28 can be optionally used with the two primary transcription factors in various combinations and play an important role in the establishment of pluripotent stem cells and the reprogramming process. In addition, the aforementioned genes are essential for pluripotency and self-renewal, and the maintenance of undifferentiation in embryonic stem cells. Transcription factors expressed from these genes function to activate one another and induce differentiation while suppressing inhibitory genes for self-renewal.

Oct-4, also known as POU domain class V, is an octamer transcription factor belonging to the POU family. This protein is known to be expressed specifically in undifferentiated cells such as embryonic stem cells and embryonic tumor cells.
Further, it plays a critical role in the maintenance of pluripotency of the stem cells. If the level of Oct-4 expression is within a normal range, the self-renewal and pluripotency of stem cells are maintained. However, an expression level of Oct-4 higher than the normal range causes the stem cells to differentiate into primitive endoderm and primitive mesoderm. On the contrary, a low expression level of Oct-4 leads the stem cells to develop into trophectoderm. Thus, Oct-4 is vital for regulating pluripotency of embryonic stem cells and cell differentiation. Oct-4 was also reported to interact with Sox2 to synergically increase expression of genes in the lower stream, i.e., Fgf4, Utf-1, Nanog, and Sox2.

The genes of the Sox family relate to unipotent stem cells as well as multipotent stem cells. Among them, Sox2 (SRY-type high mobility group box 2) transcription factor is a member of the Sox family comprising an HMG box DNA binding motif. Sox 2 is the only one, among 20 proteins of the Sox family, that plays an important role in maintaining the pluripotency of embryonic stem cells. Like Oct-4, if the expression of Sox2 is inhibited, differentiation is induced in mouse embryonic stem cells. Further, the Sox2-binding site, which is found in promoter regions of several lower-stream genes of Sox2, is often present adjacent to the Oct-4- or Nanog-binding site. Thus, the interaction between Sox2 and Oct-4 transcription factors enables the reprogrammed stem cells to maintain an undifferentiated state and to show characteristics of embryonic stem cells.

c-Myc, a helix-loop-helix/leucine zipper transcription factor, is an oncogene involved in various intracellular functions such as cell growth, differentiation, proliferation, apoptosis, and transformation into cancerous cells. c-Myc is a lower stream gene in a signal delivery mechanism by LIF(leukaemia inhibitory factor)/STAT3 and Wnt, which is a main mechanism for pluripotency maintenance. c-Myc transcription factor prevents the p21 gene from inhibiting cell proliferation by being activated by the Klf4 gene in the reprogrammed stem cells. In addition, c-Myc changes the structure of chromatin by binding to the Myc recognition site present on the genome and attracting the histone acetylase complex so that Oct-4 and Sox2 can successfully bind to the target genes.

Klf4 is a transcription factor belonging to the Kruppel-like factor (Klf) family. It was used in the production of murine and human reprogrammed stem cells by the Yamanaka group and the Jaenisch group. However, given that the Thomson group reported the establishment of human reprogrammed stem cells without Klf4, this transcription factor may not be essential for the production of human reprogrammed stem cells. Klf4 is characterized by its Kruppel-type zinc-finger and participates in inhibition of growth, thereby regulating a cell cycle. It was reported that Klf4, like c-Myc, is a lower-stream gene of STAT3 in embryonic stem cells, and its overexpression maintains Oct-4 expression, thereby inhibiting differentiation of mouse embryonic stem cells. In addition, Klf4, together with Sox2, is responsible for the regulation of expression of Lefty1, which is known as a lower-stream gene of Oct-4 in embryonic stem cells and inhibits the function of the p53 tumor suppressor gene. Thus, it is thought that Klf4 indirectly enhances the activity of Nanog, which is known as an embryo-specific gene and suppresses apoptosis induced by c-Myc to induce production of reprogrammed stem cells. It has been reported that Klf2, as well as Klf4, can be employed in the production of human reprogrammed stem cells. Further, other relevant genes, i.e., Klf1 and Klf5, can also be used in the production of human reprogrammed stem cells, although they may show lower efficiency than Klf4 or Klf2.

Nanog is an embryo-specific gene which does not act through the LIF/STAT3 mechanism. Like Oct-4 or Sox2, this independent transcription factor is involved in the maintenance of pluripotency of embryonic stem cells and suppresses GATA4 and GATA6 transcription factors responsible for differentiation into endoderm.

Lastly, LIN28, which is an mRNA-binding protein, is expressed in embryonic stem cells and embryonic tumor cells and is known to be involved in differentiation and proliferation. Although Nanog and LIN28 are not indispensible in the production of reprogrammed stem cells, the Thomson group demonstrated the establishment of reprogrammed stem cells using these two genes.

In many studies currently conducted to establish reprogrammed stem cells using the six reprogramming transcription factors, the key issue is *in vivo* transduction of the transcription factors. Thus, relevant techniques are being intensively studied. Among them, transduction with a virus vector is commonly used to deliver the reprogramming transcription factors. However, the viral vector transduction technique accompanies a side effect of tumor generation. It has been reported that when a retrovirus or lentivirus encoding any of the six reprogramming transcription factors (Oct4, Sox2, c-Myc, Klf4, Nanog, and Lin28) is inserted into a somatic gene, the gene is mutated due to integration of the external genes and the inserted c-Myc oncogene generates teratoma in 25% of the chimera generated from the reprogrammed cells.

Such a method of transducing a reprogramming transcription factor using a virus or plasmid is limited in cellular therapy due to possibilities of mutagenesis and oncogenesis. However, the method of transducing a cell-permeable reprogramming transcription factor using a protein transduction method is a safe method free of insertion of external genes. Accordingly, the inventors of the present invention aimed at producing a cell permeable reprogramming transcription factor (Cell Permeable CP-TFs) by fusing a "MTD" to a reprogramming transcription factor recombinant protein.

Meanwhile, small molecules of synthetic compounds or compounds existing in nature can be transported into cells, whereas macromolecules, such as proteins, peptides, and nucleic acids, cannot due to the large molecular weight. It is widely understood that macromolecules larger than 500 kDa are incapable of penetrating a plasma membrane, i.e., the lipid bilayer structure, of living cells. In order to overcome this problem, "macromolecule intracellular transduction technology (MITT)" was developed, which allows the delivery of therapeutically effective macromolecules into cells, thereby facilitating the development of new biomolecular drugs using peptides, proteins, and genetic materials *per se.* According to this technology, a target macromolecule is fused to a "hydrophobic MTD" and other various transporters to cells, and is synthesized or expressed and purified in the form of a recombinant protein. Then, it is administered to a subject and can be delivered to a target site rapidly and accurately where its therapeutic effect is effectively shown. *See also* Korean Patent Application No. 10-2009-7017564; U.S. Patent Application No. 12/524935; Canada Patent Application No. 2,676,797; Chinese Patent Application No. 200880003468.9; Australia Patent Application No. 2008211854; India Patent Application No. 5079/CHENP/2009; European Patent Application No. 08712219.8; and Japanese Patent Application No. 2009-547177 (which claim priority based on U.S. Provisional Patent Application No. 60/887,060). As such, MTDs allow the transport of many impermeable materials into the cells by being fused to peptides, proteins, DNA, RNA, synthetic compounds, and the like,.

Accordingly, the inventors of the present invention have developed reprogramming transcription factor recombinant proteins imparted with cell permeability by fusing six types of transcription factors (Nanog, Oct4, Sox2, Klf4, c-Myc, and Lin28) to MTDs. The inventors found that these recombinant proteins can maintain pluripotency, self-renewal, and the undifferentiated state of human somatic cells, thereby inducing reprogramming safely without insertion of exogenous genes, and contemplated the present invention.

### SUMMARY OF THE INVENTION

The objective of the present invention is to provide a cell permeable reprogramming transcription factor recombinant protein which can be introduced into a cell with high efficiency and can induce pluripotency of somatic cells, whereby induced pluripotent stem cells are established.

In order to achieve the objective above, the present invention provides a cell permeable reprogramming transcription factor recombinant protein by fusing a MTD to a reprogramming transcription factor. The recombinant protein can then introduce the reprogramming transcription factor into a cell with high efficiency.

The present invention also provides a polynucleotide encoding the cell permeable reprogramming transcription factor recombinant protein above.

The present invention further provides an expression vector comprising the polynucleotide above and a transformant prepared with such expression vector.

In addition, the present invention provides a method of producing a cell permeable reprogramming transcription factor recombinant protein comprising culturing the transformant above.

Lastly, the present invention provides a cell permeable reprogramming transcription factor containing the cell permeable reprogramming transcription factor recombinant protein above as an active ingredient, which can establish induced pluripotent stem cells safely in a high yield.

### BRIEF DESCRIPTION OF THE DRAWINGS

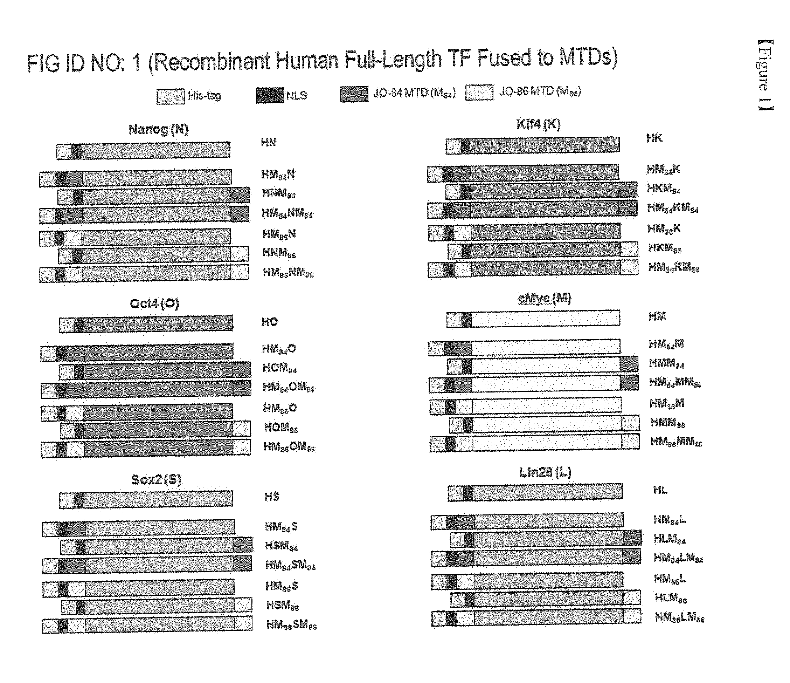

**FIG. 1** illustrates the structure of the reprogramming transcription factor recombinant proteins according to the present invention in which one of JO-84 and JO-86 MTDs is fused to each of the reprogramming transcription factors (Nanog, Oct4, Sox2, Klf4, cMyc, and Lin28).

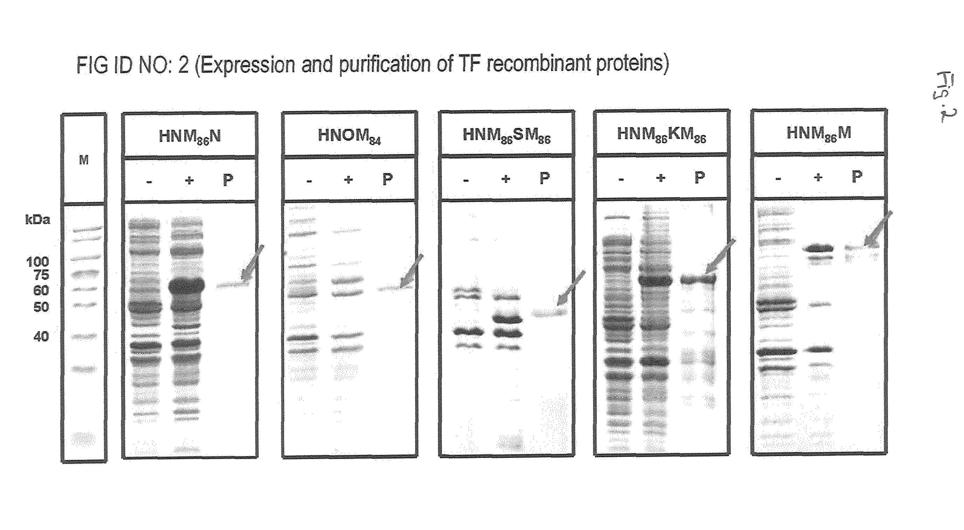

**FIG. 2** shows the results of examining the expression of the cell permeable reprogramming transcription factor recombinant proteins according to the present invention in the presence (+) and absence (-) of IPTG, a protein expression inducer.

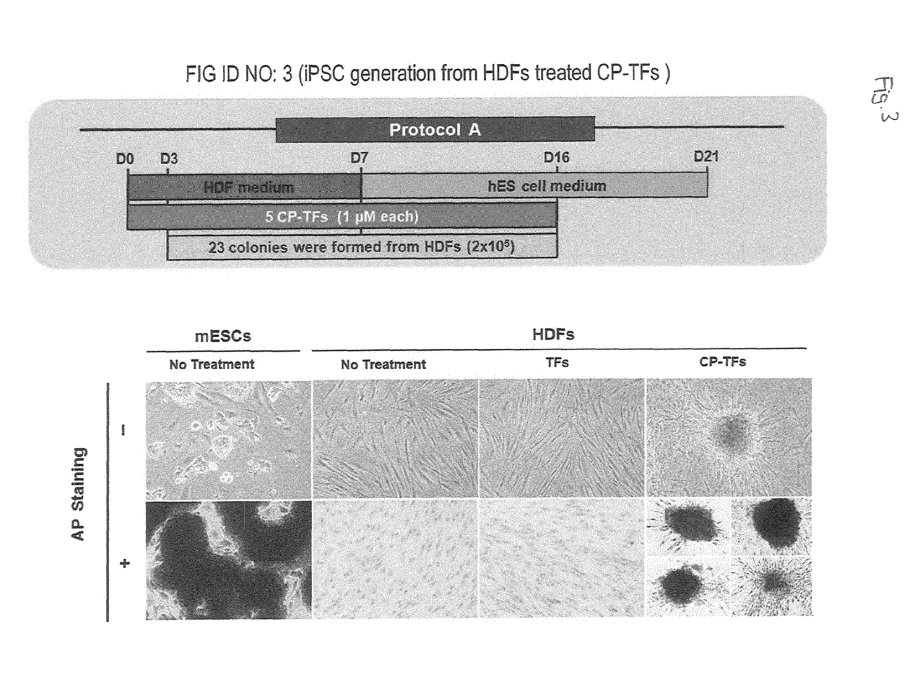

**FIG. 3** is a schematic diagram illustrating the process of making pluripotent stem cells and shows the result of alkaline phosphatase staining of cell colonies, which was obtained by treating human dermal fibroblast (HDF) cells with the recombinant protein according to the present invention, as illustrated in FIG. 3.

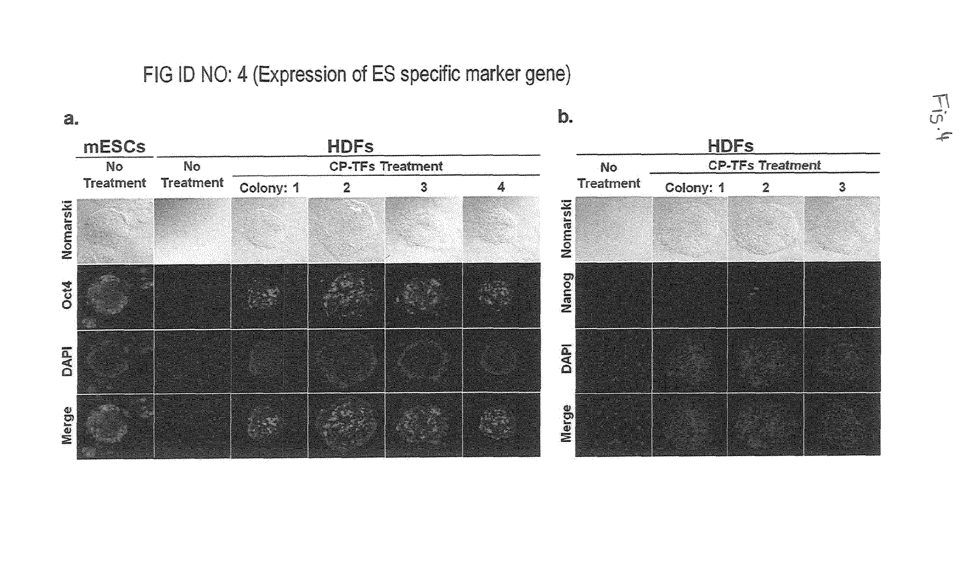

**FIG. 4** shows the result of staining of the genes expressed specifically in the nucleus of stem cells (Oct4 and Nanog) in the colonies obtained by treating human dermal fibroblast (HDF) cells with the recombinant protein according to the present invention, as illustrated in FIG. 3.

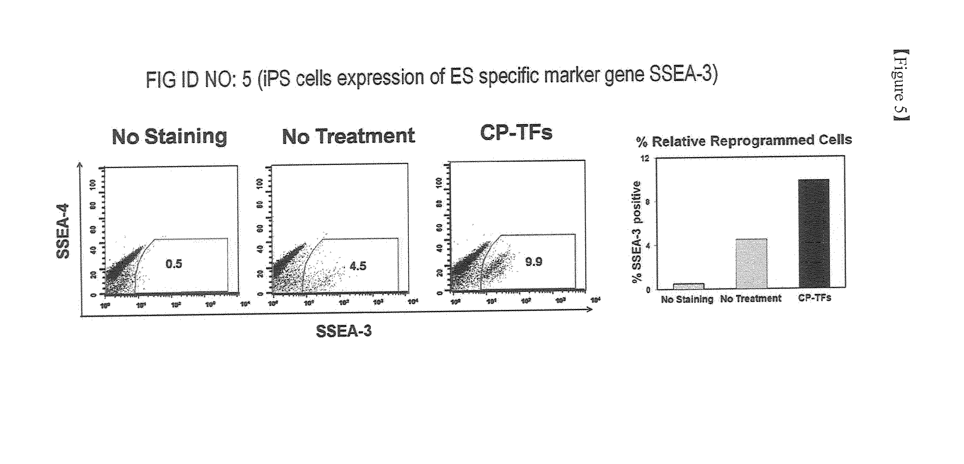

**FIG. 5** shows the result of flow cytometry analysis of the expression of a specific marker (stage-specific embryonic antigen-3: SSEA-3) on the surface of induced pluripotent stem cells obtained by treating human dermal fibroblast (HDF) cells with the recombinant protein according to the present invention, as illustrated in FIG. 3.

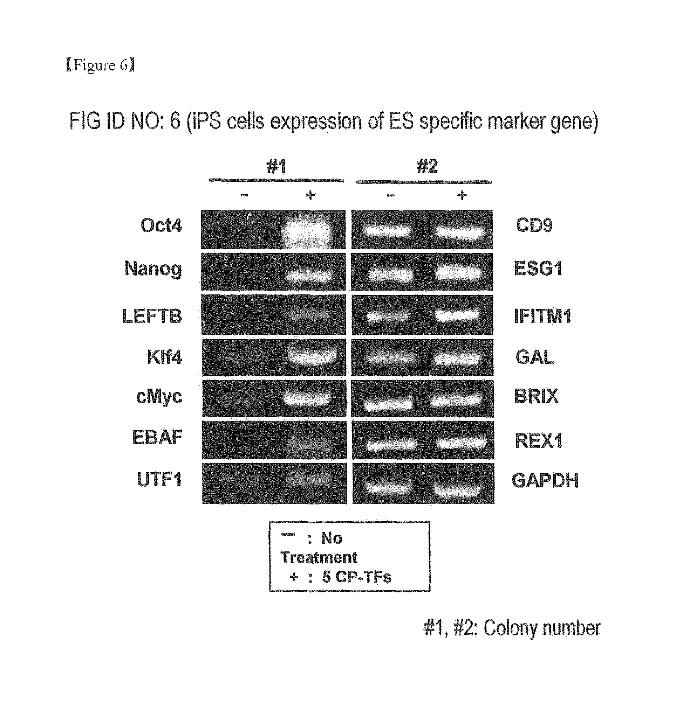

**FIG. 6** is the result of RT-PCR analysis showing the RNA expression of a stem cell specific marker in the induced pluripotent stem cells obtained by treating human dermal fibroblast (HDF) cells with the recombinant protein according to the present invention, as illustrated in FIG. 3.

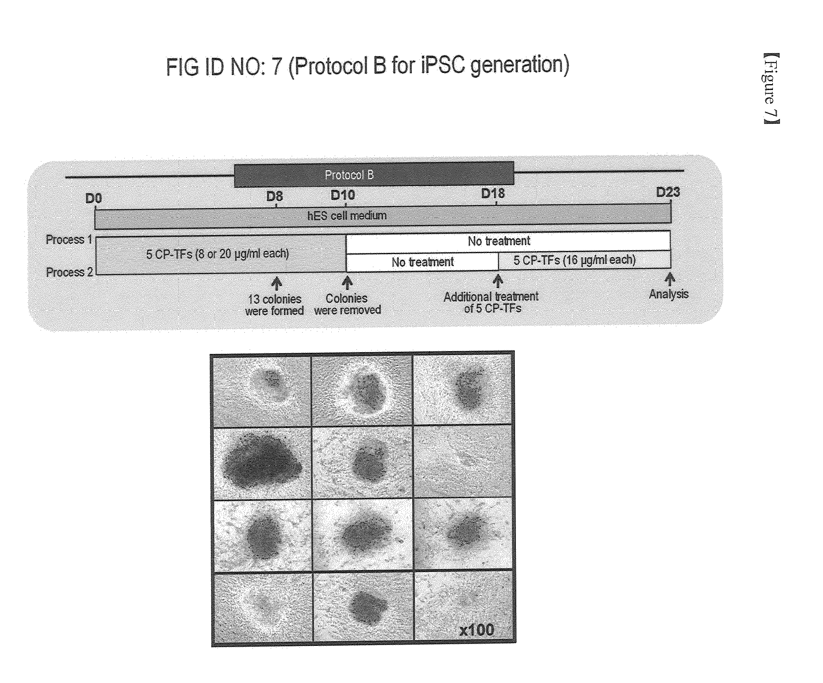

**FIG. 7** is a schematic diagram illustrating the process of making the induced pluripotent stem cells according to the present invention and a microscopic photograph visualizing the colony of the induced pluripotent stem cells obtained by treating human dermal fibroblast (HDF) cells with the recombinant protein according to the present invention, as illustrated in FIG. 7.

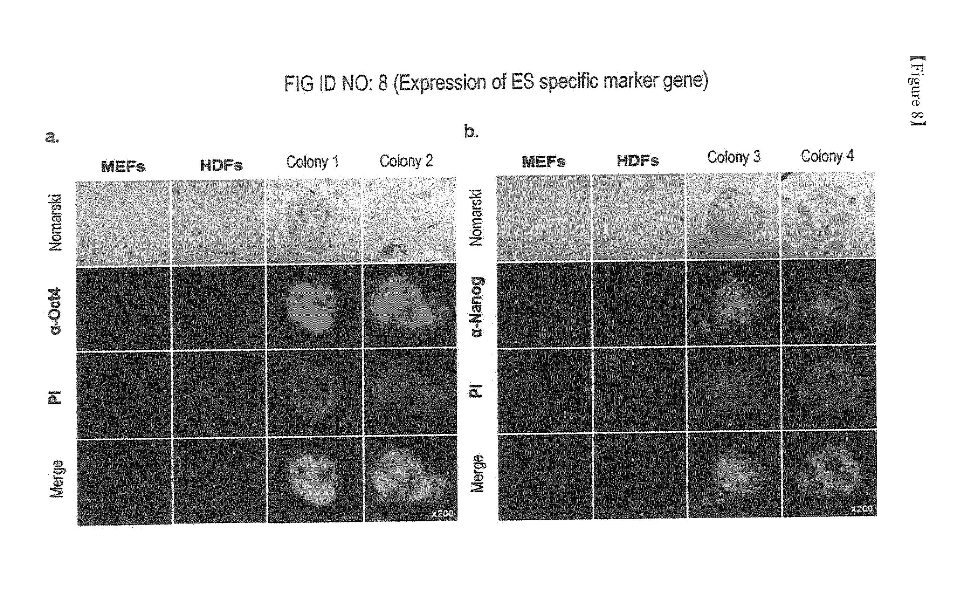

**FIG. 8** shows the result of staining of the genes expressed specifically in the nucleus of stem cells (Oct4 and Nanog) in the cell colonies obtained by treating human dermal fibroblast (HDF) cells with the recombinant protein according to the present invention, as illustrated in FIG. 7.

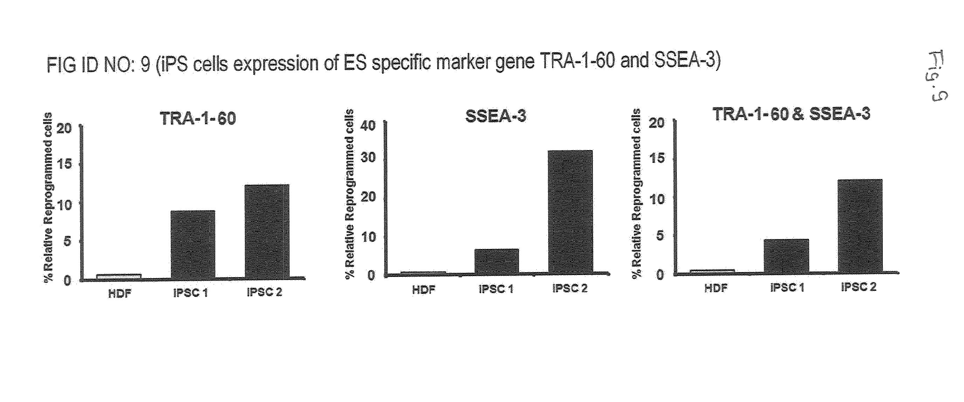

**FIG. 9** shows the result of flow cytometry analysis of the expression of two stem cell specific markers, i.e., SSEA-3 (Stage-Specific Embryonic Antigen-3) and TRA-1-60 (Tumor Rejection Antigen-1-60), on the surface of the pluripotent stem cells obtained by treating human dermal fibroblast (HDF) cells with the recombinant protein according to the present invention, as illustrated in FIG. 7.

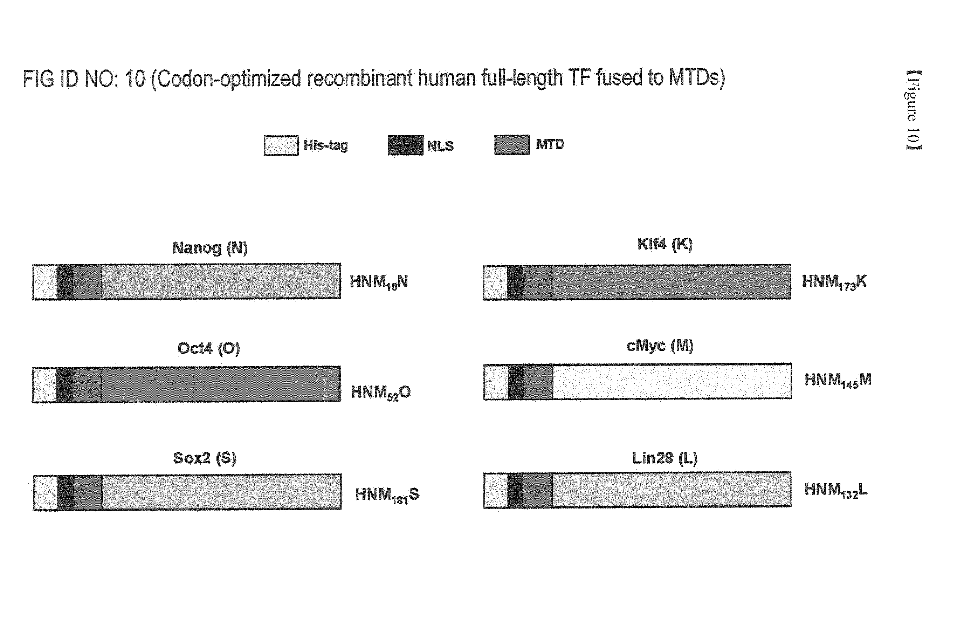

**FIG. 10** illustrates the structure of the reprogramming transcription factor recombinant proteins in which one of JO-10, JO-52, JO-132, JO-145, JO-173, and JO-181 MTDs is fused to each of the reprogramming transcription factors (Nanog, Oct4, Sox2, Klf4, cMyc, and Lin28) according to the present invention.

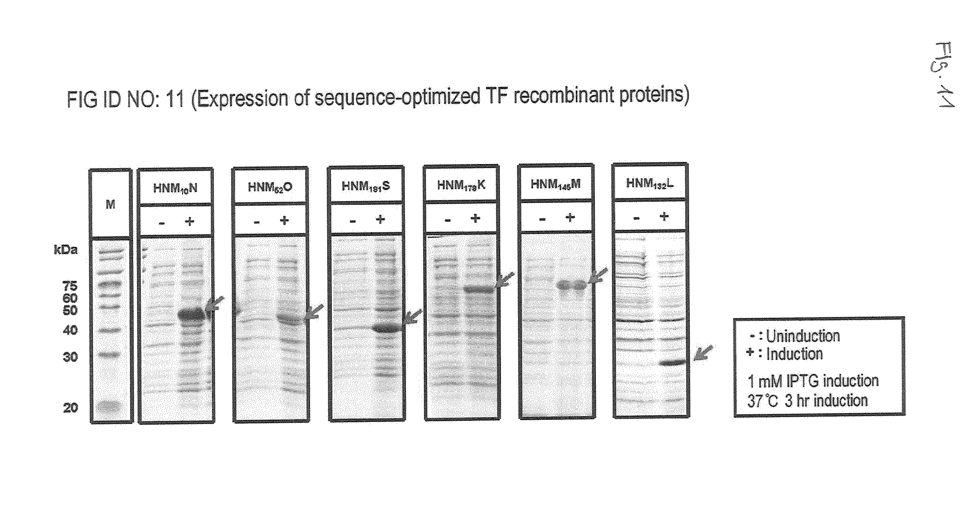

**FIG. 11** is the result of examining the expression of the reprogramming transcription factor recombinant proteins according to the present invention which had been subjected to a codon optimization to optimize the expression in the presence (+) and absence (-) of IPTG, a protein expression inducer.

### DETAILED DESCRIPTION OF THE INVENTION

The present invention provides cell permeable Nanog, Oct4, Sox2, Klf4, cMyc, and Lin28 recombinant proteins (CP-Nanog, CP-Oct4, CP-Sox2, CP-Klf4, CP-cMyc, and CP-Lin28) in which a MTD is fused to the reprogramming transcription factor, whereby the reprogramming transcription factor becomes cell permeable and can be introduced into a cell with high efficiency, and polynucleotides encoding the same.

The present invention uses proteins that acquired cell permeability by fusion of MTDs to Nanog, Oct4, Sox2, Klf4, cMyc, and Lin28 reprogramming transcription factors.

Among those, a recombinant protein in which a full length Nanog is fused to a nuclear localization sequence (NLS), i.e., NLS-Nanog (NNanog), has an amino acid sequence represented by SEQ ID NO: 20. NLS-Nanog can be encoded by, for example, a polynucleotide having a nucleotide sequence represented by SEQ ID NO: 19.

The full length Nanog recombinant proteins prepared by using a JO-84 MTD (MTD₈₄) may include the following:
NLS-MTD₈₄-Nanog (NM₈₄Nanog) has an amino acid sequence represented by SEQ ID NO: 22, and can be encoded by, for example, a polynucleotide having a nucleotide sequence represented by SEQ ID NO: 21;
NLS-Nanog-MTD₈₄ (NNanogM₈₄) has an amino acid sequence represented by SEQ ID NO: 24, and can be encoded by, for example, a polynucleotide having a nucleotide sequence represented by SEQ ID NO: 23; and
NLS-MTD₈₄-Nanog-MTD₈₄ (NM₈₄NanogM₈₄) has an amino acid sequence represented by SEQ ID NO: 26, and can be encoded by, for example, a polynucleotide having a nucleotide sequence represented by SEQ ID NO: 25.
The full-length Nanog recombinant proteins prepared by using a JO-86 MTD (MTD₈₆) may include the following:
NLS-MTD₈₆-Nanog (NM₈₆Nanog) has an amino acid sequence represented by SEQ ID NO: 28, and can be encoded by, for example, a polynucleotide having a nucleotide sequence represented by SEQ ID NO: 27;
NLS-Nanog-MTD₈₆ (NNanogM₈₆) has an amino acid sequence represented by SEQ ID NO: 30, and can be encoded by, for example, a polynucleotide having a nucleotide sequence represented by SEQ ID NO: 29; and
NLS-MTD₈₆-Nanog-MTD₈₆ (NM₈₆NanogM₈₆) has an amino acid sequence represented by SEQ ID NO: 32, and can be encoded by, for example, a polynucleotide having a nucleotide sequence represented by SEQ ID NO: 31.
In addition, the Nanog recombinant protein described above may further comprise a His-tag for easy isolation and purification.
His-NLS-Nanog (HNNanog) has an amino acid sequence represented by SEQ ID NO: 34, and can be encoded by, for example, a polynucleotide having a nucleotide sequence represented by SEQ ID NO: 33.

The full-length Nanog recombinant proteins prepared by using a JO-84 MTD (MTD₈₄) may include the following:
His-NLS-MTD₈₄-Nanog (HNM₈₄Nanog) has an amino acid sequence represented by SEQ ID NO: 36, and can be encoded by, for example, a polynucleotide having a nucleotide sequence represented by SEQ ID NO: 35;
His-NLS-Nanog-MTD₈₄ (HNNanogM₈₄) has an amino acid sequence represented by SEQ ID NO: 38, and can be encoded by, for example, a polynucleotide having a nucleotide sequence represented by SEQ ID NO: 37; and
His-NLS-MTD₈₄-Nanog-MTD₈₄ (HNM₈₄NanogM₈₄) has an amino acid sequence represented by SEQ ID NO: 40, and can be encoded by, for example, a polynucleotide having a nucleotide sequence represented by SEQ ID NO: 39.

The full-length Nanog recombinant proteins prepared by using a JO-86 MTD (MTD₈₆) may include the following:
His-NLS-MTD₈₆-Nanog (HNM₈₆Nanog) has an amino acid sequence represented by SEQ ID NO: 42, and can be encoded by, for example, a polynucleotide having a nucleotide sequence represented by SEQ ID NO: 41;
His-NLS-Nanog-MTD₈₆ (HNNanogM₈₆) has an amino acid sequence represented by SEQ ID NO: 44, and can be encoded by, for example, a polynucleotide having a nucleotide sequence represented by SEQ ID NO: 43; and
His-NLS-MTD₈₆-Nanog-MTD₈₆ (HNM₈₆NanogM₈₆) has an amino acid sequence represented by SEQ ID NO: 46, and can be encoded by, for example, a polynucleotide having a nucleotide sequence represented by SEQ ID NO: 45.
Further, His-NLS-MTD₁₀-Nanog (HNM₁₀Nanog) has an amino acid sequence represented by SEQ ID NO: 237, and can be encoded by, for example, a polynucleotide having a nucleotide sequence represented by SEQ ID NO: 236.

The full-length Oct4 recombinant proteins fused to an NLS include NLS-Oct4 (NOct4), which has an amino acid sequence represented by SEQ ID NO: 48, and can be encoded by, for example, a polynucleotide having a nucleotide sequence represented by SEQ ID NO: 47.

The full-length Oct4 recombinant proteins prepared by using a JO-84 MTD (MTD₈₄) may include the following:
NLS-MTD₈₄-Oct4 (NM₈₄Oct4) has an amino acid sequence represented by SEQ ID NO: 50, and can be encoded by, for example, a polynucleotide having a nucleotide sequence represented by SEQ ID NO: 49;
NLS-Oct4-MTD₈₄ (NOct4M₈₄) has an amino acid sequence represented by SEQ ID NO: 52, and can be encoded by, for example, a polynucleotide having a nucleotide sequence represented by SEQ ID NO: 51; and
NLS-MTD₈₄-Oct4-MTD₈₄ (NM₈₄Oct4M₈₄) has an amino acid sequence represented by SEQ ID NO: 54, and can be encoded by, for example, a polynucleotide having a nucleotide sequence represented by SEQ ID NO: 53.

The full-length Oct4 recombinant proteins prepared by using a JO-86 MTD (MTD₈₆) may include the following:
NLS-MTD₈₆-Oct4 (NM₈₆Oct4) has an amino acid sequence represented by SEQ ID NO: 56, and can be encoded by, for example, a polynucleotide having a nucleotide sequence represented by SEQ ID NO: 55;
NLS-Oct4MTD₈₆ (NOct4M₈₆) has an amino acid sequence represented by SEQ ID NO: 58, and can be encoded by, for example, a polynucleotide having a nucleotide sequence represented by SEQ ID NO: 57; and
NLS-MTD₈₆-Oct4-MTD₈₆ (NM₈₆Oct4M₈₆) has an amino acid sequence represented by SEQ ID NO: 60, and can be encoded by, for example, a polynucleotide having a nucleotide sequence represented by SEQ ID NO: 59.

In addition, the Oct4 recombinant protein described above may further comprise a His-tag for easy isolation and purification.

His-NLS-Oct4 (HNOct4) has an amino acid sequence represented by SEQ ID NO: 62, and can be encoded by, for example, a polynucleotide having a nucleotide sequence represented by SEQ ID NO: 61.

The full-length Oct4 recombinant proteins prepared by using a JO-84 MTD (MTD₈₄) may include the following:
His-NLS-MTD₈₄-Oct4 (HNM₈₄Oct4) has an amino acid sequence represented by SEQ ID NO: 64, and can be encoded by, for example, a polynucleotide having a nucleotide sequence represented by SEQ ID NO: 63;
His-NLS-Oct4-MTD₈₄ (HNOct4M₈₄) has an amino acid sequence represented by SEQ ID NO: 66, and can be encoded by, for example, a polynucleotide having a nucleotide sequence represented by SEQ ID NO: 65; and
His-NLS-MTD₈₄-Oct4-MTD₈₄ (HNM₈₄Oct4M₈₄) has an amino acid sequence represented by SEQ ID NO: 68, and can be encoded by, for example, a polynucleotide having a nucleotide sequence represented by SEQ ID NO: 67.

The full-length Oct4 recombinant proteins prepared by using a JO-86 MTD (MTD₈₆) may include the following:
His-NLS-MTD₈₆-Oct4 (HNM₈₆Oct4) has an amino acid sequence represented by SEQ ID NO: 70, and can be encoded by, for example, a polynucleotide having a nucleotide sequence represented by SEQ ID NO: 69;
His-NLS-Oct4-MTD₈₆ (HNOct4M₈₆) has an amino acid sequence represented by SEQ ID NO: 72, and can be encoded by, for example, a polynucleotide having a nucleotide sequence represented by SEQ ID NO: 71; and
His-NLS-MTD₈₆-Oct4-MTD₈₆ (HNM₈₆Oct4M₈₆ has an amino acid sequence represented by SEQ ID NO: 74, and can be encoded by, for example, a polynucleotide having a nucleotide sequence represented by SEQ ID NO: 73.

Further, His-NLS-MTD₅₂-Oct4 (HNM₅₂Oct4) has an amino acid sequence represented by SEQ ID NO: 240, and can be encoded by, for example, a polynucleotide having a nucleotide sequence represented by SEQ ID NO: 239.

The full-length Sox2 recombinant proteins fused to an NLS include NLS-Sox2 (NSox2), which has an amino acid sequence represented by SEQ ID NO: 76, and can be encoded by, for example, a polynucleotide having a nucleotide sequence represented by SEQ ID NO: 75.

The full-length Sox2 recombinant proteins prepared by using a JO-84 MTD (MTD₈₄) may include the following:
NLS-MTD₈₄-Sox2 (NM₈₄Sox2) has an amino acid sequence represented by SEQ ID NO: 78, and can be encoded by, for example, a polynucleotide having a nucleotide sequence represented by SEQ ID NO: 77;
NLS-Sox2-MTD₈₄ (NSox2M₈₄) has an amino acid sequence represented by SEQ ID NO: 80, and can be encoded by, for example, a polynucleotide having a nucleotide sequence represented by SEQ ID NO: 79; and
NLS-MTD₈₄-Sox2-MTD₈₄ (NM₈₄Sox2M₈₄) has an amino acid sequence represented by SEQ ID NO: 82, and can be encoded by, for example, a polynucleotide having a nucleotide sequence represented by SEQ ID NO: 81.

The full-length Sox2 recombinant proteins prepared by using a JO-86 MTD (MTD₈₆) may include the following:
NLS-MTD₈₆-Sox2 (NM₈₆Sox2) has an amino acid sequence represented by SEQ ID NO: 84, and can be encoded by, for example, a polynucleotide having a nucleotide sequence represented by SEQ ID NO: 83;
NLS-Sox2-MTD₈₆ (NSox2M₈₆) has an amino acid sequence represented by SEQ ID NO: 86, and can be encoded by, for example, a polynucleotide having a nucleotide sequence represented by SEQ ID NO: 85; and
NLS-MTD₈₆-Sox2-MTD₈₆ (NM₈₆Sox2M₈₆) has an amino acid sequence represented by SEQ ID NO: 88, and can be encoded by, for example, a polynucleotide having a nucleotide sequence represented by SEQ ID NO: 87.

In addition, the Sox2 recombinant proteins described above may further comprise a His-tag for easy isolation and purification.

His-NLS-Sox2 (HNSox2) has an amino acid sequence represented by SEQ ID NO: 90, and can be encoded by, for example, a polynucleotide having a nucleotide sequence represented by SEQ ID NO: 89.

The full-length Sox2 recombinant proteins prepared by using a JO-84 MTD (MTD₈₄) may include the following:
His-NLS-MTD₈₄-Sox2 (HNM₈₄Sox2) has an amino acid sequence represented by SEQ ID NO: 92, and can be encoded by, for example, a polynucleotide having a nucleotide sequence represented by SEQ ID NO: 91;
His-NLS-Sox2-MTD₈₄ (HNSox2M₈₄) has an amino acid sequence represented by SEQ ID NO: 94, and can be encoded by, for example, a polynucleotide having a nucleotide sequence represented by SEQ ID NO: 93; and
His-NLS-MTD₈₄-Sox2-MTD₈₄ (HNM₈₄Sox2M₈₄) has an amino acid sequence represented by SEQ ID NO: 96, and can be encoded by, for example, a polynucleotide having a nucleotide sequence represented by SEQ ID NO: 95.

The full-length Sox2 recombinant proteins prepared by using a JO-86 MTD (MTD₈₆) may include the following:,
His-NLS-MTD₈₆-Sox2 (HNM₈₆Sox2) has an amino acid sequence represented by SEQ ID NO: 98, and can be encoded by, for example, a polynucleotide having a nucleotide sequence represented by SEQ ID NO: 97;
His-NLS-Sox2-MTD₈₆ (HNSox2M₈₆) has an amino acid sequence represented by SEQ ID NO: 100, and can be encoded by, for example, a polynucleotide having a nucleotide sequence represented by SEQ ID NO: 99; and
His-NLS-MTD₈₆-Sox2-MTD₈₆(HNM₈₆Sox2M₈₆) has an amino acid sequence represented by SEQ ID NO: 102, and can be encoded by, for example, a polynucleotide having a nucleotide sequence represented by SEQ ID NO: 101.

Further, His-NLS-MTD₁₈₁-Sox2 (HNM₁₈₁Sox2) has an amino acid sequence represented by SEQ ID NO: 243, and can be encoded by, for example, a polynucleotide having a nucleotide sequence represented by SEQ ID NO: 242.

The full length Klf4 recombinant proteins fused to an NLS include NLS-Klf4 (NK1f4), which has an amino acid sequence represented by SEQ ID NO: 104, and can be encoded by, for example, a polynucleotide having a nucleotide sequence represented by SEQ ID NO: 103.

The full length Klf4 recombinant proteins prepared by using a JO-84 MTD (MTD₈₄) may include the following:
NLS-MTD₈₄-Klf4 (NM₈₄Klf4) has an amino acid sequence represented by SEQ ID NO: 106, and can be encoded by, for example, a polynucleotide having a nucleotide sequence represented by SEQ ID NO: 105;
NLS-Klf4-MTD₈₄ (NKlf4M₈₄) has an amino acid sequence represented by SEQ ID NO: 108, and can be encoded by, for example, a polynucleotide having a nucleotide sequence represented by SEQ ID NO: 107; and
NLS-MTD₈₄-Klf4-MTD₈₄ (NM₈₄K1f4M₈₄) has an amino acid sequence represented by SEQ ID NO: 110, and can be encoded by, for example, a polynucleotide having a nucleotide sequence represented by SEQ ID NO: 109.

The full length Klf4 recombinant proteins prepared by using a JO-86 MTD (MTD₈₆) may include the following:
NLS-MTD₈₆-Klf4 (NM₈₆Klf4) has an amino acid sequence represented by SEQ ID NO: 112, and can be encoded by, for example, a polynucleotide having a nucleotide sequence represented by SEQ ID NO: 111;
NLS-Klf4-MTD₈₆ (NKlf4M₈₆) has an amino acid sequence represented by SEQ ID NO: 114, and can be encoded by, for example, a polynucleotide having a nucleotide sequence represented by SEQ ID NO: 113; and
NLS-MTD₈₆-Klf4-MTD₈₆ (NM₈₆Klf4M₈₆) has an amino acid sequence represented by SEQ ID NO: 116, and can be encoded by, for example, a polynucleotide having a nucleotide sequence represented by SEQ ID NO: 115.

In addition, the Klf4 recombinant proteins described above may further comprise a His-tag for easy isolation and purification.

His-NLS-Klf4 (HNKlf4) has an amino acid sequence represented by SEQ ID NO: 118, and can be encoded by, for example, a polynucleotide having a nucleotide sequence represented by SEQ ID NO: 117.

The full length Klf4 recombinant proteins prepared by using a JO-84 MTD (MTD₈₄) may include the following:
His-NLS-MTD₈₄-Klf4 (HNM₈₄Klf4) has an amino acid sequence represented by SEQ ID NO: 120, and can be encoded by, for example, a polynucleotide having a nucleotide sequence represented by SEQ ID NO: 119;
His-NLS-Klf4-MTD₈₄ (HNKlf4M₈₄) has an amino acid sequence represented by SEQ ID NO: 122, and can be encoded by, for example, a polynucleotide having a nucleotide sequence represented by SEQ ID NO: 121; and
His-NLS-MTD₈₄-Klf4-MTD₈₄ (HNM₈₄Klf4M₈₄) has an amino acid sequence represented by SEQ ID NO: 124, and can be encoded by, for example, a polynucleotide having a nucleotide sequence represented by SEQ ID NO: 123.

The full length Klf4 recombinant proteins prepared by using a JO-86 MTD (MTD₈₆) may include the following:
His-NLS-MTD₈₆-Klf4 (HNM₈₆Klf4) has an amino acid sequence represented by SEQ ID NO: 126, and can be encoded by, for example, a polynucleotide having a nucleotide sequence represented by SEQ ID NO: 125;
His-NLS-Klf4-MTD₈₆ (HNKlf4M₈₆) has an amino acid sequence represented by SEQ ID NO: 128, and can be encoded by, for example, a polynucleotide having a nucleotide sequence represented by SEQ ID NO: 127; and
His-NLS-MTD₈₆-Klf4-MTD₈₆ (HNM₈₆Klf4M₈₆) has an amino acid sequence represented by SEQ ID NO: 130, and can be encoded by, for example, a polynucleotide having a nucleotide sequence represented by SEQ ID NO: 129.

Further, His-NLS-MTD₁₇₃-Klf4 (HNM₁₇₃Klf4) has an amino acid sequence represented by SEQ ID NO: 246, and can be encoded by, for example, a polynucleotide having a nucleotide sequence represented by SEQ ID NO: 245.

The full length cMyc recombinant proteins fused to a NLS include NLS-cMyc (NcMyc), which has an amino acid sequence represented by SEQ ID NO: 132, and can be encoded by, for example, a polynucleotide having a nucleotide sequence represented by SEQ ID NO: 131.

The full length cMyc recombinant proteins prepared by using a JO-84 MTD (MTD₈₄) may include the following:
NLS-MTD₈₄-cMyc (NM₈₄cMyc) has an amino acid sequence represented by SEQ ID NO: 134, and can be encoded by, for example, a polynucleotide having a nucleotide sequence represented by SEQ ID NO: 133;
NLS-cMyc-MTD₈₄ (NcMycM₈₄) has an amino acid sequence represented by SEQ ID NO: 136, and can be encoded by, for example, a polynucleotide having a nucleotide sequence represented by SEQ ID NO: 135; and
NLS-MTD₈₄-cMyc-MTD₈₄ (NM₈₄cMycM₈₄) has an amino acid sequence represented by SEQ ID NO: 138, and can be encoded by, for example, a polynucleotide having a nucleotide sequence represented by SEQ ID NO: 137.

The full length cMyc recombinant proteins prepared by using a JO-86 MTD (MTD₈₆) may include the following:
NLS-MTD₈₆-cMyc (NM₈₆cMyc) has an amino acid sequence represented by SEQ ID NO: 140, and can be encoded by, for example, a polynucleotide having a nucleotide sequence represented by SEQ ID NO: 139;
NLS-cMyc-MTD₈₆ (NcMycM₈₆) has an amino acid sequence represented by SEQ ID NO: 142, and can be encoded by, for example, a polynucleotide having a nucleotide sequence represented by SEQ ID NO: 141; and
NLS-MTD₈₆-cMyc-MTD₈₆ (NM₈₆cMycM₈₆) has an amino acid sequence represented by SEQ ID NO: 144, and can be encoded by, for example, a polynucleotide having a nucleotide sequence represented by SEQ ID NO: 143.

In addition, the cMyc recombinant proteins described above may further comprise a His-tag for easy isolation and purification.

His-NLS-cMyc (HNcMyc) has an amino acid sequence represented by SEQ ID NO: 146, and can be encoded by, for example, a polynucleotide having a nucleotide sequence represented by SEQ ID NO: 145.

The full length the cMyc recombinant proteins prepared by using a JO-84 MTD (MTD₈₄) may include the following:
His-NLS-MTD₈₄-cMyc (HNM₈₄cMyc) has an amino acid sequence represented by SEQ ID NO: 148, and can be encoded by, for example, a polynucleotide having a nucleotide sequence represented by SEQ ID NO: 147;
His-NLS-cMyc-MTD₈₄ (HNcMycM₈₄) has an amino acid sequence represented by SEQ ID NO: 150, and can be encoded by, for example, a polynucleotide having a nucleotide sequence represented by SEQ ID NO: 149; and
His-NLS-MTD₈₄-cMye-MTD₈₄ (HNM₈₄cMycM₈₄) has an amino acid sequence represented by SEQ ID NO: 152, and can be encoded by, for example, a polynucleotide having a nucleotide sequence represented by SEQ ID NO: 151.

The full length cMyc recombinant proteins prepared by using a JO-86 MTD (MTD₈₆) may include the following:
His-NLS-MTD₈₆-cMyc (HNM₈₆cMyc) has an amino acid sequence represented by SEQ ID NO: 154, and can be encoded by, for example, a polynucleotide having a nucleotide sequence represented by SEQ ID NO: 153;
His-NLS-cMyc-MTD₈₆ (HNcMycM₈₆) has an amino acid sequence represented by SEQ ID NO: 156, and can be encoded by, for example, a polynucleotide having a nucleotide sequence represented by SEQ ID NO: 155; and
His-NLS-MTD₈₆-cMyc-MTD₈₆ (HNM₈₆cMycM₈₆) has an amino acid sequence represented by SEQ ID NO: 158, and can be encoded by, for example, a polynucleotide having a nucleotide sequence represented by SEQ ID NO: 157.

Further, His-NLS-MTD₁₄₅-cMyc (HNM₁₄₅cMyc) has an amino acid sequence represented by SEQ ID NO: 249, and can be encoded by, for example, a polynucleotide having a nucleotide sequence represented by SEQ ID NO: 248.

The full length Lin28 recombinant proteins fused to an NLS may include NLS-Lin28 (NLin28), which has an amino acid sequence represented by SEQ ID NO: 160, and can be encoded by, for example, a polynucleotide having a nucleotide sequence represented by SEQ ID NO: 159.

The full length Lin28 recombinant proteins prepared by using a JO-84 MTD (MTD₈₄) may include the following:
NLS-MTD₈₄-Lin28 (NM₈₄Lin28) has an amino acid sequence represented by SEQ ID NO: 162, and can be encoded by, for example, a polynucleotide having a nucleotide sequence represented by SEQ ID NO: 161;
NLS-Lin28-MTD₈₄ (NLin28M₈₄) has an amino acid sequence represented by SEQ ID NO: 164, and can be encoded by, for example, a polynucleotide having a nucleotide sequence represented by SEQ ID NO: 163; and
NLS-MTD₈₄-Lin28-MTD₈₄ (NM₈₄Lin28M₈₄) has an amino acid sequence represented by SEQ ID NO: 166, and can be encoded by, for example, a polynucleotide having a nucleotide sequence represented by SEQ ID NO: 165.

The full length Lin28 recombinant proteins prepared by using a JO-86 MTD (MTD₈₆) may include the following:
NLS-MTD₈₆-Lin28 (NM₈₆Lin28) has an amino acid sequence represented by SEQ ID NO: 168, and can be encoded by, for example, a polynucleotide having a nucleotide sequence represented by SEQ ID NO: 167;
NLS-Lin28-MTD₈₆ (NLin28M₈₆) has an amino acid sequence represented by SEQ ID NO: 170, and can be encoded by, for example, a polynucleotide having a nucleotide sequence represented by SEQ ID NO: 169; and
NLS-MTD₈₆-Lin28-MTD₈₆ (NM₈₆Lin28M₈₆) has an amino acid sequence represented by SEQ ID NO: 172, and can be encoded by, for example, a polynucleotide having a nucleotide sequence represented by SEQ ID NO: 171.
In addition, the Lin28 recombinant proteins described above may further comprise a His-tag for easy isolation and purification.
His-NLS-Lin28 (HNLin28) has an amino acid sequence represented by SEQ ID NO: 174, and can be encoded by, for example, a polynucleotide having a nucleotide sequence represented by SEQ ID NO: 173.

The full length Lin28 recombinant proteins prepared by using a JO-84 MTD (MTD₈₄) may include the following:
His-NLS-MTD₈₄-Lin28 (HNM₈₄Lin28) has an amino acid sequence represented by SEQ ID NO: 176, and can be encoded by, for example, a polynucleotide having a nucleotide sequence represented by SEQ ID NO: 175;
His-NLS-Lin28-MTD₈₄ (HNLin28M₈₄) has an amino acid sequence represented by SEQ ID NO: 178, and can be encoded by, for example, a polynucleotide having a nucleotide sequence represented by SEQ ID NO: 177; and
His-NLS-MTD₈₄-Lin28-MTD₈₄ (HNM₈₄Lin28M₈₄) has an amino acid sequence represented by SEQ ID NO: 180, and can be encoded by, for example, a polynucleotide having a nucleotide sequence represented by SEQ ID NO: 179.

The full length the Lin28 recombinant proteins prepared by using a JO-86 MTD (MTD₈₆) may include the following:
His-NLS-MTD₈₆-Lin28 (HNM₈₆Lin28) has an amino acid sequence represented by SEQ ID NO: 182, and can be encoded by, for example, a polynucleotide having a nucleotide sequence represented by SEQ ID NO: 181;
His-NLS-Lin28-MTD₈₆ (HNLin28M₈₆) has an amino acid sequence represented by SEQ ID NO: 184, and can be encoded by, for example, a polynucleotide having a nucleotide sequence represented by SEQ ID NO: 183; and
His-NLS-MTD₈₆-Lin28-MTD₈₆ (HNM₈₆Lin28M₈₆) has an amino acid sequence represented by SEQ ID NO: 186, and can be encoded by, for example, a polynucleotide having a nucleotide sequence represented by SEQ ID NO: 185.

Further, His-NLS-MTD₁₃₂-Lin28 (HNM₁₃₂Lin28) has an amino acid sequence represented by SEQ ID NO: 252, and can be encoded by, for example, a polynucleotide having a nucleotide sequence represented by SEQ ID NO: 251.

The present invention is characterized by providing a reprogramming transcription factor, which is a macromolecule incapable of being introduced into a cell, with cell permeability by fusing it to a specific macromolecule transduction domain (hereinafter, "MTD") by MITT. Thus, the reprogramming transcription factor can be transported into a cell with high efficiency. The MTD may be fused to either one or both termini of the reprogramming transcription factor. MITT utilizing a hydrophobic polypeptide MTD derived from a secreted protein enables a cargo protein to penetrate through cell membranes by treatment of the protein only. Thus, the protein can be delivered into the nucleus.

Using this effect, reprogramming transcription factors that are not expressed in somatic cells can be delivered into cells. Thus, induced pluripotent stem cells can be safely established without insertion of exogenous genetic materials, such as viruses and plasmids, as in prior art.

The present invention has developed cell permeable reprogramming transcription factor recombinant proteins that are constructed by fusing each of the reprogramming transcription factors to a peptide domain capable of transporting a macromolecule into a cell, i.e., MTD.

The term "cell permeable reprogramming transcription factor recombinant protein" used herein refers to a covalently linked complex comprising an MTD and the reprogramming transcription factor, where they are linked by genetic fusion or chemical coupling. The term "genetic fusion" used herein refers to a linear, covalent linkage of proteins generated through genetic expression of a polynucleotide (DNA sequence) molecule encoding the proteins.

Nanog, a transcription factor capable of reprogramming somatic cells having a nucleotide sequence represented by SEQ ID NO: 1 and an amino acid sequence represented by SEQ ID NO: 2, maintains pluripotency of embryonic cells and inhibits differentiation of cells into an endoderm. This reprogramming transcription factor is composed of a domain consisting of amino acid residues 1 to 306 of the amino acid sequence of SEQ ID NO: 2.

Oct4, a transcription factor capable of reprogramming somatic cells having a nucleotide sequence represented by SEQ ID NO: 3 and an amino acid sequence represented by SEQ ID NO: 4, maintains pluripotency and regulates self-renewal of stem cells. This reprogramming transcription factor is composed of a domain consisting of amino acid residues 1 to 361 of the amino acid sequence of SEQ ID NO: 4.

Sox2, a transcription factor capable of reprogramming somatic cells having a nucleotide sequence represented by SEQ ID NO: 5 and an amino acid sequence represented by SEQ ID NO: 6, maintains pluripotency and regulates the undifferentiated state of stem cells. This reprogramming transcription factor is composed of a domain consisting of amino acid residues 1 to 318 of the amino acid sequence of SEQ ID NO: 6.

Klf4 is a transcription factor capable of reprogramming somatic cells having a nucleotide sequence represented by SEQ ID NO: 7 and an amino acid sequence represented by SEQ ID NO: 8. This reprogramming transcription factor induces reprogramming by inhibiting proliferation and regulating cell cycle. Klf4 is composed of a domain consisting of amino acid residues 1 to 471 of the amino acid sequence of SEQ ID NO: 8.

cMyc is a transcription factor capable of reprogramming somatic cells having a nucleotide sequence represented by SEQ ID NO: 9 and an amino acid sequence represented by SEQ ID NO: 10. This reprogramming transcription factor induces reprogramming by regulating cell growth, proliferation, apoptosis, and cell cycle. cMyc is composed of a domain consisting of amino acid residues 1 to 456 of the amino acid sequence of SEQ ID NO: 10.

Lin28 is a transcription factor capable of reprogramming somatic cells having a nucleotide sequence represented by SEQ ID NO: 11 and an amino acid sequence represented by SEQ ID NO: 12. This reprogramming transcription factor functions to regulate differentiation and proliferation of stem cells. Lin28 is composed of a domain consisting of amino acid residues 1 to 210 of the amino acid sequence of SEQ ID NO: 12.

As the MTD capable of being fused to the six types of reprogramming transcription factors, a cell permeable peptide having a nucleotide sequence represented by SEQ ID NO: 15 and an amino acid sequence represented by SEQ ID NO: 16 may be used.

As the MTD capable of being fused to the six types of reprogramming transcription factors, a cell permeable peptide having a nucleotide sequence represented by SEQ ID NO: 17 and an amino acid sequence represented by SEQ ID NO: 18 may be used.

As the MTD capable of being fused to the Nanog reprogramming transcription factor, a cell permeable peptide having a nucleotide sequence represented by SEQ ID NO: 223 and an amino acid sequence represented by SEQ ID NO: 224 may be used.

As the MTD capable of being fused to the Oct4 reprogramming transcription factor, a cell permeable peptide having a nucleotide sequence represented by SEQ ID NO: 225 and an amino acid sequence represented by SEQ ID NO: 226 may be used.

As the MTD capable of being fused to the Sox2 reprogramming transcription factor, a cell permeable peptide having a nucleotide sequence represented by SEQ ID NO: 233 and an amino acid sequence represented by SEQ ID NO: 234 may be used.

As the MTD capable of being fused to the Klf4 reprogramming transcription factor, a cell permeable peptide having a nucleotide sequence represented by SEQ ID NO: 231 and an amino acid sequence represented by SEQ ID NO: 232 may be used.

As the MTD capable of being fused to the cMyc reprogramming transcription factor, a cell permeable peptide having a nucleotide sequence represented by SEQ ID NO: 229 and an amino acid sequence represented by SEQ ID NO: 230 may be used.

As the MTD capable of being fused to the Lin28 reprogramming transcription factor, a cell permeable peptide having a nucleotide sequence represented by SEQ ID NO: 227 and an amino acid sequence represented by SEQ ID NO: 228 may be used.

MTDs having any one of the amino acid sequences represented by SEQ ID NOS: 16, 18, 224, 226, 228, 230, 232, or 234 described above are cell permeable polypeptides capable of mediating the transport of a biologically active molecule, such as a polypeptide, a protein domain, or a full-length protein, across the cell membrane.

The MTDs according to the present invention are designed to include a hydrophobic region having cell membrane-targeting activity by forming a helix which is derived from a signal peptide comprising three regions, i.e., an N-terminal region, a hydrophobic region, and a C-terminal region containing a secreted protein cleavage site. These MTDs can directly penetrate the cell membrane, while avoiding any cell damage, and deliver a target protein into a cell for it to exhibit its desired function.

The MTDs having any one of the amino acid sequences represented by SEQ ID NOS: 16, 18, 224, 226, 228, 230, 232, or 234 to be fused to the reprogramming transcription factors according to the present invention are summarized in Table 1 below.

**[Table 1]**

| **ID** | **Origin** | **Sequence** | **Length** | **Induction** | **Purification** | **Cell Permeability** |
|---|---|---|---|---|---|---|
| **JO-84** | ***Streptomyces coelicolor*** | **LVAALLAVL** | **9** | ++++ | ++++ | **1.9** |
| **JO-86** | ***Mycobacterium bovis*** | **LAVLAAAP** | **8** | ++++ | ++++ | **3.7** |
| **JO-10** | ***Streptomyces coelicolor*** | LGGAVVAAP VAAAVAP | **16** | ++++ | ++++ | **0.8** |
| **JO-52** | ***Homo sapiens*** | **PLLLLLPAL** | **9** | ++++ | ++++ | **1.6** |
| **JO-132** | ***Streptomyces coelicolor*** | AVVVPAIVL AAP | **12** | ++ | ++ | **1.3** |
| **JO-145** | ***Supravalvular aortic stenosis*** | AAAPVLLLL L | **10** | ++++ | ++++ | **1.3** |
| **JO-173** | ***Streptomyces coelicolor*** | **AVIPILAVP** | **9** | ++++ | ++++ | **3.7** |
| **JO-181** | ***Neisseria meningitidis*** | **AVLLLPAAA** | **9** | ++++ | ++++ | **3.5** |

The cell permeable reprogramming transcription factor recombinant proteins according to the present invention may have a structure where one of the eight MTDs above is fused to either one or both termini of the reprogramming transcription factor, and optionally, a nuclear localization sequence (NLS) derived from SV40 large T antigen or a histidine-tag (His-Tag) affinity domain can be fused to one terminus of this fused construct for easy purification.

The NLS that can be fused to the reprogramming transcription factors may include, but is not limited to, a polypeptide having an amino acid sequence represented by SEQ ID NO: 14 or encoded by a nucleotide represented by SEQ ID NO: 13. Any known NLS may be used.

In one embodiment of the present invention, one of the following MTDs is used for fusion with a reprogramming transcription factor: a JO-84 MTD having the amino acid sequence represented by SEQ ID NO: 16, which is derived from *Streptomyces coelicolor* (hereinafter, "MTD₈₄"); a JO-86 MTD having the amino acid sequence represented by SEQ ID NO: 18, which is a secreted protein derived from *Mycobacterium bovis* (hereinafter, "MTD₈₆"); a JO-10 MTD having the amino acid sequence represented by SEQ ID NO: 224, which is derived from *Streptomyces coelicolor* (hereinafter, "MTD₁₀"); a JO-52 MTD having the amino acid sequence represented by SEQ ID NO: 226, which is derived from *Homo Sapiens* (hereinafter, "MTD₅₂"); a JO-132 MTD having the amino acid sequence represented by SEQ ID NO: 228, which is derived from *Streptomyces coelicolor* (hereinafter, "MTD₁₃₂"); a JO-145 MTD having the amino acid sequence represented by SEQ ID NO: 230, which is derived from *Homo Sapiens Elastin* (hereinafter, "MTD₁₄₅"); a JO-173 MTD having the amino acid sequence represented by SEQ ID NO: 232, which is derived from *Streptomyces coelicolor* (hereinafter, "MTD₁₇₃"); and a JO-181 MTD having the amino acid sequence represented by SEQ ID NO: 234, which is derived from *Neisseria meningitidis* (hereinafter, "MTD₁₈₁").

In a preferred embodiment of the present invention, two full-length forms of reprogramming transcription factor recombinant proteins using a JO-84 MTD or a JO-86 MTD are designed for each MTD.

Referring to FIGS. 1 and 10, the Nanog recombinant protein according to the present invention may be:
1) His-NLS-MTD₈₄-Nanog (HNM₈₄Nanog), wherein His-NLS and a JO-84 MTD are fused to the N-terminus of Nanog;
2) His-NLS-Nanog-MTD₈₄ (HNNanogM₈₄), wherein His-NLS is fused to the N-terminus and a JO-84 MTD is fused to the C-terminus of Nanog;
3) His-NLS-MTD₈₄-Nanog-MTD₈₄ (HNM₈₄NanogM₈₄), wherein His-NLS-JO-84 MTD is fused to the N-terminus and a JO-84 MTD is fused to the C-terminus of Nanog;
4) His-NLS-MTD₈₆-Nanog (HNM₈₆Nanog), wherein His-NLS and a JO-86 MTD are fused to the N-terminus of Nanog;
5) His-NLS-Nanog-MTD₈₆ (HNNanogM₈₆), wherein His-NLS is fused to the N-terminus and a JO-86 MTD is fused to the C-terminus of Nanog;
6) His-NLS-MTD₈₆-Nanog-MTD₈₆ (HNM₈₆NanogM₈₆), wherein His-NLS-JO-86 MTD is fused to the N-terminus and a JO-86 MTD is fused to the C-terminus of Nanog; or
7) His-NLS-MTD₁₀-Nanog (HNM₁₀Nanog), wherein His-NLS-JO-10 MTD is fused to the N-terminus of Nanog.

Optionally, the recombinant proteins 1) to 7) above may not include His-Tag.

Referring to FIGS. 1 and 10, the Oct4 recombinant proteins of the present invention may be:
1) His-NLS-MTD₈₄-Oct4 (HNM₈₄Oct4), wherein His-NLS and JO-84 MTD are fused to the N-terminus of Oct4;
2) His-NLS-Oct4-MTD₈₄ (HNOct4M₈₄), wherein His-NLS is fused to the N-terminus and a JO-84 MTD is fused to the C-terminus of Oct4;
3) His-NLS-MTD₈₄-Oct4-MTD₈₄ (HNM₈₄Oct4M₈₄), wherein His-NLS-JO-84 MTD is fused to the N-terminus and a JO-84 MTD is fused to the C-terminus of Oct4;
4) His-NLS-MTD₈₆-Oct4 (HNM₈₆Oct4) wherein His-NLS and a JO-86 MTD are fused to the N-terminus of Oct4;
5) His-NLS-Oct4-MTD₈₆(HNOct4M₈₆), wherein His-NLS is fused to the N-terminus and a JO-86 MTD is fused to the C-terminus of Oct4;
6) His-NLS-MTD₈₆-Oct4-MTD₈₆ (HNM₈₆Oct4M₈₆), wherein His-NLS-JO-86 MTD is fused to the N-terminus and a JO-86 MTD is fused to the C-terminus of Oct4; or
7) His-NLS-MTD₅₂-Oct4 (HNM₅₂Oct4) wherein His-NLS and a JO-52 MTD are fused to the N-terminus of Oct4.

Optionally, the recombinant proteins 1) to 7) above may not include His-Tag.

Referring to FIGS. 1 and 10, the Sox2 recombinant proteins of the present invention may be:
1) His-NLS-MTD₈₄-Sox2 (HNM₈₄Sox2), wherein a His-NLS and a JO-84 MTD are fused to the N-terminus of Sox2;
2) His-NLS-Sox2-MTD₈₄ (HNSox2M₈₄), wherein a His-NLS is fused to the N-terminus and a JO-84 MTD is fused to the C-terminus of Sox2;
3) His-NLS-MTD₈₄-Sox2-MTD₈₄ (HNM₈₄Sox2M₈₄), wherein a His-NLS-JO-84 MTD is fused to the N-terminus and a JO-84 MTD is fused to the C-terminus of Sox2;
4) His-NLS-MTD₈₆-Sox2 (HNM₈₆Sox2), wherein a His-NLS and a JO-86 MTD are fused to the N-terminus of Sox2;
5) His-NLS-Sox2-MTD₈₆ (HNSox2M₈₆), wherein a His-NLS is fused to the N-terminus and a JO-86 MTD is fused to the C-terminus of Sox2;
6) His-NLS-MTD₈₆-Sox2-MTD₈₆ (HNM₈₆Sox2M₈₆), wherein a His-NLS-JO-86 MTD is fused to the N-terminus and a JO-86 MTD is fused to the C-terminus of Sox2; or
7) His-NLS-MTD₁₈₁-Sox2(HNM₁₈₁Sox2), wherein a His-NLS is fused to the N-terminus and a JO-181 MTD is fused to the C-terminus of Sox2.

Optionally, the recombinant proteins 1) to 7) above may not include His-Tag.

Referring to FIGS. 1 and 10, the Klf4 recombinant proteins of the present invention may be:
1) His-NLS-MTD₈₄-Klf4 (HNM₈₄Klf4), wherein a His-NLS and a JO-84 MTD are fused to the N-terminus of Klf4;
2) His-NLS-Klf4-MTD₈₄ (HNKlf4M₈₄), wherein a His-NLS is fused to the N-terminus and a JO-84 MTD is fused to the C-terminus of Klf4;
3) His-NLS-MTD₈₄-Klf4-MTD₈₄ (HNM₈₄Klf4M₈₄), wherein a His-NLS-JO-84 MTD is fused to the N-terminus and a JO-84 MTD is fused to the C-terminus of Klf4;
4) His-NLS-MTD₈₆-Klf4 (HNM₈₆Klf4), wherein a His-NLS and a JO-86 MTD are fused to the N-terminus of Klf4;
5) His-NLS-Klf4-MTD₈₆ (HNKlf4M₈₆), wherein a His-NLS is fused to the N-terminus and a JO-86 MTD is fused to the C-terminus of Klf4;
6) His-NLS-MTD₈₆-Klf4-MTD₈₆ (HNM₈₆Klf4M₈₆), wherein a His-NLS-JO-86 MTD is fused to the N-terminus and a JO-86 MTD is fused to the C-terminus of Klf4; or
7) His-NLS-MTD₁₇₃-Klf4 (HNM₁₇₃Klf4), wherein a His-NLS and a JO-173 MTD are fused to the N-terminus of Klf4.

Optionally, the recombinant proteins 1) to 7) above may not include His-Tag.

Referring to FIGS. 1 and 10, the cMyc recombinant proteins of the present invention may be:
1) His-NLS-MTD₈₄-cMyc (HNM₈₄cMyc), wherein a His-NLS and a JO-84 MTD are fused to the N-terminus of cMyc;
2) His-NLS-cMyc-MTD₈₄ (HNcMycM₈₄), wherein a His-NLS is fused to the N-terminus and a JO-84 MTD is fused to the C-terminus of cMyc;
3) His-NLS-MTD₈₄-cMyc-MTD₈₄ (HNM₈₄cMycM₈₄), wherein a His-NLS-JO-84 MTD is fused to the N-terminus and a JO-84 MTD is fused to the C-terminus of cMyc;
4) His-NLS-MTD₈₆-cMyc (HNM₈₆cMyc), wherein a His-NLS and a JO-86 MTD are fused to the N-terminus of cMyc,
5) His-NLS-cMyc-MTD₈₆(HNcMycM₈₆), wherein a His-NLS is fused to the N-terminus and a JO-86 MTD is fused to the C-terminus of cMyc,
6) His-NLS-MTD₈₆-cMyc-MTD₈₆ (HNM₈₆cMycM₈₆), wherein a His-NLS-JO-86 MTD is fused to the N-terminus and a JO-86 MTD is fused to the C-terminus of cMyc, or
7) His-NLS-MTD₁₄₅-cMyc (HNM₁₄₅cMyc), wherein a His-NLS and a JO-145 MTD are fused to the N-terminus of cMyc.

Optionally, the recombinant proteins 1) to 7) above may not include His-Tag.

Referring to FIGS. 1 and 10, the Lin28 recombinant proteins of the present invention may be:
1) His-NLS-MTD₈₄-Lin28 (HNM₈₄Lin28), wherein His-NLS and a JO-84 MTD are fused to the N-terminus of Lin28;
2) His-NLS-Lin28-MTD₈₄ (HNLin28M₈₄), wherein a His-NLS is fused to the N-terminus and a JO-84 MTD is fused to the C-terminus of Lin28;
3) His-NLS-MTD₈₄-Lin28-MTD₈₄ (HNM₈₄Lin28M₈₄), wherein a His-NLS-JO-84 MTD is fused to the N-terminus and a JO-84 MTD is fused to the C-terminus of Lin28;
4) His-NLS-MTD₈₆-Lin28 (HNM₈₆Lin28), wherein a His-NLS and a JO-86 MTD are fused to the N-terminus of Lin28;
5) His-NLS-Lin28-MTD₈₆ (HNLin28M₈₆), wherein a His-NLS is fused to the N-terminus and a JO-86 MTD is fused to the C-terminus of Lin28;
6) His-NLS-MTD₈₆-Lin28-MTD₈₆ (HNM₈₆Lin28M₈₆), wherein a His-NLS-JO-86 MTD is fused to the N-terminus and a JO-86 MTD is fused to the C-terminus of Lin28; or
7) His-NLS-MTD₁₃₂-Lin28 (HNM₁₃₂Lin28), wherein a His-NLS and a JO-132 MTD are fused to the N-terminus of Lin28.

Optionally, the recombinant proteins 1) to 7) above may not include His-Tag.

As a control for the cell permeable Nanog recombinant proteins, His-NLS-Nanog (HNNanog), wherein only NLS and a histidine-tag are fused to Nanog without any MTDs, is prepared. This control protein has an amino acid sequence represented by SEQ ID NO: 34 and can be encoded by a polynucleotide having a nucleotide sequence represented by SEQ ID NO: 33.

As a control for the cell permeable Oct4 recombinant proteins, His-NLS-Oct4 (HNOct4), wherein only NLS and a histidine-tag are fused to Oct4 without any MTDs, is prepared. This control protein has an amino acid sequence represented by SEQ ID NO: 62 and can be encoded by a polynucleotide having a nucleotide sequence represented by SEQ ID NO: 61.

As a control for the cell permeable Sox2 recombinant proteins, His-NLS-Sox2 (HNSox2), wherein only NLS and a histidine-tag are fused to Sox2 without any MTDs, is prepared. This control protein has an amino acid sequence represented by SEQ ID NO: 90 and can be encoded by a polynucleotide having a nucleotide sequence represented by SEQ ID NO: 89.

As a control for the cell permeable Klf4 recombinant proteins, His-NLS-Klf4 (HNKlf4), wherein only NLS and a histidine-tag are fused to Klf4 without any MTDs, is prepared. This control protein has an amino acid sequence represented by SEQ ID NO: 118 and can be encoded by a polynucleotide having a nucleotide sequence represented by SEQ ID NO: 117.

As a control for the cell permeable cMyc recombinant proteins, His-NLS-cMyc (HNcMyc), wherein only NLS and a histidine-tag are fused to cMyc without any MTDs, is prepared. This control protein has an amino acid sequence represented by SEQ ID NO: 146 and can be encoded by a polynucleotide having a nucleotide sequence represented by SEQ ID NO: 145.

As a control for the cell permeable Lin28 recombinant proteins, His-NLS-Lin28 (HNLin28), wherein only NLS and a histidine-tag are fused to Lin28 without any MTDs, is prepared. This control protein has an amino acid sequence represented by SEQ ID NO: 174 and can be encoded by a polynucleotide having a nucleotide sequence represented by SEQ ID NO: 173.

Further, the present invention provides a recombinant expression vector comprising the polynucleotide encoding the cell permeable reprogramming transcription factor recombinant proteins described above, and a transformant prepared with this expression vector.

The term "recombinant expression vector" as used herein is a vector capable of expressing a target protein or a target RNA in a suitable host cell, and refers to a genetic construct comprising necessary regulatory elements operably linked to an insert gene such that the insert can be properly expressed.

As used herein, the term "operably linked" means that a nucleotide sequence encoding a target protein or a target RNA is functionally linked to regulatory elements in a manner that allows for the expression of the nucleotide sequence. For example, a promoter can be operably linked to a nucleotide sequence encoding a target protein or RNA to influence the expression of the nucleotide sequence. An operable linkage with an recombinant expression vector can be achieved by conventional gene recombinant techniques known in the art, while site-specific DNA cleavage and ligation are carried out by using conventional enzymes.

The expression vectors that can be used in the present invention may include, but are not limited to, plasmid vectors, cosmid vectors, bacteriophage vectors, viral vectors, etc. Suitable expression vectors may include a signal sequence or a leader sequence for membrane targeting or secretion, as well as regulatory sequences such as a promoter, an operator, an initiation codon, a termination codon, a polyadenylation signal, an enhancer, and the like. It can be prepared in various ways depending on the desired purpose. The promoter may be constitutive or inducible. Further, the expression vector may include one or more selection markers for screening a host cell containing the expression vector. In the case of a replicable expression vector, it may include a nucleotide sequence of the origin of replication.

The recombinant expression vector according to the present invention as constructed above may be, for example, pET28a(+)-HNM₈₆Nanog, pET28a(+)-HNM₈₆Oct4, pET28a(+)-HNM₈₆Sox2M₈₆, pET28a(+)-HNM₈₆Klf4M₈₆, pET28a(+)-HNM₈₆cMyc. In the recombinant expression vector encoding the cell permeable reprogramming transcription factor recombinant proteins according to the present invention, a polynucleotide encoding HM₈₆Nanog where a JO-86 MTD is fused to the N-terminus of Nanog; HM₈₆Oct4 where a JO-86 MTD is fused to the N-terminus of Oct4; HM₈₆Sox2M₈₆ where a JO-86 MTD is fused to the N-terminus and the C-terminus of Sox2; HM₈₆Klf4M₈₆ where a JO-86 MTD is fused to the N-terminus and the C-terminus of Klf4; or HM₈₆cMyc where a JO-86 MTD is fused to the N-terminus of cMyc is inserted in the *Nde*I restriction site within the multiple cloning sites (MCS) of a pET-28a(+) vector (Novagen, Germany).

In one embodiment of the present invention, the nucleotide sequence of the present invention is cloned into a pET-28a(+) vector (Novagen, Germany) having a His-Tag sequence so that the six cell permeable reprogramming transcription factor recombinant proteins have 6 histidine tags, which facilitate purification, expressed at the N-terminus.

The six cell permeable reprogramming transcription factor recombinant proteins expressed in the recombinant expression vector above have a structure where any one of JO-10 MTD, JO-52 MTD, JO-84 MTD, JO-86 MTD, JO-132 MTD, JO-145 MTD, JO-173 MTD, or JO-181 MTD is fused to either one or both termini of the recombinant proteins, and a His-Tag and/or NLS are linked to the N-terminus thereof.

The present invention further provides a transformant that is obtained by transforming a host cell with the recombinant expression vector above. Host cells suitable for the present invention may preferably be *E.coli. E.coli* may be transformed with the recombinant expression vector of the present invention, for example, pET28a(+)-HNM₈₆Nanog comprising a polynucleotide encoding HM₈₆Nanog in which a JO-86 MTD is fused to the N-terminus of the Nanog. The transformant thus obtained can be used to produce the cell permeable reprogramming transcription factor recombinant protein in large amounts. Any method of introducing a nucleic acid into a host cell may be used for the transformation. Any transformation techniques well known in the art may be used. Preferably, the methods may include, but are not limited to, microprojectile bombardment, electroporation, calcium phosphate (CaHPO₄) precipitation, calcium chloride (CaCl₂) precipitation, PEG-mediated fusion, microinjection, and liposome-mediated method.

In a preferred embodiment of the present invention, *E.coli* DH5α was transformed with the recombinant protein expression vectors prepared as described above, where NLS and JO-86 MTD are fused to the six reprogramming transcription factors. The transformant containing pET28a(+)-HNM₈₆Oct was deposited with the Korean Collection for Type Cultures (KCTC), Korea Research Institute of Bioscience and Biotechnology (KRIBB), on February 17, 2010 as Deposit No. KCTC11640BP. *E.coli* DH5α was transformed with the recombinant expression vectors comprising pET28a(+)-HNM₁₀Nanog including JO-10 MTD, pET28a(+)-HNM₁₈₁Sox2 including JO-181 MTD, pET28a(+)-HNM₈₆cMyc including JO-86 MTD, pET28a(+)-HNM₁₇₃Klf4 including JO-173 MTD, and pET28a(+)-HNM₁₃₂Lin28 including JO-132 MTD to obtain transformants, respectively. These transformants were deposited with the Korean Collection for Type Cultures (KCTC), Korea Research Institute of Bioscience and Biotechnology (KRIBB), on March 10, 2010 as Deposit Nos. KCTC11660BP, KCTC11659BP, KCTC11661BP, KCTC11662BP, and KCTC11663BP, respectively.

The present invention also provides a method of producing six kinds of cell permeable reprogramming transcription factor recombinant proteins comprising culturing the transformant.

The production method above is carried out by culturing the transformant in a suitable medium under suitable conditions so that a polynucleotide encoding the cell permeable reprogramming transcription factor recombinant protein of the present invention can be expressed. The method above is well known in the art and, for example, may be carried out by inoculating a transformant in a suitable medium for growing the transformant, performing a subculture, transferring the same to a main culture medium, culturing it under suitable conditions, for example, in the presence of isopropyl-β-D-thiogalactoside (IPTG), a gene expression inducer, thereby inducing the expression of the recombinant protein. After the culture is completed, it is possible to recover a substantially pure recombinant protein from the culture solution above. The term "substantially pure" means that the recombinant protein of the present invention and the polynucleotide encoding the same are essentially free of other proteins derived from the host cell.

The recombinant protein obtained above may be recovered by various isolation and purification methods known in the art. Conventionally, cell lysates are centrifuged to remove cell debris and impurities, and then subject to precipitation by, for example, salting out (ammonium sulfate precipitation or sodium phosphate precipitation) or solvent precipitation (protein-containing fragment precipitation using acetone, ethanol, etc.). Further, dialysis, electrophoresis, and various column chromatographies may be performed. With respect to chromatography, ion exchange chromatography, gel permeation chromatography, HPLC, reverse phase HPLC, affinity column chromatography, and ultrafiltration may be used alone or in combination (Maniatis et al., Molecular Cloning: A Laboratory Manual, Cold Spring Harbor Laboratory, Cold Spring Harbor, N.Y., 1982; Sambrook et al., Molecular Cloning: A Laboratory Manual, 2d Ed., Cold Spring Harbor Laboratory Press, 1989; Deutscher, M., Guide to Protein Purification Methods Enzymology vol. 182. Academic Press. Inc., San Diego, CA, 1990).

Meanwhile, the recombinant protein expressed in the transformant transformed with the recombinant expression vector can be separated into a soluble fraction and an insoluble fraction according to the characteristics of the proteins during protein purification. If the majority of the expressed recombinant proteins are present in the soluble fraction, the recombinant protein can easily be isolated and purified according to the method described above. However, when the majority of the expressed recombinant proteins are present in the insoluble fraction, i.e., as inclusion bodies, the recombinant proteins can be solubilized using polypeptide denaturing agents, e.g., urea or surfactants, and then, centrifuged, followed by various treatments, such as dialysis, electrophoresis, and column chromatography for purification. Since there is a risk of losing the recombinant protein's activity due to structural modifications caused by solutions containing polypeptide denaturing agents, the process of purifying the recombinant protein from the insoluble fraction requires desalting and refolding steps. Namely, the desalting and refolding steps can be performed by dialysis and dilution with a solution that does not include a polypeptide denaturing agent or by centrifugation with a filter. Further, if the salt concentration of a solution used for the purification of a recombinant protein from a soluble fraction is high, such desalting and refolding steps may also be performed.

In one embodiment of the present invention, if it is confirmed that the cell permeable reprogramming transcription factor recombinant proteins of the present invention are present in the insoluble fraction as inclusion bodies, in order to purify the recombinant protein from the insoluble fraction, the insoluble fraction may be dissolved in a lysis buffer containing a non-ionic surfactant such as Triton X-100, subjected to ultrasonification, and then centrifuged to separate the precipitate. The resulting precipitate may be dissolved in a buffer containing a denaturing agent, such as urea, and centrifuged to separate the supernatant. The recombinant protein of the present invention, which is obtained by dissolving the insoluble fraction to the maximum extent with urea, was purified by means of a histidine-binding protein purification kit and subjected to dialysis, for example, by using an amicon filter for salt removal and protein refolding, thereby obtaining the purified recombinant protein of the present invention.

Further, the present invention provides a composition comprising the six cell permeable reprogramming transcription factor proteins described above. The composition according to the present invention is capable of establishing induced pluripotent stem cells when treated on somatic cells free of any reprogramming transcription factor.

This composition is capable of inducing pluripotency and self-renewal in a cell while maintaining the undifferentiated state of the cell by introducing a cell permeable reprogramming transcription factor into the nucleus of the cell with high efficiency. In addition, the composition can be usefully employed to induce reprogramming of somatic cells by activating genes specifically expressed in stem cells, thereby enabling establishment of induced pluripotent stem cells which are similar to embryonic stem cells.

Hereinafter, the embodiments of the present invention will be described in more detail with reference to the following examples. However, the examples are only provided for purposes of illustration and are not to be construed as limiting the scope of the invention.

### EXAMPLES

### EXAMPLE 1: Expression of recombinant proteins

In order to prepare gene constructs of the above-described recombinant proteins, polymerase chain reactions (PCRs) were carried out using a primer pair specifically designed for each construct and human Nanog, Oct4, Sox2, Klf4, c-Myc, and Lin28 cDNAs as a template.

The forward and reverse primers for amplifying HNNanog have nucleotide sequences represented by SEQ ID NOS: 187 and 188, respectively;

Those for amplifying HNM₈₄Nanog have nucleotide sequences represented by SEQ ID NOS: 189 and 188, respectively;

Those for amplifying HNNanogM₈₄ have nucleotide sequences represented by SEQ ID NOS: 187 and 190, respectively;

Those for amplifying HNM₈₄NanogM₈₄ have nucleotide sequences represented by SEQ ID NOS: 189 and 190, respectively;

Those for amplifying HNM₈₆Nanog have nucleotide sequences represented by SEQ ID NOS: 191 and 188, respectively;

Those for amplifying HNNanogM₈₆ have nucleotide sequences represented by SEQ ID NOS: 187 and 192, respectively;

Those for amplifying HNM₈₆NanogM₈₆ have nucleotide sequences represented by SEQ ID NOS: 191 and 192, respectively;

Those for amplifying HNOct4 have nucleotide sequences represented by SEQ ID NOS: 193 and 194, respectively;

Those for amplifying HN M₈₄Oct4 have nucleotide sequences represented by SEQ ID NOS: 195 and 194, respectively;

Those for amplifying HNOct4M₈₄ have nucleotide sequences represented by SEQ ID NOS: 193 and 190, respectively;

Those for amplifying HNM₈₄Oct4M₈₄ have nucleotide sequences represented by SEQ ID NOS: 195 and 196, respectively;

Those for amplifying HNM₈₆Oct4 have nucleotide sequences represented by SEQ ID NOS: 197 and 194, respectively;

Those for amplifying HNOct4M₈₆ have nucleotide sequences represented by SEQ ID NOS: 193 and 198, respectively;

Those for amplifying HNM₈₆Oct4M₈₆ have nucleotide sequences represented by SEQ ID NOS: 197 and 198, respectively;

Those for amplifying HNSox2 have nucleotide sequences represented by SEQ ID NOS: 199 and 200, respectively;

Those for amplifying HNM₈₄Sox2 have nucleotide sequences represented by SEQ ID NOS: 201 and 200, respectively;

Those for amplifying HNSox2M₈₄ have nucleotide sequences represented by SEQ ID NOS: 199 and 202, respectively;

Those for amplifying HNM₈₄Sox2M₈₄ have nucleotide sequences represented by SEQ ID NOS: 201 and 202, respectively;

Those for amplifying HNM₈₆Sox2 have nucleotide sequences represented by SEQ ID NOS: 203 and 200, respectively;

Those for amplifying HNSox2M₈₆ have nucleotide sequences represented by SEQ ID NOS: 199 and 204, respectively;

Those for amplifying HNM₈₆Sox2M₈₆ have nucleotide sequences represented by SEQ ID NOS: 203 and 204, respectively;

Those for amplifying HNKlf4 have nucleotide sequences represented by SEQ ID NOS: 205 and 206, respectively;

Those for amplifying HNM₈₄Klf4 have nucleotide sequences represented by SEQ ID NOS: 207 and 206, respectively;

Those for amplifying HNKlf4M₈₄ have nucleotide sequences represented by SEQ ID NOS: 205 and 208, respectively;

Those for amplifying HNM₈₄Klf4M₈₄ have nucleotide sequences represented by SEQ ID NOS: 207 and 208, respectively;

Those for amplifying HNM₈₆Klf4 have nucleotide sequences represented by SEQ ID NOS: 209 and 206, respectively;

Those for amplifying HNKlf4M₈₆ have nucleotide sequences represented by SEQ ID NOS: 205 and 210, respectively;

Those for amplifying HNM₈₆Klf4M₈₆ have nucleotide sequences represented by SEQ ID NOS: 209 and 210, respectively;

Those for amplifying HNcMyc have nucleotide sequences represented by SEQ ID NOS: 211 and 212, respectively;

Those for amplifying HNM₈₄cMyc have nucleotide sequences represented by SEQ ID NOS: 213 and 212, respectively;

Those for amplifying HNcMycM₈₄ have nucleotide sequences represented by SEQ ID NOS: 211 and 214, respectively;

Those for amplifying HNM₈₄cMycM₈₄ have nucleotide sequences represented by SEQ ID NOS: 213 and 214, respectively;

Those for amplifying HNM₈₆cMyc have nucleotide sequences represented by SEQ ID NOS: 215 and 212, respectively;

Those for amplifying HNcMycM₈₆ have nucleotide sequences represented by SEQ ID NOS: 211 and 216, respectively;

Those for amplifying HNM₈₆cMycM₈₆ have nucleotide sequences represented by SEQ ID NOS: 215 and 216, respectively;

Those for amplifying HNLin28 have nucleotide sequences represented by SEQ ID NOS: 217 and 218, respectively;

Those for amplifying HNM₈₄Lin28 have nucleotide sequences represented by SEQ ID NOS: 219 and 218, respectively;

Those for amplifying HNLin28M₈₄ have nucleotide sequences represented by SEQ ID NOS: 217 and 220, respectively;

Those for amplifying HNM₈₄Lin28M₈₄ have nucleotide sequences represented by SEQ ID NOS: 219 and 220, respectively;

Those for amplifying HNM₈₆Lin28 have nucleotide sequences represented by SEQ ID NOS: 221 and 218, respectively;

Those for amplifying HNLin28M₈₆ have nucleotide sequences represented by SEQ ID NOS: 217 and 222, respectively; and

Those for amplifying HNM₈₆Lin28M₈₆ have nucleotide sequences represented by SEQ ID NOS: 221 and 222, respectively.

The PCRs above were carried out in a 100 µl final volume reaction mixture containing 100 ng of human Nanog, Oct4, Sox2, Klf-4, c-Myc, or Lin28 cDNAs as a template, 0.2 mM (final concentration) of a dNTP mixture (dGTP, dATP, dTTP, and dCTP, each at 2 mM), 0.5 µM of primers, 10 µl of a 10x Taq buffer, and 0.5 µl of Taq polymerase (Takara, Japan). The PCR reactions were performed 30 cycles at 95□ for 45 seconds, 67□ for 45 seconds, and 72□ for 45 seconds after the initial denaturation at 95□ for 2 minutes, followed by the final amplification at 72□ for 5 minutes. After the reaction was completed, the amplified products were confirmed by electrophoresis on a 0.8% agarose gel.

After recovering the amplified recombinant fragments from the agarose gel, each recombinant fragment was extracted and purified using a commonly used kit (QIAquick Gel extraction kit, Qiagen, USA). The extracted fragments were inserted into a pGEM-T Easy vector (Promega, USA) and *E. coli* DH5α competent cells were transformed with the pGEM-T Easy vector in which an MTD-fused Nanog, Oct4, Sox2, Klf4,c-Myc, or Lin28 recombinant protein gene fragment was subcloned. The cells were plated onto an LB medium supplemented with 50 µg/ml ampicillin and cultured at 37°C overnight. The transformed *E. coli* was screened and inoculated in the LB medium again to obtain pGEM-T Easy vectors in which a Nanog, Oct4, Sox2, Klf4,c-Myc, or Lin28 recombinant protein gene was inserted. As a result, a large amount of vectors were obtained.

The recombinant fragment-inserted pGEM-T Easy vectors were treated by an *Nde*I restriction enzyme (Enzynomics, Korea) to isolate the recombinant fragments. The isolated recombinant fragments were subjected to 0.8% agarose gel electrophoresis. As a result, the successful subcloning of each recombinant fragment into the vector was confirmed.

The pGEM-T Easy vectors in which a Nanog, Oct4, Sox2, Klf4,c-Myc, or Lin28 recombinant protein gene was inserted were digested with *Nde*I at 37°C for 2 hours to obtain the recombinant fragment for each vector. After recovering the recombinant fragments from the agarose gel, each recombinant fragment was extracted and purified using a commonly used kit (QIAquick Gel extraction kit, Qiagen, USA) Meanwhile, an expression vector comprising a His-Tag and a T7 promoter, pET-28a(+) (Novagen, USA) was digested with *Nde*I under the same conditions above. Each of the recombinant fragments obtained above was admixed with the digested pET-28a(+) vector. With the addition of a T4 DNA ligase (Takara, Japan), the mixture was subjected to ligation at 16°C for 12 hours. *E. coli* DH5α supercompetent cells were transformed to obtain the recombinant protein expression vectors.

The recombinant fragment-inserted pET-28a(+) vectors were treated by an *Nde*I restriction enzyme to isolate the recombinant fragments and the isolated recombinant fragments were subjected to 0.8% agarose gel electrophoresis. As a result, the successful subcloning of each recombinant fragment into the vector was confirmed.

The recombinant protein expression vectors thus obtained were designated as pET28a(+)-HNNanog, pET28a(+)-HNM₈₄Nanog, pET28a(+)-HNNanogM₈₄, pET28a(+)-HNM₈₄NanogM₈₄, pET28a(+)-HNM₈₆Nanog, pET28a(+)-HNNanogM₈₆, pET28a(+)-HNM₈₆NanogM₈₆, pET28a(+)-HNOct4, pET28a(+)-HNM₈₄Oct4, pET28a(+)-HNOct4M₈₄, pET28a(+)-HNM₈₄Oct4M₈₄, pET28a(+)-HNM₈₆Oct4, pET28a(+)-HNOct4M₈₆, pET28a(+)-HNM₈₆Oct4M₈₆, pET28a(+)-HNSox2, pET28a(+)-HNM₈₄Sox2, pET28a(+)-HNSox2M₈₄, pET28a(+)-HNM₈₄Sox2M₈₄, pET28a(+)-HNM₈₆Sox2, pET28a(+)-HNSox2M₈₆, pET28a(+)-HNM₈₆Sox2M₈₆, pET28a(+)-HNKlf4, pET28a(+)-HNM₈₄Klf4, pET28a(+)-HNKlf4M₈₄, pET28a(+)-HNM₈₄Klf4M₈₄, pET28a(+)-HNM₈₆Klf4, pET28a(+)-14NKlf4M₈₆, pET28a(+)-HNM₈₆Klf4M₈₆, pET28a(+)-HNcMyc, pET28a(+)-HNM₈₄cMyc, pET28a(+)-HNcMycM₈₄, pET28a(+)-HNM₈₄cMycM₈₄, pET28a(+)-HNM₈₆cMyc, pET28a(+)-HNcMycM₈₆, pET28a(+)-HNM₈₆cMycM₈₆ , pET28a(+)-HNLin28, pET28a(+)-HNM₈₄Lin28, pET28a(+)-HNLin28M₈₄, pET28a(+)-HNM₈₄Lin28M₈₄, pET28a(+)-HNM₈₆Lin28, pET28a(+)-HN Lin28M₈₆, and pET28a(+)-HNM₈₆Lin28M₈₆, respectively.

*E. coli* BL21 CodonPlus (DE3) was transformed with each of the following recombinant expression vectors, i.e., pET28a(+)-HNNanog, pET28a(+)-HNM₈₄Nanog, pET28a(+)-HNNanogM₈₄, pET28a(+)-HNM₈₄NanogM₈₄, pET28a(+)-HNM₈₆Nanog, pET28a(+)-HNNanogM₈₆, pET28a(+)-HNM₈₆NanogM₈₆, pET28a(+)-HNM₁₀Nanog, pET28a(+)-HNOct4, pET28a(+)-HNM₈₄Oct4, pET28a(+)-HNOct4M₈₄, pET28a(+)-HNM₈₄Oct4M₈₄, pET28a(+)-HNM₈₆Oct4 pET28a(+)-HNOct4M₈₆, pET28a(+)-HNM₈₆Oct4M₈₆ pET28a(+)-HNM₅₂Oct4, pET28a(+)-HNSox2, pET28a(+)-HNM₈₄Sox2, pET28a(+)-HNSox2M₈₄, pET28a(+)-HNM₈₄Sox2M₈₄, pET28a(+)-HNM₈₆Sox2, pET28a(+)-HNSox2M₈₆, pET28a(+)-HNM₈₆Sox2M₈₆, pET28a(+)-HNM₁₈₁Sox2, pET28a(+)-HNKlf4, pET28a(+)-HNM₈₄Klf4, pET28a(+)-HNKlf4M₈₄, pET28a(+)-HNM₈₄Klf4M₈₄, pET28a(+)-HNM₈₆Klf4, pET28a(+)-HNKlf4M₈₆, pET28a(+)-HNM₈₆Klf4M₈₆, pET28a(+)-HNM₁₇₃Klf4, pET28a(+)-HNcMyc, pET28a(+)-HNM₈₄cMyc, pET28a(+)-HNcMycM₈₄, pET28a(+)-HNM₈₄cMycM₈₄, pET28a(+)-HNM₈₆cMyc, pET28a(+)-HNcMycM₈₆, pET28a(+)-HNM₈₆cMycM₈₆, pET28a(+)-HNM₁₄₅cMyc, pET28a(+)-HNLin28, pET28a(+)-HNM₈₄Lin28, pET28a(+)-HNLin28M₈₄, pET28a(+)-HNM₈₄Lin28M₈₄, pET28a(+)-HNM₈₆Lin28, pET28a(+)-HN Lin28M₈₆, pET28a(+)-HNM₈₆Lin28M₈₆, and pET28a(+)-HNM₁₃₂Lin28, by a heat shock method. The transformants were cultured in an LB medium containing 50 µg/ml of kanamycin. Thereafter, *E.coli* transformed with DNA encoding the recombinant protein was inoculated in 25 ml of an LB medium and cultured at 37°C overnight, and then inoculated again in 1 L of an LB medium and cultured at 37°C until the optical density OD₆₀₀ reached 0.6 to 0.7. To the culture was added 0.65 mM isopropyl-β-D-thiogalactoside (IPTG) as a protein expression inducer, followed by incubation at 37°C for an additional 3 hours. This culture was centrifuged at 4°C at a speed of 4,000 Xg for 20 minutes to remove the supernatant and bacterial cells were harvested. The harvested bacterial cells were suspended in a lysis buffer (100 mM NaH₂PO₄, 10 mM Tris-HCl, 8 M urea, pH 8.0) and the suspension was subject to sonication to disrupt the cells. The cell lysates were centrifuged at a speed of 14,000 Xg for 15 minutes to separate the insoluble fraction from the soluble fraction. The soluble and insoluble fractions thus obtained were loaded on an SDS-PAGE gel separately to analyze the protein expression profile and the degree of expression.

As shown in FIG. 2, the cell permeable reprogramming transcription factor recombinant proteins according to the present invention (about 34 kDa of Nanog, about 40 kDa of Oct4, about 35 kDa of Sox2, about 52 kDa of Klf4, and about 50 kDa of cMyc) are mostly present in the insoluble fraction as inclusion bodies. Further, it was found that expression of the protein significantly increased in the culture solution with IPTG (+) compared to that without IPTG (-).

### EXAMPLE 2: Purification of the recombinant proteins

Since the cell permeable reprogramming transcription factor recombinant proteins according to the present invention are present in the insoluble fraction as inclusion bodies, 8 M urea was used as a strong denaturing agent to separate these proteins from the insoluble fraction.

First, the BL21 CodonPlus (DE3) strains transformed with each of the expression vectors of the present invention were cultured in 1 L of an LB medium as described in Example 1 above. Each culture solution was centrifuged to harvest the bacterial cells. The obtained bacterial cells were gently suspended in 20 ml of a lysis buffer (100 mM NaH₂PO₄, 10 mM Tris-HCl, 8 M urea, pH 8.0) carefully so as to avoid forming bubbles, and homogenized at a low temperature using an ultrasonic homogenizer equipped with a microtip to destruct the cells. Here, the power was set at 25% the maximum power, while a 45 second sonication followed by a 10 second pause was repeated for 7 minutes. The sufficiently lysed inclusion bodies were centrifuged at 4°C at a speed of 4,000 Xg for 20 minutes to remove the cell precipitate and recover the supernatant. The recovered supernatant was loaded onto an Ni-NTA agarose resin where nickel (Ni) was added on nitrilotriacetic acid agarose. The Ni-NTA agarose was used after equilibration by washing with a lysis buffer prior to use. The supernatant was allowed to absorb onto the resin while slowly stirring using a rotary shaker for at least 8 hours at 4°C. The resin absorbed with the inclusion bodies containing the recombinant protein was centrifuged at 4°C at a speed of 1,000 rpm for 5 minutes to remove the reaction solution and then washed with a washing buffer (100 mM NaH₂PO₄, 10 mM Tris-HCl, 8 M urea, pH 6.3) five times to remove the non-specifically absorbed materials. Onto the washed resin was loaded an elution buffer (100 mM HaH₂PO₄, 10 mM Tris-HCl, 8 M urea, pH 4.5) in a volume that is twice the resin volume under acidic conditions of pH 4.0, followed by stirring in a shaker for 2 hours or at least 8 hours to elute the protein. In order to analyze the purity of the eluted protein, electrophoresis was carried out on a 12% SDS-PAGE gel, and subsequently, the gel was stained with Coomassie Brilliant Blue R with gentle shaking, and de-stained with a de-staining solution until the band of the target protein could be seen clearly.

As a result, as shown in FIG. 2, all of the cell permeable reprogramming transcription factor recombinant proteins fused to a JO-84 MTD or a JO-86 MTD were detected as separate bands in comparison with the band of the marker protein. It was confirmed from the results above that the cell permeable reprogramming transcription factor recombinant proteins of the present invention were purified from the insoluble fraction.

Since the recombinant proteins of the present invention purified from the insoluble fraction above were denatured with 8 M urea, a strong denaturing agent, a refolding process was carried out to convert them to an active form as described below:

First, the purified recombinant proteins were subjected to dialysis using a refolding buffer (0.55 M Guanidine HCl, 0.88 M L-arginine, 50 mM Tris-HCl, 150 mM NaCl, 1 mM EDTA, 100 mM NDSB, 2 mM glutathione oxidized, and 1 mM glutathione reduced) at 4°C for at least 72 hours to remove the denaturing agent. By doing so, the recombinant proteins were reactivated, namely, refolded. The refolding buffer in the container was changed every 24 hours. Thereafter, the activated recombinant proteins were dialyzed in a dialysis tubing (Snakeskin pleated, PIERCE) using a DMEM (Dulbecco's Modified Eagle Medium) containing 1% penicillin/streptomycin at 4°C for 9 hours while stirring. The medium in the tubing was replaced every 3 hours. The cell permeable reprogramming transcription factor recombinant proteins thus refolded were used in the following experiments.

### EXAMPLE 3: Induction of the reprogrammed stem cells and alkaline phosphatase staining (AP staining)

Human dermal fibroblast (HDF) cells were cultured to carry out induction of the reprogrammed stem cells. For an initial 7 days, the cells were cultured at 37°C in a humidified atmosphere of 5% CO₂ in an HDF medium (M106 (Cascade Biologic™) + LSGS (Cascade Biologic™)), and for a subsequent 14 days, the HDF medium was exchanged with a human embryonic stem cell medium (DMEM/F12 (HyClone®) supplemented with 20% KSR (GIBCO®), 2 mM L-glutamin (GIBCO®), 2 mM MEM Non Essential Amino Acid (GIBCO®), 0.1 mM β-mercaptoethanol (GIBCO®), and 0.1% penicillin/streptomycin (GIBCO®)). The cells were treated with the five cell permeable reprogramming transcription factor recombinant proteins at a concentration of 1 µM, and the cells treated with the MTD-free reprogramming transcription factor recombinant proteins at the same concentration were used as a control group. The treatment was conducted for 16 days, while the media and proteins were changed everyday. 16 days after the treatment, the total number of colonies formed was 23 and these colonies were used in examining the formation of reprogrammed stem cells.

In order to confirm that the colonies were reprogrammed stem cells, alkaline phosphatase (AP) staining was carried out (Sigma). Undifferentiated embryonic stem cells are characterized by a high expression level of alkaline phosphatase. Alkaline phosphatase (AP) is not only found in many organs in the body but also in the cytoplasm of an embryonic stem cell. The presence of AP can be detected by being stained in red. A capsule of Fast Blue RR salt in the alkaline phosphatase staining kit was dissolved in water to prepare a diazonium salt solution.

Naphthol AS-MX Phosphate Alkaline Solution was added to the diazonium salt solution to prepare an alkaline staining mixture. The cells in the 6-well plate were fixed with a fixative solution for about 30 seconds and rinsed gently with water for 45 seconds. With addition of the alkaline staining mixture, the cells were incubated at room temperature for 30 minutes while making sure the samples were not exposed to sunlight. Upon completion of the incubation, the cells were washed with water. Counterstaining was performed on these cells with a Mayer's Hematoxylin solution for 10 minutes and then rinsed with water for 2 minutes. Thereafter, staining was evaluated microscopically after covering the cells with a mounting solution.

As a result, as shown in FIG. 3, the undifferentiated state of the mouse embryonic stem cells was confirmed by AP staining, which is a characteristic of embryonic stem cells. The colonies, which had been formed by treatment with the cell permeable reprogramming transcription factor recombinant proteins for 16 days, also were found to be stained in red by AP staining, which is a marker indicative of cell undifferentiation.

### EXAMPLE 4: Immunocytochemistry

Colonies of the induced pluripotent stem cells were obtained by treating human dermal fibroblast cells (HDF) with the cell permeable recombinant proteins described in Example 3. The colonies were scrapped by using a capillary glass tube which had been bent into a ring shape by heating with an alcohol lamp. The successful removal of the colonies was confirmed under a stereomicroscope. The colonies were loaded for adhesion onto the 8-well chamber slides (chamber slide, NUNC) on which human dermal fibroblast cells were plated. After 1 day of stabilization, the medium was removed from the chamber slide and rinsed twice with PBS (phosphate buffered saline; WelGENE). The colonies were fixed in 2% paraformaldehyde (JUNSEI) for 20 minutes, washed twice with PBS again, and treated with 0.1% Triton X-100 for 5 minutes to make the cell membranes permeable. After rinsing with PBS twice, the cells were treated with 2% BSA (bovine serum albumin; Sigma)/PBS at room temperature for 1 hour to block non-specific protein binding. Thereafter, the colonies were treated with primary antibodies (diluted 1:1000 in 2% BSA/PBS) overnight in a refrigerator at 4□. The primary antibodies used against Nanog and Oct4 were anti-Nanog antibody (abcam) and anti-Oct4 antibody (abcam), respectively. The colonies were washed twice with PBS upon completion of the reaction and then treated with secondary antibodies (diluted 1: 500 with 2% BSA/PBS) at room temperature for 1 hour. The secondary Nanog and Oct4 antibodies used were Alexa Fluor® 488 goat anti-rabbit IgG (Alexa) and Alexa Fluor® 546 rabbit anti-goat IgG (Alexa), respectively. After rinsing the colonies with PBS three times, the nuclei were stained with 300 nM DAPI (4,6-diamidino-2-phenylindole, Sigma) at room temperature for 5 minutes in darkness. The colonies were washed with PBS three times. A mounting medium (VECTOR) was mounted thereon, and after 15 minutes, a laser scanning confocal microscopy with a Nomarski filter was used for observation. The original shape of cells and fluorescence were examined for Nanog excited at 488 nm and for Oct4 excited at 546 nm.

As a result, as shown in FIG. 4, it was found that the pluripotent stem cells induced by the cell permeable reprogramming transcription factor recombinant proteins according to the present invention expressed Nanog and Oct 4, which is regarded to be a characteristic of stem cells.

### EXAMPLE 5: Expression of a stem cell specific marker on the surface of an induced pluripotent stem cell established by a reprogramming transcription factor

The colonies that were confirmed to have an undifferentiated state by AP staining in Example 4 were treated with trypsin/EDTA (T/E, Invitrogen) at 37□ for 3 minutes to disaggregate into single cells. The cells were washed with PBS supplemented with 0.1% BSA twice and treated with Fc Blocker CD16/32 (Fc Blocker, BD Pharmingen) for 10 minutes to block non-specific binding of the Fc region to the FcR on the cell surface. The treated cells were washed with PBS supplemented with 0.1% BSA twice, and stained on ice with TRA-1-60 (Tumor Rejection Antigen, BD bioscience), at a concentration of 1 µg/1x10⁶ for 20 minutes, FTTC (fluorescein-5-isothiocyanate)-labeled SSEA-4 (Stage Specific Embryonic Antigen-4) antibody (BD bioscience), and PE (Phycoerythrin)-labeled SSEA-3 antibody (Stage Specific Embryonic Antigen-3) antibody (BD bioscience). Then, the cells were washed with PBS (Welgene) supplemented with 0.1% BSA twice. The prepared cells were analyzed with Calibur flow cytometry (Beckton-Dickinson, CA) using the CellQuest Pro cytometric analysis software (CellQuest Pro, BD).

As a result, as shown in FIG. 5, it was found that the expression of the stem cell specific surface marker of the colonies induced by the cell permeable reprogramming transcription factor recombinant proteins according to the present invention showed higher fluorescent intensity when compared to the control group.

In FIG. 5, no staining indicated the group to which the reprogramming transcription factors were not treated, and only 0.5% of the cells expressed SSEA-3. 4.5% of the cells expressed SSEA-3 in the MTD-free control group, whereas in the colonies formed by treatment with the cell permeable reprogramming transcription factors, 9.9% of the cells expressed SSEA-3.

As a result, as shown in FIG. 5, it was confirmed that TRA-1-60, the stem cell specific surface marker of the colony induced by the cell permeable reprogramming transcription factor recombinant proteins according to the present invention, showed a higher level of expression when compared to the control group by at least 5%.

### EXAMPLE 6: Reverse transcriptase polymerase chain reaction (RT-PCR)

RT-PCR was performed to confirm whether the induced reprogrammed stem cells described above showed an increased expression level of stem cell marker genes. RNAs were extracted from cells using a Trizol (iNTRON), and 5 µg of RNA was subjected to reverse transcription (Roche). A PCR was performed to examine the difference in expression levels of stem cell marker genes between the cDNAs from the group treated with the cell permeable reprogramming transcription factor recombinant proteins to induce reprogramming and those from the control group not treated with the recombinant proteins. A total of 14 genes were used as marker genes: Oct4, Nanog, LEFTB, Klf4, cMyc, EBAF, UTF1, CD9, ESG1, IFITM1, GAL, BRIX, and REX1. Further, for the control group, GAPDH was used as it is universally expressed in all types of cells. The PCR reaction was repeated 30-35 cycles at 95□ for 1 minute, 55□ for 1 minute, and 72□ for 1 minute. Electrophoresis was carried out with the PCR product on an agarose gel.

As shown in FIG. 6, it was found that the group treated with the cell permeable reprogramming transcription factor recombinant proteins exhibited a higher expression level of the genes specifically expressed in stem cells when compared to the control group.

### EXAMPLE 7: Induction of the reprogrammed stem cells

Human dermal fibroblast (HDF) cells were cultured to carry out induction of the reprogrammed stem cells. As shown in FIG. 7, the cells were cultured for a period of 23 days in a human stem cell medium (DMEM/F12 (HyClone®), 20% KSR (GIBCO®), 2mM L-glutamin (GIBCO®), 2mM MEM Non Essential Amino Acid (GIBCO©), 0.1mM β-mercaptoethanol (GIBCO®), and 0.1% penicillin/streptomycin (GIBCO®)). The cells were treated with the five cell permeable reprogramming transcription factor recombinant proteins at a concentration of 8 µg/ml or 20 µg/ml. A control group was treated with MTD-free reprogramming transcription factor recombinant proteins at the same concentration. The treatment was carried out for 10 days while the medium and the proteins were replaced daily. On the 8^{th} day after the protein treatment, a total of 13 colonies were formed. The colonies were then scrapped 10 days after the protein treatment using a capillary glass tube which had been bent into a ring shape by heating with an alcohol lamp. The successful removal of the colonies was confirmed under a stereomicroscope. Thereafter, the colonies were divided into a group with no further treatment with protein and a group to which 16 µg/ml of protein was treated for another 5 days from 8 days after the removal. Morphology of the colonies is illustrated in FIG 7. The collected colonies were used in examining the formation of reprogrammed stem cells below.

### EXAMPLE 8: Immunocytochemistry

Colonies of the induced pluripotent stem cell colony obtained by treating human dermal fibroblast cells (HDF) with the cell permeable recombinant proteins described in Example 7 were loaded for adhesion onto the 8-well chamber slides (NUNC) on which mouse dermal fibroblast cells were plated. After 1 day of stabilization, the medium was removed from the chamber slide and the slides were rinsed twice with PBS (WelGENE). The colonies were fixed in 2% paraformaldehyde (JUNSEI) for 20 minutes, washed twice with PBS again, and treated with 0.1% Triton X-100 for 5 minutes to make the cell membranes permeable. After rinsing with PBS twice, the colonies were treated with 2% BSA (Sigma)/PBS at room temperature for 1 hour to block non-specific protein binding. Thereafter, the colonies were treated with primary antibodies (diluted 1:1000 in 2% BSA/PBS) overnight in a refrigerator at 4□. The primary antibodies used were anti-Nanog antibody (abcam) and anti-Oct4 antibody (abcam). The colony was washed twice with PBS upon completion of the reaction and then treated with secondary antibodies (diluted 1:500 with 2% BSA/PBS) at room temperature for 1 hour. The secondary antibodies used against Nanog and Oct4 were Alexa Fluor^{®} 488 goat anti-rabbit IgG (Alexa) and Alexa Fluor^{®} 546 rabbit anti-goat IgG (Alexa), respectively. After rinsing with PBS three times, the nuclei were stained with 300 nM DAPI (Sigma) at room temperature for 5 minutes in darkness. The colonies were washed with PBS three times. A mounting medium (VECTOR) was mounted on the slides, and after 15 minutes, a laser scanning confocal microscopy with a Nomarski filter was used for observation. The original shape of cells and fluorescence were examined for Nanog excited at 488 nm and for Oct4 excited at 546nm .

As a result, as shown in FIG. 8, it was found that the pluripotent stem cells induced by the cell permeable reprogramming transcription factor recombinant proteins according to the present invention expressed Nanog and Oct4, which is regarded to be a characteristic of stem cells.

### EXAMPLE 9: Expression of a stem cell specific marker on the surface of an induced pluripotent stem cell established by a reprogramming transcription factor

The colonies that were confirmed to have an undifferentiated state by AP staining in the Example above were treated with trypsin/EDTA (T/E, Invitrogen) at 37□ for 3 minutes to disaggregate into single cells. The cells were washed with PBS supplemented with 0.1% BSA twice and treated with Fc Blocker CD16/32 (Fc Blocker, BD Pharmingen) for 10 minutes to block non-specific binding of the Fc region to the FcR on the cell surface. The treated cells were washed with PBS (phosphate buffered saline) supplemented with 0.1% BSA twice, and stained on ice with TRA-1-60 (Tumor Rejection Antigen, BD bioscience), at a concentration of 1 µg/1×10⁶ for 20 minutes, FTTC (fluorescein-5-isothiocyanate)-labeled SSEA-4 (Stage Specific Embryonic Antigen-4) antibody (BD bioscience), and PE (Phycoerythrin)-labeled SSEA-3 antibody (Stage Specific Embryonic Antigen-3) antibody (BD bioscience). Then, the cells were washed with PBS (Welgene) supplemented with 0.1% BSA twice. The prepared cells were analyzed with Calibur flow cytometry (Beckton-Dickinson, CA) using the CellQuest Pro cytometric analysis software (CellQuest Pro, BD).

As a result, as shown in FIG. 9, it was found that the expression of the stem cell specific surface marker in the colonies induced by the cell permeable reprogramming transcription factor recombinant proteins according to the present invention showed higher fluorescent intensity when compared to the control group.

In FIG. 9, HDF represents the control group not treated with the reprogramming transcription factors, and less than about 1% of the cells expressed SSEA-3. In the case of the colonies formed by treatment with the cell-permeable reprogramming transcription factors, about 5% and about 30% of the cells expressed SSEA-3 for each group.

As a result, as shown in FIG. 9, it was found that the expression level of TRA-160, which is a stem cell specific surface marker, was higher in the colonies induced by the cell permeable reprogramming transcription factor recombinant proteins according to the present invention when compared to the control group by about at least 10-12%.

### EXAMPLE 10: Expression of Codon optimized recombinant proteins

*E. coli* BL21 CodonPlus (DE3) was transformed with any of the following expression vectors, i.e., pET28a(+)-HNM₁₀Nanog, pET28a(+)-HNM₅₂Oct4 pET28a(+)-HNM₁₈₁Sox2, pET28a(+)-HNM₁₇₃Klf4, pET28a(+)-HNM₁₄₅cMyc, and pET28a(+)-HNM₁₃₂Lin28, in which a codon-optimized recombinant protein synthesized by Genscript was subcloned by a heat shock method, followed by incubation in an LB medium containing 50 µg/ml of kanamycin. Thereafter, the transformant was inoculated in 25 ml of an LB medium and cultured at 37°C overnight. Then, the culture was inoculated again in 1 1 of an LB medium and incubated at 37°C until the optical density OD₆₀₀ reached 0.6 to 0.7. To the culture was added 0.65 mM IPTG as a protein expression inducer, and the culture was incubated at 37°C for additional 3 hours. This culture was centrifuged at 4°C at a speed of 4,000 Xg for 20 minutes to remove the supernatant and the bacterial cells were harvested. The harvested cells were suspended in a lysis buffer (100 mM NaH₂PO₄, 10 mM Tris-HCl, 8 M urea, pH 8.0) and the suspension was subjected to sonication to disrupt the cells. The cell lysates were centrifuged at a speed of 14,000 Xg for 15 minutes to separate the insoluble fraction from the soluble fraction. The soluble and insoluble fractions thus obtained were loaded on an SDS-PAGE gel separately to analyze the protein expression profile and the degree of expression.

As a result, as shown in FIG. 11, the cell permeable reprogramming transcription factor recombinant proteins according to the present invention obtained from the expression vectors containing the recombinant protein gene constructs illustrated in FIG. 10 are mostly present in the insoluble fraction as inclusion bodies (about 34 kDa of Nanog, about 40 kDa of Oct4, about 35 kDa of Sox2, about 52 kDa of Klf4, and about 50 kDa of cMyc). Further, the expression of the target protein was found to be significantly increased in the culture solution with IPTG (+) compared to that without IPTG (-),when compared to the expression of the protein prior to the codon optimization.

### EFFECT OF THE INVENTION

The cell permeable reprogramming transcription factor recombinant proteins according to the present invention can introduce reprogramming transcription factors into a nucleus of a cell with high efficiency. The recombinant protein of the present invention can solve the problems of prior art caused by insertion of exogenous genes and is useful in the establishment of induced pluripotent stem cells with high efficiency.

## Claims

1. A cell permeable reprogramming transcription factor recombinant protein comprising a macromolecule transduction domain (MTD) fused to one terminus or both termini of a reprogramming transcription factor,
wherein the reprogramming transcription factor has an amino acid sequence selected from the group consisting of SEQ ID NOS: 2, 4, 6, 8, 10, and 12,
wherein the MTD has an amino acid sequence selected from the group consisting of SEQ ID NOS: 16 and 18.

2. The cell permeable reprogramming transcription factor recombinant protein according to Claim 1, wherein the MTD is either a JO-84 MTD having an amino acid sequence represented by SEQ ID NO: 16 or a JO-86 MTD having an amino acid sequence represented by SEQ ID NO: 18.

3. The cell permeable reprogramming transcription recombinant protein of Claim 1, wherein the recombinant protein comprises at least one selected from a nuclear localization sequence (NLS) and a histidine-tag affinity domain fused to either terminus.

4. The cell permeable reprogramming transcription factor recombinant protein according to any one of Claims 1 to 3, **characterized by** being selected from the group consisting of:
a recombinant protein wherein a JO-84 MTD having an amino acid sequence represented by SEQ ID NO: 16 is fused to the N-terminus of a reprogramming transcription factor having an amino acid sequence selected from the group consisting of SEQ ID NOS: 2, 4, 6, 8, 10, and 12;
a recombinant protein wherein a JO-84 MTD having an amino acid sequence represented by SEQ ID NO: 16 is fused to the C-terminus of a reprogramming transcription factor having an amino acid sequence selected from the group consisting of SEQ ID NOS: 2, 4, 6, 8, 10, and 12;
a recombinant protein wherein a JO-84 MTD having an amino acid sequence represented by SEQ ID NO: 16 is fused to both termini of a reprogramming transcription factor having an amino acid sequence selected from the group consisting of SEQ ID NOS: 2, 4, 6, 8, 10, and 12;
a recombinant protein wherein a JO-86 MTD having an amino acid sequence represented by SEQ ID NO: 18 is fused to the N-terminus of a reprogramming transcription factor having an amino acid sequence selected from the group consisting of SEQ ID NOS: 2, 4, 6, 8, 10, and 12;
a recombinant protein wherein a JO-86 MTD having an amino acid sequence represented by SEQ ID NO: 18 is fused to the C-terminus of a reprogramming transcription factor having an amino acid sequence selected from the group consisting of SEQ ID NOS: 2, 4, 6, 8, 10, and 12; and
a recombinant protein wherein a JO-86 MTD having an amino acid sequence represented by SEQ ID NO: 18 is fused to both termini of a reprogramming transcription factor having an amino acid sequence selected from the group consisting of SEQ ID NOS: 2, 4, 6, 8, 10, and 12.

5. The cell permeable reprogramming transcription factor recombinant protein according to any one of Claims 1 to 4, wherein the recombinant protein has an amino acid sequence selected from the group consisting of:
SEQ ID NOS: 22, 24, 26, 28, 30, or 32 for Nanog;
SEQ ID NOS: 50, 52, 54, 56, 58, or 60 for Oct4;
SEQ ID NOS: 78, 80, 82, 84, 86, or 88 for Sox2;
SEQ ID NOS: 106, 108, 110, 112, 114, or 116 for Klf4;
SEQ ID NOS: 134, 136, 138, 140, 142, or 144 for cMyc; and
SEQ ID NOS: 162, 164, 166, 168, 170, or 172 for Lin28.

6. The cell permeable reprogramming transcription factor recombinant protein according to any one of Claims 1 to 4, wherein the recombinant protein has an amino acid sequence selected from the group consisting of:
SEQ ID NOS: 36, 38, 40, 42, 44, or 46 for Nanog;
SEQ ID NOS: 64, 66, 68, 70, 72, or 74 for Oct4;
SEQ ID NOS: 92, 94, 96, 98, 100, or 102 for Sox2;
SEQ ID NOS: 120, 122, 124, 126, 128, or 130 for Klf4;
SEQ ID NOS: 148, 150, 152, 154, 156, or 158 for cMyc; and
SEQ ID NOS: 176, 178, 180, 182, 184, or 186 for Lin28.

7. A polynucleotide encoding the cell permeable reprogramming transcription factor recombinant protein according to Claim 1.

8. The polynucleotide according to Claim 7, wherein the polynucleotide has a nucleotide sequence selected from the group consisting of:
SEQ ID NOS: 21, 23, 25, 27, 29, or 31 for Nanog;
SEQ ID NOS: 49, 51, 53, 55, 57, or 59 for Oct4;
SEQ ID NOS: 77, 79, 81, 83, 85, or 87 for Sox2;
SEQ ID NOS: 105,107,109, 111, 113, or 115 for Klf4
SEQ ID NOS: 133, 135, 137, 139, 141, or 143 for cMyc; and
SEQ ID NOS: 161, 163, 165, 167, 169, or 171 for Lin28.

9. The polynucleotide according to Claim 7, wherein the polynucleotide has a nucleotide sequence selected from the group consisting of:
SEQ ID NOS: 35, 37, 39, 41, 43, or 45 for Nanog;
SEQ ID NOS: 63, 65, 67, 69, 71, or 73 for Oct4;
SEQ ID NOS: 91, 93, 95, 97, 99, or 101 for Sox2;
SEQ ID NOS: 119,121, 123,125,127, or 129 for Klf4;
SEQ ID NOS: 147, 149, 151, 153, 155, or 157 for cMyc; and
SEQ ID NOS: 175, 177, 179, 181, 183, or 185 for Lin28.

10. A recombinant expression vector comprising the polynucleotide according to Claim 7.

11. The recombinant expression vector according to Claim 10, wherein the expression vector is selected from the group consisting of pET28a(+)-HNM₈₄Nanog, pET28a(+)-HNNanogM₈₄, pET28a(+)-HNM₈₄NanogM₈₄, pET28a(+)-HNM₈₆Nanog, pET28a(+)-HNNanogM₈₆, pET28a(+)-HNM₈₆NanogM₈₆, pET28a(+)-HNM₈₄Oct4, pET28a(+)-HNOct4M_{84,} pET28a(+)-HNM₈₄Oct4M₈₄, pET28a(+)-HNM₈₆Oct4, pET28a(+)-HNOct4M₈₆, pET28a(+)-HNM₈₆Oct4M₈₆, pET28a(+)-HNM₈₄Sox2, pET28a(+)-HNSox2M₈₄, pET28a(+)-HNM₈₄Sox2M₈₄, pET28a(+)-HNM₈₆Sox2, pET28a(+)-HNSox2M₈₆, pET28a(+)-HNM₈₆Sox2M₈₆, pET28a(+)-HNM₈₄Klf4, pET28a(+)-HNKlf4M₈₄, pET28a(+)-HNM₈₄Klf4M₈₄, pET28a(+)-HNM₈₆Klf4, pET28a(+)-HNKlf4M₈₆, pET28a(+)-HNM₈₆Klf4M₈₆, pET28a(+)-HNM₈₄cMyc, pET28a(+)-HNcMycM₈₄, pET28a(+)-HNM₈₄cMycM₈₄, pET28a(+)-HNM₈₆cMyc, pET28a(+)-HNcMycM₈₆, pET28a(+)-HNM₈₆cMycM₈₆, pET28a(+)-HNM₈₄Lin28, pET28a(+)-HNLin28M₈₄, pET28a(+)-HNM₈₄Lin28M₈₄, pET28a(+)-HNM₈₆Lin28, pET28a(+)-HNLin28M₈₆, and pET28a(+)-HNM₈₆Lin28M_{86.}

12. A transformant which is obtained by transformation with the recombinant expression vector according to Claim 10.

13. The transformant according to Claim 12, wherein the transformant is *E. coli* DH5α/HNM₈₆Oct4 (KCTC 11640BP) orDH5α/HNM₈₆cMyc (KCTC 11661BP).

14. A method of producing the cell permeable reprogramming transcription factor recombinant protein according to Claim 1 comprising the steps of:
1) culturing the transformant according to Claim 12 so that the cell permeable reprogramming transcription factor recombinant protein is expressed; and
2) recovering the expressed cell permeable reprogramming transcription factor recombinant protein from the culture.

15. A cell permeable reprogramming transcription factor recombinant protein comprising a macromolecule transduction domain (MTD) fused to the recombinant protein, wherein
the MTD having an amino acid sequence represented by SEQ ID NO: 224 is fused to the N-terminus of SEQ ID NO: 2;
the MTD having an amino acid sequence represented by SEQ ID NO: 226 is fused to the N-terminus of SEQ ID NO: 4;
the MTD having an amino acid sequence represented by SEQ ID NO: 234 is fused to the N-terminus of SEQ ID NO: 6;
the MTD having an amino acid sequence represented by SEQ ID NO: 232 is fused to the N-terminus of SEQ ID NO: 8;
the MTD having an amino acid sequence represented by SEQ ID NO: 230 is fused to the N-terminus of SEQ ID NO: 10; or
the MTD having an amino acid sequence represented by SEQ ID NO: 228 is fused to the N-terminus of SEQ ID NO: 12.

16. The cell permeable reprogramming transcription factor recombinant protein according to Claim 15, wherein the MTD is any one of the following:
a JO-10 MTD having an amino acid sequence represented by SEQ ID NO: 224;
a JO-52 MTD having an amino acid sequence represented by SEQ ID NO: 226;
a JO-132 MTD having an amino acid sequence represented by SEQ ID NO: 228;
a JO-145 MTD having an amino acid sequence represented by SEQ ID NO: 230 a JO-173 MTD having an amino acid sequence represented by SEQ ID NO: 232; or
a JO-181 MTD having an amino acid sequence represented by SEQ ID NO: 234.

17. The cell permeable reprogramming transcription factor recombinant protein according to Claim 15, wherein the recombinant protein comprises at least one selected from a nuclear localization sequence (NLS) or a histidine-tag affinity domain fused at either terminus of the recombinant protein.

18. The cell permeable reprogramming transcription factor recombinant protein according to any of Claims 15 to 17,
wherein the recombinant protein has an amino acid sequence selected from the group consisting of SEQ ID NOS: 237, 240, 243, 246, 249, and 252.

19. A polynucleotide encoding the cell permeable reprogramming transcription factor recombinant protein according to Claim 15.

20. The polynucleotide according to Claim 19, **characterized by** having a nucleotide sequence selected from the group consisting of SEQ ID NOS: 236, 239, 242, 245, 248, and 251.

21. A recombinant expression vector comprising the polynucleotide according to Claim 19.

22. The recombinant expression vector according to Claim 21, wherein the recombinant expression vector is selected from the group consisting of pET28a(+)-HNM₁₀Nanog, pET28a(+)-HNM₅₂Oct4 pET28a(+)-HNM₁₈₁Sox2, pET28a(+)-HNM₁₇₃Klf4, pET28a(+)-HNM₁₄₅cMye, and pET28a(+)-HNM₁₃₂Lin28.

23. A transformant which is obtained by transformation with the recombinant expression vector according to Claim 21.

24. The transformant according to Claim 23, wherein the transformant is *E.*
*coli* DH5α/HNM₁₀Nanog (KCTC 11660BP), DH5α/HNM₁₈₁Sox2 (KCTC 11659BP), DH5α/HNM₁₇₃Klf4 (KCTC 11662BP), or DH5α/HNM₁₃₂Lin28 (KCTC 11663BP).

25. A method of producing the cell permeable reprogramming transcription factor recombinant protein according to Claim 15 comprising the steps of:
1) culturing the transformant according to Claim 23 so that the cell permeable reprogramming transcription factor recombinant protein is expressed; and
2) recovering the expressed cell permeable reprogramming transcription factor recombinant protein from the culture.
